(19) Europäisches Patentamt
European Patent Office
Office européen des brevets

(11) EP 3 013 859 B1

# (12) EUROPEAN PATENT SPECIFICATION

(45) Date of publication and mention of the grant of the patent:
**30.05.2018 Bulletin 2018/22**

(21) Application number: **14733614.3**

(22) Date of filing: **25.06.2014**

(51) Int Cl.:
***A61K 39/395*** *(2006.01)*

(86) International application number:
**PCT/EP2014/063443**

(87) International publication number:
**WO 2014/207064 (31.12.2014 Gazette 2014/53)**

## (54) BISPECIFIC MOLECULES CAPABLE OF SPECIFICALLY BINDING TO BOTH CTLA-4 AND CD40

BISPEZIFISCHE MOLEKÜLE ZUR SPEZIFISCHEN BINDUNG AN CTLA-4 UND CD40

MOLÉCULES BISPÉCIFIQUES CAPABLES DE SE LIER SPÉCIFIQUEMENT À LA FOIS À CTLA-4 ET CD40

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.06.2013 GB 201311487**

(43) Date of publication of application:
**04.05.2016 Bulletin 2016/18**

(73) Proprietor: **Alligator Bioscience AB**
**223 81 Lund (SE)**

(72) Inventors:
- **ELLMARK, Peter**
  **SE 226 49 Lund (SE)**
- **FUREBRING, Christina**
  **SE 224 72 Lund (SE)**
- **NORLEN, Per**
  **SE 216 11 Limhamn (SE)**

(74) Representative: **Potter Clarkson LLP**
**The Belgrave Centre**
**Talbot Street**
**Nottingham NG1 5GG (GB)**

(56) References cited:
**WO-A1-2011/061487**

- **JOHN KIRKWOOD ET AL.: "Immunotherapy of cancer in 2012", CA: A CANCER JOURNAL FOR CLINICIANS, vol. 62, no. 5, September 2012 (2012-09), pages 309-335, XP055059122,**
- **PETER ELLMARK ET AL.: "Pre-assembly of the extracellular domains of CD40 is not necessary for rescue of mouse B cells from anti-immunoglobulin M-induced apoptosis", IMMUNOLOGY, vol. 108, no. 4, 1 April 2003 (2003-04-01) , pages 452-457, XP055135455, ISSN: 0019-2805, DOI: 10.1046/j.1365-2567.2003.01622.x cited in the application**
- **ROBERT PEACH ET AL.: "Both extracellular immunoglobulin-like domains of CD80 contains residues critical for binding T cell surface receptors CTLA-4 and CD28.", JOURNAL OF BIOLOGICAL CHEMISTRY, vol. 270, no. 36, 8 September 1995 (1995-09-08), pages 21181-21187, XP002912152, cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 013 859 B1

## Description

### Field of the Invention

The present invention relates to bispecific molecules that specifically bind to both CD40 and CTLA-4, and in particular to both human CD40 and human CTLA-4.

### Background to the Invention

Cancer is a leading cause of premature deaths in the developed world. The aim of immunotherapy in cancer is to mount an effective immune response by the body against a tumour. This may be achieved by, for example, breaking tolerance against tumour antigen, augmenting anti-tumor immune responses, and stimulating local cytokine responses at the tumor site. The key effector cell of a long lasting anti-tumor immune response is the activated tumor specific effector T cell. Incomplete activation of effector T cells by, for example, dendritic cells can cause T-cell anergy, which results in an inefficient anti-tumor response, whereas adequate induction by dendritic cells can generate a potent expansion of activated effector T cells, redirecting the immune response towards the tumor.

The cell surface CD40 receptor molecule is a member of the tumour necrosis factor receptor superfamily (TNFR) and is a key regulator in both innate and adaptive immune responses. It is expressed on human antigen presenting cells, in particular B cells, dendritic cells and macrophages, as well as on normal cells, such as fibroblasts, smooth muscle cells, endothelial cells and epithelial cells. Moreover, is it expressed on a wide range of tumor cells including all B-lymphomas, 30 - 70% of solid tumours, melanomas and carcinomas.

The natural ligand of CD40, designated CD154 or CD40L, is mainly expressed on mature T lymphocytes. CD40L-mediated signalling triggers several biological events, including immune cell activation, proliferation, and production of cytokines and chemokines. Thus, stimulation via the CD40 receptor enhances cellular and immune functions. Its role in cell-mediated immune responses is well known. For example, the activation of dendritic cells via CD40 stimulation, induces activation of effector T cells. Treatment with CD40 agonists may thus provide the means to redirect the immune response and expand effector T cells directed to tumour

Antitumour effects have been reported for some anti-CD40 antibodies, with several mechanisms having been identified. An indirect effect is observed for CD40 negative tumors, involving the activation of antigen presenting cells, in particular increased activity by tumor specific cytotoxic T lymphocytes and natural killer cells (NK cells). A direct antitumor mechanism is observed for CD40 positive tumours, wherein the CD40 antibody binding to tumour cells induces cell apoptosis. These mechanisms for anti-tumour activity may be complemented by the stimulation of a humoral response leading to enhanced antibody mediated cellular cytotoxicity (ADCC). However, the systemic administration of anti-CD40 antibodies has alo been associated with adverse side effects, such as shock syndrome and cytokine release syndrome.

The T cell receptor CTLA-4, serves as a negative regulator of T cell activation, and is upregulated on the T-cell surface following initial activation. The ligands of the CTLA-4 receptor, which are expressed by antigen presenting cells are the B7 proteins . The corresponding ligand receptor pair which is responsible for the upregulation of T cell activation is CD28 - B7. Signalling via CD28 constitutes a costimulatory pathway, and follows upon the activation of T cells, through the T cell receptor recognizing antigenic peptide presented by the MHC complex.

By blocking the CTLA-4 interaction to the B7-1 and, or B7-2 ligands, one of the normal check points of the immune response may be removed. Clinical studies have demonstrated that CTLA-4 blockade generates anti-tumor effects. However, as with CD40, administration of anti-CTLA-4 antibodies has been associated with toxic side-effects.

There exists a need for an alternative to the existing mono-specific drugs which target either CD40 or CTLA-4.

WO 2011/061487 describes an immunomodulatory agent for use in the local treatment of tumours, wherein the treatment comprises patient-specific optimisation of the dose of the immunomodulatory agent. WO 2011/061487 also describes methods for the local treatment of tumours as well as methods for optimising treatments for the same.

Kirkwood et al 2012, CA Cancer J Clin 2012;62:309-335, describes the status of immunotherapy of Cancer in 2012. The improved understanding of immunotherapy and the mechanisms underlying immunity in cancer has fuelled an expanding array of new therapeutic agents for a variety of cancers. Principles that guide the development and application of immunotherapy include antibodies, cytokines, vaccines, and cellular therapies.

### Summary of the Invention

The present inventors have produced novel bispecific binding molecules which target two immunoregulatory receptors, CTLA-4 and CD40. The CTLA-4 and CD40 are preferably human, but may be CTLA-4 and/or CD40 from another mammal such as a non-human primate or a mouse. The non-human primate may be, for example, a cynomolgus monkey.

The suppression of immune responses observed in cancer patients may be overcome by the blocking the CTLA-4 mediated inhibition of T-cells specific for tumor antigens and, simultaneously, the targeting of CD40 will potentiate dendritic cells, B lymphocytes and macrophages cells expressing CD40. This will promote further immune responses towards tumours.

The bispecific binding molecules of the present invention may provide additional therapeutic effect by physically linking the CD40 expressing antigen presenting cells and the CTLA-4 expressing cells T cells, potentially creating a cell-cell synapse not normally found in nature. This will result in a very powerful immune activation for the immediate generation of tumoricidal activity. The bispecific binding molecules of the present invention may display higher potency and/or efficacy in cancer immunotherapy than treatment regimens which include a combination of mono-specific drugs targeting either CTLA-4 or CD40.

The bispecific binding molecule of the present invention is a polypeptide. Thus, the present invention provides a polypeptide capable of specifically binding to both human CTLA-4 and human CD40, said binding molecule comprising B1 and B2, wherein:

B1 is an antibody, or antigen binding fragment thereof, specific for human CD40, and which is a CD40 agonist; and
B2 is a polypeptide binding domain specific for human CTLA-4, which comprises or consists of (i) the amino acid sequence of SEQ ID NO: 3; or (ii) an amino acid sequence in which at least one amino acid is changed when compared to the amino acid sequence of SEQ ID NO: 3 provided that said binding domain binds to human CTLA-4 with higher affinity than wild-type human CD86. The CD40 binding domain(s) of B1 and the CTLA-4-binding domain of B2 may be the only binding domains in the polypeptide of the invention.

Also provided is a polypeptide of the invention for use in a method of treating or preventing a disease or condition in an individual.

Also provided is a polypeptide of the invention for use in the manufacture of a medicament for treating or preventing a disease or condition in an individual.

Also provided is a polynucleotide encoding a polypeptide of the invention, and a vector or cell comprising a said polynucleotide. Also provided is a method of producing a polypeptide of the invention, comprising expressing a said polynucleotide in a cell.

Also provided is a composition comprising a polypeptide of the invention and at least one pharmaceutically acceptable diluent or carrier.

## Brief Description of the Sequence Listing

SEQ ID NO: 1 is the amino acid sequence of human CTLA-4 (corresponding to GenBank: AAD00698.1)
SEQ ID NO: 2 is the amino acid sequence of human CD28 (corresponding to GenBank: AAA51944.1)
SEQ ID NO: 3 is the amino acid sequence of the monomeric extracellular domainof human wildtype CD86, excluding a 23 amino acid signal sequence from the N terminus.
SEQ ID NO: 4 is the amino acid sequence of the monomeric extracellular and transmembrane domains of human wildtype CD86, including N-terminal signal sequence. All numbering of amino acid positions herein is based on the positions in SEQ ID NO: 4 starting from the N terminus. Thus, the Alanine at the N terminus of SEQ ID NO: 3 is numbered 24.
SEQ ID NO: 5 is the amino acid sequence of a mutant form of the extracellular domain of human CD86 disclosed in Peach et al (Journal of Biological Chemistry 1995, vol 270(36), 21181-21187). H at position 79 of the wild type sequence is susbstituted with A in the corresponding position for the sequence of SEQ ID NO: 5. This change is referred to herein as H79A. Equivalent nomenclature is used throughout for other amino acid substitutions referred to herein. Numbering of positions is based on SEQ ID NO: 4 as outlined above.
SEQ ID NOs: 6 to 24 are the amino acid sequences of specific proteins of the invention.
SEQ ID NOs: 25 to 43 are nucleotide sequences encoding the amino acid sequences of each of SEQ ID NOs 6 to 24, respectively
SEQ ID NO: 44 is the the full length amino acid sequence of human CD86 (corresponding to GenBank: ABK41931.1)
SEQ ID NO: 45 is the amino acid sequence of human CD40 (corresponding to GenBank: AAH12419.1)
SEQ ID NOs: 46 to 49 are various linkers which may be used in the polypeptides of the invention.
SEQ ID NO: 50, 51 and 52 are the amino acid sequences of CDRs 1, 2 and 3 respectively of the heavy chain of the antibody A2-54.
SEQ ID NO: 53, 54 and 55 are the amino acid sequences of CDRs 1, 2 and 3 respectively of the light chain of the antibody A2-54.
SEQ ID NOs: 56 to 60 and 110 to 114 are the amino acid sequences of exemplary polypeptides of the invention.

SEQ ID NO: 61 is an amino acid sequence of the heavy chain of the antibody A2-54.
SEQ ID NO: 62 is an amino acid sequence of the the light chain of the antibody A2-54.
SEQ ID NO: 63 is a nucleotide sequence encoding SEQ ID NO. 61.
SEQ ID NO: 64 is a nucleotide sequence encoding SEQ ID NO. 62.
SEQ ID NOs: 65 to 69 and 115 to 119 are nucleotide sequences encoding SEQ ID NOs: 56 to 60 and 110 to 114, respectively.
SEQ ID NOs: 70, 72, 74, 76, 78, 80, 82, 84, 86 and 88 are amino acid sequences of the heavy chain of antibodies disclosed herein.
SEQ ID NOs: 71, 73, 75, 77, 79, 81, 83, 85, 87 and 89 are amino acid sequences of the light chain of antibodies disclosed herein.
SEQ ID NOs: 90, 92, 94, 96, 98, 100, 102, 104, 106 and 108 are nucleic acid sequences encoding heavy chain sequences of antibodies disclosed herein.
SEQ ID NOs: 91, 93, 95, 97, 99, 101, 103, 105, 107 and 109 are nucleic acid sequences encoding heavy chain sequences of antibodies disclosed herein.
SEQ ID NO: 120 is the amino acid sequence of murine CTLA-4 (corresponding to UniProtKB/Swiss-Prot: P09793.1).
SEQ ID NO: 121 is the amino acid sequence of murine CD28 (corresponding to GenBank: AAA37395.1).

## Brief Description of the Figures

Figure 1 shows the CTLA-4 binding properties of CTLA-4 binding domains of polypeptides the invention as determined by an ELISA binding assay.
Figure 2 shows the CTLA-4 binding properties of CTLA-4 binding domains of polypeptides of the invention as determined by an ELISA inhibition assay.
Figure 3 shows a schematic representation of the structure of exemplary polypeptides of the invention: A is 956/530 and 957/530; B is 959/530 and 960/530; C is 958/531. Anti-CD40 antibody variable domains are filled in black; constant domains in white. CTLA-A binding domains are shaded with diagonal lines.
Figure 4 provides a schematic representation of human wild-type CD86 amino acid sequences disclosed herein. A is the amino acid sequence of the monomeric soluble extracellular domain of human CD86 without N-terminal signal sequence (SEQ ID NO: 3); B is the amino acid sequence of the monomeric extracellular and transmembrane domains of human wildtype CD86, including N-terminal signal sequence (SEQ ID NO: 4); C is the full length amino acid sequence of human CD86 (Genbank ABK41931.1; SEQ ID NO: 44). The sequence in A may optionally lack Alanine and Proline at the N terminus, that is positions 24 and 25, shown in bold. Signal sequences in B and C are underlined. Numbering of amino acid positions is based on SEQ ID NOs: 4 and 44, starting from the N terminus.
Figure 5 shows the CTLA-4 binding properties of exemplary polypeptides of the invention as determined by an ELISA binding assay.
Figure 6 shows the CD40 binding properties of exemplary polypeptides of the invention as determined by an ELISA binding assay.
Figure 7 shows the ability of exemplary polypeptides of the invention to simultaneously bind to both CTLA-4 and CD40 in an ELISA assay.
Figure 8 shows the ability of exemplary polypeptides of the invention to simultaneously bind to both CTLA-4 and CD40 as measured using Surface Plasmon Resonance (SPR).
Figure 9 is a representative FACS plot showing the interaction between CD40-expressing and CTLA-4 expressing cells as a result of binding by an exemplary polypeptide of the invention.
Figure 10 shows the ability of an exemplary polypeptide of the invention to induce immune activation, as indicated by B cell proliferation. A synergistic effect is observed for the polypeptide of the invention 957/530.
Figure 11 shows the results of an inhibition ELISA demonstrating that a polypeptide of the invention has binding affinity of a similar magnitude for both human and murine CTLA-4.
Figure 12A - C show the CD40 binding properties of exemplary polypeptides of the invention as determined by an ELISA binding assay.
Figure 13A-F show the CTLA-4 binding properties of exemplary polypeptides of the invention as determined by an ELISA binding assay.
Figure 14 shows the the ability of exemplary polypeptides of the invention to simultaneously bind to both CTLA-4 and CD40 as measured using Surface Plasmon Resonance (SPR).
Figure 15 shows the in vivo anti-tumour effect of an exemplary polypeptide of the invention. Increased survival (top panel) and decreased tumour volume (bottom panel) relative to controls is demonstrated.
Figure 16 shows that treatment with an exemplary polypeptide of the invention established immunological memory in vivo. Previously treated mice have increased survival (top panel) and decreased tumour volume (bottom panel) relative to controls.

Figure 17 shows that exemplary polypeptides of the invention have increased efficacy at inducing B cell proliferation, when compared to the corresponding monospecific antibodies. The y axis shows the mean fold change in B cell proliferation for each polypeptide of the invention relative to its corresponding monospecific antibody.

Figure 18 shows that exemplary polypeptides of the invention have increased efficacy at inducing activation of immune cells in human PBMCs, when compared to the corresponding monospecific antibodies. The y axis shows the mean fold change in IL2 production for each polypeptide of the invention relative to its corresponding monospecific antibody.

Figure 19 shows that an exemplary polypeptide of the invention triggers B cell activation via CD40 that is enhanced by simultaneous cross linking with CTLA-4 on another cell population (*in trans*), as indicated by increased CD83 expression (y axis).

## Detailed Description of the Invention

It is to be understood that different applications of the disclosed products and methods may be tailored to the specific needs in the art. It is also to be understood that the terminology used herein is for the purpose of describing particular embodiments of the invention only, and is not intended to be limiting.

In addition as used in this specification and the appended claims, the singular forms "a", "an", and "the" include plural referents unless the content clearly dictates otherwise. Thus, for example, reference to "an inhibitor" includes two or more such inhibitors, or reference to "an oligonucleotide" includes two or more such oligonucleotides and the like.

A "polypeptide" is used herein in its broadest sense to refer to a compound of two or more subunit amino acids, amino acid analogs, or other peptidomimetics. The term "polypeptide" thus includes short peptide sequences and also longer polypeptides and proteins. As used herein, the term "amino acid" refers to either natural and/or unnatural or synthetic amino acids, including both D or L optical isomers, and amino acid analogs and peptidomimetics.

*The polypeptide of the invention part B1 - antibody specific for CD40*

B1 is an antibody, or antigen binding fragment thereof, specific for human CD40, and which is a CD40 agonist. The term "antibody" as referred to herein includes whole antibodies and any antigen binding fragment (*i.e.,* "antigen-binding portion") or single chains thereof. An antibody refers to a glycoprotein comprising at least two heavy (H) chains and two light (L) chains inter-connected by disulfide bonds, or an antigen binding portion thereof. Each heavy chain is comprised of a heavy chain variable region (abbreviated herein as VH) and a heavy chain constant region. Each light chain is comprised of a light chain variable region (abbreviated herein as VL) and a light chain constant region. The variable regions of the heavy and light chains contain a binding domain that interacts with an antigen. The VH and VL regions can be further subdivided into regions of hypervariability, termed complementarity determining regions (CDR), interspersed with regions that are more conserved, termed framework regions (FR). The constant regions of the antibodies may mediate the binding of the immunoglobulin to host tissues or factors, including various cells of the immune system (*e.g.,* effector cells) and the first component (Clq) of the classical complement system.

The term "antigen-binding fragment" of an antibody refers to one or more fragments of an antibody that retain the ability to specifically bind to an antigen, such as CD40. It has been shown that the antigen-binding function of an antibody can be performed by fragments of a full-length antibody. Examples of binding fragments encompassed within the term "antigen-binding portion" of an antibody include a Fab fragment, a $F(ab')_2$ fragment, a Fab' fragment, a Fd fragment, a Fv fragment, a dAb fragment and an isolated complementarity determining region (CDR). Single chain antibodies such as scFv and heavy chain antibodies such as VHH and camel antibodies are also intended to be encompassed within the term "antigen-binding portion" of an antibody. These antibody fragments may be obtained using conventional techniques known to those of skill in the art, and the fragments may be screened for utility in the same manner as intact antibodies.

The polypeptide of the invention may incorporate any anti-CD40 antibody, or antigen binding fragment therof, which is a CD40 agonist. Such an anti-CD40 antibody may be produced by an suitable means. For example, such an antibody may be prepared, expressed, created or isolated by recombinant means, such as (a) antibodies isolated from an animal (*e.g.,* a mouse) that is transgenic or transchromosomal for the immunoglobulin genes of interest or a hybridoma prepared therefrom, (b) antibodies isolated from a host cell transformed to express the antibody of interest, *e.g.,* from a transfectoma, (c) antibodies isolated from a recombinant, combinatorial antibody library, and (d) antibodies prepared, expressed, created or isolated by any other means that involve splicing of immunoglobulin gene sequences to other DNA sequences. Alternatively an anti-CD40 antibody may be produced by methods comprising immunising a non-human animal with CD40, or immunising human lymphocytes *in vitro* with CD40.

The antibody typically comprises at least one heavy chain variable domain ($V_H$) and at least one light chain variable domain (VL). The antibody may comprise an Fc region, preferably a human Fc region, or a variant of a said region. The Fc region may be an IgG1, IgG2, IgG3 or IgG4 region, preferably an IgG1 or IgG4 region. A variant of an Fc region typically binds to Fc receptors, sucha as FcgammaR and/or neonatal Fc receptor (FcRn) with altered affinity providing for improved function and/or half life of the polypeptide. The half life may be either increased or a decreased relative to the half life of a polypeptide comprising a native Fc region.

Heavy and light chain amino acid sequences of preferred antibodies for incorporation in the polypeptide of the invention are shown in table A. Within each sequence the CDR sequences are underlined. The sequences are all presented in the orientation N to C from left to right. Thus, reading from left to right within each sequence, CDR1 is the first underlined, CDR2 is the second underlined, and CDR3 is the third underlined sequence. Constant domains are shown in italics.

**Table A**

| SEQ ID | Chain | Sequence |
|---|---|---|
| Antibody A2-54 | | |
| 61 | A2-54 Variable Heavy, VH-gamma1 | EVQLLESGGGLVQPGGSLRLSCAASGFTFSDYYMSWVRQAPGKGLEWISSISSNGIYIYYADSLKGRFTISRDNSKNTLYLQMNSLRAEDTAVYYCARAPVDYSNPSGMDVWGQGTLVTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNH* |
| | | *YTQKSLSLSPGK* |
| 62 | A2-54 Variable Light, VL-kappa | QSVLTQPPSASGTPGQRVTISCSGSSSNIGSNTVNWYQQLPGTAPKLLIYRNNQRPSGVPDRFSGSKSGTSASLAISGLRSEDEADYYCAAWDDSLSGWRFGGGTKLTVL*GGTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC* |
| Antibody 1107/1145 | | |
| 70 | Antibody clone, 1107, variable heavy, VH-gamma1 | EVQLLESGGGLVQPGGSLRLSCAASGFTFSSYAMSWVRQAPGKGLEWVSAISGSGGSTYYADSVKGRFTISRDNSKNTLYLQMNSLRAEDTAVYYCARRVWGFDYWGQGTLVTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK* |
| 71 | Antibody clone 1145, variable light, VL-kappa | DIQMTQSPSSLSASVGDRVTITCRASQSISSYLNWYQQKPGKAPKLLIYAASSLQSGVPSRFSGSGSGTDFTLTISSLQPEDFATYYCQQYGVYPFTFGQGTKLEIK*RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC* |
| Antibody 1136/1143 | | |
| 72 | Antibody clone, 1136, variable heavy, VH-gamma1 | EVQLLESGGGLVQPGGSLRLSCAASGFTFSSYAMSWVRQAPGKGLEWVSAISGSGGSTYYADSVKGRFTISRDNSKNTLYLQMNSLRAEDTAVYYCARYVFGIDYWGQGTLVTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK* |

(continued)

| | | |
|---|---|---|
| Antibody 1136/1143 | | |
| 73 | Antibody clone 1143, variable light, VL-kappa | DIQMTQSPSSLSASVGDRVTITCRASQSISSYLNWYQQKPGKAPKLLIYAASSLQ<br>SGVPSRFSGSGSGTDFTLTISSLQPEDFATYYCQQAYYAGLFTFGQGTKLEIK*RT*<br>*VAAPSVFIFPPSDEQLKSGTASVVCLLNNFYPREAKVQWKVDNALQSGNSQESVT*<br>*EQDSKDSTYSLSSTLTLSKADYEKHKVYACEVTHQGLSSPVTKSFNRGEC* |
| Antibody 1132/1139 | | |
| 74 | Antibody clone 1132, variable heavy, VH-gamma1 | EVQLLESGGGLVQPGGSLRLSCAASGFTFSSYAMSWVRQAPGKGLEWVSGIGSYG<br>GGTYYADSVKGRFTISRDNSKNTLYLQMNSLRAEDTAVYYCARYVNFGMDYWGQG<br>TLVTVSS*ASTKGPSVFPLAPSSKSTSGGTAALGCLVKDYFPEPVTVSWNSGALTS*<br>*GVHTFPAVLQSSGLYSLSSVVTVPSSSLGTQTYICNVNHKPSNTKVDKKVEPKSC*<br>*DKTHTCPPCPAPELLGGPSVFLFPPKPKDTLMISRTPEVTCVVVDVSHEDPEVKF*<br>*NWYVDGVEVHNAKTKPREEQYNSTYRVVSVLTVLHQDWLNGKEYKCKVSNKALPA*<br>*PIEKTISKAKGQPREPQVYTLPPSRDELTKNQVSLTCLVKGFYPSDIAVEWESNG*<br>*QPENNYKTTPPVLDSDGSFFLYSKLTVDKSRWQQGNVFSCSVMHEALHNHYTQKS*<br>*LSLSPGK* |
| 75 | Antibody clone 1139, variable light, VL-kappa | DIQMTQSPSSLSASVGDRVTITCRASQSISSYLNWYQQKPGKAPKLLIYAASSLQ<br>SGVPSRFSGSGSGTDFTLTISSLQPEDFATYYCQQYGRNPPTFGQGTKLEIK*RTV*<br>*AAPSVFIFPPSDEQLKSGTASVVCLLNNFYPREAKVQWKVDNALQSGNSQESVTE*<br>*QDSKDSTYSLSSTLTLSKADYEKHKVYACEVTHQGLSSPVTKSFNRGEC* |
| Antibody 1140/1141 | | |
| 76 | Antibody clone 1140, variable heavy, VH-gamma1 | EVQLLESGGGLVQPGGSLRLSCAASGFTFSSYAMSWVRQAPGKGLEWVSAISGSG<br>GSTYYADSVKGRFTISRDNSKNTLYLQMNSLRAEDTAVYYCARGPVYSSVFDYWG<br>QGTLVTVSS*ASTKGPSVFPLAPSSKSTSGGTAALGCLVKDYFPEPVTVSWNSGAL*<br>*TSGVHTFPAVLQSSGLYSLSSVVTVPSSSLGTQTYICNVNHKPSNTKVDKKVEPK*<br>*SCDKTHTCPPCPAPELLGGPSVFLFPPKPKDTLMISRTPEVTCVVVDVSHEDPEV*<br>*KFNWYVDGVEVHNAKTKPREEQYNSTYRVVSVLTVLHQDWLNGKEYKCKVSNKAL* |
| | | *PAPIEKTISKAKGQPREPQVYTLPPSRDELTKNQVSLTCLVKGFYPSDIAVEWES*<br>*NGQPENNYKTTPPVLDSDGSFFLYSKLTVDKSRWQQGNVFSCSVMHEALHNHYTQ*<br>*KSLSLSPGK* |
| 77 | Antibody clone 1141, variable light, VL-kappa | DIQMTQSPSSLSASVGDRVTITCRASQSISSYLNWYQQKPGKAPKLLIYAASSLQ<br>SGVPSRFSGSGSGTDFTLTISSLQPEDFATYYCQQSYSTPYTFGQGTKLEIK*RTV*<br>*AAPSVFIFPPSDEQLKSGTASVVCLLNNFYPREAKVQWKVDNALQSGNSQESVTE*<br>*QDSKDSTYSLSSTLTLSKADYEKHKVYACEVTHQGLSSPVTKSFNRGEC* |
| Antibody 1150/1152 | | |
| 78 | Antibody clone 1150, variable heavy, VH-gamma1 | EVQLLESGGGLVQPGGSLRLSCAASGFTFSSYAMSWVRQAPGKGLEWVSGIGGSS<br>SYTSYADSVKGRFTISRDNSKNTLYLQMNSLRAEDTAVYYCARYYSYHMDYWGQG<br>TLVTVSS*ASTKGPSVFPLAPSSKSTSGGTAALGCLVKDYFPEPVTVSWNSGALTS*<br>*GVHTFPAVLQSSGLYSLSSVVTVPSSSLGTQTYICNVNHKPSNTKVDKKVEPKSC*<br>*DKTHTCPPCPAPELLGGPSVFLFPPKPKDTLMISRTPEVTCVVVDVSHEDPEVKF*<br>*NWYVDGVEVHNAKTKPREEQYNSTYRVVSVLTVLHQDWLNGKEYKCKVSNKALPA*<br>*PIEKTISKAKGQPREPQVYTLPPSRDELTKNQVSLTCLVKGFYPSDIAVEWESNG*<br>*QPENNYKTTPPVLDSDGSFFLYSKLTVDKSRWQQGNVFSCSVMHEALHNHYTQKS*<br>*LSLSPGK* |
| 79 | Antibody clone 1152, variable light, VL-kappa | DIQMTQSPSSLSASVGDHVTITCRASQSISSYLNWYQQKPGKAPKLLIYAASSLQ<br>SGVPSRFSGSGSGTDFTLTISSLQPEDFATYYCQQYGSAPPTFGQGTKLEIK*RTV*<br>*AAPSVFIFPPSDEQLKSGTASVVCLLNNFYPREAKVQWKVDNALQSGNSQESVTE*<br>*QDSKDSTYSLSSTLTLSKADYEKHKVYACEVTHQGLSSPVTKSFNRGEC* |

(continued)

| | | |
|---|---|---|
| Antibody 1134/1141 | | |
| 80 | Antibody clone 1134, variable heavy, VH-gamma1 | EVQLLESGGGLVQPGGSLRLSCAASGFTFSSYAMSWVRQAPGKGLEWVSSIYSGGGGTSYADSVKGRFTISRDNSKNTLYLQMNSLRAEDTAVYYCARGPAYSSFFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 81 | Antibody clone 1141, variable light, VL-kappa | DIQMTQSPSSLSASVGDRVTITCRASQSISSYLNWYQQKPGKAPKLLIYAASSLQSGVPSRFSGSGSGTDFTLTISSLQPEDFATYYCQQSYSTPYTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| Antibody 1146/1147 | | |
| 82 | Antibody clone 1146, variable heavy, VH-gamma1 | EVQLLESGGGLVQPGGSLRLSCAASGFTFSSYAMSWVRQAPGKGLEWVSAISGSGGSTYYADSVKGRFTISRDNSKNTLYLQMNSLRAEDTAVYYCARRVFGFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 83 | Antibody clone 1147, variable light, VL-kappa | DIQMTQSPSSLSASVGDRVTITCRASQSISSYLNWYQQKPGKAPKLLIYAASSLQSGVPSRFSGSGSGTDFTLTISSLQPEDFATYYCQQYYYYPFTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| Antibody 1142/1135 | | |
| 84 | Antibody clone 1142, | EVQLLESGGGLVQPGGSLRLSCAASGFTFSSYAMSWVRQAPGKGLEWVSAISGSGGSTYYADSVKGRFTISRDNSKNTLYLQMNSLRAEDTAVYYCARGPAYSTVLDYWGQGTLVTVSSASTKGPSVFPLAPSSKSTSGGTAALGCLVKDYFPEPVTVSWNSGAL |
| | variable heavy, VH-gamma1 | TSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 85 | Antibody clone 1135, variable light, VL-kappa | DIQMTQSPSSLSASVGDRVTITCRASQSISSYLNWYQQKPGKAPKLLIYAASSLQSGVPSRFSGSGSGTDFTLTISSLQPEDFATYYCQQSYSTPYTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |

(continued)

| | | |
|---|---|---|
| Antibody 1148/1149 | | |
| 86 | Antibody clone 1148, variable heavy, VH-gamma1 | EVQLLESGGGLVQPGGSLRLSCAASGFTFSSYAMSWVRQAPGKGLEWVSAISGSGGSTYYADSVKGRFTISRDNSKNTLYLQMNSLRAEDTAVYYCARAVFGFDYWGQGTLVTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK* |
| 87 | Antibody clone 1149, variable light, VL-kappa | DIQMTQSPSSLSASVGDRVTITCRASQSISSYLNWYQQKPGKAPKLLIYAASSLQSGVPSRFSGSGSGTDFTLTISSLQPEDFATYYCQQAYYFPHTFGQGTKLEIK*RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC* |
| Antibody 1138/1135 | | |
| 88 | Antibody clone 1138, variable heavy, VH-gamma1 | EVQLLESGGGLVQPGGSLRLSCAASGFTFSSYAMSWVRQAPGKGLEWVSAISGSGGSTYYADSVKGRFTISRDNSKNTLYLQMNSLRAEDTAVYYCARGFVYSSYIDYWGQGTLVTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK* |
| 89 | Antibody clone 1135, variable light, VL-kappa | DIQMTQSPSSLSASVGDRVTITCRASQSISSYLNWYQQKPGKAPKLLIYAASSLQSGVPSRFSGSGSGTDFTLTISSLQPEDFATYYCQQSYSTPYTFGQGTKLEIK*RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC* |

The CDRs for the heavy chain of antibody A2-54 are also shown in SEQ ID NOs: 50, 51 and 52. The CDRs for the light chain are also shown in SEQ ID NOs: 53, 54 and 55. All six CDRs of A2-54 are also shown in the following table.

| SEQ ID | CDR | Sequence | SEQ ID | CDR | Sequence |
|---|---|---|---|---|---|
| 50 | VH1 | GFTFSDYY | 53 | VL1 | SSNIGSNT |
| 51 | VH2 | ISSNGIYI | 54 | VL2 | RNN |
| 52 | VH3 | ARAPVDYSNPSGMDV | 55 | VL3 | AAWDDSLSG |

The antibody may comprise the amino acid sequence of any one of the heavy chains for which the sequences are shown in Table A, or a fragment or variant of any thereof. The antibody may comprise the amino acid sequence ofany one of the light chains for which the sequences are shown in Table A, or a fragment or variant of any thereof. A preferred fragment of a said heavy chain or a said light chain is the variable region.

The antibody may comprise both the heavy chain (or a fragment or variant thereof) and the light chain (or a fragment or variant thereof) of any one of the antibodies shown in Table A.

The antibody may comprise a fragment of one of the heavy or light chain amino acid sequences shown in Table A. For example, an antibody of the invention may comprise a fragment of at least 7, at least 8, at least 9, at least 10, at least 12, at least 15, at least 18, at least 20 or at least 25 consecutive amino acids from said amino acid sequence. Such a fragment will preferably retain one or more of the functions discussed below, such as the ability to bind to CD40.

The antibody may comprise one, two, three, four, five or six CDR sequences from any one of the light chain or heavy chain sequences shown in Table A. The antibody may comprise one or more heavy chain CDR sequences and alternatively or additionally one or more light chain CDR sequences selected from the CDR sequences shown in Table A. The antibody may comprise one, two or all three of the heavy chain CDR sequences of any one of the antibodies shown in Table A, and alternatively or additionally one, two or all three of the light chain CDR sequences of the same said antibody as shown in Table A. An antibody of the invention preferably comprises all six CDR sequences of an antibody as shown in Table A. For example an antibody of the invention may comprise all six CDR sequences of antibody 1107/1145 as shown in Table A.

The antibody may alternatively be or may comprise a variant of one of the specific sequences recited above. For example, a variant may be a substitution, deletion or addition variant of any of the above amino acid sequences.

A variant antibody may comprise 1, 2, 3, 4, 5, up to 10, up to 20, up to 30 or more amino acid substitutions and/or deletions from the specific sequences and fragments discussed above. "Deletion" variants may comprise the deletion of individual amino acids, deletion of small groups of amino acids such as 2, 3, 4 or 5 amino acids, or deletion of larger amino acid regions, such as the deletion of specific amino acid domains or other features. "Substitution" variants preferably involve the replacement of one or more amino acids with the same number of amino acids and making conservative amino acid substitutions. For example, an amino acid may be substituted with an alternative amino acid having similar properties, for example, another basic amino acid, another acidic amino acid, another neutral amino acid, another charged amino acid, another hydrophilic amino acid, another hydrophobic amino acid, another polar amino acid, another aromatic amino acid or another aliphatic amino acid. Some properties of the 20 main amino acids which can be used to select suitable substituents are as follows:

| | | | |
|---|---|---|---|
| Ala | aliphatic, hydrophobic, neutral | Met | hydrophobic, neutral |
| Cys | polar, hydrophobic, neutral | Asn | polar, hydrophilic, neutral |
| Asp | polar, hydrophilic, charged (-) | Pro | hydrophobic, neutral |
| Glu | polar, hydrophilic, charged (-) | Gln | polar, hydrophilic, neutral |
| Phe | aromatic, hydrophobic, neutral | Arg | polar, hydrophilic, charged (+) |
| Gly | aliphatic, neutral | Ser | polar, hydrophilic, neutral |
| His | aromatic, polar, hydrophilic, charged (+) | Thr | polar, hydrophilic, neutral |
| Ile | aliphatic, hydrophobic, neutral | Val | aliphatic, hydrophobic, neutral |
| Lys | polar, hydrophilic, charged(+) | Trp | aromatic, hydrophobic, neutral |
| Leu | aliphatic, hydrophobic, neutral | Tyr | aromatic, polar, hydrophobic |

Preferred "derivatives" or "variants" include those in which instead of the naturally occurring amino acid the amino acid which appears in the sequence is a structural analog thereof. Amino acids used in the sequences may also be derivatized or modified, e.g. labelled, providing the function of the antibody is not significantly adversely affected.

Derivatives and variants as described above may be prepared during synthesis of the antibody or by post-production modification, or when the antibody is in recombinant form using the known techniques of site- directed mutagenesis, random mutagenesis, or enzymatic cleavage and/or ligation of nucleic acids.

Preferably variant antibodies have an amino acid sequence which has more than 60%, or more than 70%, e.g. 75 or 80%, preferably more than 85%, e.g. more than 90 or 95% amino acid identity to the $V_L$ or $V_H$ domain, or a fragment thereof, of an antibody disclosed herein. This level of amino acid identity may be seen across the full length of the relevant SEQ ID NO sequence or over a part of the sequence, such as across 20, 30, 50, 75, 100, 150, 200 or more amino acids, depending on the size of the full length polypeptide.

In connection with amino acid sequences, "sequence identity" refers to sequences which have the stated value when assessed using ClustalW (Thompson *et al.,* 1994, supra) with the following parameters:

Pairwise alignment parameters -Method: accurate, Matrix: PAM, Gap open penalty: 10.00, Gap extension penalty: 0.10;

Multiple alignment parameters -Matrix: PAM, Gap open penalty: 10.00, % identity for delay: 30, Penalize end gaps: on, Gap separation distance: 0, Negative matrix: no, Gap extension penalty: 0.20, Residue-specific gap penalties: on, Hydrophilic gap penalties: on, Hydrophilic residues: GPSNDQEKR. Sequence identity at a particular residue is intended to include identical residues which have simply been derivatized.

The present invention thus provides antibodies having specific heavy and light chain amino acid sequences and variants and fragments thereof which maintain the function or activity of these chains.

Accordingly, the antibody may comprise:

(a) a heavy chain amino acid sequence of any one of SEQ ID NOs: 61, 70, 72, 74, 76, 78, 80, 82, 84, 86 or 88;

(b) a fragment of at least 7 amino acids of (a), such as the variable region, wherein the antibody retains the ability to specifically bind to CD40; or

(c) a variant of (a) having at least 70% amino acid sequence identity to a sequence of (a), wherein the antibody retains the ability to specifically bind to CD40.

The antibody may comprise:

(a) a light chain amino acid sequence of any one of SEQ ID NOs: 62, 71, 73, 75, 77, 79, 81, 83, 85, 87 or 89;

(b) a fragment of at least 7 amino acids of (a), such as the variable region, wherein the antibody retains the ability to specifically bind to CD40; or

(c) a variant of (a) having at least 70% amino acid sequence identity to a sequence of (a), wherein the antibody retains the ability to specifically bind to CD40.

The antibody may bind to the same epitope as any of the specific antibodies, fragments and variants described herein. Preferably it binds to the same epitope as an antibody as shown in Table A, or an antibody possessing all six CDRs of an antibody as shown in Table A.

The antibody, or antigen binding fragment therof, has certain preferred binding characteristics and functional effects, which are explained in more detail below. Said antibody, or antigen binding fragment thereof, preferably retrains these binding characteristics and functional effects when incorporated as part of a polypeptide of the invention.

The antibody preferably specifically binds to CD40, that is it binds to CD40 but does not bind, or binds at a lower affinity, to other molecules. The term CD40 as used herein typically refers to human CD40. The sequence of human CD40 is set out in SEQ ID NO: 45. The antibody may have some binding affinity for CD40 from other mammals, such as CD40 from a non-human primate (for example cynomolgus monkey) or a mouse. The antibody preferably binds to human CD40 when localised on the surface of a cell.

The antibody has the ability to bind to CD40 in its native state and in particular to CD40 localised on the surface of a cell. Preferably, the antibody will bind specifically to CD40. That is, an antibody of the invention will preferably bind to CD40 with greater binding affinity than that at which it binds to another molecule.

By "localised on the surface of a cell" it is meant that CD40 is associated with the cell such that one or more region of CD40 is present on the outer face of the cell surface. For example, CD40 may be inserted into the cell plasma membrane (i.e. orientated as a transmembrane protein) with one or more regions presented on the extracellular surface. This may occur in the course of expression of CD40 by the cell. Thus, in one embodiment, "localised on the surface of a cell" may mean "expressed on the surface of a cell." Alternatively, CD40 may be outside the cell with covalent and/or ionic interactions localising it to a specific region or regions of the cell surface.

The antibody may modulate the activity of a cell expressing CD40, wherein said modulation is an increase or decrease in the activity of said cell. The cell is typically a dendritic cell or a B cell.

Professional APCs, such as dendritic cells, are activated when signaling via CD40 occurs, which triggers several biological events, including immune cell activation, proliferation, and production of cytokines and chemokines. Methods for determining dendritic cell activation associated with CD40 are known in the art (discussed, for example, in Schonbeck et al., 2001, Cell Mol Life Sci., 58:40-43; van Kooten et al., 2000, J. Leuk., Biol., 67: 2-17) and are described further below.

Stimulation of human B cells with recombinant CD40L or anti-CD40 antibodies induces up-regulation of surface markers, such as CD23, CD30, CD80, CD86, Fas and MHC II, secretion of soluble cytokines, e.g. IL-6, TNF-$\gamma$ and TNF-$\alpha$, and homeotypic aggregation. Methods for determining CD40-related B cell activation are known in the art and are described further below.

Methods and assays for determining the ability of an antibody to modulate the activity of dendritic cells and B cells are well known in the art. For example, the activation of dendritic cells may be assessed by measuring the level of cell surface markers such as CD86 and CD80 and/or by measuring anti-CD40 antibody-induced secretion of IFN$\gamma$ from T cells, wherein in an increase in any of these parameters indicates increased activation and a decrease represents decreased activation. Similarly, the ability of an antibody to modulate the activity of B cells may be assessed by measuring the level of cell surface markers (such as CD86) and/or by measuring anti-CD40 antibody-induced B cell proliferation , wherein in an increase in any of these parameters indicates increased activation and a decrease represents decreased activation.

The terms "binding activity" and "binding affinity" are intended to refer to the tendency of an antibody molecule to bind or not to bind to a target. Binding affinity may be quantified by determining the dissociation constant (Kd) for an antibody and its target. Similarly, the specificity of binding of an antibody to its target may be defined in terms of the comparative dissociation constants (Kd) of the antibody for its target as compared to the dissociation constant with respect to the antibody and another, non-target molecule.

Typically, the Kd for the antibody with respect to the target will be 2-fold, preferably 5-fold, more preferably 10-fold less than Kd with respect to the other, non-target molecule such as unrelated material or accompanying material in the environment. More preferably, the Kd will be 50- fold less, even more preferably 100-fold less, and yet more preferably 200-fold less.

The value of this dissociation constant can be determined directly by well-known methods, and can be computed even for complex mixtures by methods such as those, for example, set forth in Caceci et al. (Byte 9:340-362, 1984). For example, the Kd may be established using a double-filter nitrocellulose filter binding assay such as that disclosed by Wong & Lohman (Proc. Natl. Acad. Sci. USA 90, 5428-5432, 1993). Other standard assays to evaluate the binding ability of ligands such as antibodies towards targets are known in the art, including for example, ELISAs, Western blots, RIAs, and flow cytometry analysis. The binding kinetics (*e.g.,* binding affinity) of the antibody also can be assessed by standard assays known in the art, such as by Biacore™ system analysis.

A competitive binding assay can be conducted in which the binding of the antibody to the target is compared to the binding of the target by another, known ligand of that target, such as another antibody. The concentration at which 50% inhibition occurs is known as the Ki. Under ideal conditions, the Ki is equivalent to Kd. The Ki value will never be less than the Kd, so measurement of Ki can conveniently be substituted to provide an upper limit for Kd.

An antibody of the invention is preferably capable of binding to its target with an affinity that is at least two-fold, 10-fold, 50-fold, 100-fold or greater than its affinity for binding to another non-target molecule.

The antibody may be a human antibody. The term "human antibody", as used herein, is intended to include antibodies having variable regions in which both the framework and CDR regions are derived from human germline immunoglobulin sequences. Furthermore, if the antibody contains a constant region, the constant region also is derived from human germline immunoglobulin sequences. The human antibodies of the invention may include amino acid residues not encoded by human germline immunoglobulin sequences (*e.g.,* mutations introduced by random or site-specific mutagenesis *in vitro* or by somatic mutation *in vivo*). However, the term "human antibody", as used herein, is not intended to include antibodies in which CDR sequences derived from the germline of another mammalian species, such as a mouse, have been grafted onto human framework sequences.

*The polypeptide of the invention part B2 - binding domain specific for CTLA-4*

CD86 and CD80 may be referred to herein as B7 proteins (B7-2 and B7-1 respectively). These proteins are expressed on the surface of antigen presenting cells and interact with the T cell receptors CD28 and CTLA-4. The binding of the B7 molecules to CD28 promotes T cell activation while binding of B7 molecules to CTLA-4 switches off the activation of the T cell. The interaction between the B7 proteins with CD28 and/or CTLA-4 constitute a costimulatory signalling pathway which plays an important role in immune activation and regulation. Thus, the B7 molecules are part of a pathway, amenable to manipulation in order to uncouple immune inhibition, thereby enhancing immunity in patients.

The CD86 protein is a monomer and consists of two extracellular immunoglobulin superfamily domains. The receptor binding domain of CD86 has a typical IgV-set structure, whereas the membrane proximal domain has a C1-set like structure. The structure of CD80 and CD86 have been determined on their own or in complex with CTLA-4. The contact residues on the CD80 and CD86 molecules are in the soluble extracellular domain, and mostly located in the beta-sheets and not in the (CDR-like) loops.

SEQ ID NO: 3 is the amino acid sequence of the monomeric soluble extracellular domain of human wild-type CD86. This wild type sequence may optionally lack Alanine and Proline at the N terminus, that is positions 24 and 25. These amino acids may be referred to herein as A24 and P25 respectively.

Part B2 of the polypeptide of the invention is a polypeptide binding domain specific for CTLA-4. Said binding domain may also bind to CD28. The term CTLA-4 as used herein typically refers to human CTLA-4 and the term CD28 as used herein typically refers to human CD28. The sequences of human CTLA-4 and human CD28 are set out in SEQ ID NOs: 1 and 2 respectively. Part B2 of the polypeptide of the present invention may have some binding affinity for CTLA-4 or CD28 from other mammals, for example primate or murine CTLA-4 or CD28.

Part B2 of the polypeptide of the invention has the ability to bind to CTLA-4 in its native state and in particular to CTLA-4 localised on the surface of a cell. By "localised on the surface of a cell" it is meant that CTLA-4 is associated with the cell such that one or more region of CTLA-4 is present on the outer face of the cell surface. For example, CTLA-4 may be inserted into the cell plasma membrane (i.e. orientated as a transmembrane protein) with one or more regions presented on the extracellular surface. This may occur in the course of expression of CTLA-4 by the cell. Thus, in one embodiment, "localised on the surface of a cell" may mean "expressed on the surface of a cell." Alternatively, CTLA-4 may be outside the cell with covalent and/or ionic interactions localising it to a specific region or regions of the cell surface.

Part B2 of the polypeptide of the invention may comprise or consist of:

(i) the amino acid sequence of SEQ ID NO: 3; or
(ii) an amino acid sequence in which at least one amino acid is changed when compared to the amino acid sequence of SEQ ID NO: 3 provided that said binding domain binds to human CTLA-4 with higher affinity than wild-type human CD86.

In other words, B2 is a polypeptide binding domain specific for human CTLA-4 which comprises or consists of (i) the monomeric soluble extracellular domain of human wild-type CD86, or (ii) a polypeptide variant of said soluble extracellular domain, provided that said polypeptide variant binds to human CTLA-4 with higher affinity than wild-type human CD86.

Accordingly, part B2 of the polypeptide of the invention may have the same target binding properties as human wild-type CD86, or may have different target binding properties compared to the target binding properties of human wild-type CD86. For the purposes of comparing such properties, "human wild-type CD86" typically refers to the monomeric soluble extracellular domain of human wild-type CD86 as described in the preceding section.

Human wild-type CD86 specifically binds to two targets, CTLA-4 and CD28. Accordingly, the binding properties of part B2 of the polypeptide of the invention may be expressed as an individual measure of the ability of the polypeptide to bind to each of these targets. For example, a polypeptide variant of the monomeric extracellular domain of human wild-type CD86 preferably binds to CTLA-4 with a higher binding affinity than that of wild-type human CD86 for CTLA-4. Such a polypeptide may optionally also bind to CD28 with a lower binding affinity than that of wild-type human CD86 for CD28.

Standard assays to evaluate the binding ability of ligands towards targets are well known in the art, including for example, ELISAs, Western blots, RIAs, and flow cytometry analysis. The binding kinetics (*e.g.,* binding affinity) of the polypeptide also can be assessed by standard assays known in the art, such as by Surface Plasmon Resonance analysis (SPR).

The terms "binding activity" and "binding affinity" are intended to refer to the tendency of a polypeptide molecule to bind or not to bind to a target. Binding affinity may be quantified by determining the dissociation constant (Kd) for a polypeptide and its target. A lower Kd is indicative of a higher affinity for a target. Similarly, the specificity of binding of a polypeptide to its target may be defined in terms of the comparative dissociation constants (Kd) of the polypeptide for its target as compared to the dissociation constant with respect to the polypeptide and another, non-target molecule.

The value of the dissociation constant Kd can be determined directly by well-known methods, and can be computed even for complex mixtures by methods such as those, for example, set forth in Caceci et al. (Byte 9:340-362, 1984). For example, the Kd may be established using a double-filter nitrocellulose filter binding assay such as that disclosed by Wong & Lohman (Proc. Natl. Acad. Sci. USA 90, 5428-5432, 1993). A competitive binding assay can be conducted in which the binding of the polypeptide to the target is compared to the binding of the target by another, known ligand of that target, such as another polypeptide. In this case, the soluble extracellular domain of wild-type human CD86 (optionally linked to a detectable domain such as an Fc domain or an Ig domain at the N or C terminus) is a suitable alternative ligand. The concentration at which 50% inhibition occurs is known as the Ki. Under ideal conditions, the Ki is equivalent to Kd. The Ki value will never be less than the Kd, so measurement of Ki can conveniently be substituted to provide an upper limit for Kd.

Alternative measures of binding affinity include EC50 or IC50. In this context EC50 indicates the concentration at which a polypeptide achieves 50% of its maximum binding to a fixed quantity of target. IC50 indicates the concentration at which a polypeptide inhibits 50% of the maximum binding of a fixed quantity of competitor to a fixed quantity of target. In both cases, a lower level of EC50 or IC50 indicates a higher affinity for a target. The EC50 and IC50 values of a ligand for its target can both be determined by well-known methods, for example ELISA. Suitable assays to assess the EC50 and IC50 of polypeptides are set out in the Examples.

Part B2 of the polypeptide of the invention is a polypeptide binding domain specific for CTLA-4. This means that it binds to CTLA-4 preferably with a greater binding affinity than that at which it binds to another molecule. Part B2 preferably binds to CTLA-4 with with the same or with a higher affinity than that of wild-type human CD86 for CTLA-4.

Preferably, the Kd of part B2 of the polypeptide of the invention for human CTLA-4 will be at least 2-fold, at least 2.5-fold, at least 3-fold, at least 3.5-fold, at least 4-fold, at least 4.5-fold, at least 5-fold, at least 5.5-fold, at least 8-fold or at least 10-fold less than the Kd of wild-type human CD86 for human CTLA-4. Most preferably, the Kd of part B2 for human CTLA-4 will be at least 5-fold or at least 10-fold less than the Kd of wild-type human CD86 for human CTLA-4. A preferred method for determining the Kd of a polypeptide for CTLA-4 is SPR analysis, e.g. with a Biacore™ system. Suitable protocols for the SPR analysis of polypeptides are set out in the Examples.

Preferably, the EC50 of part B2 of the polypeptide of the invention for human CTLA-4 will be at least 1.5-fold, at least 2-fold, at least 3-fold, at least 5-fold, at least 10-fold, at least 12-fold, at least 14-fold, at least 15-fold, at least 17-fold, at least 20-fold, at least 25-fold or at least 50-fold less than the EC50 of wild-type human CD86 for human CTLA-4 under the same conditions. Most preferably, the EC50 of part B2 for human CTLA-4 will be at least 10-fold or at least 25-fold less than the EC50 of wild-type human CD86 for human CTLA-4 under the same conditions. A preferred method for determining the EC50 of a polypeptide for CTLA-4 is via ELISA. Suitable ELISA assays for use in the assessment of the EC50 of polypeptides are set out in the Examples.

Preferably, the IC50 of part B2 of the polypeptide of the invention when competing with wild-type human CD86 for binding to human CTLA-4 will be at least 2-fold, at least 3-fold, at least 4-fold, at least 5-fold, at least 10-fold, at least 13-fold, at least 15-fold, at least 50-fold, at least 100-fold, or at least 300-fold less than the IC50 of wild-type human CD86 under the same conditions. Most preferably, the IC50 of part B2 will be at least 10-fold or at least 300-fold less than the IC50 of wild-type human CD86 under the same conditions. A preferred method for determining the IC50 of a polypeptide of the invention is via ELISA. Suitable ELISA assays for use in the assessment of the IC50 of polypeptides of the invention are set out in the Examples.

Part B2 of the polypeptide of the invention may also bind specifically to CD28. That is, part B2 may bind to CD28 with greater binding affinity than that at which it binds to another molecule, with the exception of CTLA-4. Part B2 may bind to human CD28 with a lower affinity than that of wild-type human CD86 for human CD28. Preferably, the Kd of part B2 for human CD28 will be at least 2-fold, preferably at least 5-fold, more preferably at least 10-fold higher than the Kd of wild-type human CD86 for human CD28.

The binding properties of part B2 of the polypeptide of the invention may also be expressed as a relative measure of the ability of a polypeptide to bind to the two targets, CTLA-4 and CD28. That is, the binding properties of part B2 may be expressed as a relative measure of the ability of the polypeptide to bind to CTLA-4 versus its ability to bind to CD28. Preferably part B2 has an increased relative ability to bind to CTLA-4 versus CD28, when compared to the corresponding relative ability of human wild-type CD86 to bind to CTLA-4 versus CD28.

When the binding affinity of a polypeptide for both CTLA-4 and CD28 is assessed using the same parameter (e.g. Kd, EC50), then the relative binding ability of the polypeptide for each target may be expressed as a simple ratio of the values of the parameter for each target. This ratio may be referred to as the binding ratio or binding strength ratio of a polypeptide. For many parameters used to assess binding affinity (e.g. Kd, EC50), a lower value indicates a higher affinity. When this is the case, the ratio of binding affinities for CTLA-4 versus CD28 is preferably expressed as a single numerical value calculated according to the following formula:

$$\textit{Binding ratio} = [\textit{binding affinity for CD28}] \div [\textit{binding affinity for CTLA-4}]$$

Alternatively, if binding affinity is assessed using a parameter for which a higher value indicates a higher affinity, the inverse of the above formula is preferred. In either context, part B2 of the polypeptide of the invention preferably has a higher binding ratio than human wild-type CD86. It will be appreciated that direct comparison of the binding ratio for a given polypeptide to the binding ratio for another polypeptide typically requires that the same parameters be used to assess the binding affinities and calculate the binding ratios for both polypeptides.

Preferably, the binding ratio for a polypeptide is calculated by determining the Kd of the polypeptide for each target and then calculating the ratio in accordance with the formula [Kd for CD28] ÷ [Kd for CTLA-4]. This ratio may be referred to as the Kd binding ratio of a polypeptide. A preferred method for determining the Kd of a polypeptide for a target is SPR analysis, e.g. with a Biacore™ system. Suitable protocols for the SPR analysis of polypeptides of the invention are set out in the Examples. The binding ratio of part B2 of the polypeptide of the invention calculated according to this method is preferably at least 2-fold or at least 4-fold higher than the binding ratio of wild-type human CD86 calculated according to the same method.

Alternatively, the binding ratio for a polypeptide may be calculated by determining the EC50 of the polypeptide for each target and then calculating the ratio in accordance with the formula [EC50 for CD28] ÷ [EC50 for CTLA-4]. This ratio may be referred to as the EC50 binding ratio of a polypeptide. A preferred method for determining the EC50 of a polypeptide for a target is via ELISA. Suitable ELISA assays for use in the assessment of the EC50 of polypeptides of the invention are set out in the Examples. The binding ratio of part B2 of the polypeptide of the invention calculated according to this method is at least 2-fold, at least 3-fold, at least 4-fold, at least 5-fold, at least 6-fold, at least 7-fold, at least 8-fold, at least 9-fold or at least 10-fold higher than the binding ratio of wild-type human CD86 calculated according to the same method.

Part B2 of the polypeptide of the invention may have the ability to cross-compete with another polypeptide for binding to CTLA-4. For example, part B2 may cross-compete with a polypeptide having the amino acid sequence of any one of SEQ ID NOs: 6 to 24 for binding to CTLA-4. Such cross-competing polypeptides may be identified in standard binding assays. For example, SPR analysis (e.g. with a Biacore™ system), ELISA assays or flow cytometry may be used to demonstrate cross-competition.

In addition to the above functional characteristics, part B2 of the polypeptide of the invention has certain preferred structural characteristics. Part B2 either comprises or consists of (i) the monomeric soluble extracellular domain of human wild-type CD86, or (ii) a polypeptide variant of said soluble extracellular domain, provided that said polypeptide variant binds to human CTLA-4 with higher affinity than wild-type human CD86.

A polypeptide variant of the monomeric soluble extracellular domain of human wild-type CD86 comprises or consists of an amino acid sequence which is derived from that of human wild-type CD86, specifically the amino acid sequence of the soluble extracellular domain of human wild-type CD86 (SEQ ID NO: 3), optionally lacking A24 and P25. In particular, a variant comprises an amino acid sequence in which at least one amino acid is changed when compared to the amino acid sequence of SEQ ID NO: 3 (or said sequence lacking A24 and P25). By "changed" it is meant that at least one amino acids is deleted, inserted, or substituted compared to the amino acid sequence of SEQ ID NO: 3 (or said sequence lacking A24 and P25). By "deleted" it is meant that the at least one amino acid present in the amino acid sequence of SEQ ID NO: 3 (or said sequence lacking A24 and P25) is removed, such that the amino acid sequence is shortened by one amino acid. By "inserted" it is meant that the at least one additional amino acid is introduced into the amino acid sequence of SEQ ID NO: 3 (or said sequence lacking A24 and P25), such that the amino acid sequence is lengthened by one amino acid. By "substituted" it is meant that the at least one amino acid in the amino acid sequence of SEQ ID NO: 3 (or said sequence lacking A24 and P25) is replaced with an alternative amino acid.

Amino acids herein may be referred to by full name, three letter code or single letter code, as set out below.

| | | | | | |
|---|---|---|---|---|---|
| Alanine | Ala | A | Leucine | Leu | L |
| Arginine | Arg | R | Lysine | Lys | K |
| Asparagine | Asn | N | Methionine | Met | M |
| Aspartic acid | Asp | D | Phenylalanine | Phe | F |
| Cysteine | Cys | C | Proline | Pro | P |
| Glutamic acid | Glu | E | Serine | Ser | s |
| Glutamine | Gln | Q | Threonine | Thr | T |
| Glycine | Gly | G | Typtophan | Trp | w |
| Histidine | His | H | Tyrosine | Tyr | Y |
| Isoleucine | Ile | I | Valine | Val | V |

Typically, at least 1, 2, 3, 4, 5, 6, 7, 8 or 9 amino acids are changed when compared to the amino acid sequence of SEQ ID NO: 3 (or said sequence lacking A24 and P25). Typically, no more than 10, 9, 8, 7, 6, 5, 4, 2 or 1 amino acids are changed when compared to the amino acid sequence of SEQ ID NO: 3 (or said sequence lacking A24 and P25). It will be appreciated that any of these lower limits may be combined with any of these upper limits to define a range for the permitted number of changes compared to the amino acid sequence of SEQ ID NO: 3 (or said sequence lacking A24 and P25). Thus, for example, a polypeptide of the invention may comprise an amino acid sequence in which the permitted number of amino acid changes compared to the amino acid sequence of SEQ ID NO: 3 (or said sequence lacking A24 and P25) is in the range 2 to 3, 2 to 4, 2 to 5, 2 to 6, 2 to 7, 2 to 8, 2 to 9, 2 to 10, 3 to 4, 3 to 5, 3 to 6, and so on.

It is particularly preferred that at least 2 amino acids are changed when compared to the amino acid sequence of SEQ ID NO: 3 (or said sequence lacking A24 and P25). Preferably, the permitted number of amino acid changes compared to the amino acid sequence of SEQ ID NO: 3(or said sequence lacking A24 and P25) is in the range 2 to 9, 2 to 8 or 2 to 7.

The numbers and ranges set out above may be achieved with any combination of deletions, insertions or substitutions compared to the amino acid sequence of SEQ ID NO: 3(or said sequence lacking A24 and P25). For example, there may be only deletions, only insertions, or only substitutions compared to the amino acid sequence of SEQ ID NO: 3 (or said sequence lacking A24 and P25), or any mixture of deletions, insertions or substitutions. Preferably the variant comprises an amino acid sequence in which all of the changes compared to the amino acid sequence of SEQ ID NO: 3 (or said sequence lacking A24 and P25) are substitutions. That is, a sequence in which no amino acids are deleted or inserted compared to the sequence of SEQ ID NO: 3 (or said sequence lacking A24 and P25). In the amino acid sequence of a preferred variant, 1, 2, 3, 4, 5, 6, 7, or 8 amino acids are substituted when compared to the amino acid sequence of SEQ ID NO: 3 (or said sequence lacking A24 and P25) and no amino acids are deleted or inserted compared to the sequence of SEQ ID NO: 3 (or said sequence lacking A24 and P25).

Preferably the changes compared to the sequence of SEQ ID NO: 3 (or said sequence lacking A24 and P25) are in the FG loop region (positions 114 to 121) and/or the beta sheet region of SEQ ID NO: 3. The strands of the beta sheet region have the following positions in SEQ ID NO: 3: A:27-31, B:36-37, C:54-58, C':64-69, C":72-74, D:86-88, E:95-97, F:107-113, G:122-133.

Most preferably, the changes compared to the sequence of SEQ ID NO: 3 (or said sequence lacking A24 and P25) are in one or more of the positions selected from 32, 48, 49, 54, 74, 77, 79, 103, 107, 111, 118, 120, 121, 122, 125, 127 or 134. All numbering of amino acid positions herein is based on counting the amino acids in SEQ ID NO: 4 starting from the N terminus. Thus, the first position at the N terminus of SEQ ID NO: 3 is numbered 24 (see schematic diagram in Figure 4).

Particularly preferred insertions include a single additional amino acid inserted between positions 116 and 117 and/or a single additional amino acid inserted between positions 118 and 119. The inserted amino acid is preferably Tyrosine (Y), Serine (S), Glycine (G), Leucine (L) or Aspartic Acid (D).

A particularly preferred substitution is at position 122, which is Arginine (R). The polypeptide of the invention preferably includes an amino acid sequence in which position 122 is substituted compared to the amino acid sequence of SEQ ID NO: 3 (or said sequence lacking A24 and P25). The most preferred substitution at position 122 is to replace Arginine (R) with Lysine (K) or Asparagine (N), ranked in order of preference. This substitution may be referred to as R122K/N.

Other preferred substitutions are at positions 107, 121, and 125, which are Leucine (L), Isoleucine (I) and Glutamic acid (Q), respectively. In addition to the substitution at position 122, the polypeptide of the invention preferably includes an amino acid sequence in which at least one of the amino acids at positions 107, 121 and 125 is also substituted compared to the amino acid sequence of SEQ ID NO: 3 (or said sequence lacking A24 and P25). The amino acid sequence of the polypeptide of the invention may also be substituted at one or more of positions 32, 48, 49, 54, 64, 74, 77, 79, 103, 111, 118, 120, 127 and 134.

The most preferred substitution at position 107 is to replace Leucine (L) with Isoleucine(I), Phenylalanine(F) or Arginine(R), ranked in order of preference. This substitution may be referred to as L107I/F/R. Similar notation is used for other substitutions described herein. The most preferred substitution at position 121 is to replace Isoleucine (I) with Valine (V). This substitution may be referred to as 1121V. The most preferred substitution at position 125 is to replace Glutamine (Q) with Glutamic acid (E). This substitution may be referred to as Q125E.

Other substitutions which may be preferred in the amino acid sequence of the polypeptide of the invention include: F32I, Q48L, S49T, V54I, V64I, K74I/R, S77A, H79D/S/A, K103E, I111V, T118S, M120L, N127S/D and A134T.

Particularly preferred variants of said soluble extracellular domain of human wild-type CD86 comprise or consist of any one of the following amino acid sequences of SEQ ID NOs: 6 to 24

| SEQ ID NO. | DESIG NATIO N | SEQUENCE |
|---|---|---|
| 6 | 900 | LKIQAYFNETADLPCQFANSQNQSLSELVVFWQDQENLVLNEVYLGKEKFDSVDSK YMGRTSFDSDSWTLRLHNLQIKDKGIYQCVIHHKKPSGLVKIHEMNSELSVLA |
| 7 | 901 | LKIQAYFNETADLPCQFANSQNLTLSELVVFWQDQENLVLNEVYLGKEKFDSVHSK YMGRTSFDSDSWTLRLHNLQIKDKGIYQCVIHHKKPTGMIKIHEMNSELSVLT |
| 8 | 904 | LKIQAYFNETADLPCQFANSQNQSLSELIVFWQDQENLVLNEVYLGKERFDAVDSK YMGRTSFDSDSWTLRLHNLQIKDKGIYQCIIHHKKPSGMVKIHQMDSELSVLA |
| 9 | 906 | LKIQAYINETADLPCQFANSQNLSLSELVVFWQDQENLVLNEVYLGKERFDSVDSK YMGRTSFDSDSWTLRLHNLQIKDKGFYQCIIHHKKPTGLVKIHEMNSELSVLA |
| 10 | 907 | LKIQAYFNETADLPCQFANSQNQSLSELVVFWQDQENLVLNEVYLGKEKFDSVHSK YMGRTSFDSDSWTLRLHNLQIKDKGLYQCIIHHKKPTGMIKIHEMNSELSVLA |
| 11 | 908 | LKIQAYFNETADLPCQFANSQNQSLSELVVFWQDQENLVLNEVYLGKEKFDSVHSK YMGRTSFDSDSWTLRLHNLQIKDKGIYQCIIHHKKPTGMVKIHEMNSELSVLA |
| 12 | 910 | LKIQAYFNETADLPCQFANSQNQSLSELVVFWQDQENLVLNEVYLGKEKFDSVDSK YMGRTSFDSDSWTLRLHNLQIKDKGIYQCIIHHKKPTGMVKIHEMNSELSVLA |
| 13 | 915 | LKIQAYFNETADLPCQFANSQNQSLSELVVFWQDQENLILNEVYLGKEKFDSVDSK YMGRTSFDSDSWTLRLHNLQIKDKGFYQCIIHHKKPSGLIKIHQMDSELSVLA |
| 14 | 938 | LKIQAYFNETADLPCQFANSQNQSLSELVVFWQDQENLILNEVYLGKEKFDSVHSK YMGRTSFDSDSWTLRLHNLQIKDKGIYQCIIHHKKPTGMVKIHQMNSELSVLA |
| 15 | 1038 | APLKIQAYFNETADLPCQFANSQNLSLSELVVFWQDQENLVLNEVYLGKEKFDSVD SKYMGRTSFDSDSWTLRLHNLQIKDKGIYQCIIHHKKPTGMVKIHEMNSELSVLA |
| 16 | 1039 | APLKIQAYFNETADLPCQFANSQNLSLSELVVFWQDQENLVLNEVYLGKEKFDSVS SKYMGRTSFDSDSWTLRLHNLQIKDKGIYQCIIHHKKPSGMVKIHQMDSELSVLA |
| 17 | 1040 | APLKIQAYFNETADLPCQFANSQNLSLSELVVFWQDQENLVLNEVYLGKERFDSVD SKYMGRTSFDSDSWTLRLHNLQIKDKGRYQCIIHHKKPTGMINIHQMNSELSVLA |
| 18 | 1041 | APLKIQAYLNETADLPCQFANSQNLSLSELVVFWQDQENLVLNEVYLGKEKFDSVD SKYMGRTSFDSDSWTLRLHNLQIKDKGIYQCIIHHKKPTGLVKIHEMNSELSVLA |

(continued)

| SEQ ID NO. | DESIG NATIO N | SEQUENCE |
|---|---|---|
| 19 | 1042 | APLKIQAYFNETADLPCQFANSQNLSLSELVVFWQDQENLVLNEVYLGKEIFDSVS SKYMGRTSFDSDSWTLRLHNLQIKDKGIYQCIIHHKKPSGMVKIHQMDSELSVLA |
| 20 | 1043 | APLKIQAYFNETADLPCQFANSQNLSLSELVVFWQDQENLVLNEVYLGKEKFDSVD SKYMGRTSFDSDSWTLRLHNLQIKDKGIYQCIIHHKKPTGMIKIHEMNSELSVLA |
| 21 | 1044 | APLKIQAYFNETADLPCQFANSQNLTLSELVVFWQDQENLVLNEVYLGKEKFDSVS SKYMGRTSFDSDSWTLRLHNLQIKDKGIYQCIIHHKKPTGMIKIHEMSSELSVLA |
| 22 | 1045 | APLKIQAYFNETADLPCQFANSQNLTLSELVVFWQDQENLVLNEVYLGKEKFDSVD SKYMGRTSFDSDSWTLRLHNLQIKDKGLYQCIIHHKKPTGLVKIHEMNSELSVLA |
| 23 | 1046 | APLKIQAYFNETADLPCQFANSQNQSLSELVVFWQDQENLVLNEVYLGKEKFDSVD SKYMGRTSFDSDSWTLRLHNLQIEDKGIYQCIIHHKKPSGMVKIHQMDSELSVLA |
| 24 | 1047 | APLKIQAYFNETADLPCQFANSQNLSLSELVVFWQDQENLVLNEVYLGKEKFDSVD SKYMGRTSFDSDSWTLRLHNLQIKDKGIYQCIIHHKKPTGLVKIHEMNSELSVLA |

The amino acid sequences shown in SEQ ID NOs: 6 to 14 may optionally include the additional residues AP at the N-terminus . The amino acid sequences shown in SEQ ID NOs: 15 to 24 may optionally lack the residues AP at the N-terminus. In either case, these residues correspond to A24 and P25 of SEQ ID NO: 3.

Part B2 of the polypeptide of the invention may comprise or consist of any of the above-described variants of said soluble extracellular domain of human wild-type CD86. That is, part B2 of the polypeptide of the invention may comprise or consist of the amino acid sequence of any one of SEQ ID NOs: 6 to 24.

The binding domain may modulate signalling from CTLA-4, for example when administered to a cell expressing CTLA-4, such as a T cell. Preferably the binding domain reduces, i.e. inhibits or blocks, said signalling and thereby increases the activation of said cell. Changes in CTLA-4 signalling and cell activation as a result of administration of a test agent (such as the binding domain) may be determined by any suitable method. Suitable methods include assaying for the ability of membrane-bound CD86 (e.g. on Raji cells) to bind and signal through CTLA-4 expressed on the surface of T cells, when in the presence of a test agent or in the presence of a suitable control. An increased level of T cell IL-2 production or an increase in T cell proliferation in the presence of the test agent relative to the level of T cell IL-2 production and/or T cell proliferation in the presence of the control is indicative of reduced signalling through CTLA-4 and increased cell activation. A typical assay of this type is disclosed in Example 9 of US20080233122.

*The polypeptide of the invention*

The polypeptide of the invention is capable of specifically binding to both human CTLA-4 and human CD40, and comprises B1 and B2, wherein:

B1 is an antibody, or antigen binding fragment thereof, specific for human CD40, and which is a CD40 agonist; and B2 is a polypeptide binding domain specific for human CTLA-4, which comprises or consists of:

(i) the amino acid sequence of SEQ ID NO: 3; or
(ii) an amino acid sequence in which at least one amino acid is changed when compared to the amino acid sequence of SEQ ID NO: 3 provided that said binding domain binds to human CTLA-4 with higher affinity than wild-type human CD86.

By capable of specifically binding to both CTLA-4 and CD40, it is meant that part B1 specifically binds to CD40 and part B2 specifically binds to CTLA-4, in accordance with the definitions provided for each part above. Preferably the binding characteristics of parts B1 and B2 for their respective targets are unchanged or substantially unchanged when they are present as part of a polypeptide of the invention, when compared to said characteristics for parts B1 and B2 when present as separate entities. The binding characteristics of parts B1 and B2 when present as part a polypeptide of the invention may be assessed by any suitable assay. In particular, the assays set out above for each separate part may also be applied to B1 and B2 when they are present as part of a polypeptide of the invention. Suitable assays for assessed the binding characteristics of polypeptides of the invention are also set out in the Examples.

Part B1 of the polypeptide of the invention is an antibody, or antigen-binding fragment thereof, which typically comprises at least one heavy chain (H) and/or at least one light chain (L). Part B2 of the polypeptide of the invention may be attached to any part of B1, but may typically be attached to said at least one heavy chain (H) or least one light chain (L), preferably at either the N or the C terminus. Part B2 of the polypeptide of the invention may be so attached either directly or indirectly via any suitable linking molecule (a linker).

Part B1 preferably comprises at least one heavy chain (H) and at least one light chain (L) and part B2 is preferably attached to the N or the C terminus of either said heavy chain (H) or said light chain (L). An exemplary antibody of B1 consists of two identical heavy chains (H) and two identical light chains (L). Such an antibody is typically arranged as two arms, each of which has one H and one L joined as a heterodimer, and the two arms are joined by disulfide bonds between the H chains. Thus, the antibody is effectively a homodimer formed of two H-L heterodimers. Part B2 of the polypeptide of the invention may be attached to both H chains or both L chains of such an antibody, or to just one H chain, or just one L chain.

The polypeptide of the invention may therefore alternatively be described as an anti-CD40 antibody, or an antigen binding fragment thereof, to which is attached at least one polypeptide binding domain specific for CTLA-4, which comprises or consists of the monomeric soluble extracellular domain of human wild-type CD86 or a variant thereof. The binding domains of B1 and B2 may be the only binding domains in the polypeptide of the invention.

The polypeptide of the invention may comprise a polypeptide arranged according to any one of the following formulae, written in the direction N-C:

(a) H-(X)n-B2;
(b) B2-(X)n-H;
(c) L-(X)n-B2; or
(d) B2-(X)n-L

wherein H is the heavy chain of an antibody (i.e. of B1), L is the light chain of an antibody (i.e. of B1), X is a linker and n is 0 or 1. Where the linker (X) is a peptide, it typically has the amino acid sequence SGGGGSGGGGS, SGGGGSGGGGSAP, NFSQP, KRTVA or (SG)m, where m = 1 to 7.

The present invention also provides a polypeptide which consists of a polypeptide arranged according to any of formulae (a) to (d). Said polypeptide may be provided as a monomer or may be present as a component of a multimeric protein, such as an antibody. Said polypeptide may be isolated. Examples of such polypeptides are provided as SEQ ID NOs: 56 to 60 and 110 to 114.

Part B2 may be attached to any part of a polypeptide of the invention, or to a linker, by any suitable means. For example, the various parts of the polypeptide may be joined by chemical conjugation, such as with a peptide bond. Thus the polypeptide of the invention may comprise or consist of a fusion protein comprising B1 (or a component part therof) and B2, optionally joined by a peptide linker. In such a fusion protein, the CD40-binding domain or domains of B1 and the CTLA-4-binding domain or domains of B2 may be the only binding domains.

Other methods for conjugating molecules to polypeptides are known in the art. For example, carbodiimide conjugation (Bauminger & Wilchek (1980) Methods Enzymol. 70, 151-159) may be used to conjugate a variety of agents, including doxorubicin, to antibodies or peptides. The water-soluble carbodiimide, 1-ethyl-3-(3-dimethylaminopropyl) carbodiimide (EDC) is particularly useful for conjugating a functional moiety to a binding moiety. As a further example, conjugation may be achieved by sodium periodate oxidation followed by reductive alkylation of appropriate reactants, or by glutaraldehyde cross-linking. However, it is recognised that, regardless of which method is selected, a determination should preferably be made that parts B1 and B2 retain or substantially retain their target binding properties when present as parts of the polypeptide of the invention.

The same techniques may be used to link the polypeptide of the invention(directly or indirectly) to another molecule. The other molecule may be a a therapeutic agent or a detectable label. Suitable therapeutic agents include a cytotoxic moiety or a drug.

A polypeptide of the invention may be provided in isolated or substantially isolated form. By substantially isolated, it is meant that there may be substantial, but not total, isolation of the polypeptide from any surrounding medium. The polypeptides may be mixed with carriers or diluents which will not interfere with their intended use and still be regarded as substantially isolated.

Exemplary polypeptides of the invention may comprise or consist of the amino acid sequence of any one of SEQ ID NOs: 56 to 60 as shown below.

| SEQ ID | |
|---|---|
| 56 | Bispecific polypeptide 956/530 |

(continued)

| SEQ ID | |
|---|---|
| | QSVLTQPPSASGTPGQRVTISCSGSSSNIGSNTVNWYQQLPGTAPKLLIYRNNQRPSGVPDRFSG<br>SKSGTSASLAISGLRSEDEADYYCAAWDDSLSGWRFGGGTKLTVLGGTVAAPSVFIFPPSDEQLK<br>SGTASVVCLLNNFYPREAKVQWKVDNALQSGNSQESVTEQDSKDSTYSLSSTLTLSKADYEKHKV<br>YACEVTHQGLSSPVTKSFNRGECSGGGGSGGGGS**LKIQAYFNETADLPCQFANSQNQSLSELIVF**<br>**WQDQENLVLNEVYLGKERFDAVDSKYMGRTSFDSDSWTLRLHNLQIKDKGIYQCIIHHKKPSGMV**<br>**KIHQMDSELSVLA**<br>-A2-54 light chain,VL-kappa<br>-Linker (underlined)SGGGGSGGGGS<br>-904: CD86 mutant molecule (bold)<br>ANTIBODY PREFERBALY ASSEMBLES WITH A2-54 heavy chain |
| 57 | Bispecific polypeptide 957/530 |
| | QSVLTQPPSASGTPGQRVTISCSGSSSNIGSNTVNWYQQLPGTAPKLLIYRNNQRPSGVPDRFSG<br>SKSGTSASLAISGLRSEDEADYYCAAWDDSLSGWRFGGGTKLTVLGGTVAAPSVFIFPPSDEQLK<br>SGTASVVCLLNNFYPREAKVQWKVDNALQSGNSQESVTEQDSKDSTYSLSSTLTLSKADYEKHKV<br>YACEVTHQGLSSPVTKSFNRGECSGGGGSGGGGSAP**LKIQAYFNETADLPCQFANSQNQSLSELI**<br>**VFWQDQENLVLNEVYLGKERFDAVDSKYMGRTSFDSDSWTLRLHNLQIKDKGIYQCIIHHKKPSG**<br>**MVKIHQMDSELSVLA**<br>-A2-54 light chain,VL-kappa<br>-linker: (underlined)SGGGGSGGGGS<br>-Amino acids inserted: AP<br>-904: CD86 mutant molecule (bold)<br>ANTIBODY PREFERBALY ASSEMBLES WITH A2-54 heavy chain |
| 58 | Bispecific polypeptide 958/531<br>**LKIQAYFNETADLPCQFANSQNQSLSELIVFWQDQENLVLNEVYLGKERFDAVDSKYMGRTSFDS**<br>**DSWTLRLHNLQIKDKGIYQCIIHHKKPSGMVKIHQMDSELSVLA**NFSQPEVQLLESGGGLVQPGG<br>SLRLSCAASGFTFSDYYMSWVRQAPGKGLEWISSISSNGIYIYYADSLKGRFTISRDNSKNTLYL<br>QMNSLRAEDTAVYYCARAPVDYSNPSGMDVWGQGTLVTVSSASTKGPSVFPLAPSSKSTSGGTAA<br>LGCLVKDYFPEPVTVSWNSGALTSGVHTFPAVLQSSGLYSLSSVVTVPSSSLGTQTYICNVNHKP<br>SNTKVDKKVEPKSCDKTHTCPPCPAPELLGGPSVFLFPPKPKDTLMISRTPEVTCVVVDVSHEDP<br>EVKFNWYVDGVEVHNAKTKPREEQYNSTYRVVSVLTVLHQDWLNGKEYKCKVSNKALPAPIEKTI<br>SKAKGQPREPQVYTLPPSRDELTKNQVSLTCLVKGFYPSDIAVEWESNGQPENNYKTTPPVLDSD<br>GSFFLYSKLTVDKSRWQQGNVFSCSVMHEALHNHYTQKSLSLSPGK<br>-904: mutant CD86 molecule(bold)<br>-linker: (underlined) NFSQP<br>-A2-54 Heavy Chain, VH-gammal<br>ANTIBODY PREFERBALY ASSEMBLES WITH A2-54 light chain |
| 59 | Bispecific polypeptide 959/530<br>**LKIQAYFNETADLPCQFANSQNQSLSELIVFWQDQENLVLNEVYLGKERFDAVDSKYMGRTSFDS**<br>**DSWTLRLHNLQIKDKGIYQCIIHHKKPSGMVKIHQMDSELSVLA**KRTVAQSVLTQPPSASGTPGQ<br>RVTISCSGSSSNIGSNTVNWYQQLPGTAPKLLIYRNNQRPSGVPDRFSGSKSGTSASLAISGLRS<br>EDEADYYCAAWDDSLSGWRFGGGTKLTVLGGTVAAPSVFIFPPSDEQLKSGTASVVCLLNNFYPR<br>EAKVQWKVDNALQSGNSQESVTEQDSKDSTYSLSSTLTLSKADYEKHKVYACEVTHQGLSSPVTK<br>SFNRGEC<br>-904: mutant CD86 molecule(bold)<br>-Linker: (underlined) KRTVA<br>-A2-54 Variable Light chain,VL-kappa<br>ANTIBODY PREFERBALY ASSEMBLES WITH A2-54 heavy chain |
| 60 | Bispecific polypeptide 960/530 |

(continued)

| SEQ ID | |
|---|---|
| | **LKIQAYFNETADLPCQFANSQNQSLSELIVFWQDQENLVLNEVYLGKERFDAVDSKYMGRTSFDSDSWTLRLHNLQIKDKGIYQCIIHHKKPSGMVKIHQMDSELSVLA**NFSQPQSVLTQPPSASGTPGQRVTISCSGSSSNIGSNTVNWYQQLPGTAPKLLIYRNNQRPSGVPDRFSGSKSGTSASLAISGLRSEDEADYYCAAWDDSLSGWRFGGGTKLTVLGGTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC<br>-904: mutant CD86 molecule(bold)<br>-linker: (underlined) NFSQP<br>-A2-54 Variable Light chain, VL-kappa<br>ANTIBODY PREFERBALY ASSEMBLES WITH A2-54 heavy chain |
| 110 | Bispecific polypeptide 1107/1145<br>DIQMTQSPSSLSASVGDRVTITCRASQSISSYLNWYQQKPGKAPKLLIYAASSLQSGVPSRFSGSGSGTDFTLTISSLQPEDFATYYCQQYGVYPFTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECSGGGGSGGGGS**APLKIQAYFNETADLPCQFANSQNLSLSELVVFWQDQENLVLNEVYLGKERFDSVDSKYMGRTSFDSDSWTLRLHNLQIKDKGRYQCIIHHKKPTGMINIHQMNSELSVLA**<br>-clone 1145 light chain,VL-kappa<br>-Linker (underlined)SGGGGSGGGGS<br>-1040: CD86 mutant molecule (bold)<br>ANTIBODY PREFERBALY ASSEMBLES WITH HEAVY CHAIN 1107 |
| 111 | Bispecific polypeptide 1136/1143<br>DIQMTQSPSSLSASVGDRVTITCRASQSISSYLNWYQQKPGKAPKLLIYAASSLQSGVPSRFSGSGSGTDFTLTISSLQPEDFATYYCQQAYYAGLFTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECSGGGGSGGGGSAP**LKIQAYFNETADLPCQFANSQNLSLSELVVFWQDQENLVLNEVYLGKERFDSVDSKYMGRTSFDSDSWTLRLHNLQIKDKGRYQCIIHHKKPTGMINIHQMNSELSVLA**<br>-clone 1143 light chain,VL-kappa<br>-Linker (underlined)SGGGGSGGGGS<br>-Amino acids inserted: AP<br>-1040: CD86 mutant molecule (bold)<br>ANTIBODY PREFERBALY ASSEMBLES WITH HEAVY CHAIN 1136 |
| 112 | Bispecific polypeptide 1132/1139<br>DIQMTQSPSSLSASVGDRVTITCRASQSISSYLNWYQQKPGKAPKLLIYAASSLQSGVPSRFSGSGSGTDFTLTISSLQPEDFATYYCQQYGRNPPTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECSGGGGSGGGGSAP**LKIQAYFNETADLPCQFANSQNLSLSELVVFWQDQENLVLNEVYLGKERFDSVDSKYMGRTSFDSDSWTLRLHNLQIKDKGRYQCIIHHKKPTGMINIHQMNSELSVLA**<br>-clone 1139 light chain,VL-kappa<br>-Linker (underlined)SGGGGSGGGGS<br>-Amino acids inserted: AP<br>-1040: CD86 mutant molecule (bold)<br>ANTIBODY PREFERBALY ASSEMBLES WITH HEAVY CHAIN 1132 |
| | |
| 113 | Bispecific polypeptides 1140/1141 and 1134/1141 |

(continued)

| SEQ ID | |
|---|---|
| | DIQMTQSPSSLSASVGDRVTITCRASQSISSYLNWYQQKPGKAPKLLIYAASSLQSGVPSRFSGS<br>GSGTDFTLTISSLQPEDFATYYCQQSYSTPYTFGQGTKLEIKRTVAAPSVFIFPPSDEQLKSGTA<br>SVVCLLNNFYPREAKVQWKVDNALQSGNSQESVTEQDSKDSTYSLSSTLTLSKADYEKHKVYACE<br>VTHQGLSSPVTKSFNRGECSGGGGSGGGGSAP**LKIQAYFNETADLPCQFANSQNLSLSELVVFWQ**<br>**DQENLVLNEVYLGKERFDSVDSKYMGRTSFDSDSWTLRLHNLQIKDKGRYQCIIHHKKPTGMINI**<br>**HQMNSELSVLA**<br>-clone 1141 light chain,VL-kappa<br>-Linker (underlined)SGGGGSGGGGS<br>-Amino acids inserted: AP<br>-1040: CD86 mutant molecule (bold)<br>FOR 1140/1141: ANTIBODY ASSEMBLES WITH HEAVY CHAIN 1140<br>FOR 1134/1141: ANTIBODY ASSEMBLES WITH HEAVY CHAIN 1134 |
| 114 | 1150/1152<br>DIQMTQSPSSLSASVGDHVTITCRASQSISSYLNWYQQKPGKAPKLLIYAASSLQSGVPSRFSGS<br>GSGTDFTLTISSLQPEDFATYYCQQYGSAPPTFGQGTKLEIKRTVAAPSVFIFPPSDEQLKSGTA<br>SVVCLLNNFYPREAKVQWKVDNALQSGNSQESVTEQDSKDSTYSLSSTLTLSKADYEKHKVYACE<br>VTHQGLSSPVTKSFNRGECSGGGGSGGGGSAP**LKIQAYFNETADLPCQFANSQNLSLSELVVFWQ**<br>**DQENLVLNEVYLGKERFDSVDSKYMGRTSFDSDSWTLRLHNLQIKDKGRYQCIIHHKKPTGMINI**<br>**HQMNSELSVLA**<br>-clone 1152 light chain,VL-kappa<br>-Linker (underlined)SGGGGSGGGGS<br>-Amino acids inserted: AP<br>-1040: CD86 mutant molecule (bold)<br>ANTIBODY PREFERABLY ASSEMBLES WITH HEAVY CHAIN 1150 |

The polypeptide of the invention may be produced by any suitable means. For example, all or part of the polypeptide may be expressed as a fusion protein by a cell comprising a nucleotide which encodes said polypeptide.

Alternatively parts B1 and B2 may be produced separately and then subsequently joined together. Joining may be achieved by any suitable means, for example using the chemical conjugation methods and linkers outlined above. Separate production of parts B1 and B2 may be achieved by any suitable means. For example by expression from separate nucleotides optionally in separate cells, as is explained in more detail below.

*Polynucleotides, vectors and cells*

The invention also relates to polynucleotides that encode a polypeptide of the invention . Thus, a polynucleotide of the invention may encode any polypeptide as described herein. The terms "nucleic acid molecule" and "polynucleotide" are used interchangeably herein and refer to a polymeric form of nucleotides of any length, either deoxyribonucleotides or ribonucleotides, or analogs thereof. Non-limiting examples of polynucleotides include a gene, a gene fragment, messenger RNA (mRNA), cDNA, recombinant polynucleotides, plasmids, vectors, isolated DNA of any sequence, isolated RNA of any sequence, nucleic acid probes, and primers. A polynucleotide of the invention may be provided in isolated or substantially isolated form. By substantially isolated, it is meant that there may be substantial, but not total, isolation of the polypeptide from any surrounding medium. The polynucleotides may be mixed with carriers or diluents which will not interfere with their intended use and still be regarded as substantially isolated.

A nucleic acid sequence which "encodes" a selected polypeptide is a nucleic acid molecule which is transcribed (in the case of DNA) and translated (in the case of mRNA) into a polypeptide *in vivo* when placed under the control of appropriate regulatory sequences. The boundaries of the coding sequence are determined by a start codon at the 5' (amino) terminus and a translation stop codon at the 3' (carboxy) terminus. For the purposes of the invention, such nucleic acid sequences can include, but are not limited to, cDNA from viral, prokaryotic or eukaryotic mRNA, genomic sequences from viral or prokaryotic DNA or RNA, and even synthetic DNA sequences. A transcription termination sequence may be located 3' to the coding sequence.

Representative polynucleotides which encode examples of a heavy chain or light chain amino acid sequence of an antibody may comprise or consist of any one of the sequences set out below.

| SEQ ID | | |
|---|---|---|
| | Antibody A2-54 | |
| 63 | A2-54 heavy chain, VH-gamma1 | GAGGTGCAGCTGCTGGAGTCCGGAGGAGGCCTGGTGCAGCCCGGCGGCAGCCTGAGAC<br>TGAGCTGTGCCGCCAGCGGATTCACCTTCTCCGACTACTATATGAGCTGGGTGAGGCA<br>GGCCCCAGGCAAGGGCCTGGAGTGGATTTCCTCCATCAGCAGCAATGGGATCTACATT<br>TACTACGCCGACAGCCTGAAGGGCAGGTTCACAATCAGCAGGGACAACTCTAAGAACA<br>CACTGTACCTGCAGATGAACTCCCTGCGCGCCGAGGACACCGCCGTGTATTACTGTGC<br>CAGGGCCCCCGTGGACTACTCTAATCCCAGCGGCATGGACGTGTGGGGCCAGGGCACC<br>CTGGTGACAGTGAGCTCAGGTGAGTCGTACGCTAGCAAGCTTTCTGGGGCAGGCCAGG<br>CCTGACCTTGGCTTTGGGGCAGGGAGGGGGCTAAGGTGAGGCAGGTGGCGCCAGCCAG<br>GTGCACACCCAATGCCCATGAGCCCAGACACTGGACGCTGAACCTCGCGGACAGTTAA<br>GAACCCAGGGGCCTCTGCGCCCTGGGCCCAGCTCTGTCCCACACCGCGGTCACATGGC<br>ACCACCTCTCTTGCAGCCTCCACCAAGGGCCCATCGGTCTTCCCCCTGGCACCCTCCT<br>CCAAGAGCACCTCTGGGGGCACAGCGGCCCTGGGCTGCCTGGTCAAGGACTACTTCCC<br>CGAACCGGTGACGGTGTCGTGGAACTCAGGCGCCCTGACCAGCGGCGTGCACACCTTC<br>CCGGCTGTCCTACAGTCCTCAGGACTCTACTCCCTCAGCAGCGTGGTGACCGTGCCCT<br>CCAGCAGCTTGGGCACCCAGACCTACATCTGCAACGTGAATCACAAGCCCAGCAACAC<br>CAAGGTGGACAAGAAAGTTGGTGAGAGGCCAGCACAGGGAGGGAGGGTGTCTGCTGGA<br>AGCCAGGCTCAGCGCTCCTGCCTGGACGCATCCCGGCTATGCAGCCCCAGTCCAGGGC<br>AGCAAGGCAGGCCCCGTCTGCCTCTTCACCCGGAGGCCTCTGCCCGCCCCACTCATGC<br>TCAGGGAGAGGGTCTTCTGGCTTTTTCCCCAGGCTCTGGGCAGGCACAGGCTAGGTGC<br>CCCTAACCCAGGCCCTGCACACAAAGGGGCAGGTGCTGGGCTCAGACCTGCCAAGAGC<br>CATATCCGGGAGGACCCTGCCCCTGACCTAAGCCCACCCCAAAGGCCAAACTCTCCAC<br>TCCCTCAGCTCGGACACCTTCTCTCCTCCCAGATTCCAGTAACTCCCAATCTTCTCTC<br>TGCAGAGCCCAAATCTTGTGACAAAACTCACACATGCCCACCGTGCCCAGGTAAGCCA<br>GCCCAGGCCTCGCCCTCCAGCTCAAGGCGGGACAGGTGCCCTAGAGTAGCCTGCATCC<br>AGGGACAGGCCCCAGCCGGGTGCTGACACGTCCACCTCCATCTCTTCCTCAGCACCTG<br>AACTCCTGGGGGGACCGTCAGTCTTCCTCTTCCCCCCAAAACCCAAGGACACCCTCAT<br>GATCTCCCGGACCCCTGAGGTCACATGCGTGGTGGTGGACGTGAGCCACGAAGACCCT<br>GAGGTCAAGTTCAACTGGTACGTGGACGGCGTGGAGGTGCATAATGCCAAGACAAAGC<br>CGCGGGAGGAGCAGTACAACAGCACGTACCGTGTGGTCAGCGTCCTCACCGTCCTGCA<br>CCAGGACTGGCTGAATGGCAAGGAGTACAAGTGCAAGGTCTCCAACAAAGCCCTCCCA<br>GCCCCCATCGAGAAAACCATCTCCAAAGCCAAAGGTGGGACCCGTGGGGTGCGAGGGC<br>CACATGGACAGAGGCCGGCTCGGCCCACCCTCTGCCCTGAGAGTGACCGCTGTACCAA<br>CCTCTGTCCCTACAGGGCAGCCCCGAGAACCACAGGTGTACACCCTGCCCCCATCCCG<br>GGATGAGCTGACCAAGAACCAGGTCAGCCTGACCTGCCTGGTCAAAGGCTTCTATCCC<br>AGCGACATCGCCGTGGAGTGGGAGAGCAATGGGCAGCCGGAGAACAACTACAAGACCA<br>CGCCTCCCGTGCTGGACTCCGACGGCTCCTTCTTCCTCTACAGCAAGCTCACCGTGGA<br>CAAGAGCAGGTGGCAGCAGGGGAACGTCTTCTCATGCTCCGTGATGCATGAGGCTCTG<br>CACAACCACTACACGCAGAAGAGCCTCTCCCTGTCTCCGGGTAAATGA<br>GAGGTGCAGCTGCTGGAGTCCGGAGGAGGCCTGGTGCAGCCCGGCGGCAGCCTGAGAC<br>TGAGCTGTGCCGCCAGCGGATTCACCTTCTCCGACTACTATATGAGCTGGGTGAGGCA |

(continued)

| SEQ ID | | |
|---|---|---|
| | Antibody A2-54 | |
| | | GGCCCCAGGCAAGGGCCTGGAGTGGATTTCCTCCATCAGCAGCAATGGGATCTACATT<br>TACTACGCCGACAGCCTGAAGGGCAGGTTCACAATCAGCAGGGACAACTCTAAGAACA<br>CACTGTACCTGCAGATGAACTCCCTGCGCGCCGAGGACACCGCCGTGTATTACTGTGC<br>CAGGGCCCCCGTGGACTACTCTAATCCCAGCGGCATGGACGTGTGGGGCCAGGGCACC<br>CTGGTGACAGTGAGCTCAGGTGAGTCGTACGCTAGCAAGCTTTCTGGGGCAGGCCAGG<br>CCTGACCTTGGCTTTGGGGCAGGGAGGGGGCTAAGGTGAGGCAGGTGGCGCCAGCCAG<br>GTGCACACCCAATGCCCATGAGCCCAGACACTGGACGCTGAACCTCGCGGACAGTTAA<br>GAACCCAGGGGCCTCTGCGCCCTGGGCCCAGCTCTGTCCCACACCGCGGTCACATGGC<br>ACCACCTCTCTTGCAGCCTCCACCAAGGGCCCATCGGTCTTCCCCCTGGCACCCTCCT<br>CCAAGAGCACCTCTGGGGGCACAGCGGCCCTGGGCTGCCTGGTCAAGGACTACTTCCC<br>CGAACCGGTGACGGTGTCGTGGAACTCAGGCGCCCTGACCAGCGGCGTGCACACCTTC<br>CCGGCTGTCCTACAGTCCTCAGGACTCTACTCCCTCAGCAGCGTGGTGACCGTGCCCT<br>CCAGCAGCTTGGGCACCCAGACCTACATCTGCAACGTGAATCACAAGCCCAGCAACAC<br>CAAGGTGGACAAGAAAGTTGGTGAGAGGCCAGCACAGGGAGGGAGGGTGTCTGCTGGA<br>AGCCAGGCTCAGCGCTCCTGCCTGGACGCATCCCGGCTATGCAGCCCCAGTCCAGGGC<br>AGCAAGGCAGGCCCCGTCTGCCTCTTCACCCGGAGGCCTCTGCCCGCCCCACTCATGC<br>TCAGGGAGAGGGTCTTCTGGCTTTTTCCCCAGGCTCTGGGCAGGCACAGGCTAGGTGC<br>CCCTAACCCAGGCCCTGCACACAAAGGGGCAGGTGCTGGGCTCAGACCTGCCAAGAGC<br>CATATCCGGGAGGACCCTGCCCCTGACCTAAGCCCACCCCAAAGGCCAAACTCTCCAC<br>TCCCTCAGCTCGGACACCTTCTCTCCTCCCAGATTCCAGTAACTCCCAATCTTCTCTC<br>TGCAGAGCCCAAATCTTGTGACAAAACTCACACATGCCCACCGTGCCCAGGTAAGCCA<br>GCCCAGGCCTCGCCCTCCAGCTCAAGGCGGGACAGGTGCCCTAGAGTAGCCTGCATCC<br>AGGGACAGGCCCCAGCCGGGTGCTGACACGTCCACCTCCATCTCTTCCTCAGCACCTG<br>AACTCCTGGGGGGACCGTCAGTCTTCCTCTTCCCCCCAAAACCCAAGGACACCCTCAT<br>GATCTCCCGGACCCCTGAGGTCACATGCGTGGTGGTGGACGTGAGCCACGAAGACCCT<br>GAGGTCAAGTTCAACTGGTACGTGGACGGCGTGGAGGTGCATAATGCCAAGACAAAGC<br>CGCGGGAGGAGCAGTACAACAGCACGTACCGTGTGGTCAGCGTCCTCACCGTCCTGCA<br>CCAGGACTGGCTGAATGGCAAGGAGTACAAGTGCAAGGTCTCCAACAAAGCCCTCCCA<br>GCCCCCATCGAGAAAACCATCTCCAAAGCCAAAGGTGGGACCCGTGGGGTGCGAGGGC<br>CACATGGACAGAGGCCGGCTCGGCCCACCCTCTGCCCTGAGAGTGACCGCTGTACCAA<br>CCTCTGTCCCTACAGGGCAGCCCCGAGAACCACAGGTGTACACCCTGCCCCCATCCCG<br>GGATGAGCTGACCAAGAACCAGGTCAGCCTGACCTGCCTGGTCAAAGGCTTCTATCCC<br>AGCGACATCGCCGTGGAGTGGGAGAGCAATGGGCAGCCGGAGAACAACTACAAGACCA<br>CGCCTCCCGTGCTGGACTCCGACGGCTCCTTCTTCCTCTACAGCAAGCTCACCGTGGA<br>CAAGAGCAGGTGGCAGCAGGGGAACGTCTTCTCATGCTCCGTGATGCATGAGGCTCTG<br>CACAACCACTACACGCAGAAGAGCCTCTCCCTGTCTCCGGGTAAATGA<br>CAGTCCGTGCTGACCCAGCCACCCTCCGCCAGCGGCACCCCTGGCCAGCGGGTGACCA<br>TCTCTTGTAGCGGCAGCAGCTCCAACATCGGAAGCAATACCGTGAATTGGTACCAGCA<br>GCTGCCCGGCACCGCCCCCAAGCTGCTGATCTACCGCAATAACCAGAGGCCCTCCGGC<br>GTGCCCGACAGGTTCAGCGGCAGCAAGTCTGGCACCTCCGCCTCTCTGGCCATTAGCG<br>GACTGCGGAGCGAGGACGAGGCCGATTACTACTGCGCCGCCTGGGACGACTCCCTGTC<br>CGGGTGGCGCTTTGGAGGCGGCACAAAGCTGACCGTGCTGGGAGGTGAGTAGAACGTA<br>CGCTAGCAAGCTTGATATCGAATTCTAAACTCTGAGGGGGTCGGATGACGTGGCCATT<br>CTTTGCCTAAAGCATTGAGTTTACTGCAAGGTCAGAAAAGCATGCAAAGCCCTCAGAA<br>TGGCTGCAAAGAGCTCCAACAAAACAATTTAGAACTTTATTAAGGAATAGGGGGAAGC<br>TAGGAAGAAACTCAAAACATCAAGATTTTAAATACGCTTCTTGGTCTCCTTGCTATAA<br>TTATCTGGGATAAGCATGCTGTTTTCTGTCTGTCCCTAACATGCCCTGTGATTATCCG<br>CAAACAACACACCCAAGGGCAGAACTTTGTTACTTAAACACCATCCTGTTTGCTTCTT<br>TCCTCAGGAACTGTGGCTGCACCATCTGTCTTCATCTTCCCGCCATCTGATGAGCAGT<br>TGAAATCTGGAACTGCCTCTGTTGTGTGCCTGCTGAATAACTTCTATCCCAGAGAGGC<br>CAAAGTACAGTGGAAGGTGGATAACGCCCTCCAATCGGGTAACTCCCAGGAGAGTGTC<br>ACAGAGCAGGACAGCAAGGACAGCACCTACAGCCTCAGCAGCACCCTGACGCTGAGCA<br>AAGCAGACTACGAGAAACACAAAGTCTACGCCTGCGAAGTCACCCATCAGGGCCTGAG<br>CTCGCCCGTCACAAAGAGCTTCAACAGGGGAGAGTGT |

(continued)

| SEQ ID | | |
|---|---|---|
| | Antibody A2-54 | |
| 64 | A2-54 light chain, VL-kappa | |
| 1107/ 1145 | | |
| 90 | Antibody clone 1107, heavychain VH | GAGGTGCAGCTGTTGGAGAGCGGGGGAGGCTTGGTACAGCCTGGGGGGTCCCTGCGCC<br>TCTCCTGTGCAGCCAGCGGATTCACCTTTAGCAGCTATGCCATGAGCTGGGTCCGCCA<br>GGCTCCAGGGAAGGGGCTGGAGTGGGTCTCAGCTATTAGTGGTAGTGGTGGTAGCACA<br>TACTATGCAGACTCCGTGAAGGGCCGGTTCACCATCTCCCGTGACAATTCCAAGAACA<br>CGCTGTATCTGCAAATGAACAGCCTGCGTGCCGAGGACACGGCTGTATATTATTGTGC<br>GCGCCGTGTTTGGGGTTTTGACTATTGGGGCCAGGGAACCCTGGTCACCGTCTCCTCA<br>GACATCCAGATGACCCAGTCTCCATCCTCCCTGAGCGCATCTGTAGGAGACCGCGTCA |
| 91 | Antibody | |
| | clone 1145, variable light, VL-kappa | CCATCACTTGCCGGGCAAGTCAGAGCATTAGCAGCTATTTAAATTGGTATCAGCAGAA<br>ACCAGGGAAAGCCCCTAAGCTCCTGATCTATGCTGCATCCAGTTTGCAAAGTGGGGTC<br>CCATCACGTTTCAGTGGCAGTGGAAGCGGGACAGATTTCACTCTCACCATCAGCAGTC<br>TGCAACCTGAAGATTTTGCAACTTATTACTGTCAACAGTACGGTGTTTACCCGTTCAC<br>TTTTGGCCAGGGGACCAAGCTGGAGATCAAACgtgagtcgtacgctagcaagcttgat<br>atcgaattctaaactctgagggggtcggatgacgtggccattctttgcctaaagcatt<br>gagtttactgcaaggtcagaaaagcatgcaaagccctcagaatggctgcaaagagctc<br>caacaaaacaatttagaactttattaaggaatagggggaagctaggaagaaactcaaa<br>acatcaagattttaaatacgcttcttggtctccttgctataattatctgggataagca<br>tgctgttttctgtctgtccctaacatgccctgtgattatccgcaaacaacacacccaa<br>gggcagaactttgttacttaaacaccatcctgtttgcttctttcctcagGAACTGTGG<br>CTGCACCATCTGTCTTCATCTTCCCGCCATCTGATGAGCAGTTGAAATCTGGAACTGC<br>CTCTGTTGTGTGCCTGCTGAATAACTTCTATCCCAGAGAGGCCAAAGTACAGTGGAAG<br>GTGGATAACGCCCTCCAATCGGGTAACTCCCAGGAGAGTGTCACAGAGCAGGACAGCA<br>AGGACAGCACCTACAGCCTCAGCAGCACCCTGACGCTGAGCAAAGCAGACTACGAGAA<br>ACACAAAGTCTACGCCTGCGAAGTCACCCATCAGGGCCTGAGCTCGCCCGTCACAAAG<br>AGCTTCAACAGGGGAGAGTGT |
| 1136/1143 | | |
| 92 | Antibody clone 1136, heavychain VH | |

(continued)

| SEQ ID | | |
|---|---|---|
| | Antibody A2-54 | |
| 93 | Antibody clone 1143, variable light, VL-kappa | GAGGTGCAGCTGTTGGAGAGCGGGGGAGGCTTGGTACAGCCTGGGGGGTCCCTGCGCC<br>TCTCCTGTGCAGCCAGCGGATTCACCTTTAGCAGCTATGCCATGAGCTGGGTCCGCCA<br>GGCTCCAGGGAAGGGGCTGGAGTGGGTCTCAGCTATTAGTGGTAGTGGTGGTAGCACA<br>TACTATGCAGACTCCGTGAAGGGCCGGTTCACCATCTCCCGTGACAATTCCAAGAACA<br>CGCTGTATCTGCAAATGAACAGCCTGCGTGCCGAGGACACGGCTGTATATTATTGTGC<br>GCGCTACGTTTTCGGTATTGACTATTGGGGCCAGGGAACCCTGGTCACCGTCTCCTCA<br>GACATCCAGATGACCCAGTCTCCATCCTCCCTGAGCGCATCTGTAGGAGACCGCGTCA<br>CCATCACTTGCCGGGCAAGTCAGAGCATTAGCAGCTATTTAAATTGGTATCAGCAGAA<br>ACCAGGGAAAGCCCCTAAGCTCCTGATCTATGCTGCATCCAGTTTGCAAAGTGGGGTC<br>CCATCACGTTTCAGTGGCAGTGGAAGCGGGACAGATTTCACTCTCACCATCAGCAGTC<br>TGCAACCTGAAGATTTTGCAACTTATTACTGTCAACAGGCTTACTACGCTGGTCTGTT<br>CACTTTTGGCCAGGGGACCAAGCTGGAGATCAAACgtgagtcgtacgctagcaagctt<br>gatatcgaattctaaactctgaggggtcggatgacgtggccattctttgcctaaagc<br>attgagtttactgcaaggtcagaaaagcatgcaaagccctcagaatggctgcaaagag<br>ctccaacaaaacaatttagaactttattaaggaataggggaagctaggaagaaactc<br>aaaacatcaagattttaaatacgcttcttggtctccttgctataattatctgggataa<br>gcatgctgttttctgtctgtccctaacatgccctgtgattatccgcaaacaacacacc<br>caagggcagaactttgttacttaaacaccatcctgtttgcttctttcctcagGAACTG<br>TGGCTGCACCATCTGTCTTCATCTTCCCGCCATCTGATGAGCAGTTGAAATCTGGAAC<br>TGCCTCTGTTGTGTGCCTGCTGAATAACTTCTATCCCAGAGAGGCCAAAGTACAGTGG<br>AAGGTGGATAACGCCCTCCAATCGGGTAACTCCCAGGAGAGTGTCACAGAGCAGGACA<br>GCAAGGACAGCACCTACAGCCTCAGCAGCACCCTGACGCTGAGCAAAGCAGACTACGA<br>GAAACACAAAGTCTACGCCTGCGAAGTCACCCATCAGGGCCTGAGCTCGCCCGTCACA<br>AAGAGCTTCAACAGGGGAGAGTGT |
| 1132/1139 | | |
| 94 | Antibody clone 1132, heavychain VH | |
| 95 | Antibody clone 1139, variable light, VL-kappa | GAGGTGCAGCTGTTGGAGAGCGGGGGAGGCTTGGTACAGCCTGGGGGGTCCCTGCGCC<br>TCTCCTGTGCAGCCAGCGGATTCACCTTTAGCAGCTATGCCATGAGCTGGGTCCGCCA<br>GGCTCCAGGGAAGGGGCTGGAGTGGGTCTCAGGTATTGGTTCTTACGGTGGTGGTACA<br>TACTATGCAGACTCCGTGAAGGGCCGGTTCACCATCTCCCGTGACAATTCCAAGAACA<br>CGCTGTATCTGCAAATGAACAGCCTGCGTGCCGAGGACACGGCTGTATATTATTGTGC<br>GCGCTACGTTAACTTCGGTATGGACTATTGGGGCCAGGGAACCCTGGTCACCGTCTCC<br>TCA<br>GACATCCAGATGACCCAGTCTCCATCCTCCCTGAGCGCATCTGTAGGAGACCGCGTCA<br>CCATCACTTGCCGGGCAAGTCAGAGCATTAGCAGCTATTTAAATTGGTATCAGCAGAA<br>ACCAGGGAAAGCCCCTAAGCTCCTGATCTATGCTGCATCCAGTTTGCAAAGTGGGGTC<br>CCATCACGTTTCAGTGGCAGTGGAAGCGGGACAGATTTCACTCTCACCATCAGCAGTC<br>TGCAACCTGAAGATTTTGCAACTTATTACTGTCAACAGTACGGTCGTAACCCGCCCAC<br>TTTTGGCCAGGGGACCAAGCTGGAGATCAAACgtgagtcgtacgctagcaagcttgat<br>atcgaattctaaactctgaggggtcggatgacgtggccattctttgcctaaagcatt<br>gagtttactgcaaggtcagaaaagcatgcaaagccctcagaatggctgcaaagagctc<br>caacaaaacaatttagaactttattaaggaataggggaagctaggaagaaactcaaa<br>acatcaagattttaaatacgcttcttggtctccttgctataattatctgggataagca<br>tgctgttttctgtctgtccctaacatgccctgtgattatccgcaaacaacacacccaa |

(continued)

| SEQ ID | | |
|---|---|---|
| | Antibody A2-54 | |
| | | gggcagaactttgttacttaaacaccatcctgtttgcttctttcctcagGAACTGTGG<br>CTGCACCATCTGTCTTCATCTTCCCGCCATCTGATGAGCAGTTGAAATCTGGAACTGC<br>CTCTGTTGTGTGCCTGCTGAATAACTTCTATCCCAGAGAGGCCAAAGTACAGTGGAAG<br>GTGGATAACGCCCTCCAATCGGGTAACTCCCAGGAGAGTGTCACAGAGCAGGACAGCA<br>AGGACAGCACCTACAGCCTCAGCAGCACCCTGACGCTGAGCAAAGCAGACTACGAGAA<br>ACACAAAGTCTACGCCTGCGAAGTCACCCATCAGGGCCTGAGCTCGCCCGTCACAAAG<br>AGCTTCAACAGGGGAGAGTGT |
| | 1140/1141 | |
| 96 | Antibody clone 1140, heavychain VH | |
| 97 | Antibody clone 1141, variable light, VL-kappa | GAGGTGCAGCTGTTGGAGAGCGGGGGAGGCTTGGTACAGCCTGGGGGGTCCCTGCGCC<br>TCTCCTGTGCAGCCAGCGGATTCACCTTTAGCAGCTATGCCATGAGCTGGGTCCGCCA<br>GGCTCCAGGGAAGGGGCTGGAGTGGGTCTCAGCTATTAGTGGTAGTGGTGGTAGCACA<br>TACTATGCAGACTCCGTGAAGGGCCGGTTCACCATCTCCCGTGACAATTCCAAGAACA<br>CGCTGTATCTGCAAATGAACAGCCTGCGTGCCGAGGACACGGCTGTATATTATTGTGC<br>GCGCGGTCCGGTTTACTCTTCTGTTTTTGACTATTGGGGCCAGGGAACCCTGGTCACC<br>GTCTCCTCA<br>GACATCCAGATGACCCAGTCTCCATCCTCCCTGAGCGCATCTGTAGGAGACCGCGTCA<br>CCATCACTTGCCGGGCAAGTCAGAGCATTAGCAGCTATTTAAATTGGTATCAGCAGAA<br>ACCAGGGAAAGCCCCTAAGCTCCTGATCTATGCTGCATCCAGTTTGCAAAGTGGGGTC<br>CCATCACGTTTCAGTGGCAGTGGAAGCGGGACAGATTTCACTCTCACCATCAGCAGTC<br>TGCAACCTGAAGATTTTGCAACTTATTACTGTCAACAGAGTTACAGTACCCCTTATAC<br>TTTTGGCCAGGGGACCAAGCTGGAGATCAAACgtgagtcgtacgctagcaagcttgat<br>atcgaattctaaactctgagggggtcggatgacgtggccattctttgcctaaagcatt<br>gagtttactgcaaggtcagaaaagcatgcaaagccctcagaatggctgcaaagagctc<br>caacaaaacaatttagaactttattaaggaataggggaagctaggaagaaactcaaa<br>acatcaagattttaaatacgcttcttggtctccttgctataattatctgggataagca<br>tgctgttttctgtctgtccctaacatgccctgtgattatccgcaaacaacacacccaa<br>gggcagaactttgttacttaaacaccatcctgtttgcttctttcctcagGAACTGTGG<br>CTGCACCATCTGTCTTCATCTTCCCGCCATCTGATGAGCAGTTGAAATCTGGAACTGC<br>CTCTGTTGTGTGCCTGCTGAATAACTTCTATCCCAGAGAGGCCAAAGTACAGTGGAAG<br>GTGGATAACGCCCTCCAATCGGGTAACTCCCAGGAGAGTGTCACAGAGCAGGACAGCA<br>AGGACAGCACCTACAGCCTCAGCAGCACCCTGACGCTGAGCAAAGCAGACTACGAGAA<br>ACACAAAGTCTACGCCTGCGAAGTCACCCATCAGGGCCTGAGCTCGCCCGTCACAAAG<br>AGCTTCAACAGGGGAGAGTGT |
| | | 1150/1152 |
| 98 | Antibody clone 1150, heavychain VH | |

(continued)

| SEQ ID | | |
|---|---|---|
| | Antibody A2-54 | |
| 99 | Antibody clone 1152, variable light, VL-kappa | GAGGTGCAGCTGTTGGAGAGCGGGGGAGGCTTGGTACAGCCTGGGGGGTCCCTGCGCCTCTCCTGTGCAGCCAGCGGATTCACCTTTAGCAGCTATGCCATGAGCTGGGTCCGCCAGGCTCCAGGGAAGGGGCTGGAGTGGGTCTCAGGTATTGGTGGTTCTTCTTCTTACACATCTTATGCAGACTCCGTGAAGGGCCGGTTCACCATCTCCCGTGACAATTCCAAGAACACGCTGTATCTGCAAATGAACAGCCTGCGTGCCGAGGACACGGCTGTATATTATTGTGCGCGCTACTACTCTTACCATATGGACTATTGGGGCCAGGGAACCCTGGTCACCGTCTCCTCA<br>GACATCCAGATGACCCAGTCTCCATCCTCCCTGAGCGCATCTGTAGGAGACCaCGTCACCATCACTTGCCGGGCAAGTCAGAGCATTAGCAGCTATTTAAATTGGTATCAGCAGAAACCAGGGAAAGCCCCTAAGCTCCTGATCTATGCTGCATCCAGTTTGCAAAGTGGGGTCCCATCACGTTTCAGTGGCAGTGGAAGCGGGACAGATTTCACTCTCACCATCAGCAGTCTGCAACCTGAAGATTTTGCAACTTATTACTGTCAACAGTACGGTTCTGCTCCGCCCACTTTTGGCCAGGGGACCAAGCTGGAGATCAAACgtgagtcgtacgctagcaagcttgatatcgaattctaaactctgaggggggtcggatgacgtggccattctttgcctaaagcattgagtttactgcaaggtcagaaaagcatgcaaagccctcagaatggctgcaaagagctccaacaaaacaatttagaactttattaaggaataggggggaagctaggaagaaactcaaaacatcaagattttaaatacgcttcttggtctccttgctataattatctgggataagcatgctgttttctgtctgtccctaacatgccctgtgattatccgcaaacaacacacccaagggcagaactttgttacttaaacaccatcctgtttgcttctttcctcagGAACTGTGGCTGCACCATCTGTCTTCATCTTCCCGCCATCTGATGAGCAGTTGAAATCTGGAACTGCCTCTGTTGTGTGCCTGCTGAATAACTTCTATCCCAGAGAGGCCAAAGTACAGTGGAAGGTGGATAACGCCCTCCAATCGGGTAACTCCCAGGAGAGTGTCACAGAGCAGGACAGCAAGGACAGCACCTACAGCCTCAGCAGCACCCTGACGCTGAGCAAAGCAGACTACGAGAAACACAAAGTCTACGCCTGCGAAGTCACCCATCAGGGCCTGAGCTCGCCCGTCACAAAGAGCTTCAACAGGGGAGAGTGT |
| 1134/1141 | | |
| 100 | Antibody | GAGGTGCAGCTGTTGGAGAGCGGGGGAGGCTTGGTACAGCCTGGGGGGTCCCTGCGCC |
| | clone 1134, heavychain VH | |

(continued)

| SEQ ID | | |
|---|---|---|
| | Antibody A2-54 | |
| 101 | Antibody clone 1141, variable light, VL-kappa | TCTCCTGTGCAGCCAGCGGATTCACCTTTAGCAGCTATGCCATGAGCTGGGTCCGCCA<br>GGCTCCAGGGAAGGGGCTGGAGTGGGTCTCATCTATTTACTCTGGTGGTGGTGGTACA<br>TCTTATGCAGACTCCGTGAAGGGCCGGTTCACCATCTCCCGTGACAATTCCAAGAACA<br>CGCTGTATCTGCAAATGAACAGCCTGCGTGCCGAGGACACGGCTGTATATTATTGTGC<br>GCGCGGTCCGGCTTACTCTTCTTTCTTTGACTATTGGGGCCAGGGAACCCTGGTCACC<br>GTCTCCTCA<br>GACATCCAGATGACCCAGTCTCCATCCTCCCTGAGCGCATCTGTAGGAGACCGCGTCA<br>CCATCACTTGCCGGGCAAGTCAGAGCATTAGCAGCTATTTAAATTGGTATCAGCAGAA<br>ACCAGGGAAAGCCCCTAAGCTCCTGATCTATGCTGCATCCAGTTTGCAAAGTGGGGTC<br>CCATCACGTTTCAGTGGCAGTGGAAGCGGGACAGATTTCACTCTCACCATCAGCAGTC<br>TGCAACCTGAAGATTTTGCAACTTATTACTGTCAACAGAGTTACAGTACCCCTTATAC<br>TTTTGGCCAGGGGACCAAGCTGGAGATCAAACgtgagtcgtacgctagcaagcttgat<br>atcgaattctaaactctgagggggtcggatgacgtggccattctttgcctaaagcatt<br>gagtttactgcaaggtcagaaaagcatgcaaagccctcagaatggctgcaaagagctc<br>caacaaaacaatttagaactttattaaggaatagggggaagctaggaagaaactcaaa<br>acatcaagattttaaatacgcttcttggtctccttgctataattatctgggataagca<br>tgctgttttctgtctgtccctaacatgccctgtgattatccgcaaacaacacacccaa<br>gggcagaactttgttacttaaacaccatcctgtttgcttctttcctcagGAACTGTGG<br>CTGCACCATCTGTCTTCATCTTCCCGCCATCTGATGAGCAGTTGAAATCTGGAACTGC<br>CTCTGTTGTGTGCCTGCTGAATAACTTCTATCCCAGAGAGGCCAAAGTACAGTGGAAG<br>GTGGATAACGCCCTCCAATCGGGTAACTCCCAGGAGAGTGTCACAGAGCAGGACAGCA<br>AGGACAGCACCTACAGCCTCAGCAGCACCCTGACGCTGAGCAAAGCAGACTACGAGAA<br>ACACAAAGTCTACGCCTGCGAAGTCACCCATCAGGGCCTGAGCTCGCCCGTCACAAAG<br>AGCTTCAACAGGGGAGAGTGT |
| | | 1146/1147 |
| 102 | Antibody clone 1146, heavychain VH | GAGGTGCAGCTGTTGGAGAGCGGGGGAGGCTTGGTACAGCCTGGGGGGTCCCTGCGCC<br>TCTCCTGTGCAGCCAGCGGATTCACCTTTAGCAGCTATGCCATGAGCTGGGTCCGCCA<br>GGCTCCAGGGAAGGGGCTGGAGTGGGTCTCAGCTATTAGTGGTAGTGGTGGTAGCACA<br>TACTATGCAGACTCCGTGAAGGGCCGGTTCACCATCTCCCGTGACAATTCCAAGAACA<br>CGCTGTATCTGCAAATGAACAGCCTGCGTGCCGAGGACACGGCTGTATATTATTGTGC<br>GCGCCGTGTTTTCGGTTTTGACTATTGGGGCCAGGGAACCCTGGTCACCGTCTCCTCA<br><br>GACATCCAGATGACCCAGTCTCCATCCTCCCTGAGCGCATCTGTAGGAGACCGCGTCA<br>CCATCACTTGCCGGGCAAGTCAGAGCATTAGCAGCTATTTAAATTGGTATCAGCAGAA<br>ACCAGGGAAAGCCCCTAAGCTCCTGATCTATGCTGCATCCAGTTTGCAAAGTGGGGTC<br>CCATCACGTTTCAGTGGCAGTGGAAGCGGGACAGATTTCACTCTCACCATCAGCAGTC<br>TGCAACCTGAAGATTTTGCAACTTATTACTGTCAACAGTACTACTACTACCCGTTCAC<br>TTTTGGCCAGGGGACCAAGCTGGAGATCAAA |
| 103 | Antibody clone 1147, variable light VL | |
| | | 1142/1135 |

(continued)

| SEQ ID | | |
|---|---|---|
| | Antibody A2-54 | |
| 104 | Antibody clone 1142, heavychain VH | GAGGTGCAGCTGTTGGAGAGCGGGGGAGGCTTGGTACAGCCTGGGGGGTCCCTGCGCC TCTCCTGTGCAGCCAGCGGATTCACCTTTAGCAGCTATGCCATGAGCTGGGTCCGCCA GGCTCCAGGGAAGGGGCTGGAGTGGGTCTCAGCTATTAGTGGTAGTGGTGGTAGCACA TACTATGCAGACTCCGTGAAGGGCCGGTTCACCATCTCCCGTGACAATTCCAAGAACA CGCTGTATCTGCAAATGAACAGCCTGCGTGCCGAGGACACGGCTGTATATTATTGTGC GCGCGGTCCGGCTTACTCTACTGTTTTGGACTATTGGGGCCAGGGAACCCTGGTCACC GTCTCCTCA<br><br>GACATCCAGATGACCCAGTCTCCATCCTCCCTGAGCGCATCTGTAGGAGACCGCGTCA CCATCACTTGCCGGGCAAGTCAGAGCATTAGCAGCTATTTAAATTGGTATCAGCAGAA ACCAGGGAAAGCCCCTAAGCTCCTGATCTATGCTGCATCCAGTTTGCAAAGTGGGGTC CCATCACGTTTCAGTGGCAGTGGAAGCGGGACAGATTTCACTCTCACCATCAGCAGTC TGCAACCTGAAGATTTTGCAACTTATTACTGTCAACAGAGTTACAGTACCCCTTATAC TTTTGGCCAGGGGACCAAGCTGGAGATCAAA |
| 105 | Antibody clone 1135, variable light VL | |
| | | 1148/1149 |
| 106 | Antibody clone 1148, heavychain VH | GAGGTGCAGCTGTTGGAGAGCGGGGGAGGCTTGGTACAGCCTGGGGGGTCCCTGCGCC TCTCCTGTGCAGCCAGCGGATTCACCTTTAGCAGCTATGCCATGAGCTGGGTCCGCCA GGCTCCAGGGAAGGGGCTGGAGTGGGTCTCAGCTATTAGTGGTAGTGGTGGTAGCACA TACTATGCAGACTCCGTGAAGGGCCGGTTCACCATCTCCCGTGACAATTCCAAGAACA CGCTGTATCTGCAAATGAACAGCCTGCGTGCCGAGGACACGGCTGTATATTATTGTGC |
| | | GCGCGCTGTTTTCGGTTTTGACTATTGGGGCCAGGGAACCCTGGTCACCGTCTCCTCA |
| 107 | Antibody clone 1149, variable light VL | GACATCCAGATGACCCAGTCTCCATCCTCCCTGAGCGCATCTGTAGGAGACCGCGTCA CCATCACTTGCCGGGCAAGTCAGAGCATTAGCAGCTATTTAAATTGGTATCAGCAGAA ACCAGGGAAAGCCCCTAAGCTCCTGATCTATGCTGCATCCAGTTTGCAAAGTGGGGTC CCATCACGTTTCAGTGGCAGTGGAAGCGGGACAGATTTCACTCTCACCATCAGCAGTC TGCAACCTGAAGATTTTGCAACTTATTACTGTCAACAGGCTTACTACTTCCCGCACAC TTTTGGCCAGGGGACCAAGCTGGAGATCAAA |
| | | 1138/1135 |
| 108 | Antibody clone 1138, heavychain VH | GAGGTGCAGCTGTTGGAGAGCGGGGGAGGCTTGGTACAGCCTGGGGGGTCCCTGCGCC TCTCCTGTGCAGCCAGCGGATTCACCTTTAGCAGCTATGCCATGAGCTGGGTCCGCCA GGCTCCAGGGAAGGGGCTGGAGTGGGTCTCAGCTATTAGTGGTAGTGGTGGTAGCACA TACTATGCAGACTCCGTGAAGGGCCGGTTCACCATCTCCCGTGACAATTCCAAGAACA CGCTGTATCTGCAAATGAACAGCCTGCGTGCCGAGGACACGGCTGTATATTATTGTGC GCGCGGTTTCGTTTACTCTTCTTACATTGACTATTGGGGCCAGGGAACCCTGGTCACC GTCTCCTCA |
| 109 | Antibody clone 1135, variable light VL | GACATCCAGATGACCCAGTCTCCATCCTCCCTGAGCGCATCTGTAGGAGACCGCGTCA CCATCACTTGCCGGGCAAGTCAGAGCATTAGCAGCTATTTAAATTGGTATCAGCAGAA ACCAGGGAAAGCCCCTAAGCTCCTGATCTATGCTGCATCCAGTTTGCAAAGTGGGGTC CCATCACGTTTCAGTGGCAGTGGAAGCGGGACAGATTTCACTCTCACCATCAGCAGTC TGCAACCTGAAGATTTTGCAACTTATTACTGTCAACAGAGTTACAGTACCCCTTATAC TTTTGGCCAGGGGACCAAGCTGGAGATCAAA |
| | | |

Representative polynucleotides which encode examples of part B2 may comprise or consist of any one of SEQ ID NOS: 25 to 43 as set out below.

| SEQ ID | | |
|---|---|---|
| 25 | 900 | CTCAAAATCCAAGCGTACTTCAACGAAACTGCAGACTTACCGTGTCAGTTTGCCAA TTCGCAGAATCAAAGCCTGAGCGAACTGGTGGTTTTCTGGCAGGATCAGGAGAACC TGGTTCTGAACGAAGTCTATCTGGGCAAAGAGAAATTCGACAGCGTGGACAGCAAG TATATGGGCCGCACCAGCTTTGATAGCGACAGCTGGACCCTGCGTCTGCACAATCT GCAAATCAAAGATAAGGGTATCTACCAGTGCGTGATCCACCATAAGAAGCCGAGCG GTCTGGTGAAGATTCACGAGATGAACTCCGAGTTGTCTGTCCTGGCG |
| 26 | 901 | CTCAAAATCCAAGCGTACTTCAACGAAACTGCAGACTTACCGTGTCAGTTTGCCAA TTCGCAGAATCTGACCCTGAGCGAACTGGTGGTTTTCTGGCAGGATCAGGAGAACC TGGTTCTGAACGAAGTCTATCTGGGCAAAGAGAAATTCGACAGCGTGCATAGCAAG TATATGGGCCGCACCAGCTTTGATAGCGACAGCTGGACCCTGCGTCTGCACAATCT GCAAATCAAAGATAAGGGTATCTACCAGTGCGTGATCCACCATAAGAAGCCGACGG GTATGATTAAGATTCACGAGATGAACTCCGAGTTGTCTGTCCTGACC |
| 27 | 904 | CTCAAAATCCAAGCGTACTTCAACGAAACTGCAGACTTACCGTGTCAGTTTGCCAA TTCGCAGAATCAAAGCCTGAGCGAACTGATCGTTTTCTGGCAGGATCAGGAGAACC TGGTTCTGAACGAAGTCTATCTGGGCAAAGAGCGGTTCGACGCCGTGGACAGCAAG TATATGGGCCGCACCAGCTTTGATAGCGACAGCTGGACCCTGCGTCTGCACAATCT GCAAATCAAAGATAAGGGTATCTACCAGTGCATTATCCACCATAAGAAGCCGAGCG GTATGGTGAAGATTCACCAAATGGACTCCGAGTTGTCTGTCCTGGCG |
| 28 | 906 | CTCAAAATCCAAGCGTACATCAACGAAACTGCAGACTTACCGTGTCAGTTTGCCAA TTCGCAGAATCTGAGCCTGAGCGAACTGGTGGTTTTCTGGCAGGATCAGGAGAACC TGGTTCTGAACGAAGTCTATCTGGGCAAAGAGCGGTTCGACAGCGTGGACAGCAAG TATATGGGCCGCACCAGCTTTGATAGCGACAGCTGGACCCTGCGTCTGCACAATCT GCAAATCAAAGATAAGGGTTTCTACCAGTGCATTATCCACCATAAGAAGCCGACGG GTCTGGTGAAGATTCACGAGATGAACTCCGAGTTGTCTGTCCTGGCG |
| 29 | 907 | CTCAAAATCCAAGCGTACTTCAACGAAACTGCAGACTTACCGTGTCAGTTTGCCAA TTCGCAGAATCAAAGCCTGAGCGAACTGGTGGTTTTCTGGCAGGATCAGGAGAACC TGGTTCTGAACGAAGTCTATCTGGGCAAAGAGAAATTCGACAGCGTGCATAGCAAG TATATGGGCCGCACCAGCTTTGATAGCGACAGCTGGACCCTGCGTCTGCACAATCT |
| | | GCAAATCAAAGATAAGGGTCTGTACCAGTGCATTATCCACCATAAGAAGCCGACGG GTATGATTAAGATTCACGAGATGAACTCCGAGTTGTCTGTCCTGGCG |
| 30 | 908 | CTCAAAATCCAAGCGTACTTCAACGAAACTGCAGACTTACCGTGTCAGTTTGCCAA TTCGCAGAATCAAAGCCTGAGCGAACTGGTGGTTTTCTGGCAGGATCAGGAGAACC TGGTTCTGAACGAAGTCTATCTGGGCAAAGAGAAATTCGACAGCGTGCATAGCAAG TATATGGGCCGCACCAGCTTTGATAGCGACAGCTGGACCCTGCGTCTGCACAATCT GCAAATCAAAGATAAGGGTATCTACCAGTGCATTATCCACCATAAGAAGCCGACGG GTATGGTGAAGATTCACGAGATGAACTCCGAGTTGTCTGTCCTGGCG |
| 31 | 910 | CTCAAAATCCAAGCGTACTTCAACGAAACTGCAGACTTACCGTGTCAGTTTGCCAA TTCGCAGAATCAAAGCCTGAGCGAACTGGTGGTTTTCTGGCAGGATCAGGAGAACC TGGTTCTGAACGAAGTCTATCTGGGCAAAGAGAAATTCGACAGCGTGGACAGCAAG TATATGGGCCGCACCAGCTTTGATAGCGACAGCTGGACCCTGCGTCTGCACAATCT GCAAATCAAAGATAAGGGTATCTACCAGTGCATTATCCACCATAAGAAGCCGACGG GTATGGTGAAGATTCACGAGATGAACTCCGAGTTGTCTGTCCTGGCG |
| 32 | 915 | CTCAAAATCCAAGCGTACTTCAACGAAACTGCAGACTTACCGTGTCAGTTTGCCAA TTCGCAGAATCAAAGCCTGAGCGAACTGGTGGTTTTCTGGCAGGATCAGGAGAACC TGATCCTGAACGAAGTCTATCTGGGCAAAGAGAAATTCGACAGCGTGGACAGCAAG TATATGGGCCGCACCAGCTTTGATAGCGACAGCTGGACCCTGCGTCTGCACAATCT GCAAATCAAAGATAAGGGTTTCTACCAGTGCATTATCCACCATAAGAAGCCGAGCG GTCTGATTAAGATTCACCAAATGGACTCCGAGTTGTCTGTCCTGGCG |

(continued)

| SEQ ID | | |
|---|---|---|
| 33 | 938 | CTCAAAATCCAAGCGTACTTCAACGAAACTGCAGACTTACCGTGTCAGTTTGCCAA<br>TTCGCAGAATCTGAGCCTGAGCGAACTGGTGGTTTTCTGGCAGGATCAGGAGAACC<br>TGATCCTGAACGAAGTCTATCTGGGCAAAGAGCGGTTCGACAGCGTGCATAGCAAG<br>TATATGGGCCGCACCAGCTTTGATAGCGACAGCTGGACCCTGCGTCTGCACAATCT<br>GCAAATCAAAGATAAGGGTCTGTACCAGTGCATTATCCACCATAAGAAGCCGAGCG<br>GTATGGTGAAGATTCACGAGATGAACTCCGAGTTGTCTGTCCTGGCG |
| 34 | 1038 | GCCCCCCTCAAAATCCAAGCGTACTTCAACGAAACTGCAGACTTACCGTGTCAGTT<br>TGCCAATTCGCAGAATCTGAGCCTGAGCGAACTGGTGGTTTTCTGGCAGGATCAGG<br>AGAACCTGGTTCTGAACGAAGTCTATCTGGGCAAAGAGAAATTCGACAGCGTGGAC<br>AGCAAGTATATGGGCCGCACCAGCTTTGATAGCGACAGCTGGACCCTGCGTCTGCA<br>CAATCTGCAAATCAAAGATAAGGGTATCTACCAGTGCATTATCCACCATAAGAAGC<br>CGACGGGTATGGTGAAGATTCACGAGATGAACTCCGAGTTGTCTGTCCTGGCG |
| 35 | 1039 | GCCCCCCTCAAAATCCAAGCGTACTTCAACGAAACTGCAGACTTACCGTGTCAGTT<br>TGCCAATTCGCAGAATCTGAGCCTGAGCGAACTGGTGGTTTTCTGGCAGGATCAGG<br>AGAACCTGGTTCTGAACGAAGTCTATCTGGGCAAAGAGAAATTCGACAGCGTGAGT<br>AGCAAGTATATGGGCCGCACCAGCTTTGATAGCGACAGCTGGACCCTGCGTCTGCA<br>CAATCTGCAAATCAAAGATAAGGGTATCTACCAGTGCATTATCCACCATAAGAAGC<br>CGAGCGGTATGGTGAAGATTCACCAAATGGACTCCGAGTTGTCTGTCCTGGCG |
| 36 | 1040 | GCCCCCCTCAAAATCCAAGCGTACTTCAACGAAACTGCAGACTTACCGTGTCAGTT<br>TGCCAATTCGCAGAATCTGAGCCTGAGCGAACTGGTGGTTTTCTGGCAGGATCAGG<br>AGAACCTGGTTCTGAACGAAGTCTATCTGGGCAAAGAGCGGTTCGACAGCGTGGAC<br>AGCAAGTATATGGGCCGCACCAGCTTTGATAGCGACAGCTGGACCCTGCGTCTGCA<br>CAATCTGCAAATCAAAGATAAGGGTAGGTACCAGTGCATTATCCACCATAAGAAGC<br>CGACGGGTATGATTAATATTCACCAAATGAACTCCGAGTTGTCTGTCCTGGCG |
| 37 | 1041 | GCCCCCCTCAAAATCCAAGCGTACCTCAACGAAACTGCAGACTTACCGTGTCAGTT<br>TGCCAATTCGCAGAATCTGAGCCTGAGCGAACTGGTGGTTTTCTGGCAGGATCAGG<br>AGAACCTGGTTCTGAACGAAGTCTATCTGGGCAAAGAGAAATTCGACAGCGTGGAC<br>AGCAAGTATATGGGCCGCACCAGCTTTGATAGCGACAGCTGGACCCTGCGTCTGCA<br>CAATCTGCAAATCAAAGATAAGGGTATCTACCAGTGCATTATCCACCATAAGAAGC<br>CGACGGGTCTGGTGAAGATTCACGAGATGAACTCCGAGTTGTCTGTCCTGGCG |
| 38 | 1042 | GCCCCCCTCAAAATCCAAGCGTACTTCAACGAAACTGCAGACTTACCGTGTCAGTT<br>TGCCAATTCGCAGAATCTGAGCCTGAGCGAACTGGTGGTTTTCTGGCAGGATCAGG<br>AGAACCTGGTTCTGAACGAAGTCTATCTGGGCAAAGAGATTTTCGACAGCGTGAGT<br>AGCAAGTATATGGGCCGCACCAGCTTTGATAGTGACAGCTGGACCCTGCGTCTGCA<br>CAATCTGCAAATCAAAGATAAGGGTATCTACCAGTGCATTATCCACCATAAGAAGC<br>CGAGCGGTATGGTGAAGATTCACCAAATGGACTCCGAGTTGTCTGTCCTGGCG |
| 39 | 1043 | GCCCCCCTCAAAATCCAAGCGTACTTCAACGAAACTGCAGACTTACCGTGTCAGTT<br>TGCCAATTCGCAGAATCTGAGCCTGAGCGAACTGGTGGTTTTCTGGCAGGATCAGG<br>AGAACCTGGTTCTGAACGAAGTCTATCTGGGCAAAGAGAAATTCGACAGCGTGGAT<br>AGCAAGTATATGGGCCGCACCAGCTTTGATAGCGACAGCTGGACCCTGCGTCTGCA<br>CAATCTGCAAATCAAAGATAAGGGTATCTACCAGTGCATTATCCACCATAAGAAGC |
| | | CGACGGGTATGATTAAGATTCACGAGATGAACTCCGAGTTGTCTGTCCTGGCG |
| 40 | 1044 | GCCCCCCTCAAAATCCAAGCGTACTTCAACGAAACTGCAGACTTACCGTGTCAGTT<br>TGCCAATTCGCAGAATCTGACCCTGAGCGAACTGGTGGTTTTCTGGCAGGATCAGG<br>AGAACCTGGTTCTGAACGAAGTCTATCTGGGCAAAGAGAAATTCGACAGCGTGTCT<br>AGCAAGTATATGGGCCGCACCAGCTTTGATAGCGACAGCTGGACCCTGCGTCTGCA<br>CAATCTGCAAATCAAAGATAAGGGTATCTACCAGTGCATTATCCACCATAAGAAGC<br>CGACGGGTATGATTAAGATTCACGAGATGAGCTCCGAGTTGTCTGTCCTGGCG |

(continued)

| SEQ ID | | |
|---|---|---|
| 41 | 1045 | GCCCCCCTCAAAATCCAAGCGTACTTCAACGAAACTGCAGACTTACCGTGTCAGTT<br>TGCCAATTCGCAGAATCTGACCCTGAGCGAACTGGTGGTTTTCTGGCAGGATCAGG<br>AGAACCTGGTTCTGAACGAAGTCTATCTGGGCAAAGAGAAATTCGACAGCGTGGAC<br>AGCAAGTATATGGGCCGCACCAGCTTTGATAGCGACAGCTGGACCCTGCGTCTGCA<br>CAATCTGCAAATCAAAGATAAGGGTCTGTACCAGTGCATTATCCACCATAAGAAGC<br>CGACGGGTCTGGTGAAGATTCACGAGATGAACTCCGAGTTGTCTGTCCTGGCG |
| 42 | 1046 | GCCCCCCTCAAAATCCAAGCGTACTTCAACGAAACTGCAGACTTACCGTGTCAGTT<br>TGCCAATTCGCAGAATCAAAGCCTGAGCGAACTGGTGGTTTTCTGGCAGGATCAGG<br>AGAACCTGGTTCTGAACGAAGTCTATCTGGGCAAAGAGAAATTCGACAGCGTGGAC<br>AGCAAGTATATGGGCCGCACCAGCTTTGATAGCGACAGCTGGACCCTGCGTCTGCA<br>CAATCTGCAAATCGAAGATAAGGGTATCTACCAGTGCATTATCCACCATAAGAAGC<br>CGAGCGGTATGGTGAAGATTCACCAAATGGACTCCGAGTTGTCTGTCCTGGCG |
| 43 | 1047 | GCCCCCCTCAAAATCCAAGCGTACTTCAACGAAACTGCAGACTTACCGTGTCAGTT<br>TGCCAATTCGCAGAATCTGAGCCTGAGCGAACTGGTGGTTTTCTGGCAGGATCAGG<br>AGAACCTGGTTCTGAACGAAGTCTATCTGGGCAAAGAGAAATTCGACAGCGTGGAC<br>AGCAAGTATATGGGCCGCACCAGCTTTGATAGCGACAGCTGGACCCTGCGTCTGCA<br>CAATCTGCAAATCAAAGATAAGGGTATCTACCAGTGCATTATCCACCATAAGAAGC<br>CGACGGGTCTGGTGAAGATTCACGAGATGAACTCCGAGTTGTCTGTCCTGGCG |

Representative polynucleotides which encode the polypeptides of SEQ ID NOs: 56 to 50 and 110 to 114 may comprise or consist of any one of SEQ ID NOS: 65 to 69 and 115 to 119 as set out below.

| | | |
|---|---|---|
| 65 | Bispecific molecule 956/530: A2-54 light chain, VL-kappa linker: SGGGGSGGGGS 904, CD86 mutant molecule | CAGTCCGTGCTGACCCAGCCACCCTCCGCCAGCGGCACCCCTGGCCAGCGGGT<br>GACCATCTCTTGTAGCGGCAGCAGCTCCAACATCGGAAGCAATACCGTGAATT<br>GGTACCAGCAGCTGCCCGGCACCGCCCCCAAGCTGCTGATCTACCGCAATAAC<br>CAGAGGCCCTCCGGCGTGCCCGACAGGTTCAGCGGCAGCAAGTCTGGCACCTC<br>CGCCTCTCTGGCCATTAGCGGACTGCGGAGCGAGGACGAGGCCGATTACTACT<br>GCGCCGCCTGGGACGACTCCCTGTCCGGGTGGCGCTTTGGAGGCGGCACAAAG<br>CTGACCGTGCTGGGAGGTGAGTAGAACGTACGCTAGCAAGCTTGATATCGAAT<br>TCTAAACTCTGAGGGGGTCGGATGACGTGGCCATTCTTTGCCTAAAGCATTGA<br>GTTTACTGCAAGGTCAGAAAAGCATGCAAAGCCCTCAGAATGGCTGCAAAGAG<br>CTCCAACAAAACAATTTAGAACTTTATTAAGGAATAGGGGGAAGCTAGGAAGA<br>AACTCAAAACATCAAGATTTTAAATACGCTTCTTGGTCTCCTTGCTATAATTA<br>TCTGGGATAAGCATGCTGTTTTCTGTCTGTCCCTAACATGCCCTGTGATTATC<br>CGCAAACAACACACCCAAGGGCAGAACTTTGTTACTTAAACACCATCCTGTTT<br>GCTTCTTTCCTCAGGAACTGTGGCTGCACCATCTGTCTTCATCTTCCCGCCAT<br>CTGATGAGCAGTTGAAATCTGGAACTGCCTCTGTTGTGTGCCTGCTGAATAAC<br>TTCTATCCCAGAGAGGCCAAAGTACAGTGGAAGGTGGATAACGCCCTCCAATC<br>GGGTAACTCCCAGGAGAGTGTCACAGAGCAGGACAGCAAGGACAGCACCTACA<br>GCCTCAGCAGCACCCTGACGCTGAGCAAAGCAGACTACGAGAAACACAAAGTC<br>TACGCCTGCGAAGTCACCCATCAGGGCCTGAGCTCGCCCGTCACAAAGAGCTT<br>CAACAGGGGAGAGTGTAGCGGAGGAGGAGGAAGCGGAGGAGGAGGAAGCCTCA<br>AGATTCAGGCGTACTTTAACGAAACAGCGGACCTCCCGTGCCAATTCGCCAAT<br>AGCCAGAATCAATCGCTCAGTGAGCTCATCGTGTTTTGGCAAGATCAGGAAAA<br>CCTTGTCTTGAATGAGGTCTATCTGGGTAAAGAGAGATTCGACGCCGTAGATT<br>CCAAGTACATGGGGAGGACTTCGTTCGATTCAGATTCGTGGACGCTGAGATTG<br>CATAATCTCCAAATCAAAGACAAAGGGATCTACCAGTGCATCATTCACCACAA<br>AAAGCCTAGCGGAATGGTGAAGATCCATCAGATGGACAGCGAATTGTCAGTTT<br>TGGCC |
| 66 | Bispecific Molecule 957/530: A2-54 | CAGTCCGTGCTGACCCAGCCACCCTCCGCCAGCGGCACCCCTGGCCAGCGGGT<br>GACCATCTCTTGTAGCGGCAGCAGCTCCAACATCGGAAGCAATACCGTGAATT<br>GGTACCAGCAGCTGCCCGGCACCGCCCCCAAGCTGCTGATCTACCGCAATAAC<br>CAGAGGCCCTCCGGCGTGCCCGACAGGTTCAGCGGCAGCAAGTCTGGCACCTC |

(continued)

| | | |
|---|---|---|
| | light chain, VL-kappa linker: SGGGGSGGGGS Amino acids inserted AP. 904, mutant CD86 molecule | CGCCTCTCTGGCCATTAGCGGACTGCGGAGCGAGGACGAGGCCGATTACTACT GCGCCGCCTGGGACGACTCCCTGTCCGGGTGGCGCTTTGGAGGCGGCACAAAG CTGACCGTGCTGGGAGGTGAGTAGAACGTACGCTAGCAAGCTTGATATCGAAT TCTAAACTCTGAGGGGGTCGGATGACGTGGCCATTCTTTGCCTAAAGCATTGA GTTTACTGCAAGGTCAGAAAAGCATGCAAAGCCCTCAGAATGGCTGCAAAGAG CTCCAACAAAACAATTTAGAACTTTATTAAGGAATAGGGGGAAGCTAGGAAGA AACTCAAAACATCAAGATTTTAAATACGCTTCTTGGTCTCCTTGCTATAATTA TCTGGGATAAGCATGCTGTTTTCTGTCTGTCCCTAACATGCCCTGTGATTATC CGCAAACAACACACCCAAGGGCAGAACTTTGTTACTTAAACACCATCCTGTTT GCTTCTTTCCTCAGGAACTGTGGCTGCACCATCTGTCTTCATCTTCCCGCCAT CTGATGAGCAGTTGAAATCTGGAACTGCCTCTGTTGTGTGCCTGCTGAATAAC TTCTATCCCAGAGAGGCCAAAGTACAGTGGAAGGTGGATAACGCCCTCCAATC GGGTAACTCCCAGGAGAGTGTCACAGAGCAGGACAGCAAGGACAGCACCTACA GCCTCAGCAGCACCCTGACGCTGAGCAAAGCAGACTACGAGAAACACAAAGTC TACGCCTGCGAAGTCACCCATCAGGGCCTGAGCTCGCCCGTCACAAAGAGCTT CAACAGGGGAGAGTGTAGCGGAGGAGGAGGAAGCGGAGGAGGAGGAAGCGCCC CCCTCAAGATTCAGGCGTACTTTAACGAAACAGCGGACCTCCCGTGCCAATTC GCCAATAGCCAGAATCAATCGCTCAGTGAGCTCATCGTGTTTTGGCAAGATCA GGAAAACCTTGTCTTGAATGAGGTCTATCTGGGTAAAGAGAGATTCGACGCCG TAGATTCCAAGTACATGGGGAGGACTTCGTTCGATTCAGATTCGTGGACGCTG AGATTGCATAATCTCCAAATCAAAGACAAAGGGATCTACCAGTGCATCATTCA CCACAAAAAGCCTAGCGGAATGGTGAAGATCCATCAGATGGACAGCGAATTGT CAGTTTTGGCC |

(continued)

| | | |
|---|---|---|
| 67 | Bispecific molecule 958/531: 904, mutant CD86 molecule linker: NFSQP A2-54 Heavy Chain, VH-gammal | CTCAAGATTCAGGCGTACTTTAACGAAACAGCGGACCTCCCGTGCCAATTCGC<br>CAATAGCCAGAATCAATCGCTCAGTGAGCTCATCGTGTTTTGGCAAGATCAGG<br>AAAACCTTGTCTTGAATGAGGTCTATCTGGGTAAAGAGAGATTCGACGCCGTA<br>GATTCCAAGTACATGGGGAGGACTTCGTTCGATTCAGATTCGTGGACGCTGAG<br>ATTGCATAATCTCCAAATCAAAGACAAAGGGATCTACCAGTGCATCATTCACC<br>ACAAAAAGCCTAGCGGAATGGTGAAGATCCATCAGATGGACAGCGAATTGTCA<br>GTTTTGGCCAACTTCAGCCAGCCCGAGGTGCAGCTGCTGGAGTCCGGAGGAGG<br>CCTGGTGCAGCCCGGCGGCAGCCTGAGACTGAGCTGTGCCGCCAGCGGATTCA<br>CCTTCTCCGACTACTATATGAGCTGGGTGAGGCAGGCCCCAGGCAAGGGCCTG<br>GAGTGGATTTCCTCCATCAGCAGCAATGGGATCTACATTTACTACGCCGACAG<br>CCTGAAGGGCAGGTTCACAATCAGCAGGGACAACTCTAAGAACACACTGTACC<br>TGCAGATGAACTCCCTGCGCGCCGAGGACACCGCCGTGTATTACTGTGCCAGG<br>GCCCCCGTGGACTACTCTAATCCCAGCGGCATGGACGTGTGGGGCCAGGGCAC<br>CCTGGTGACAGTGAGCTCAGGTGAGTCGTACGCTAGCAAGCTTTCTGGGGCAG<br>GCCAGGCCTGACCTTGGCTTTGGGGCAGGGAGGGGGCTAAGGTGAGGCAGGTG<br>GCGCCAGCCAGGTGCACACCCAATGCCCATGAGCCCAGACACTGGACGCTGAA<br>CCTCGCGGACAGTTAAGAACCCAGGGGCCTCTGCGCCCTGGGCCCAGCTCTGT<br>CCCACACCGCGGTCACATGGCACCACCTCTCTTGCAGCCTCCACCAAGGGCCC<br>ATCGGTCTTCCCCCTGGCACCCTCCTCCAAGAGCACCTCTGGGGGCACAGCGG<br>CCCTGGGCTGCCTGGTCAAGGACTACTTCCCCGAACCGGTGACGGTGTCGTGG<br>AACTCAGGCGCCCTGACCAGCGGCGTGCACACCTTCCCGGCTGTCCTACAGTC<br>CTCAGGACTCTACTCCCTCAGCAGCGTGGTGACCGTGCCCTCCAGCAGCTTGG<br>GCACCCAGACCTACATCTGCAACGTGAATCACAAGCCCAGCAACACCAAGGTG<br>GACAAGAAAGTTGGTGAGAGGCCAGCACAGGGAGGGAGGGTGTCTGCTGGAAG<br>CCAGGCTCAGCGCTCCTGCCTGGACGCATCCCGGCTATGCAGCCCCAGTCCAG<br>GGCAGCAAGGCAGGCCCCGTCTGCCTCTTCACCCGGAGGCCTCTGCCCGCCCC<br>ACTCATGCTCAGGGAGAGGGTCTTCTGGCTTTTTCCCCAGGCTCTGGGCAGGC<br>ACAGGCTAGGTGCCCCTAACCCAGGCCCTGCACACAAAGGGGCAGGTGCTGGG<br>CTCAGACCTGCCAAGAGCCATATCCGGGAGGACCCTGCCCCTGACCTAAGCCC<br>ACCCCAAAGGCCAAACTCTCCACTCCCTCAGCTCGGACACCTTCTCTCCTCCC<br>AGATTCCAGTAACTCCCAATCTTCTCTCTGCAGAGCCCAAATCTTGTGACAAA<br>ACTCACACATGCCCACCGTGCCCAGGTAAGCCAGCCCAGGCCTCGCCCTCCAG<br>CTCAAGGCGGGACAGGTGCCCTAGAGTAGCCTGCATCCAGGGACAGGCCCCAG<br>CCGGGTGCTGACACGTCCACCTCCATCTCTTCCTCAGCACCTGAACTCCTGGG<br>GGGACCGTCAGTCTTCCTCTTCCCCCCAAAACCCAAGGACACCCTCATGATCT<br>CCCGGACCCCTGAGGTCACATGCGTGGTGGTGGACGTGAGCCACGAAGACCCT<br>GAGGTCAAGTTCAACTGGTACGTGGACGGCGTGGAGGTGCATAATGCCAAGAC<br>AAAGCCGCGGGAGGAGCAGTACAACAGCACGTACCGTGTGGTCAGCGTCCTCA<br>CCGTCCTGCACCAGGACTGGCTGAATGGCAAGGAGTACAAGTGCAAGGTCTCC |
| | | AACAAAGCCCTCCCAGCCCCCATCGAGAAAACCATCTCCAAAGCCAAAGGTGG<br>GACCCGTGGGGTGCGAGGGCCACATGGACAGAGGCCGGCTCGGCCCACCCTCT<br>GCCCTGAGAGTGACCGCTGTACCAACCTCTGTCCCTACAGGGCAGCCCCGAGA<br>ACCACAGGTGTACACCCTGCCCCCATCCCGGGATGAGCTGACCAAGAACCAGG<br>TCAGCCTGACCTGCCTGGTCAAAGGCTTCTATCCCAGCGACATCGCCGTGGAG<br>TGGGAGAGCAATGGGCAGCCGGAGAACAACTACAAGACCACGCCTCCCGTGCT<br>GGACTCCGACGGCTCCTTCTTCCTCTACAGCAAGCTCACCGTGGACAAGAGCA<br>GGTGGCAGCAGGGGAACGTCTTCTCATGCTCCGTGATGCATGAGGCTCTGCAC<br>AACCACTACACGCAGAAGAGCCTCTCCCTGTCTCCGGGTAAATGAG |

(continued)

| | | |
|---|---|---|
| 68 | Bispecific molecule 959/530: 904, mutant CD86 molecule linker: KRTVA A2-54 Variable Light chain, VL-kappa | CTCAAGATTCAGGCGTACTTTAACGAAACAGCGGACCTCCCGTGCCAATTCGCCAATAGCCAGAATCAATCGCTCAGTGAGCTCATCGTGTTTTGGCAAGATCAGGAAAACCTTGTCTTGAATGAGGTCTATCTGGGTAAAGAGAGATTCGACGCCGTAGATTCCAAGTACATGGGGAGGACTTCGTTCGATTCAGATTCGTGGACGCTGAGATTGCATAATCTCCAAATCAAAGACAAAGGGATCTACCAGTGCATCATTCACCACAAAAAGCCTAGCGGAATGGTGAAGATCCATCAGATGGACAGCGAATTGTCAGTTTTGGCCAAGAGAACCGTGGCCCAGTCCGTGCTGACCCAGCCACCCTCCGCCAGCGGCACCCCTGGCCAGCGGGTGACCATCTCTTGTAGCGGCAGCAGCTCCAACATCGGAAGCAATACCGTGAATTGGTACCAGCAGCTGCCCGGCACCGCCCCCAAGCTGCTGATCTACCGCAATAACCAGAGGCCCTCCGGCGTGCCCGACAGGTTCAGCGGCAGCAAGTCTGGCACCTCCGCCTCTCTGGCCATTAGCGGACTGCGGAGCGAGGACGAGGCCGATTACTACTGCGCCGCCTGGGACGACTCCCTGTCCGGGTGGCGCTTTGGAGGCGGCACAAAGCTGACCGTGCTGGGAGGTGAGTAGAACGTACGCTAGCAAGCTTGATATCGAATTCTAAACTCTGAGGGGGTCGGATGACGTGGCCATTCTTTGCCTAAAGCATTGAGTTTACTGCAAGGTCAGAAAAGCATGCAAAGCCCTCAGAATGGCTGCAAAGAGCTCCAACAAAACAATTTAGAACTTTATTAAGGAATAGGGGGAAGCTAGGAAGAAACTCAAAACATCAAGATTTTAAATACGCTTCTTGGTCTCCTTGCTATAATTATCTGGGATAAGCATGCTGTTTTCTGTCTGTCCCTAACATGCCCTGTGATTATCCGCAAACAACACACCCAAGGGCAGAACTTTGTTACTTAAACACCATCCTGTTTGCTTCTTTCCTCAGGAACTGTGGCTGCACCATCTGTCTTCATCTTCCCGCCATCTGATGAGCAGTTGAAATCTGGAACTGCCTCTGTTGTGTGCCTGCTGAATAACTTCTATCCCAGAGAGGCCAAAGTACAGTGGAAGGTGGATAACGCCCTCCAATCGGGTAACTCCCAGGAGAGTGTCACAGAGCAGGACAGCAAGGACAGCACCTACAGCCTCAGCAGCACCCTGACGCTGAGCAAAGCAGACTACGAGAAACACAAAGTCTACGCCTGCGAAGTCACCCATCAGGGCCTGAGCTCGCCCGTCACAAAGAGCTTCAACAGGGGAGAGTGT |
| 69 | Bispecific molecule 960/530: 904, mutant CD86 molecule linker: NFSQP A2-54 Variable Light, VL-kappa | CTCAAGATTCAGGCGTACTTTAACGAAACAGCGGACCTCCCGTGCCAATTCGCCAATAGCCAGAATCAATCGCTCAGTGAGCTCATCGTGTTTTGGCAAGATCAGGAAAACCTTGTCTTGAATGAGGTCTATCTGGGTAAAGAGAGATTCGACGCCGTAGATTCCAAGTACATGGGGAGGACTTCGTTCGATTCAGATTCGTGGACGCTGAGATTGCATAATCTCCAAATCAAAGACAAAGGGATCTACCAGTGCATCATTCACCACAAAAAGCCTAGCGGAATGGTGAAGATCCATCAGATGGACAGCGAATTGTCAGTTTTGGCCAACTTCAGCCAGCCCCAGTCCGTGCTGACCCAGCCACCCTCCGCCAGCGGCACCCCTGGCCAGCGGGTGACCATCTCTTGTAGCGGCAGCAGCTCCAACATCGGAAGCAATACCGTGAATTGGTACCAGCAGCTGCCCGGCACCGCCCCCAAGCTGCTGATCTACCGCAATAACCAGAGGCCCTCCGGCGTGCCCGACAGGTTCAGCGGCAGCAAGTCTGGCACCTCCGCCTCTCTGGCCATTAGCGGACTGCGGAGCGAGGACGAGGCCGATTACTACTGCGCCGCCTGGGACGACTCCCTGTCCGGGTGGCGCTTTGGAGGCGGCACAAAGCTGACCGTGCTGGGAGGTGAGTAGAACGTACGCTAGCAAGCTTGATATCGAATTCTAAACTCTGAGGGGGTCGGATGACGTGGCCATTCTTTGCCTAAAGCATTGAGTTTACTGCAAGGTCAGAAAAGCATGCAAAGCCCTCAGAATGGCTGCAAAGAGCTCCAACAAAACAATTTAGAACTTTATTAAGGAATAGGGGGAAGCTAGGAAGAAACTCAAAACATCAAGATTTTAAATACGCTTCTTGGTCTCCTTGCTATAATTATCTGGGATAAGCATGCTGTTTTCTGTCTGTCCCTAACATGCCCTGTGATTATCCGCAAACAACACACCCAAGGGCAGAACTTTGTTACTTAAACACCATCCTGTTTGCTTCTTTCCTCAGGAACTGTGGCTGCACCATCTGTCTTCATCTTCCCGCCATCTGATGAGCAGTTGAAATCTGGAACTGCCTCTGTTGTGTGCCTGCTGAATAACTTCTATCCCAGAGAGGCCAAAGTACAGTGGAAGGTGGATAACGCCCTCCAATCGGGTAACTCCCAGGAGAGTGTCACAGAGCAGGACAGCAAGGACAGCACCTACAGCCTCAGCAGCACCCTGACGCTGAGCAAAGCAGACTACGAGAAACACAAAGTCTACGCCTGCGAAGTCACCCATCAGGGCCTGAGCTCGCCCGTCACAAAGAGCTTCAACAGGGGAGAGTGT |
| 115 | Bispecific | GACATCCAGATGACCCAGTCTCCATCCTCCCTGAGCGCATCTGTAGGAGACCG |

(continued)

| | | |
|---|---|---|
| | molecule 1107/1145 : clone 1145 light chain, VL-kappa, Linker SGGGGSGGGGS , 1040: CD86 mutant molecule | CGTCACCATCACTTGCCGGGCAAGTCAGAGCATTAGCAGCTATTTAAATTGGTATCAGCAGAAACCAGGGAAAGCCCCTAAGCTCCTGATCTATGCTGCATCCAGTTTGCAAAGTGGGGTCCCATCACGTTTCAGTGGCAGTGGAAGCGGGACAGATTTCACTCTCACCATCAGCAGTCTGCAACCTGAAGATTTTGCAACTTATTACTGTCAACAGTACGGTGTTTACCCGTTCACTTTTGGCCAGGGGACCAAGCTGGAGATCAAACgtgagtcgtacgctagcaagcttgatatcgaattctaaactctgagggggtcggatgacgtggccattctttgcctaaagcattgagtttactgcaaggtcagaaaagcatgcaaagccctcagaatggctgcaaagagctccaacaaaacaatttagaactttattaaggaatagggggaagctaggaagaaactcaaaacatcaagattttaaatacgcttcttggtctccttgctataattatctgggataagcatgctgttttctgtctgtccctaacatgccctgtgattatccgcaaacaacacacccaagggcagaactttgttacttaaacaccatcctgtttgcttctttcctcagGAACTGTGGCTGCACCATCTGTCTTCATCTTCCCGCCATCTGATGAGCAGTTGAAATCTGGAACTGCCTCTGTTGTGTGCCTGCTGAATAACTTCTATCCCAGAGAGGCCAAAGTACAGTGGAAGGTGGATAACGCCCTCCAATCGGGTAACTCCCAGGAGAGTGTCACAGAGCAGGACAGCAAGGACAGCACCTACAGCCTCAGCAGCACCCTGACGCTGAGCAAAGCAGACTACGAGAAACACAAAGTCTACGCCTGCGAAGTCACCCATCAGGGCCTGAGCTCGCCCGTCACAAAGAGCTTCAACAGGGGAGAGTGTAGCGGAGGAGGAGGAAGCGGAGGAGGAGGAAGCGCCCCCCTCAAAATCCAAGCGTACTTCAACGAAACTGCAGACTTACCGTGTCAGTTTGCCAATTCGCAGAATCTGAGCCTGAGCGAACTGGTGGTTTTCTGGCAGGATCAGGAGAACCTGGTTCTGAACGAAGTCTATCTGGGCAAAGAGCGGTTCGACAGCGTGGACAGCAAGTATATGGGCCGCACCAGCTTTGATAGCGACAGCTGGACCCTGCGTCTGCACAATCTGCAAATCAAAGATAAGGGTAGGTACCAGTGCATTATCCACCATAAGAAGCCGACGGGTATGATTAATATTCACCAAATGAACTCCGAGTTGTCTGTCCTGGCG |
| 116 | Bispecific molecule 1136/1143 : clone 1143 light chain,VL-kappa, Linker SGGGGSGGGGS , 1040: CD86 mutant molecule | GACATCCAGATGACCCAGTCTCCATCCTCCCTGAGCGCATCTGTAGGAGACCGCGTCACCATCACTTGCCGGGCAAGTCAGAGCATTAGCAGCTATTTAAATTGGTATCAGCAGAAACCAGGGAAAGCCCCTAAGCTCCTGATCTATGCTGCATCCAGTTTGCAAAGTGGGGTCCCATCACGTTTCAGTGGCAGTGGAAGCGGGACAGATTTCACTCTCACCATCAGCAGTCTGCAACCTGAAGATTTTGCAACTTATTACTGTCAACAGGCTTACTACGCTGGTCTGTTCACTTTTGGCCAGGGGACCAAGCTGGAGATCAAACgtgagtcgtacgctagcaagcttgatatcgaattctaaactctgaggggggtcggatgacgtggccattctttgcctaaagcattgagtttactgcaaggtcagaaaagcatgcaaagccctcagaatggctgcaaagagctccaacaaaacaatttagaactttattaaggaatagggggaagctaggaagaaactcaaaacatcaagattttaaatacgcttcttggtctccttgctataattatctgggataagcatgctgttttctgtctgtccctaacatgccctgtgattatccgcaaacaacacacccaagggcagaactttgttacttaaacaccatcctgtttgcttctttcctcagGAACTGTGGCTGCACCATCTGTCTTCATCTTCCCGCCATCTGATGAGCAGTTGAAATCTGGAACTGCCTCTGTTGTGTGCCTGCTGAATAACTTCTATCCCAGAGAGGCCAAAGTACAGTGGAAGGTGGATAACGCCCTCCAATCGGGTAACTCCCAGGAGAGTGTCACAGAGCAGGACAGCAAGGACAGCACCTACAGCCTCAGCAGCACCCTGACGCTGAGCAAAGCAGACTACGAGAAACACAAAGTCTACGCCTGCGAAGTCACCCATCAGGGCCTGAGCTCGCCCGTCACAAAGAGCTTCAACAGGGGAGAGTGTAGCGGAGGAGGAGGAAGCGGAGGAGGAGGAAGCGCCCCCCTCAAAATCCAAGCGTACTTCAACGAAACTGCAGACTTACCGTGTCAGTTTGCCAATTCGCAGAATCTGAGCCTGAGCGAACTGGTGGTTTTCTGGCAGGATCAGGAGAACCTGGTTCTGAACGAAGTCTATCTGGGCAAAGAGCGGTTCGACAGCGTGGACAGCAAGTATATGGGCCGCACCAGCTTTGATAGCGACAGCTGGACCCTGCGTCTGCACAATCTGCAAATCAAAGATAAGGGTAGGTACCAGTGCATTATCCACCATAAGAAGCCGACGGGTATGATTAATATTCACCAAATGAACTCCGAGTTGTCTGTCCTGGCG |

(continued)

| | | |
|---|---|---|
| 117 | Bispecific molecule 1132/1139 : clone 1139 light chain,VL-kappa, Linker SGGGGSGGGGS , 1040: CD86 mutant | GACATCCAGATGACCCAGTCTCCATCCTCCCTGAGCGCATCTGTAGGAGACCG CGTCACCATCACTTGCCGGGCAAGTCAGAGCATTAGCAGCTATTTAAATTGGT ATCAGCAGAAACCAGGGAAAGCCCCTAAGCTCCTGATCTATGCTGCATCCAGT TTGCAAAGTGGGGTCCCATCACGTTTCAGTGGCAGTGGAAGCGGGACAGATTT CACTCTCACCATCAGCAGTCTGCAACCTGAAGATTTTGCAACTTATTACTGTC AACAGTACGGTCGTAACCCGCCCACTTTTGGCCAGGGGACCAAGCTGGAGATC AAACgtgagtcgtacgctagcaagcttgatatcgaattctaaactctgagggg gtcggatgacgtggccattctttgcctaaagcattgagtttactgcaaggtca gaaaagcatgcaaagccctcagaatggctgcaaagagctccaacaaaacaatt tagaactttattaaggaataggggaagctaggaagaaactcaaaacatcaag attttaaatacgcttcttggtctccttgctataattatctgggataagcatgc |
| | molecule | tgttttctgtctgtccctaacatgccctgtgattatccgcaaacaacacaccc aagggcagaactttgttacttaaacaccatcctgtttgcttctttcctcagGA ACTGTGGCTGCACCATCTGTCTTCATCTTCCCGCCATCTGATGAGCAGTTGAA ATCTGGAACTGCCTCTGTTGTGTGCCTGCTGAATAACTTCTATCCCAGAGAGG CCAAAGTACAGTGGAAGGTGGATAACGCCCTCCAATCGGGTAACTCCCAGGAG AGTGTCACAGAGCAGGACAGCAAGGACAGCACCTACAGCCTCAGCAGCACCCT GACGCTGAGCAAAGCAGACTACGAGAAACACAAAGTCTACGCCTGCGAAGTCA CCCATCAGGGCCTGAGCTCGCCCGTCACAAAGAGCTTCAACAGGGGAGAGTGT AGCGGAGGAGGAGGAAGCGGAGGAGGAGGAAGCGCCCCCCTCAAAATCCAAGC GTACTTCAACGAAACTGCAGACTTACCGTGTCAGTTTGCCAATTCGCAGAATC TGAGCCTGAGCGAACTGGTGGTTTTCTGGCAGGATCAGGAGAACCTGGTTCTG AACGAAGTCTATCTGGGCAAAGAGCGGTTCGACAGCGTGGACAGCAAGTATAT GGGCCGCACCAGCTTTGATAGCGACAGCTGGACCCTGCGTCTGCACAATCTGC AAATCAAAGATAAGGGTAGGTACCAGTGCATTATCCACCATAAGAAGCCGACG GGTATGATTAATATTCACCAAATGAACTCCGAGTTGTCTGTCCTGGCG |
| 118 | Bispecific molecule 1140/1141: clone 1141 light chain,VL-kappa, Linker SGGGGSGGGGS , 1040: CD86 mutant molecule | GACATCCAGATGACCCAGTCTCCATCCTCCCTGAGCGCATCTGTAGGAGACCG CGTCACCATCACTTGCCGGGCAAGTCAGAGCATTAGCAGCTATTTAAATTGGT ATCAGCAGAAACCAGGGAAAGCCCCTAAGCTCCTGATCTATGCTGCATCCAGT TTGCAAAGTGGGGTCCCATCACGTTTCAGTGGCAGTGGAAGCGGGACAGATTT CACTCTCACCATCAGCAGTCTGCAACCTGAAGATTTTGCAACTTATTACTGTC AACAGAGTTACAGTACCCCTTATACTTTTGGCCAGGGGACCAAGCTGGAGATC AAACgtgagtcgtacgctagcaagcttgatatcgaattctaaactctgagggg gtcggatgacgtggccattctttgcctaaagcattgagtttactgcaaggtca gaaaagcatgcaaagccctcagaatggctgcaaagagctccaacaaaacaatt tagaactttattaaggaataggggaagctaggaagaaactcaaaacatcaag attttaaatacgcttcttggtctccttgctataattatctgggataagcatgc tgttttctgtctgtccctaacatgccctgtgattatccgcaaacaacacaccc aagggcagaactttgttacttaaacaccatcctgtttgcttctttcctcagGA ACTGTGGCTGCACCATCTGTCTTCATCTTCCCGCCATCTGATGAGCAGTTGAA ATCTGGAACTGCCTCTGTTGTGTGCCTGCTGAATAACTTCTATCCCAGAGAGG CCAAAGTACAGTGGAAGGTGGATAACGCCCTCCAATCGGGTAACTCCCAGGAG AGTGTCACAGAGCAGGACAGCAAGGACAGCACCTACAGCCTCAGCAGCACCCT GACGCTGAGCAAAGCAGACTACGAGAAACACAAAGTCTACGCCTGCGAAGTCA CCCATCAGGGCCTGAGCTCGCCCGTCACAAAGAGCTTCAACAGGGGAGAGTGT AGCGGAGGAGGAGGAAGCGGAGGAGGAGGAAGCGCCCCCCTCAAAATCCAAGC GTACTTCAACGAAACTGCAGACTTACCGTGTCAGTTTGCCAATTCGCAGAATC TGAGCCTGAGCGAACTGGTGGTTTTCTGGCAGGATCAGGAGAACCTGGTTCTG AACGAAGTCTATCTGGGCAAAGAGCGGTTCGACAGCGTGGACAGCAAGTATAT GGGCCGCACCAGCTTTGATAGCGACAGCTGGACCCTGCGTCTGCACAATCTGC AAATCAAAGATAAGGGTAGGTACCAGTGCATTATCCACCATAAGAAGCCGACG GGTATGATTAATATTCACCAAATGAACTCCGAGTTGTCTGTCCTGGCG |

(continued)

| | | |
|---|---|---|
| 119 | Bispecific molecule 1151/1150 : clone 1151 light chain,VL-kappa, Linker SGGGGSGGGGS , 1040: CD86 mutant molecule | GACATCCAGATGACCCAGTCTCCATCCTCCCTGAGCGCATCTGTAGGAGACCa CGTCACCATCACTTGCCGGGCAAGTCAGAGCATTAGCAGCTATTTAAATTGGT ATCAGCAGAAACCAGGGAAAGCCCCTAAGCTCCTGATCTATGCTGCATCCAGT TTGCAAAGTGGGGTCCCATCACGTTTCAGTGGCAGTGGAAGCGGGACAGATTT CACTCTCACCATCAGCAGTCTGCAACCTGAAGATTTTGCAACTTATTACTGTC AACAGTACGGTTCTGCTCCGCCCACTTTTGGCCAGGGGACCAAGCTGGAGATC AAACgtgagtcgtacgctagcaagcttgatatcgaattctaaactctgagggg gtcggatgacgtggccattctttgcctaaagcattgagtttactgcaaggtca gaaaagcatgcaaagccctcagaatggctgcaaagagctccaacaaaacaatt tagaactttattaaggaatagggggaagctaggaagaaactcaaaacatcaag attttaaatacgcttcttggtctccttgctataattatctgggataagcatgc tgttttctgtctgtccctaacatgccctgtgattatccgcaaacaacacaccc aagggcagaactttgttacttaaacaccatcctgtttgcttctttcctcagGA ACTGTGGCTGCACCATCTGTCTTCATCTTCCCGCCATCTGATGAGCAGTTGAA ATCTGGAACTGCCTCTGTTGTGTGCCTGCTGAATAACTTCTATCCCAGAGAGG CCAAAGTACAGTGGAAGGTGGATAACGCCCTCCAATCGGGTAACTCCCAGGAG AGTGTCACAGAGCAGGACAGCAAGGACAGCACCTACAGCCTCAGCAGCACCCT GACGCTGAGCAAAGCAGACTACGAGAAACACAAAGTCTACGCCTGCGAAGTCA CCCATCAGGGCCTGAGCTCGCCCGTCACAAAGAGCTTCAACAGGGGAGAGTGT AGCGGAGGAGGAGGAAGCGGAGGAGGAGGAAGCGCCCCCCTCAAAATCCAAGC GTACTTCAACGAAACTGCAGACTTACCGTGTCAGTTTGCCAATTCGCAGAATC |
| | | TGAGCCTGAGCGAACTGGTGGTTTTCTGGCAGGATCAGGAGAACCTGGTTCTG AACGAAGTCTATCTGGGCAAAGAGCGGTTCGACAGCGTGGACAGCAAGTATAT GGGCCGCACCAGCTTTGATAGCGACAGCTGGACCCTGCGTCTGCACAATCTGC AAATCAAAGATAAGGGTAGGTACCAGTGCATTATCCACCATAAGAAGCCGACG GGTATGATTAATATTCACCAAATGAACTCCGAGTTGTCTGTCCTGGCG |

A suitable polynucleotide sequence may alternatively be a variant of one of these specific polynucleotide sequences. For example, a variant may be a substitution, deletion or addition variant of any of the above nucleic acid sequences. A variant polynucleotide may comprise 1, 2, 3, 4, 5, up to 10, up to 20, up to 30, up to 40, up to 50, up to 75 or more nucleic acid substitutions and/or deletions from the sequences given in the sequence listing.

Suitable variants may be at least 70% homologous to a polynucleotide of any one of nucleic acid sequences disclosed herein, preferably at least 80 or 90% and more preferably at least 95%, 97% or 99% homologous thereto. Preferably homology and identity at these levels is present at least with respect to the coding regions of the polynucleotides. Methods of measuring homology are well known in the art and it will be understood by those of skill in the art that in the present context, homology is calculated on the basis of nucleic acid identity. Such homology may exist over a region of at least 15, preferably at least 30, for instance at least 40, 60, 100, 200 or more contiguous nucleotides. Such homology may exist over the entire length of the unmodified polynucleotide sequence.

Methods of measuring polynucleotide homology or identity are known in the art. For example the UWGCG Package provides the BESTFIT program which can be used to calculate homology (e.g. used on its default settings) (Devereux et al (1984) Nucleic Acids Research 12, p387-395).

The PILEUP and BLAST algorithms can also be used to calculate homology or line up sequences (typically on their default settings), for example as described in Altschul S.F. (1993) J Mol Evol 36:290-300; Altschul, S, F et al (1990) J Mol Biol 215:403-10.

Software for performing BLAST analysis is publicly available through the National Centre for Biotechnology Information (http://www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al,* supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89:10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90:5873-5787. One measure of similarity provided by the BLAST algorithm is the smallest sum probability (P(N)), which provides an indication of the probability by which a match between two nucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

The homologue may differ from a sequence in the relevant polynucleotide by less than 3, 5, 10, 15, 20 or more mutations (each of which may be a substitution, deletion or insertion). These mutations may be measured over a region of at least 30, for instance at least 40, 60 or 100 or more contiguous nucleotides of the homologue.

In one embodiment, a variant sequence may vary from the specific sequences given in the sequence listing by virtue of the redundancy in the genetic code. The DNA code has 4 primary nucleic acid residues (A, T, C and G) and uses these to "spell" three letter codons which represent the amino acids the proteins encoded in an organism's genes. The linear sequence of codons along the DNA molecule is translated into the linear sequence of amino acids in the protein(s) encoded by those genes. The code is highly degenerate, with 61 codons coding for the 20 natural amino acids and 3 codons representing "stop" signals. Thus, most amino acids are coded for by more than one codon - in fact several are coded for by four or more different codons. A variant polynucleotide of the invention may therefore encode the same polypeptide sequence as another polynucleotide of the invention, but may have a different nucleic acid sequence due to the use of different codons to encode the same amino acids.

A polypeptide of the invention may thus be produced from or delivered in the form of a polynucleotide which encodes, and is capable of expressing, it.

Polynucleotides of the invention can be synthesised according to methods well known in the art, as described by way of example in Sambrook et al (1989, Molecular Cloning - a laboratory manual; Cold Spring Harbor Press).

The nucleic acid molecules of the present invention may be provided in the form of an expression cassette which includes control sequences operably linked to the inserted sequence, thus allowing for expression of the polypeptide of the invention *in vivo.* These expression cassettes, in turn, are typically provided within vectors (e.g., plasmids or recombinant viral vectors). Such an expression cassette may be administered directly to a host subject. Alternatively, a vector comprising a polynucleotide of the invention may be administered to a host subject. Preferably the polynucleotide is prepared and/or administered using a genetic vector. A suitable vector may be any vector which is capable of carrying a sufficient amount of genetic information, and allowing expression of a polypeptide of the invention.

Also disclosed herein are expression vectors that comprise such polynucleotide sequences. Such expression vectors are routinely constructed in the art of molecular biology and may for example involve the use of plasmid DNA and appropriate initiators, promoters, enhancers and other elements, such as for example polyadenylation signals which may be necessary, and which are positioned in the correct orientation, in order to allow for expression of a peptide of the invention. Other suitable vectors would be apparent to persons skilled in the art. By way of further example in this regard we refer to Sambrook *et al.*

Also disclosed herein are cells that have been modified to express a polypeptide of the invention. Such cells include transient, or preferably stable higher eukaryotic cell lines, such as mammalian cells or insect cells, lower eukaryotic cells, such as yeast or prokaryotic cells such as bacterial cells. Particular examples of cells which may be modified by insertion of vectors or expression cassettes encoding for a polypeptide of the invention include mammalian HEK293T, CHO, HeLa, NS0 and COS cells. Preferably the cell line selected will be one which is not only stable, but also allows for mature glycosylation and cell surface expression of a polypeptide.

Such cell lines may be cultured using routine methods to produce a polypeptide of the invention, or may be used therapeutically or prophylactically to deliver antibodies of the invention to a subject. Alternatively, polynucleotides, expression cassettes or vectors of the invention may be administered to a cell from a subject *ex vivo* and the cell then returned to the body of the subject.

*Pharmaceutical Formulations, Therapeutic uses and Patient Groups*

In another aspect, the present invention provides compositions comprising molecules of the invention, such as the polypeptides described herein. For example, the invention provides a composition comprising one or more molecules of the invention, such as one or more polypeptides of the invention, and at least one pharmaceutically acceptable carrier.

As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for parenteral, e.g. intravenous, intramuscular or subcutaneous administration (*e.g.,* by injection or infusion). Depending on the route of administration, the polypeptide may be coated in a material to protect the polypeptide from the action of acids and other natural conditions that may inactivate or denature the polypeptide.

Preferred pharmaceutically acceptable carriers comprise aqueous carriers or diluents. Examples of suitable aqueous carriers that may be employed in the compositions of the invention include water, buffered water and saline. Examples of other carriers include ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants. In many cases, it will be preferable to include isotonic agents, for example, sugars, polyalcohols such as mannitol, sorbitol, or sodium chloride in the composition.

A composition of the invention also may include a pharmaceutically acceptable anti-oxidant. These compositions may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Therapeutic compositions typically must be sterile and stable under the conditions of manufacture and storage. The composition can be formulated as a solution, microemulsion, liposome, or other ordered structure suitable to high drug concentration.

Sterile injectable solutions can be prepared by incorporating the active agent (e.g. polypeptide) in the required amount in an appropriate solvent with one or a combination of ingredients enumerated above, as required, followed by sterilization microfiltration. Generally, dispersions are prepared by incorporating the active agent into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active agent plus any additional desired ingredient from a previously sterile-filtered solution thereof.

Particularly preferred compositions are formulated for systemic administration or for local administration. Local administration may be at the site of a tumour or into a tumour draining lymph node. The composition may prefereably be formulated for sustained release over a period of time. Thus the composition may be provided in or as part of a matrix facilitating sustained release. Preferred sustained release matrices may comprise a Montanide or $\gamma$-Polyglutamic acid (PGA) nanoparticles. Localised release of a polypeptide of the invention, optionally over a sustained period of time, may reduce potential autoimmune side-effects associated with administration of a CTLA-4 antagonist.

Compositions of the invention may comprise additional active ingredients as well as a polypeptide of the invention. As mentioned above, compositions of the invention may comprise one or more polypeptides of the invention. They may also comprise additional therapeutic or prophylactic agents.

Also disclosed herein are kits comprising polypeptides or other compositions of the invention and instructions for use. The kit may further contain one ore more additional reagents, such as an additional therapeutic or prophylactic agent as discussed above.

The polypeptides in accordance with the present invention maybe used in therapy or prophylaxis. In therapeutic applications, polypeptides or compositions are administered to a subject already suffering from a disorder or condition, in an amount sufficient to cure, alleviate or partially arrest the condition or one or more of its symptoms. Such therapeutic treatment may result in a decrease in severity of disease symptoms, or an increase in frequency or duration of symptom-free periods. An amount adequate to accomplish this is defined as "therapeutically effective amount". In prophylactic applications, polypeptides or compositions are administered to a subject not yet exhibiting symptoms of a disorder or condition, in an amount sufficient to prevent or delay the development of symptoms. Such an amount is defined as a "prophylactically effective amount". The subject may have been identified as being at risk of developing the disease or condition by any suitable means.

In particular, polypeptides of the invention may be useful in the treatment or prevention of cancer. Accordingly, the invention provides a polypeptide of the invention for use in the treatment or prevention of cancer. The invention also provides an polypeptide of the invention for use in the manufacture of a medicament for the treatment or prevention of cancer.

The cancer may be prostate cancer, breast cancer, colorectal cancer, pancreatic cancer, ovarian cancer, lung cancer, cervical cancel, rhabdomyosarcoma, neuroblastoma, multiple myeloma, leukemia, acute lymphoblastic leukemia, melanoma, bladder cancer, gastric cancer, head and neck cancer, liver cancer, skin cancer, lymphoma or glioblastoma.

A polypeptide of the present invention, or a composition comprising said polypeptide, may be administered via one or more routes of administration using one or more of a variety of methods known in the art. As will be appreciated by the skilled artisan, the route and/or mode of administration will vary depending upon the desired results. Systemic administration or local administration are preferred. Local administration may be at the site of a tumour or into a tumour draining lymph node. Preferred modes of administration for polypeptides or compositions of the invention include intravenous, intramuscular, intradermal, intraperitoneal, subcutaneous, spinal or other parenteral modes of administration, for example by injection or infusion. The phrase "parenteral administration" as used herein means modes of administration other than enteral and topical administration, usually by injection. Alternatively, a polypeptide or composition of the invention can be administered via a non-parenteral mode, such as a topical, epidermal or mucosal mode of administration.

A suitable dosage of a polypeptide of the invention may be determined by a skilled medical practitioner. Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular polypeptide employed, the route of administration, the time of administration, the rate of excretion of the polypeptide, the duration of the treatment, other drugs, compounds and/or materials used in combination with the particular compositions employed, the age, sex, weight, condition, general health and prior medical history of the patient being treated, and like factors well known in the medical arts.

A suitable dose of a polypeptide of the invention may be, for example, in the range of from about 0.1 μg/kg to about 100mg/kg body weight of the patient to be treated. For example, a suitable dosage may be from about 1 μg/kg to about 10mg/kg body weight per day or from about 10 g/kg to about 5 mg/kg body weight per day.

Dosage regimens may be adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Polypeptides may be administered in a single dose or in multiple doses. The multiple doses may be administered via the same or different routes and to the same or different locations. Alternatively, polypeptides can be administered as a sustained release formulation as described above, in which case less frequent administration is required. Dosage and frequency may vary depending on the half-life of the polypeptide in the patient and the duration of treatment that is desired. The dosage and frequency of administration can also vary depending on whether the treatment is prophylactic or therapeutic. In prophylactic applications, a relatively low dosage may be administered at relatively infrequent intervals over a long period of time. In therapeutic applications, a relatively high dosage may be administered, for example until the patient shows partial or complete amelioration of symptoms of disease.

Combined administration of two or more agents may be achieved in a number of different ways. In one embodiment, the polypeptide and the other agent may be administered together in a single composition. In another embodiment, the polypeptide and the other agent may be administered in separate compositions as part of a combined therapy. For example, the modulator may be administered before, after or concurrently with the other agent.

A polypeptide or composition of the invention may also be used in a method of increasing the activation of a population of cells expressing human CD40 and human CTLA-4, the method comprising administering to said population of cells a polypeptide or composition as disclosed herein under conditions suitable to permit interaction between said cell and said polypeptide. The population of cells typically comprises at least some cells which express CTLA-4, typically T cells, and at least some cells which express CD40, typically an antigen presenting cell such as a B cell. The method is typically carried out *ex vivo.*

The present invention is further illustrated by the following examples which should not be construed as further limiting.

## Examples

### Examples 1 to 4 - development of polypeptide variants of CD86 for part B2

### Example 1 - First selection

*Introduction*

The starting material for the production of the polypeptide variants was the monomeric soluble extracellular binding domain of human CD86. That is, the CTLA-4 binding domain of human CD86. The amino acid sequence of this domain and a structural model of CD86 in complex with CTLA-4 (Schwartz et al; Nature 2001; 410(6828) p604-608) was used to design 4 different starting phage display libraries of candidate polypeptides: AL-1014-01, AL-1014-02, AL-1014-03 and AL-1014-04. The phage display libraries were produced using standard protocols using nucleotide sequences encoding the candidate polypeptides. The amino acid sequences of the candidate polypeptides were designed as set out below.

*Library design*

The primary purpose for the design of library AL-1014-01 was to provide an increased binding surface of the binding domain of CD86 for the interaction with CTLA-4. To this end, various residues in the FG loop of CD86 (positions 114 to 121, numbering as in Figure 4) were selected for mutation. Two insertions were also introduced to allow for a elongation of the FG-loop. The positions and introduced mutations relative to the wild-type sequence of CD86 are summarised in Table 1 below. The variability that was allowed in each position is displayed. Nucleotides encoding all of the possible polypeptides which result from all of the possible combinations of the mutations shown in Table 1 were designed and used to produce the AL-1014-01 phage display library, in accordance with standard protocols.

TABLE1

| Amino acid sequence number according to SEQ ID NO: 1 | | | | | | | | | | | | |
|---|---|---|---|---|---|---|---|---|---|---|---|---|
| 113 | 114 | 115 | 116 | insert | 117 | 118 | insert | 119 | 120 | 121 | 122 | |
| ***H*** | ***H*** | ***K*** | ***K*** | | ***P*** | ***T*** | | ***G*** | ***M*** | ***I*** | ***R*** | **Wt-sequence** |
| Y | Y | Y | Y | Y | Y | Y | Y | Y | Y | Y | Y | **Amino acids allowed in each position (restricted variability)** |
| S | S | S | S | S | S | S | S | S | S | S | S | |
| G | G | G | G | G | G | G | G | | G | G | G | |
| L | L | L | L | L | L | L | L | L | L | L | L | |
| D | D | D | D | D | D | D | D | D | D | D | D | |
| | K | | | | R | K | | | | K | | |

In library AL-1014-02 amino acid positions in the binding surfaces of CD86 facing CTLA-4 were allowed to vary between the wild type amino acid and a very limited number of homologous residues to introduce minimal structural perturbations in the beta-sheet surface of CD86. The positions and introduced mutations of the library AL-1014-02 are shown in Table 2. A mutation in position 79 has been reported to contribute favorably to the binding of CD86 to CTLA-4 (Peach et al; Journal of Biological Chemistry 1995; 270 p21181-21187), and this position was also allowed to vary in the library. The variability that was allowed in each position is displayed. Nucleotides encoding all of the possible polypeptides which result from all of the possible combinations of the mutations shown in Table 2 were designed and used to produce the AL-1014-02 phage display library, in accordance with standard protocols. Table 2 also shows the IUPAC-IUB code that was used to represent the indicated mutations in the corresponding nucleotide sequences. IUPAC-IUB codes are typically used to define multiple nucleotide possibilities in a given position and are based on a recognised set of nomeclature rules for incompletely specified bases in nucleotide sequences (see Biochem. J., 1985, 229, 281-286; Eur. J. Biochem., 1985, 150, 1-5; J. Biol. Chem., 1986, 261, 13-17; Mol. Biol. Evol., 1986, 3, 99-108; Nucl. Acids Res., 1985, 13, 3021-3030; Proc. Nat. Acad. Sci. (U. S.), 1986, 83, 4-8; and Biochemical Nomenclature and Related Documents 2nd edition, Portland Press, 1992, pp 122-126). The IUPAC-IUB codes used in these experiments are summarised below Table 2.

TABLE 2

| Amino acid sequence position in human CD86 - numbering as in Figure 4 | | | | | | | | | | | | | | | | | | | | | |
|---|---|---|---|---|---|---|---|---|---|---|---|---|---|---|---|---|---|---|---|---|---|
| saa | rtt | Twc | Saa | rtt | gam | Twc | arg | kct | san | htc | Saa | Vtc | san | asc | gst | mtg | rtt | arg | saa | rac | IUB code |
| 48 | 54 | 56 | 58 | 64 | 67 | 69 | 74 | 77 | 79 | 107 | 109 | 111 | 113 | 118 | 119 | 120 | 121 | 122 | 125 | 127 | a.a. position |
| **Q** | **V** | **F** | **Q** | **V** | **E** | **Y** | **K** | **S** | **H** | **L** | **Q** | **I** | **H** | **T** | **G** | **M** | **I** | **R** | **Q** | **N** | **Wt-sequ** |
| E | I | Y | E | I | D | F | R | A | D | I | E | L | D | S | A | L | V | K | E | D | **Amino acids allowed in each position (restricted variability)** |
| | | | | | | | | | E | F | | V | E | | | | | | | | |
| | | | | | | | | | Q | | | | Q | | | | | | | | |
| | | | | | | | | | A | | | | A | | | | | | | | |

IUPAC-IUB codes

| IUB Code | N | V | B | H | D | K | S | W | M | Y | R |
|---|---|---|---|---|---|---|---|---|---|---|---|
| Nucleotide bases | A,C,G,T | G,A,C | G,T,C | A,T,C | G,A,T | G,T | G,C | A,T | A,C | C,T | A,G |

Two more libraries were designed. In these two libraries, the entire IgGV domain of CD86 was randomized by taking the nucleotide sequence encoding wild type CD86 and using an error prone PCR method set-up to minimize mutational bias. The resulting mutant nucleotide sequences were used to produce phage display libraries in accordance with standard protocols.

Three different random mutagenized libraries were generated of which two were selected. AL-1014-03 was taken from randomization round 2 and randomization round 3 provided library AL-1014-04.

AL-1014-1, AL-1014-2, AL-1014-3 and AL-1014-04 were produced by cloning into a phagemid vector and phage stocks were generated according to standard protocols.

*Selection strategy*

The selection strategy applied in this Example consisted of 6 rounds (R1 to R6) which are summarised in Table 3.

TABLE 3

| Selection round | Ag conc | Incubation (min) | Wash (min) | Non bio-Ag | Temp | pH | CD28 |
|---|---|---|---|---|---|---|---|
| **R1** | 50 nM | 60 (with hCTLA4-bio) + 30 (with beads) | Standard | | | | |

(continued)

| Selection round | Ag conc | Incubation (min) | Wash (min) | Non bio-Ag | Temp | pH | CD28 |
|---|---|---|---|---|---|---|---|
| **R2** | 5 nM | 20 (with hCTLA4-bio) + 30 (with beads) | Standard | | | | 300 nM * |
| **R3** | 20 nM mCTLA4 | 60 (with mCTLA4-bio) + 30 (with beads) | Standard | | | | |
| **R4** | 5 nM | 10 (with hCTLA4-bio) + 20 (with beads) | Standard +10 | | 60 °C, 3 hours | | 200 nM ** |
| **R5** | 5 nM | 5 (with hCTLA4-bio) + 10 (non-bio CTLA4) + 20 (with beads) | Standard +30 | | | | 200 nM** |
| **R6** | 2 nM | 5 (with hCTLA4-bio) + 10 (non-bio CTLA4)(*1000) + 15 (with beads) | Standard +120 | X | | pH 6.3<br>pH 5.5 | 200 nM** |
| * Pre-selection with CD28-Fc<br>** CD28-Fc added at the same time as bio-CTLA4 | | | | | | | |

The first round of selection (R1) enriched the members of starting libraries AL-1014-01, AL-1014-02, AL-104-03 and AL-1014-04 for binders to biotinylated CTLA4-Fcγ (50nM). The enrichment for functional binders was confirmed by sequencing.

The output from R1 was a pool of phage clones from each starting library, enriched for functional binders. The output from libraries AL-104-03 and AL-1014-04 was subsequently combined into a new library AL-1014-05. The output from libraries AL-1014-01, AL-1014-02 and AL-1014-04 was combined into a further new library, AL-1014-06. Members of libraries AL-1014-05 and AL-1014-06 were then subjected to 5 additional rounds of screening (R2 to R6), to select for increased binding to CTLA-4 and decreased binding to CD28.

The strategy for the additional 5 rounds of selection was to apply selection pressure specificially aimed at improving the properties of affinity, on-rate, off-rate, selectivity, multimerization and epitope maintenance.

Selection for increased affinity was achieved by lowering the antigen concentration in each round. The selections started at 50 nM CTLA-4 lowering to 5 nM which was maintained in rounds R2, R4 and R5, followed by a final round (R6) in which the CTLA-4 concentration was 2 nM. Selection for increased on-rate was achieved by shortening of incubation time with biotinylated CTLA-4. Selection for decreased off-rate was achieved by increasing the stringency during the wash step. Selection for increased selectivity for CTLA-4 (and reduced binding affinity to CD28) was achieved by incubating in excess of unbiotinylated CD28-Fc (in R2, R4, R5 and R6). Retained binding affinity to mouse CTLA-4 was ensured by including a round (R3) in which biotinylated murine CTLA-4 was used in place of human CTLA-4 as the selection antigen. This step was included to make sure that the affinity to mouse CTLA4 was kept to enable proof-of-concept experiments in mice models. Selecting for avidity effects was avoided by introducing unbiotinylated CTLA-4 five minutes after the start of incubation with biotinylated CTLA-4 (in R5 and R6). Addition of unbiotinylated Fcγ (IgG) to the selection buffer was performed to avoid selecting for binders to the Fc-fusion part of the CTLA-4 antigen.

Decreased temperature sensitivity and potentially increased melting point was obtained by introducing a selection step at increased temperature (60°C) (R4). Increased affinity at low pH was addressed by introducing a selection round at a lower pH (R6).

After the selection rounds individual phage clones from R5 and R6 were analysed by high throughput screening in a conventional affinity ELISA assay. The assay was a sandwich ELISA which measured binding of phages to either CTLA-4 or CD28. In short, 96-well flat bottom high binding plates (Greiner #655074) were coated with either CTLA4-Fcγ (Orencia, Apoteket) or CD28- Fcγ (R&D systems 342-CD) by incubating overnight at 4°C. The plates were washed (Wash buffer: PBS+0,05% Tween 20 (PBST) Medicago #09-9410-100) and then blocked in PBST+3% Milk powder (Semper). The plates were washed again and sample or controls were added to the wells. The samples were incubated for 1h at room temperature and then washed. Detection antibody, mouse-anti-M13-HRP (GE Healthcare, #27-9421-01) was added and the plates were subsequently developed using SuperSignal Pico Chemiluminescent substrate (Thermo #37069) and detected with an Envision reader (Perkin Elmer).

Example 2 - Expression and recloning of selected polypeptides

The phage clones selected from Example 1 were re-cloned according to standard protocols into a fusion protein-format, with each clone fused to y2/y4 Fc. The fusion proteins were expressed in HEK293 cells. Supernatants culure of the cells were collected and the expressed fusion proteins were assayed using both ELISA and Biacore™ according to the methods set out below. The results are shown in Table 4, which also shows the mutations present in each clone. The best performing clone in the ELISA assay (907) had an EC50 binding ratio almost 10 times higher than wild-type protein CD86. The best performing clone in the Biacore™ assay (904) had a Kd binding ratio more than 4 times higher than wild type protein CD86.

*Binding ELISA*

96-well flat bottom high binding plates (Greiner #655074) were coated with either CTLA4-Fc (Fitzgerald #30R-CD152) or CD28-Fc (R&D systems 342-CD) by incubating overnight at 4 °C. The plates were washed (Wash buffer: PBS+0,05% Tween 20 (PBST) Medicago #09-9410-100) and then blocked in PBST+3% BSA (Merck, #1.12018.0100). The plates were washed again and sample or controls (serially diluted 1/5 from 200 -0,001 μg/ml) were added to the wells. The samples were incubated for 1h at room temperature and then washed. Detection antibody, goat-anti-human IgG Fcy-HRP (Jackson, #109-035-098) was added and the plates were subsequently developed using SuperSignal Pico Chemiluminescent substrate (Thermo #37069) and detected with an Envision reader (Perkin Elmer). EC50 values were calculated for both CTLA4 and CD28. The binding ratio (EC50 binding ratio = [EC50 for CD28] ÷ [EC50 for CTLA-4]) was calculated for each polypeptide and is shown in Table 4.

*Biacore™*

Either CTLA4-Fc (Fitzgerald #30R-CD152) or CD28-Fc (R&D systems 342-CD) was immobilized to the Biacore™ senshorship, CM5, using conventional amine coupling. The CD86 mutant molecules and controls (serially diluted 1/2 100-1,5 nM) were analyzed for binding in HBs-P (GE, BR-1003-69) at a flow rate 30 μl/ml. The association was followed for 3 minutes and the dissociation for 10 minutes. Regeneration was performed twice using 5 mM NaOH for 30 seconds. The kinetic parameters and the affinity constants were calculated using BIAevaluation 4.1 software. The binding ratio (Kd binding ratio = [Kd for CD28] ÷ [Kd for CTLA-4]) was calculated for each polypeptide and is shown in Table 4.

TABLE 4

| Clone | Mutated positions and amino acid change (positions numbered as in Figure 4) | EC50 binding ratio | Kd binding ratio |
|---|---|---|---|
| 900 | H79D,L107I,I111V,T118S, M120L, I121V, R122K, Q125E | 3.5 | ND* |
| 901 | Q48L,S49T,L107I,I111V,R122K,Q125E,A134T | 17.2 | 2.7 |
| 904 | V54I,K74R,S77A,H79D,L107I,T118S,I121V,R122K,N127D | 12.2 | 6.8 |
| 906 | F32I,Q48L,K74R, H79D,L107F, M120L,I121V,R122K,Q125E | 16.2 | 0.8 |
| 907 | R122K,Q125E | 30.5 | 5.6 |
| 908 | L107I,I121V,R122K,Q125E | 6.2 | 4.7 |
| 910 | H79D,L107I,I121V,R122K,Q125E | 7.7 | 5.1 |

(continued)

| Clone | Mutated positions and amino acid change (positions numbered as in Figure 4) | EC50 binding ratio | Kd binding ratio |
|---|---|---|---|
| 915 | V64I,H79D,L107F,T118S,M120L,R122K,N127D | 9.9 | 1.9 |
| 938 | V64I,L107I,I121V,R122K | 2.0 | 5.5 |
| | Wild type | 3.4 | 1.6 |
| * No detectable binding was seen in the BIAcore analysis | | | |

Table 5 summarises the mutations and positions in each of the selected clones. The full amino acid sequences for clones 900, 901, 904, 906, 907, 908, 910, 915 and 938 are provided as SEQ ID NOs: 6 to 14, respectively.

TABLE 5

| | F32 I | Q48L | S49 T | V54 I | V64I | K74R | S77A | H79D | L107 I/F |
|---|---|---|---|---|---|---|---|---|---|
| 900 | | | | | | | | D | I |
| 901 | | L | T | | | | | | I |
| 904 | | | | I | | R | A | D | I |
| 906 | I | L | | | | R | | D | F |
| 907 | | | | | | | | | |
| 908 | | | | | | | | | I |
| 910 | | | | | | | | D | I |
| 915 | | | | | I | | | D | F |
| 938 | | | | | I | | | | I |

| | I111 V | T118S | M120L | I121 V | R122K | Q125E | N127D | A134T |
|---|---|---|---|---|---|---|---|---|
| 900 | V | x | L | v | K | E | | |
| 901 | V | | | | K | E | | T |
| 904 | | S | | v | K | | D | |
| 906 | | | L | v | K | E | | |
| 907 | | | | | K | E | | |
| 908 | | | | v | K | E | | |
| 910 | | | | v | K | E | | |
| 915 | | S | L | | K | | D | |
| 938 | | | | v | K | | | |

Example 3 - Expanded library diversity and repeated selection and screening.

*Library production*

The starting material for two further libraries, AL-1014-11 and AL-1014-12, were six clones identified in Examples 1 and 2, namely 901, 904, 906,907, 908, 915. Additional diversity was generated by error-prone PCR with mutated oligos included in the reassembly PCR step, in accordance with protocols described in WO2002048351, WO200309734, and WO2007057682. The applied oligos comprised mutations in hotspot regions of the CD86 molecules. Approximately 20 clones from each library were sequenced. The two libraries contained recombined clones, error prone PCR generated clones, and clones produced by the introduction of mutated oligos. Each clone contained on average 3 - 11 mutations compared to the wild-type sequence of CD86.

*Selection strategy*

Selection rounds R2 to R6 as described in Example 1 were applied to both libraries AL-1014-11 and AL-1014-12. Clones selected in the last two rounds were sequenced to confirm that recombination and novel mutations were achieved.

*Assesment of selected clones*

Approximately 1250 clones from the last two selection rounds were expressed as phage stocks and analyzed for binding to CTLA-4 and CD28 by the same high throughput screening ELISA as described in Example 1 (data not shown). Clones were ranked based on their binding to CTLA-4 and CD28. The top ten clones were selected based on the criteria of high binding to CTLA-4 and low binding to CD28. The sequence of these clones was determined and each was expressed from HEK293 cells as a gamma2/gamma4 fusion, as described in Example 2.

Table 6 summarises the mutations and positions in each of the top ten clones. The full amino acid sequences for clones 1038, 1039, 1040, 1041, 1042, 1043, 1044, 1045, 1046 and 1047 are provided as SEQ ID NOs: 15 to 24, respectively.

TABLE 6

| | F32L | Q48L | S49T | K74I/R | H79D/S/A | K103E | L107I/R |
|---|---|---|---|---|---|---|---|
| 1038 | | L | | | D | | I |
| 1039 | | L | | | S | | I |
| 1040 | | L | | R | D | | R |
| 1041 | L | L | | | D | | I |
| 1042 | | L | | I | S | | I |
| 1043 | | L | | | D | | I |
| 1044 | | L | T | | S | | I |
| 1045 | | L | T | | D | | |
| 1046 | | | | | D | E | I |
| 1047 | | L | | | D | | I |

| | T118S | M120L | I121V | R122K/N | Q125E | N127S/D |
|---|---|---|---|---|---|---|
| 1038 | | | V | K | E | |
| 1039 | S | | V | K | | D |
| 1040 | | | | N | | |
| 1041 | | L | V | K | E | |
| 1042 | S | | V | K | | D |
| 1043 | | | | K | E | |
| 1044 | | | | K | E | S |
| 1045 | | L | V | K | E | |
| 1046 | S | | V | K | | D |
| 1047 | | L | V | K | E | |

## Example 4 - Further assessment of top ten polypeptides

The polypeptides from each of the ten clones selected in Example 3 were further characterised as follows.

*Expression and purification*

Plasmids encoding each clone were transfected into Freestyle 293-T cells (Invitrogen) according to standard protocols and supernatant were harvested on day 6. Polypeptides and controls were affinity purified using protein A columns (GE Healthcare #17-0402-01).

*Binding ELISA*

Similar to the protocol described in Example 2, 96-well flat bottom high binding plates (Greiner #655074) were coated with either CTLA4-Fc (Fitzgerald #30R-CD152) or CD28-Fc (kindly provided by Simon Davis, Oxford University) by incubating overnight at 4 °C. The plates were washed (Wash buffer: PBS+0,05% Tween 20 (PBST) Medicago #09-9410-100) and then blocked in PBST+3% BSA (Merck, #1.12018.0100). The plates were washed again and sample or controls (serially diluted 1/3 from 50 000-0001 nM) were added to the wells. The samples were incubated for 1h at room temperature and then washed. Detection antibody, goat-anti-human IgG Fcγ-HRP (Jackson, #109-035-098) was added and the plates were subsequently developed using SuperSignal Pico Chemiluminescent substrate (Thermo #37069) and detected with an Envision reader (Perkin Elmer).

The results of the CTLA-4 binding ELISA are shown in Figure 1. EC50 values were calculated and are shown in Table 7. All molecules showed better EC50 values than both wild type and H79A, with an improvement of between 2 and 67-fold. Binding to CD28 was too low for detection (data not shown).

TABLE 7

| Sample | EC50 |
|---|---|
| 1038 | 0.14 |
| 1039 | 0.039 |
| 1040 | 0.0076 |
| 1041 | 0.087 |
| 1042 | 0.29 |
| 1043 | 0.035 |
| 1044 | 0.029 |
| 1045 | 0.047 |
| 1046 | 0.019 |
| 1047 | 0.037 |
| Wild type | 0.51 |
| Prior Art | 0.81 |
| Negative control | No activity |

*Inhibition ELISA*

96-well flat bottom plate high binding plates (Greiner #655074) were coated with wildtype CD86-Fc (R&D Systems #7625-B2) by incubating overnight at 4 °C. The plates were washed (Wash buffer: PBS+0,05% Tween 20 (PBST) Medicago #09-9410-100) and then blocked in PBST+3% BSA (Merck, #1.12018.0100). The sample (CD86 mutant or wild type protein; serially diluted 1/4 from 30000 to 0.3ng/ml) was incubated with biotinylated-CTLA4 (Fitzgerald #30R-CD152) in room temperature 1 h, the mixture was then added to the blocked wells in the ELISA plate. Detection was performed with Streptavidin-HRP (Pierce #21126) and the plates were subsequently developed using SuperSignal Pico Chemiluminescent substrate (Thermo #37069) and detected with Envision reader (Perkin Elmer). The results are shown in Figure 2. IC50 values were calculated and are shown in Table 8. All molecules showed better IC50 value than both wild type and H79A, the IC of the best mutant CD86 molecule was improved over 100 fold compared to wild type.

TABLE 8

| Sample | IC50 |
|---|---|
| 1040 | 0.049 |
| 1041 | 3.1 |
| 1042 | 4.3 |
| 1043 | 4.0 |
| 1044 | 1.4 |
| 1045 | 2.6 |
| 1046 | 1.1 |
| 1047 | 0.98 |
| Wild type | 15 |
| H79A | 25 |
| Negative control | No activity |

*Biacore™*

As in Example 2, either CTLA4-Fc (Fitzgerald #30R-CD152) or CD28-Fc (kindly provided by Simon Davis, Oxford University)was immobilized to the Biacore™ senshorship, CM5, using conventional amine coupling. The CD86 mutant molecules and controls (serially diluted 1/3 1000-4 nM) were analyzed for binding in HBs-P (GE, BR-1003-69) at a flow rate 30 μl/ml. The association was followed for 3 minutes and the dissociation for 10 minutes. Regeneration was performed twice using 5 mM NaOH for 30 seconds. The kinetic parameters and the affinity constants were calculated using BIAevaluation 4.1 software.

The CTLA-4 results for each selected clone and wild type CD86 to CTLA-4 are summarised in Table 9. The mutations present in each clone are also shown. A clone including only the H79A mutation from Peach et al (Journal of Biological Chemistry 1995, vol 270(36), 21181-21187) is also included for comparison. The CD28 results are not shown, since in most cases affinity to CD28 was too weak to be determined by this protocol. However, where it was determined, the affinity of the selected CD86 clones for CD28 was at least 100 times lower than that for CTLA-4.

It was also determined that the selected clones also bind to murine CTLA-4 (data not shown).

TABLE 9

| Clone | Mutated positions and amino acid change (positions numbered as in Figure 4) | ka (1/Ms) | kd (1/s) | Kd (nM) |
|---|---|---|---|---|
| 1038 | Q48L, H79D, L107I, I121V, R122K, Q125E | 1.0e6 | 0.012 | 12 |
| 1039 | Q48L, H79S, L107I, T118S, I121V, R122K, N127D | 1.0e6 | 8.5e-3 | 8 |
| 1040 | Q48L, K74R, H79D, L107R, R122N | 1.0e6 | 3.2e-3 | 3 |
| 1041 | F32L, Q48L, H79D, L107I, M120L, I121V, R122K,125E | 7.0e5 | 8.4e-3 | 12 |
| 1042 | Q48L, K74I, H79S, L107I, T118S, 1121V, R122K, N127D | 4.4e5 | 0.011 | 25 |
| 1043 | Q48L, H79D, L107I, R122K, Q125E | 1.1e6 | 0.011 | 10 |
| 1044 | Q48L, S49T, H79S, L107I, R122K, Q125E, N127S | 1.1e6 | 9.4e-3 | 8 |
| 1045 | Q48L, S49T, H79D, M120L, I121V, R122K, Q125E | 9.4e5 | 8.3e-3 | 9 |
| 1046 | H79D, K103E, L107I, T118S, I121V, R122K, N127D | 1.4e6 | 8.0e-3 | 6 |
| 1047 | Q48L, H79D, L107I, M120L, I121V, R122K, Q125E | 8.5e5 | 8.4e-3 | 10 |
| Wild type | | 4.6e5 | 0.023 | 50 |
| H79A | | 3,4e5 | 0,022 | 63 |

Example 5 - cloning and construction of exemplary bispecific molecules

Bispecific polypeptides in accordance with the invention were produced according to the schematic diagram shown in Figure 3 and as described Table 10.

TABLE 10

| Reference no. | Description of molecule |
|---|---|
| 956 / 530 | Amino acid sequence of 904 (B2; SEQ ID NO: 8) joined by linker SGGGGSGGGGS (X; SEQ ID NO: 6) to the C terminal of the light chain of antibody A2-54 (B1) - antibody otherwise assembled as normal with heavy chain of A2-54. |
| 957 / 530 | Amino acid sequence AP-904 (B2; SEQ ID NO: 8 with additional AP at N terminus) joined by linker SGGGGSGGGGS (X; SEQ ID NO: 46) to the C terminal of the light chain of antibody A2-54 (B1) - antibody otherwise assembled as normal with heavy chain of A2-54. |
| 958 / 531 | Amino acid sequence of 904 (B2; SEQ ID NO: 8) joined by linker NFSQP (X; SEQ ID NO: 48) to the N terminal of the heavy chain of antibody A2-54 (B1) - antibody otherwise assembled as normal with light chain of A2-54. |

(continued)

| Reference no. | Description of molecule |
|---|---|
| 959 / 530 | Amino acid sequence of 904 (B2; SEQ ID NO: 8) joined by linker KRTVA (X; SEQ ID NO: 49) to the N terminal of the light chain of antibody A2-54 (B1) - antibody otherwise assembled as normal with heavy chain of A2-54. |
| 960 / 530 | Amino acid sequence of 904 (B2; SEQ ID NO: 8) joined by linker NFSQP (X; SEQ ID NO: 48) to the N terminal of the light chain of antibody A2-54 (B1) - antibody otherwise assembled as normal with heavy chain of A2-54. |

Sequences encoding a mutated human CD86 variable soluble domain (B2), a linker (X) and restriction enzyme sites were designed in silico. The sequences were ordered from Eurofins MWG Operon (Ebersberg, Germany) and cloned into a vector containing the heavy or the light chain of a CD40 binding antibody (A2-54; Ellmark et al.,Immunology, 108, 452-457, 2003). The vector was then transfected into Freestyle 293 cells (Invitrogen Corp., Carlsbad, US) according to the manufacturer's instructions. The transfected cells were cultivated and harvested. The bispecific constructs produced by the cells were purified on protein A columns (HisTrap protein A, GE Helthcare) according to standard methods.

Example 6 - characterisation of exemplary bispecific molecules

The isoelectric point of the polypeptides produced in Example 5 was determined using GP-MAW software and is shown below.

| Bispecific antibody | Isoelectric point (pI) |
|---|---|
| 956/530 | 7.8 |
| 957/530 | 7.8 |
| 958/531 | 7.8 |
| 959/530 | 8.7 |
| 960/530 | 7.8 |

Dynamic Light Scattering, DLS, was used to study aggregation behavior and to confirm molecular weights in accordance with standard protocols. No aggregation was observed for two representative polypeptides, 956/530 and 957/530, tested at a concentration of 1mg/ml. The observed Mw for 956/530 and 957/530 was 265 kDa and 282kDa, respectively.

Dynamic light scattering was measured using Malvern Zetasizer Nano series ZEN1600 (Worcestershire, UK) and Malvern Low-volume quartz batch cuvette (ZEN2112) according to manufacturer's instructions. 200μl sterile and dust free pipette tips from Biozym, cat no 720328, were used at each transfer step. Interfering dust particles were removed by centrifugation of the samples for 5 min at 13000 rpm prior to analysis.

Example 7 - demonstration of dual binding specificity of exemplary bispecific molecules *Binding to CTLA-4*

Binding of the polypeptides produced in Example 5 to CTLA-4 was assessed by standard binding ELISA. In short, 96-well flat bottom plate high binding plates (Greiner #655074) were coated with CTLA4-Fc (Fitzgerald #30R-CD152) by incubating overnight at 4 °C. The plates were washed (Wash buffer: PBS+0,05% Tween 20 (PBST) Medicago #09-9410-100) and then blocked in PBST+3% BSA (Merck, #1.12018.0100). The plates were washed again and samples or control (serially diluted 1/ from 50 000-0001 nM) were added to the wells. A monospecific antibody for CD40 (530/531: A2-54) was used as the control. The samples were incubated for 1h at room temperature and then washed. Detection antibody, goat-anti-human IgG Fcy-HRP (Jackson, #109-035-098) was added and the plates were subsequently developed using SuperSignal Pico Chemiluminescent substrate (Thermo #37069) and detected with Envision reader (Perkin Elmer). Results are shown in Figure 5. In Figure 5A the binding of bispecific molecules at different concentrations to CTLA-4 coated onto an ELISA plate is shown. In Figure 5B, the binding of bispecific molecules at one fixed concentration (100ng/ml) to CTLA-4 coated onto an ELISA plate is shown. The bi-specific molecules bind specifically to CTLA-4.

*Binding to CD40*

Binding of the polypeptides produced in Example 5 to CD40 was assessed by standard binding ELISA. In short, 96-well flat bottom plate high binding plates (Greiner #655074) were coated with CD40-Fc (Ancell # 504-820) by incubating overnight at 4 °C. The plates were washed (Wash buffer: PBS+0,05% Tween 20 (PBST) Medicago #09-9410-100) and then blocked in PBST+3% BSA (Merck, #1.12018.0100). The plates were washed again and samples or control (serial dilutions 1/2, starting at 0.2 ug/ml) wereadded to the wells. The isolated polypeptide of amino acid sequence SEQ ID NO: 8 (soluble CTLA-4 binding domain reference 904) was used as a control as it binds specifically to only CTLA-4.

The samples were incubated for 1h at room temperature and then washed. Detection antibody, goat-anti-human IgG Fcγ-HRP (Jackson, #109-035-098) was added and the plates were subsequently developed using SuperSignal Pico Chemiluminescent substrate (Thermo #37069) and detected with Envision reader (Perkin Elmer). Results are shown in Figure 6. In Figure 6A the binding of bispecific molecules at different concentrations to CD40 coated onto an ELISA plate is shown. In Figure 6B, the binding of bispecific molecules at one fixed concentration (100ng/ml) to CD40 coated onto an ELISA plate is shown. The bispecific molecules bind specifically to CD40.

*Dual binding to CD40 and CTLA-4*

A dual ELISA was developed to assess simultaneous binding of the polypeptides produced in Example 5 to both CD40 and CTLA-4. In short, 96-well flat bottom plate high binding plates (Greiner #655074) were coated with CD40-Fc (Ancell # 504-820) by incubating overnight at 4 °C. The plates were washed (Wash buffer: PBS+0,05% Tween 20 (PBST) Medicago #09-9410-100) and then blocked in PBST+3% BSA (Merck, #1.12018.0100). The plates were washed again and samples (957/530; 956/530) or control (0.2 ug/ml) were added to the wells. Mono-specific anti-CD40 antibody was used as the control (A2-54). The samples were incubated for 1h at room temperature and then washed. Then biotinylated CTLA4-Fc (Orencia, Bristol-Mayers Squibb) (serially diluted 1/4 from3.4 - 215nM) was added. Streptavidin-HRP (Pierce #21126) was then added and the plates were subsequently developed using SuperSignal Pico Chemiluminescent substrate (Thermo #37069) and detected with Envision reader (Perkin Elmer). Results are shown in Figure 7 and demonstrate that the bispecific molecules bind to the two antigens simultaneously.

*Surface Plasmon Resonance analysis*

The ability of the bispecifc molecule to bind simulataneously to CTLA4 and CD40 was further confirmed by Biacore™ analysis. CD40-Fc (R&D #1493-CD) was immobilized to the BIAcore sensorship, CM5, using conventional amine coupling. 0.5 uM Bispecific construct (957/530) was injected over immobilized CD40 for 3 minutes and the dissociation was followed for 3 minutes. 2.17 uM CTLA4-Fc (Orencia, Bristol-Mayers Squibb) was injected over the CD40-Bispecific complex for 3 minutes and the dissociation phase was followed for 3 minutes. The chip was finally regenerated using 10 mM Glycine, pH 1.7. A Biacore 3000 instrument was used according to the manufacturer's protocols. Results are shown in Figure 8. The black line shows binding of the bispecific molecule 957/530 to CD40 (time 75s), and, upon injection of soluble CTLA-4 (time 400s), simultaneous binding to CD40 and CTLA-4. The dashed line shows the binding of the control Ab, a monospecific antibody (530/531, A2-54) that only binds to CD40. The results demonstrate that the bi-specific molecule is able to simultaneously bind specifically to CD40 and to CTLA-4.

Example 8 - demonstration of dual binding specificity of exemplary bispecific molecules to targets expressed by cells

The ability of the bispecific antibodies to simultaneously bind to human CD40 expressed on WEHI cells and to human CTLA-4 expressed on HEK cells was determined in vitro by flow cytometry analysis. In short, WEHI-CD40 cells were labeled with PKH67 (Sigma-Aldrich # MIDI67) and HEK-CTLA4 cells were labeled with PKH26 (Sigma-Aldrich # MINI26), the cells were washed with PBS-2%BSA. $0{,}5x10^6$ per cell type were mixed with bispecific polypeptide (957/530) for 30 minutes at room temperature. The cells were washed and analyzed on a FACScalibur instrument, according to the manufacturer's instruction (Becton Dickinson, USA) and then the mean fluorescence intensity ("MFI") was determined. A representative FACS plot is shown in Figure 9. This confirms that simultaneous binding to the two targets by the bispecific polypeptide occurs in vitro, and that this bispecific binding results in cell-cell contact between CTLA-4 expressing cells and CD40 expressing cells. This was further confirmed by Fluorescence Microscopy (not shown).

Example 9 - demonstration of the ability of exemplary bispecific molecules to induce proliferation in human B cells

Buffy coats from peripheral blood were obtained from healthy blood donors at Uppsala University Hospital Blood Center and Lund University Hospital in Sweden. Human PBMCs were isolated by Ficoll-Paque density gradient centrifugation. CD19+ B cells were isolated with MACS microbeads (Miltenyi Biotec) according to manufacturer's instructions. The CD19+ cells (5 x $10^4$/well, >95% purity) were cultured in R10 medium with 10 ng/ml of IL-4 and dilution series of either a polypeptide of the invention (957/530), or a monospecific antibody for CD40 (530/531: A2-54), or the isolated polypeptide of amino acid sequence SEQ ID NO: 8 (soluble CTLA-4 binding domain reference 904) was used as a control as it binds specifically to only CTLA-4. After 48 to 72 h, the metabolic activity was measured with CellTiter-Glo (Promega, WI, USA). The EC50 values were calculated using GraphPad Prism (GraphPad Software, Inc.). The results are shown in Figure 10. The bispecific polypeptide of the invention produces a synergistic increase in proliferation in human B cells.

Example 10 - cross-reactivity to cynomolgus CD40

The ability of exemplary bispecific polypeptides to bind to cells expressing cynomolgus CD40 was evaluated by measuring binding to HEK cells (ECACC, Salisbury, UK), transfected with the genes encoding the extracellular part of cynomolgus CD40. These HEK cells express cynomolgus CD40 on their surface. The concentrations of the bispecific polypeptides were 1 and 10 μg/ml. All 5 of the bispecific polypeptides tested bound to cynomolgus monkey CD40. The results are shown in the table below.

| Bispecific Polypeptide | Cynomolgus monkey cross reactivity |
|---|---|
| 1107/1145 (SEQ 110 with 1107 Heavy chain) | + |
| 1132/1139 (SEQ 112 with 1132 Heavy chain) | + |
| 1140/1141 (SEQ 113 with 1140 Heavy chain) | + |
| 1150/1152 (SEQ 114 with 1150 Heavy chain) | + |
| 1134/1141 (SEQ 113 with 1134 Heavy chain) | + |

Example 11 - Cross reactivity to murine CTLA-4

The relative affinity for murine and human CTLA-4 of an exemplary bispecific polypeptide of the invention was investigated using an inhibition ELISA binding assay. The bispecific polypeptide tested was 1140/1141 (SEQ 113 assembled with 1140 heavy chain). The CTLA-4-binding part of this polypeptide is the 1040 mutant CD86 molecule. The CTLA-4 binding properties of this CD86 molecule are not affected by conjugation to antibody.

In brief, 96-well flat bottom plate high binding plates (Greiner #655074) were coated with human CTLA-4 (Fitzgerald) incubating overnight at 4 °C. The plates were washed (Wash buffer: PBS+0,05% Tween 20 (PBST) Medicago #09-9410-100) and then blocked in PBST+3% BSA (Merck, #1.12018.0100).

The sample (exemplary polypeptide) was pre-incubated at room temperature for 1 hour with soluble biotinylated human CTLA4 (Fitzgerald #30R-CD152) or soluble murine CTLA-4 (R&D systems) at different concentrations (serial dilutions ¼ from 30000 to 0.3ng/ml).

The mixture was then added to the blocked wells in the ELISA plate. Detection was performed with Streptavidin-HRP (Pierce #21126) and the plates were subsequently developed using SuperSignal Pico Chemiluminescent substrate (Thermo #37069) and detected with Envision reader (Perkin Elmer). The results are shown in Figure 11. The observed inhibition curves with murine and human CTLA-4 demonstrate that the binding affinity of the exemplary polypeptide to the two forms of CTLA-4 is of a similar magnitude.

Example 12 - Binding to human CD40

The ability of different exemplary bispecific polypeptides to bind to human CD40 was assayed by standard binding ELISA. The protocol was as described in Example 7 above. Results are shown as dose response curves in Figures 12A, B and C. The EC50 values ranged from 0.3 to 14 nM. The tested bispecific polypeptides include 1107/1145 (dimer of SEQ 110 with 1107 Heavy chain), 1136/1143 (dimer of SEQ 111 with 1143 Heavy chain), 1132/1139 (dimer of SEQ 112 with 1132 Heavy chain), 1140/1141 (dimer of SEQ 113 with 1140 Heavy chain), 1150/1152 (dimer of SEQ 114 with 1150 Heavy chain) and 1134/1141 (dimer SEQ 113 with 1134 Heavy chain).

Example 13 - Binding to human CTLA-4

Six different exemplary bispecific polypeptides were tested for their ability to bind to human CD40 using standard binding ELISA. The protocol was as described in Example 7 above. Results are shown as dose response curves in Figures 13A to F. An exemplary bispecific molecule from Example 7 (957/530) was included for comparison, as well as the anti-CD40 mono-specific antibody, A2-54 as a negative control. All six tested polypeptides had an EC50 value calculated to be in the range approximately 0.02 - 0.05 μg/ml. Since all six share the same CD86 variant (1040), this indicates that different antibody conjugates do not affect the ability of this variant to bind CTLA-4. The tested bispecific polypeptides were 1107/1145 (dimer of SEQ 110 with 1107 Heavy chain), 1136/1143 (dimer of SEQ 111 with 1143 Heavy chain), 1132/1139 (dimer of SEQ 112 with 1132 Heavy chain), 1140/1141 (dimer of SEQ 113 with 1140 Heavy chain), 1150/1152 (dimer of SEQ 114 with 1150 Heavy chain) and 1134/1141 (dimer SEQ 113 with 1134 Heavy chain).

Example 14 - Dual binding to CD40 and CTLA-4

The six different exemplary bispecific polypeptides from Example 13 were tested for their ability to bind to both CD40 and CTLA-4. This was assessed by Surface Plasmon Resonance using the same protocol as in Example 7 above. The results are shown in Figure 14. The kinetic parameters and the affinity constants were calculated using BIAevaluation 4.1 software and are shown in the tables below.

Affinity constants for binding to human CD40 as determined by Surface Plamson Resonance (Biacore).

| polypeptide | ka (1/Ms) | kd (1/s) | KD (M) |
|---|---|---|---|
| 1107/1145 | 3,47E+05 | 1,17E-04 | 3,36E-09 |
| 1132/1139 | 1,36E+06 | 8,44E-03 | 6,20E-09 |
| 1140/1141 | 4,70E+05 | 3,98E-03 | 8,47E-09 |
| 1150/1152 | 1,28E+06 | 3,26E-03 | 2,54E-09 |
| 1134/1141 | 1,34E+05 | 1,89E-03 | 1,41E-08 |
| 1136/1143 | 1,45E+05 | 5,63E-03 | 3,89E-08 |

Affinity constants for binding to human CTLA-4 as determined by Surface Plamson Resonance (Biacore).

| polypeptide | ka (1/Ms) | kd (1/s) | KD (M) |
|---|---|---|---|
| 1107/1145 | 1,02E+06 | 3,10E-03 | 3,05E-09 |
| 1132/1139 | 1,06E+06 | 3,11E-03 | 2,92E-09 |
| 1140/1141 | 7,97E+05 | 3,15E-03 | 3,95E-09 |
| 1150/1152 | 7,65E+05 | 3,12E-03 | 4,07E-09 |

Example 15 -bispecific polypeptide has an anti-tumor effect in vivo

The anti-tumor effect of exemplary bispecific polypeptide 1140/1141 (dimer of SEQ 113 with 1140 Heavy chain) was studied by administering the polypeptide to human CD40 transgenic mice inoculated with Murine bladder transitional cell carcinoma cancer cell line, Mouse Bladder-49 (MB49) cells. The treatment produced a statistically significant anti-tumor efficacy.

The animal model used for this experiment was created by injection of $2.5x10^5$ MB49 cells in the flank of hCD40tg mice. Bispecific polypeptide was administrated peritumorally with doses of 200pmol (42 μg) on days 7 and 10 after inoculation, and tumor measurements were taken by caliper. The tumor growth and survival was followed over time.

The treatment resulted in a significant anti-tumor efficacy, with improved survival and reduced tumour volume, as shown in Figures 15A and B.

Example 16 -bispecific polypeptide establishes anti-tumour immunological memory in vivo

Mice previously treated for bladder cancer and cured with the bispecific polypeptide 1140/1141 were re-challenged with bladder cancer cells. The treatment with the bispecific antibody clone was shown to have established an immunological memory for bladder cancer and hence immunity to tumors when the animals were re-challenged.

For this experiment, MB49 re-challenge was performed by injection of $2.5x10^5$ cells in the flank of hCD40tg mice that had previously been cured of MB49 tumors by treatment with 1140/1141 (as in Example 15). Naive (i.e. not previously treated with polypeptide or inoculated) hCD40tg mice were used as controls. The tumor growth taken by caliper and survival was followed over time.

As shown in Figures 16A and B, the previously treated, re-challenged mice had 100% survival and the tumors regressed completely. Thus, the data shows that treatment with the bispecific polypeptide induces statistically significant immunity to tumor re-challenge with MB49 in hCD40tg mice. This demonstrates the presence of immunological memory. This result is of clinical relevance, since such anti-tumour immunological memory is necessary to establish a long lasting treatment effect particularly against metastatic tumors.

Example 17 - B cell proliferation assay

The six different exemplary bispecific polypeptides from Example 13 were tested for their ability to stimulate human B cells. Each bispecific polypeptide was directly compared to the corresponding monospecific antibody lacking a CTLA-4 binding domain.

Human B cells from leukocyte filters were purified using RosetteSep Human B cell Enrichment Cocktail (Stemcell Technologies) and density separation using Ficoll-Paque Plus (GE Healthcare). B cell purity was assessed by staining with anti-human CD19-PE ab (Milteney). B cell purity varied between the different donors of the 3 experiments with a mean B cell purity of 69%.

B cells were seeded out on 96-wells plates together with different concentrations of the CD40-CD86 antibodies and human IL-4. The plates were incubated in a moisture chamber at 37C 5% $C0^2$ for 3 days. Metabolic activity was measured with Cell titer Glo (Promega) luminescence that measures the ATP content of the wells, which reflects B cell proliferation. S2C6 was used as a reference antibody. S2C6 is a murine anti-human CD40 antibody with agonistic effect in the B cell proliferation assay (Koho, Can Imm Imm 1984).

Dose-response curves of each antibody were plotted using graph Pad prism 4.0. The efficacy of each antibody was normalized by dividing the TOP value (as calculated using variable dose response curve fitting) of the antibody with the TOP value of the reference antibody S2C6.

As shown in Figure 17, the bispecific polypeptides displayed a higher efficacy in the B-cell proliferation assay than their corresponding monoclonal anti-CD40 antibody, indicating that the CTLA-4 binding part influences the ability to stimulate B-cell proliferation. This effect could be due to presence of cells (other than B cells) that express CD28 or CTLA-4 that may bind to the CD86-part of the antibody and thereby induce crosslinking of the antibody. The resulting increased immune activation has the potential to result in a stronger immune activation *in vivo*, generating an improved anti-tumor activity.

Example 18 - Activation of immune cells in human PBMCs

Human PBMCs contain antigen presenting cells expressing CD40 and T cells expressing CTLA-4. Two of the exemplary bispecific molecules from Example 13 (1134/1141 and 1140/1141) were assayed for their ability to activate these immune cells. Each bispecific polypeptide was directly compared to the corresponding monospecific antibody lacking a CTLA-4 binding domain.

Human PBMCs from leukocyte filters or buffy blood (2-4 donors/experiment) were purified using density separation with Ficoll-Paque Plus (GE Healthcare). PBMCs were seeded out on CD3 pre-coated (overnight) 96-wells plates together with different concentrations of the bispecific polypeptide or the corresponding monospecific antibody. Staphylococcal enterotoxin A (SEA), a T cell stimulator, was added to the culture medium. The plates were incubated in a moisture chamber at 37C 5% $C0^2$ for 2 days and the IL-2 concentration in the supernatant was measured using a human IL-2 ELISA kit (BD Pharmingen). The IL-2 production at each tested concentration was calculated from the IL-2 ELISA standard curve.

The mean fold change of each bispecific polypeptide relative to the corresponding monospecific antibody was calculated by dividing the mean of the two highest values (TOP value) of each bispecific polypeptide with the TOP value of the corresponding monospecific antibody. The two bispecific polypeptides generated a strong T cell activation as measured by IL-2 production. The mean fold change in IL-2 production was significantly higher relative to the monospecific antibodies, as shown in Figure 18.

It has been determined (data not shown) that the anti-CD40 antibody portion of bispecific polypeptides 1134/1141 and 1140/1141 is not capable of blocking CD40L binding to the CD40 receptor. These anti-CD40 antibodies (and any which bind to the same epitope on CD40) thus have particularly favorable properties for use in a bispecific format with a CTLA-4 binding domain. Binding to this particular epitope on CD40 in a bispecific format enables a surprisingly strong immune activation, which has the potential to generate a strong anti-tumor effect in vivo.

Example 19- CTLA-4 induced activation of B cells

Immune activation was further investiaged by incubating cells_transformed to express human CTLA-4 and B cells together with exemplary bispecific polypeptides.

2,5 x $10^3$ HEK wild type cells (HEK-wt) or HEK cells transduced to produce CTLA-4 (CTLA4-HEK) were seeded in the wells of a 96-well plate. Different concentrations of an exemplary bispecific polypeptide from Example 13 (1140/1141) were added to the cell containing wells and to empty wells, prior to pre-incubation in a moisture chamber at 37°C, 5% C02. After 2h of incubation, 5 x $10^3$ B cells isolated from leukocyte filters of 2 healthy donors were added to each well together with IL-4 (10 ng/ml).

After 48 hours of incubation, cells were harvested and B cell activation was analyzed by co-staining of antibodies against anti-human CD19-PE (B cells) and the activation marker: anti-human CD83-APC (BD Biosciences). The mean fluorescence intensity (MFI) of CD83 expression was measured by flow cytometry on a FACSVerse, the data was analyzed using FlowJo software and statistical analysis was performed using Graphpad Prism 4.

As shown in Figure 19, the efficacy of the up-regulation of CD83 was increased 4-14 fold upon co-culturing of the bispecific polypeptide with CTLA4-HEK and B cells as compared to controls. The controls were either co-culturing of the bispecific polypeptide with HEK-wt and B cells, or the B cells alone.

It is known that many CD40 agonists require cross-linking via the Fc part of the antibody to induce optimal activity (Li et al 20011, Science, White et al 2011 J Immunol)). The CTLA-4 binding part of the bispecific polypeptide may provide this increase in cross-linking resulting in increased immune activation. The increased immune activation may result in increased anti-tumor effects in vivo.

## Claims

1. A polypeptide capable of specifically binding to both CTLA-4 and CD40, said polypeptide comprising B1 and B2, wherein:

B1 is an antibody, or antigen binding fragment thereof, specific for CD40, and which is a CD40 agonist; and B2 is a polypeptide binding domain specific for CTLA-4, which comprises or consists of:

i) the amino acid sequence of SEQ ID NO: 3; or
ii) an amino acid sequence in which at least one amino acid is changed when compared to the amino acid sequence of SEQ ID NO: 3 provided that said binding domain binds to human CTLA-4 with higher affinity than wild-type human CD86.

**2.** A polypeptide according to claim 1 in which B1 comprises at least one heavy chain (H) and at least one light chain (L) and B2 is attached to said at least one heavy chain (H) or at least one light chain (L).

**3.** A polypeptide according to claim 2 in which B1 comprises:

- at least one heavy chain (H) and at least one light chain (L) and B2 is attached to either the heavy chain or the light chain; or
- two identical heavy chains (H) and two identical light chains (L) and B2 is attached to both heavy chains or to both light chains.

**4.** A polypeptide according to any one of the preceding claims which comprises a polypeptide arranged according to the following formula, written in the direction N-C:

(a) H-(X)n-B2;
(b) B2-(X)n-H;
(c) L-(X)n-B2; or
(d) B2-(X)n-L

wherein X is a linker and n is 0 or 1,
or a polypeptide which consists of a polypeptide arranged according to any one of formulae (a) to (d).

**5.** A polypeptide according to claim 4, wherein X is a peptide with the amino acid sequence SGGGGSGGGGS, SGGGGSGGGGSAP, NFSQP, KRTVA or (SG)m, where m = 1 to 7.

**6.** A polypeptide according to any one of the preceding claims, wherein 1, 2, 3, 4, 5, 6, 7, 8, 9 or 10 amino acids in said amino acid sequence of B2 (ii) are substituted when compared to the amino acid sequence of SEQ ID NO: 3.

**7.** A polypeptide according to claim 6, wherein at least one of said amino acid substitutions in said amino acid sequence of said first binding domain is at position 122.

**8.** A polypeptide according to claim 7, wherein said amino acid sequence is also substituted in at least one of positions 107, 121, 125, 32, 48, 49, 54, 74, 77, 79, 103, 111, 118, 120, 127 and 134.

**9.** A polypeptide according to claim 7 or 8, wherein the substitutions in each position are selected from the following: F32I, Q48L, S49T, V54I, V64/I, K74I/R, S77A, H79D/S/A, K103E, L107I/F/R, I111V, T118S, M120L, I121V, R122K/N, Q125E, N127S/D and A134T.

**10.** A polypeptide according to any one of the preceding claims wherein said amino acid sequence of B2 comprises or consists of an amino acid sequence selected from any one of SEQ ID NOs 8, 6, 7 and 9 to 24.

**11.** A polypeptide according to any one of the preceding claims, wherein B1 comprises the heavy chain CDR sequences of an antibody selected from the group consisting of: A2-54 (SEQ ID NO: 61); 1107/1145 (SEQ ID NO: 70); 1136/1143 (SEQ ID NO: 72); 1132/1139 (SEQ ID NO: 74); 1140/1141 (SEQ ID NO: 76); 1150/1152 (SEQ ID NO: 78); 1134/1141 (SEQ ID NO: 80); 1146/1147 (SEQ ID NO: 82); 1142/1135 (SEQ ID NO: 84); 1148/1149 (SEQ ID NO: 86) and 1138/1135 (SEQ ID NO: 88); and the light chain CDR sequences of an antibody selected from the group consisting of: A2-54 (SEQ ID NO: 62); 1107/1145 (SEQ ID NO: 71); 1136/1143 (SEQ ID NO: 73); 1132/1139 (SEQ ID NO: 75); 1140/1141 (SEQ ID NO: 77); 1150/1152 (SEQ ID NO: 79); 1134/1141 (SEQ ID NO: 81); 1146/1147 (SEQ ID NO: 83); 1142/1135 (SEQ ID NO: 85); 1148/1149 (SEQ ID NO: 87) and 1138/1135 (SEQ ID NO: 89).

**12.** A polypeptide according to any one of the preceding claims, wherein B1 comprises an human Fc region or a variant of a said region, where the region is an IgG1, IgG2, IgG3 or IgG4 region, preferably an IgG1 or IgG4 region.

**13.** A polypeptide according to any one of the preceding claims, which comprises or consists of the amino acid sequence of any one of SEQ ID NOs: 56 to 60 or 110 to 114.

**14.** A polypeptide according to any one of the preceding claims for use in a method for treating or preventing a disease or condition in an individual.

**15.** A polypeptide for use according to claim 14, wherein the disease or condition is cancer.

**16.** A polypeptide for use according to claim 15, wherein the method comprises administering the polypeptide systemically or locally, such as at the site of a tumour or into a tumour draining lymph node.

**17.** A polypeptide for use according to claim 15 or 16 wherein the cancer is prostate cancer, breast cancer, colorectal cancer, pancreatic cancer, ovarian cancer, lung cancer, cervical cancel, rhabdomyosarcoma, neuroblastoma, multiple myeloma, leukemia, acute lymphoblastic leukemia, melanoma, bladder cancer, gastric cancer, head and neck cancer, liver cancer, skin cancer, lymphoma or glioblastoma.

**18.** A polynucleotide encoding a polypeptide according to any one of claims 1 to 13.

**19.** A polypeptide according to any one of claims 1 to 13 conjugated to an additional therapeutic moiety.

**20.** A composition comprising a polypeptide according to any one of claims 1 to 13 and at least one pharmaceutically acceptable diluent or carrier.

**Patentansprüche**

**1.** Polypeptid, das in der Lage ist, sowohl an CTLA-4 als auch an CD40 spezifisch zu binden, wobei das Polypeptid B1 und B2 umfasst, wobei:

B1 ein Antikörper oder ein Antigen-bindendes Fragment davon ist, spezifisch für CD40 ist und ein CD40-Agonist ist; und
B2 eine Polypeptidbindungsdomäne ist, die für CTLA-4 spezifisch ist und die Folgendes umfasst oder aus Folgenden besteht:

i) der Aminosäuresequenz von SEQ ID NR.: 3; oder
ii) einer Aminosäuresequenz, in der mindestens eine Aminosäure verglichen mit der Aminosäuresequenz von SEQ ID NR.: 3 verändert ist, vorausgesetzt, dass die Bindungsdomäne mit höherer Affinität an humanes CTLA-4 bindet als humanes Wildtyp-CD86.

**2.** Polypeptid nach Anspruch 1, in dem B1 mindestens eine schwere Kette (H) und mindestens eine leichte Kette (L) umfasst und B2 an der mindestens einen schweren Kette (H) oder an mindestens einer leichten Kette (L) befestigt ist.

**3.** Polypeptid nach Anspruch 2, in dem B1 Folgendes umfasst:

- mindestens eine schwere Kette (H) und mindestens eine leichte Kette (L) und B2 entweder an der schweren Kette oder der leichten Kette befestigt ist; oder
- zwei identische schwere Ketten (H) und zwei identische leichte Ketten (L) und B2 an beiden schweren Ketten oder an beiden leichten Ketten befestigt ist.

**4.** Polypeptid nach einem der vorhergehenden Ansprüche, das ein Polypeptid umfasst, welches nach der folgenden Formel angeordnet ist, geschrieben in der Richtung von N nach C:

(a) H-(X)n-B2;
(b) B2-(X)n-H;
(c) L-(X)n-B2; oder
(d) B2-(X)n-L,

wobei X ein Linker ist und n 0 oder 1 ist,
oder ein Polypeptid, das aus einem Polypeptid besteht, das nach einer der Formeln (a) bis (d) angeordnet ist.

5. Polypeptid nach Anspruch 4, wobei X ein Peptid mit der Aminosäuresequenz SGGGGSGGGGS, SGGGGSGGGGSAP, NFSQP, KRTVA oder (SG)m ist, wobei m = 1 bis 7 ist.

6. Polypeptid nach einem der vorhergehenden Ansprüche, wobei 1, 2, 3, 4, 5, 6, 7, 8, 9 oder 10 Aminosäuren in der Aminosäuresequenz von B2 (ii) im Vergleich mit der Aminosäuresequenz von SEQ ID NR.: 3 substituiert sind.

7. Polypeptid nach Anspruch 6, wobei sich mindestens eine der Aminosäuresubstitutionen in der Aminosäuresequenz der ersten Bindungsdomäne an Position 122 befindet.

8. Polypeptid nach Anspruch 7, wobei die Aminosäuresequenz auch an mindestens einer der Positionen 107, 121, 125, 32, 48, 49, 54, 74, 77, 79, 103, 111, 118, 120, 127 und 134 substituiert ist.

9. Polypeptid nach Anspruch 7 oder 8, wobei die Substitutionen an jeder Position aus den Folgenden ausgewählt sind: F32I, Q48L, S49T, V54I, V64/I, K74I/R, S77A, H79D/S/A, K103E, L107I/F/R, I111V, T118S, M120L, I121V, R122K/N, Q125E, N127S/D und A134T.

10. Polypeptid nach einem der vorhergehenden Ansprüche, wobei die Aminosäuresequenz von B2 eine Aminosäuresequenz umfasst oder aus einer Aminosäuresequenz besteht, die aus einer der SEQ ID NR. 8, 6, 7 und 9 bis 24 ausgewählt ist.

11. Polypeptid nach einem der vorhergehenden Ansprüche, wobei B1 die CDR-Sequenzen der schweren Kette eines Antikörpers umfasst, die aus der Gruppe ausgewählt sind, welche aus den Folgenden besteht: A2-54 (SEQ ID NR.: 61); 1107/1145 (SEQ ID NR.: 70); 1136/1143 (SEQ ID NR.: 72); 1132/1139 (SEQ ID NR.: 74); 1140/1141 (SEQ ID NR.: 76); 1150/1152 (SEQ ID NR.: 78); 1134/1141 (SEQ ID NR.: 80); 1146/1147 (SEQ ID NR.: 82); 1142/1135 (SEQ ID NR.: 84); 1148/1149 (SEQ ID NR.: 86) und 1138/1135 (SEQ ID NR.: 88), und die CDR-Sequenzen der leichten Kette eines Antikörpers, die aus der Gruppe ausgewählt sind, welche aus den Folgenden besteht: A2-54 (SEQ ID NR.: 62); 1107/1145 (SEQ ID NR.: 71); 1136/1143 (SEQ ID NR.: 73); 1132/1139 (SEQ ID NR.: 75); 1140/1141 (SEQ ID NR.: 77); 1150/1152 (SEQ ID NR.: 79); 1134/1141 (SEQ ID NR.: 81); 1146/1147 (SEQ ID NR.: 83); 1142/1135 (SEQ ID NR.: 85); 1148/1149 (SEQ ID NR.: 87) und 1138/1135 (SEQ ID NR.: 89).

12. Polypeptid nach einem der vorhergehenden Ansprüche, wobei B1 eine humane Fc-Region oder eine Variante einer solchen Region umfasst, wobei die Region eine IgG1-, IgG2-, IgG3- oder IgG4-Region ist, vorzugsweise eine IgG1- oder IgG4-Region.

13. Polypeptid nach einem der vorhergehenden Ansprüche, welches die Aminosäuresequenz von einer von SEQ ID NR. 56 bis 60 oder 110 bis 114 umfasst oder daraus besteht.

14. Polypeptid nach einem der vorhergehenden Ansprüche zur Verwendung in einem Verfahren zum Behandeln oder Verhindern einer Krankheit oder eines Zustands in einem Individuum.

15. Polypeptid zur Verwendung nach Anspruch 14, wobei es sich bei der Krankheit bzw. bei dem Zustand um Krebs handelt.

16. Polypeptid zur Verwendung nach Anspruch 15, wobei das Verfahren das systemische oder lokale Verabreichen des Polypeptids umfasst, wie beispielsweise an der Stelle eines Tumors oder in einen tumorableitenden Lymphknoten.

17. Polypeptid zur Verwendung nach Anspruch 15 oder 16, wobei es sich bei dem Krebs um Prostatakrebs, Brustkrebs, kolorektalen Krebs, Bauchspeicheldrüsenkrebs, Ovarialkrebs, Lungenkrebs, Gebärmutterhalskrebs, Rhabdomyosarkom, Neuroblastom, multiples Myelom, Leukämie, akute lymphoblastische Leukämie, Melanom, Blasenkrebs, Magenkrebs, Kopf- und Hals-Krebs, Leberkrebs, Hautkrebs, Lymphom oder Glioblastom handelt.

18. Polynukleotid, welches ein Polypeptid nach einem der Ansprüche 1 bis 13 kodiert.

19. Polypeptid nach einem der Ansprüche 1 bis 13, konjugiert an eine zusätzliche therapeutische Einheit.

**20.** Zusammensetzung, umfassend ein Polypeptid nach einem der Ansprüche 1 bis 13 und mindestens ein pharmazeutisch akzeptables Verdünnungsmittel oder mindestens einen pharmazeutisch akzeptablen Träger.

## Revendications

**1.** Polypeptide susceptible de se lier spécifiquement à la fois au CTLA-4 et au CD40, ledit polypeptide contenant B1 et B2, dans lequel :

B1 est un anticorps, ou un fragment de liaison à un antigène de celui-ci, propre au CD40, et qui est un agoniste du CD40 ; et
B2 est un domaine de liaison du polypeptide spécifique au CTLA-4, qui comprend ou qui est constitué de :

i) la séquence d'acides aminés de SEQ ID N° 3 ; ou
ii) une séquence d'acides aminés dans laquelle au moins un acide aminé est modifié par rapport à la séquence d'acides aminés de SEQ ID N° 3 à condition que ledit domaine de liaison se lie au CTL-4 humain avec plus d'affinité que le CD86 humain de type sauvage.

**2.** Polypeptide selon la revendication 1, dans lequel B1 comprend au moins une chaîne lourde (H) et au moins une chaîne légère (L) et où B2 est lié à ladite au moins une chaîne lourde (H) ou à l'au moins une chaîne légère (L).

**3.** Polypeptide selon la revendication 2, dans lequel B1 comprend :

- au moins une chaîne lourde (H) et au moins une chaîne légère (L) et où B2 est lié soit à la chaîne lourde soit à la chaîne légère ; ou
- deux chaînes lourdes identiques (H) et deux chaînes légères identiques (L) et où B2 est lié à la fois aux chaînes lourdes et aux chaînes légères.

**4.** Polypeptide selon l'une quelconque des revendications précédentes, qui comprend un polypeptide répondant à la formule suivante, écrite dans la direction N-C :

(a) H-(X)n-B2 ;
(b) B2-(X)n-H ;
(c) L-(X)n-B2 ; ou
(d) B2-(X)n-L

dans laquelle X représente un liant et où n représente 0 ou 1,
ou un polypeptide constitué d'un polypeptide répondant à l'une quelconque des formules (a) à (d).

**5.** Polypeptide selon la revendication 4, dans lequel X représente un peptide comportant la séquence d'acides aminés SGGGGSGGGGS, SGGGGSGGGGSAP, NFSQP, KRTVA ou (SG)m, où m = 1 à 7.

**6.** Polypeptide selon l'une quelconque des revendications précédentes, dans lequel 1, 2, 3, 4, 5, 6, 7, 8, 9 ou 10 acides aminés de ladite séquence d'acides aminés de B2 (ii) sont substitués par rapport à la séquence d'acides aminés de SEQ ID N° 3.

**7.** Polypeptide selon la revendication 6, dans lequel au moins une des substitutions d'acides aminés dans ladite séquence d'acides aminés dudit premier domaine de liaison est en position 122.

**8.** Polypeptide selon la revendication 7, dans lequel ladite séquence d'acides aminés est également substituée dans au moins une des positions 107, 121, 125, 32, 48, 49, 54, 74, 77, 79, 103, 111, 118, 120, 127 et 134.

**9.** Polypeptide selon la revendication 7 ou 8, dans lequel les substitutions dans chaque position sont choisies parmi les suivantes : F32I, Q48L, S49T, V54I, V64/I, K74I/R, S77A, H79D/S/A, K103E, L107I/F/R, I111V, T118S, M120L, I121V, R122K/N, Q125E, N127S/D et A134T.

**10.** Polypeptide selon l'une quelconque des revendications précédentes, dans lequel ladite séquence d'acides aminés de B2 comprend une séquence d'acides aminés choisie parmi l'une quelconque des SEQ ID N° 8, 6, 7 et 9 à 24 ou est constituée de celle-ci.

**11.** Polypeptide selon l'une quelconque des revendications précédentes, dans lequel B1 contient les séquences CDR de chaîne lourde d'un anticorps choisi dans l'ensemble constitué de : A2-54 (SEQ ID N° 61) ; 1107/1145 (SEQ ID N° 70) ; 1136/1143 (SEQ ID N° 72) ; 1132/1139 (SEQ ID N° 74) ; 1140/1141 (SEQ ID N° 76) ; 1150/1152 (SEQ ID N° 78) ; 1134/1141 (SEQ ID N° 80) ; 1146/1147 (SEQ ID N°82) ; 1142/1135 (SEQ ID N° 84) ; 1148/1149 (SEQ ID N° 86) et 1138/1135 (SEQ ID N° 88) ; et les séquences CDR de chaîne légère d'un anticorps choisi dans l'ensemble constitué de : A2-54 (SEQ ID N° 62) ; 1107/1145 (SEQ ID N° 71) ; 1136/1143 (SEQ ID N° 73) ; 1132/1139 (SEQ ID N° 75) ; 1140/1141 (SEQ ID N° 77) ; 1150/1152 (SEQ ID N° 79) ; 1134/1141 (SEQ ID N° 81) ; 1146/1147 (SEQ ID N°83) ; 1142/1135 (SEQ ID N° 85) ; 1148/1149 (SEQ ID N° 87) et 1138/1135 (SEQ ID N° 89).

**12.** Polypeptide selon l'une quelconque des revendications précédentes, dans lequel B1 comprend une région Fc humaine ou un variant d'une telle région, la région étant une région IgG1, IgG2, IgG3 ou IgG4, de préférence une région IgG1 ou IgG4.

**13.** Polypeptide selon l'une quelconque des revendications précédentes, qui comprend la séquence d'acides aminés de l'une quelconque parmi SEQ ID N° 56 à 60 ou 110 à 114 ou qui est constitué de celle-ci.

**14.** Polypeptide selon l'une quelconque des revendications précédentes, à utiliser dans un procédé de traitement ou de prévention d'une maladie ou d'un état chez un individu.

**15.** Polypeptide à utiliser selon la revendication 14, la maladie ou l'état étant un cancer.

**16.** Polypeptide à utiliser selon la revendication 15, le procédé comprenant l'administration du polypeptide de manière systémique ou locale, comme au site d'une tumeur ou dans un noeud lymphatique de drainage de tumeur.

**17.** Polypeptide à utiliser selon la revendication 15 ou 16, le cancer étant un cancer de la prostate, un cancer du sein, un cancer colorectal, un cancer du pancréas, un cancer des ovaires, un cancer des poumons, un cancer du col de l'utérus, un rhabdomyosarcome, un neuroblastome, un myélome multiple, une leucémie, une leucémie lymphoblastique aiguë, un mélanome, un cancer de la vessie, un cancer de l'estomac, un cancer des voies aérodigestives supérieures, un cancer du foie, un cancer de la peau, un lymphome ou un glioblastome.

**18.** Polynucléotide codant un polypeptide conforme à l'une quelconque des revendications 1 à 13.

**19.** Polynucléotide conforme à l'une quelconque des revendications 1 à 13 conjugué à un autre fragment thérapeutique.

**20.** Composition comprenant un polypeptide conforme à l'une des revendications 1 à 13 et au moins un diluant ou un véhicule pharmaceutiquement acceptable.

FIGURE 1

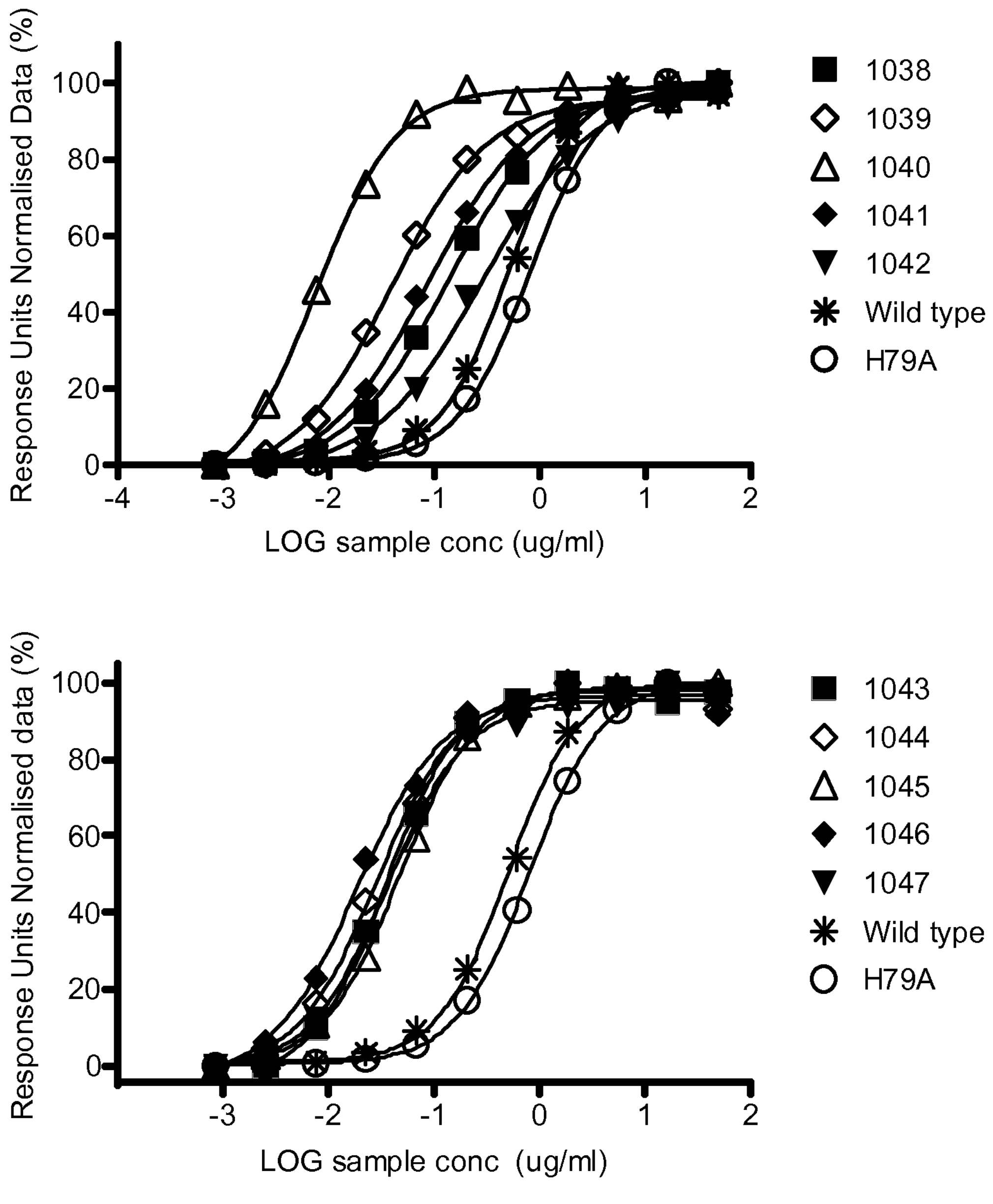
Response Units Normalised Data (%)
100
80
60
40
20
0
-4
-3
-2
-1
0
1
2
LOG sample conc (ug/ml)
1038
1039
1040
1041
1042
Wild type
H79A
Response Units Normalised data (%)
100
80
60
40
20
0
-3
-2
-1
0
1
2
LOG sample conc (ug/ml)
1043
1044
1045
1046
1047
Wild type
H79A

FIGURE 2

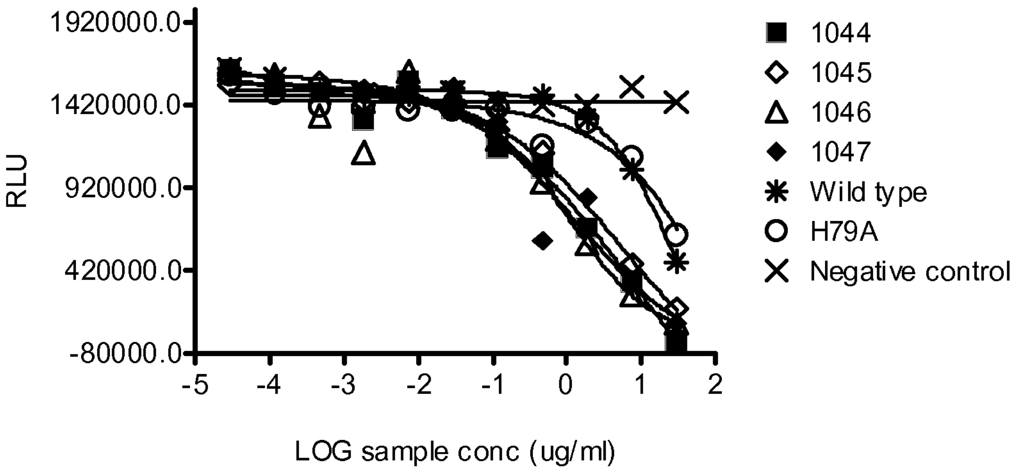
1920000.0
1420000.0
920000.0
420000.0
-80000.0
RLU
-5
-4
-3
-2
-1
0
1
2
LOG sample conc (ug/ml)
1044
1045
1046
1047
Wild type
H79A
Negative control

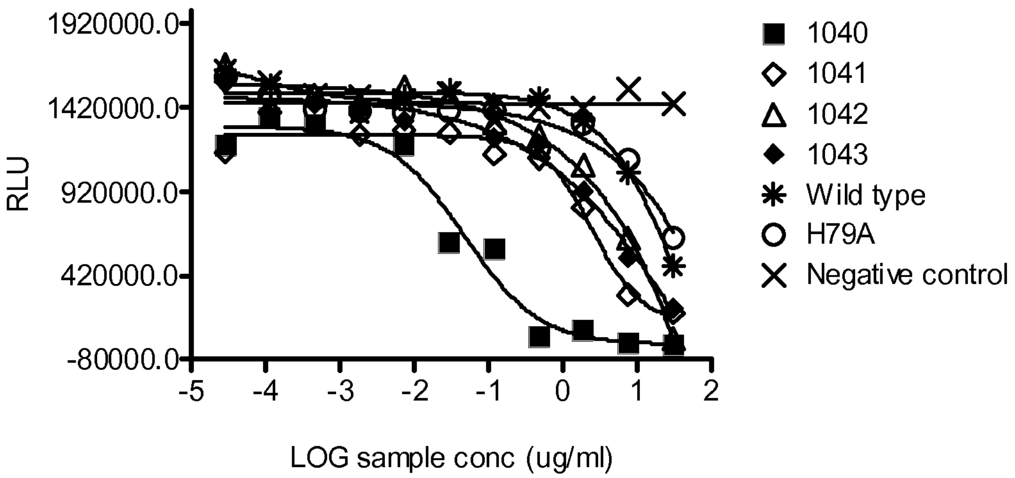
1920000.0
1420000.0
920000.0
420000.0
-80000.0
RLU
-5
-4
-3
-2
-1
0
1
2
LOG sample conc (ug/ml)
1040
1041
1042
1043
Wild type
H79A
Negative control

FIGURE 3

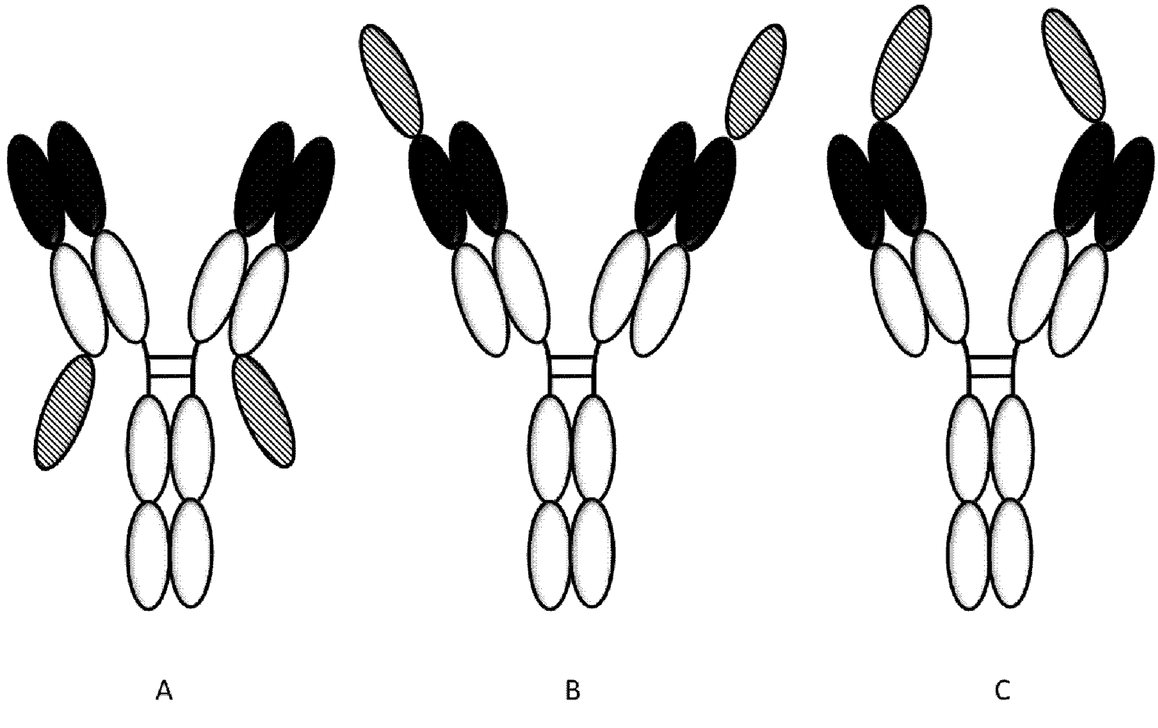

FIGURE 4

A

```
                    24
                    |
                    APLKIQA YFNETADLPC QFANSQNQSL SELVVFWQDQ     60
ENLVLNEVYL GKEKFDSVHS KYMGRTSFDS DSWTLRLHNL QIKDKGLYQC IIHHKKPTGM    120
IRIHQMNSEL SVLA                                                      134
```

B

```
MDPQCTMGLS NILFVMAFLL SGAAPLKIQA YFNETADLPC QFANSQNQSL SELVVFWQDQ     60
ENLVLNEVYL GKEKFDSVHS KYMGRTSFDS DSWTLRLHNL QIKDKGLYQC IIHHKKPTGM    120
IRIHQMNSEL SVLANFSQPE IVPISNITEN VYINLTCSSI HGYPEPKKMS VLLRTKNSTI    180
EYDGIMQKSQ DNVTELYDVS ISLSVSFPDV TSNMTIFCIL ETDKTRLLSS PFSIELEDPQ    240
PPPDHIP                                                              247
```

C

```
MDPQCTMGLS NILFVMAFLL SGAAPLKIQA YFNETADLPC QFANSQNQSL SELVVFWQDQ     60
ENLVLNEVYL GKEKFDSVHS KYMGRTSFDS DSWTLRLHNL QIKDKGLYQC IIHHKKPTGM    120
IRIHQMNSEL SVLANFSQPE IVPISNITEN VYINLTCSSI HGYPEPKKMS VLLRTKNSTI    180
EYDGIMQKSQ DNVTELYDVS ISLSVSFPDV TSNMTIFCIL ETDKTRLLSS PFSIELEDPQ    240
PPPDHIPWIT AVLPTVIICV MVFCLILWKW KKKKRPRNSY KCGTNTMERE ESEQTKKREK    300
IHIPERSDEA QRVFKSSKTS SCDKSDTCF
```

FIGURE 5

A

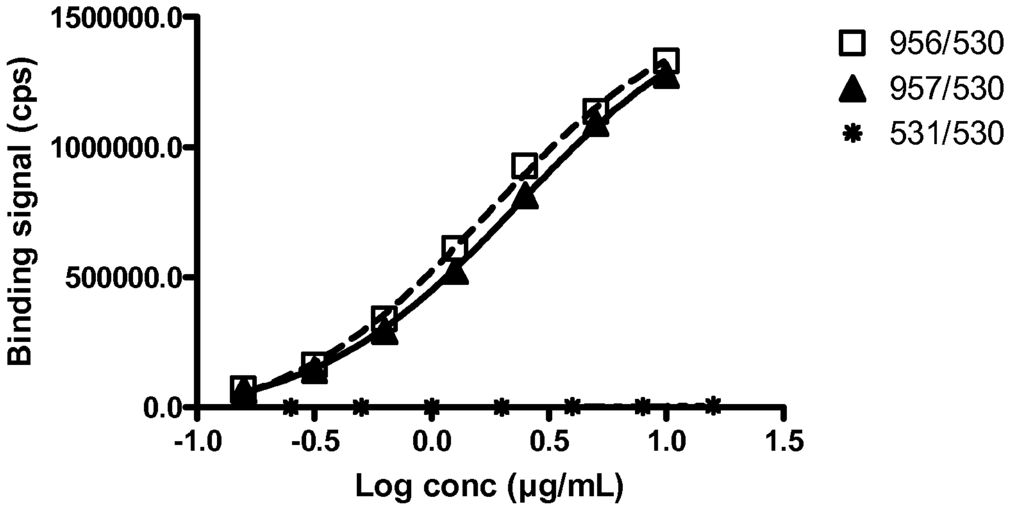
1500000.0
1000000.0
500000.0
0.0
Binding signal (cps)
-1.0
-0.5
0.0
0.5
1.0
1.5
Log conc (µg/mL)
956/530
957/530
531/530

B

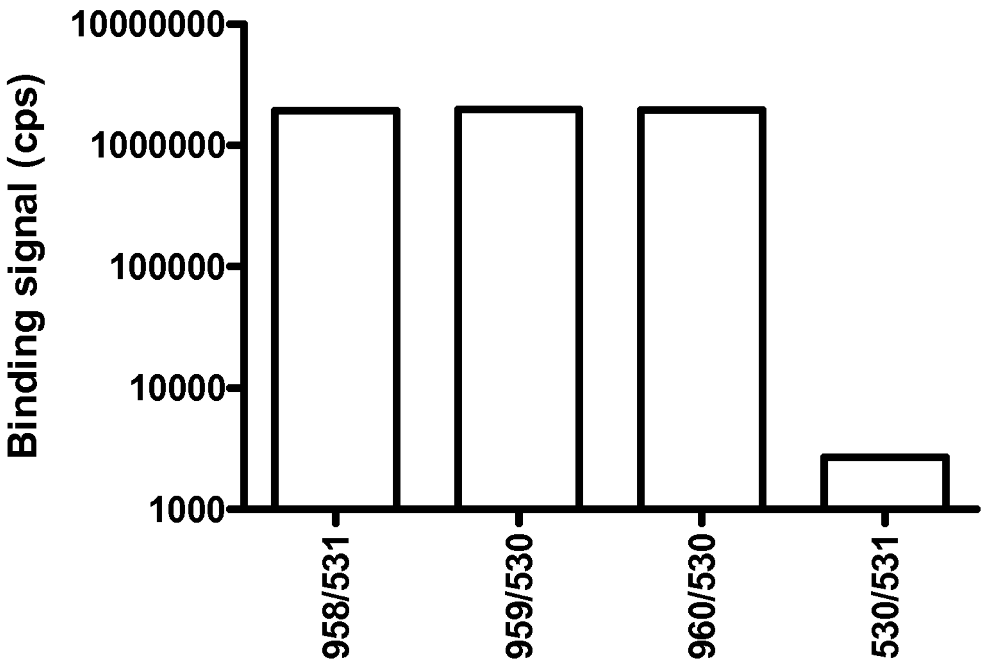
10000000
1000000
100000
10000
1000
Binding signal (cps)
958/531
959/530
960/530
530/531

FIGURE 6

A

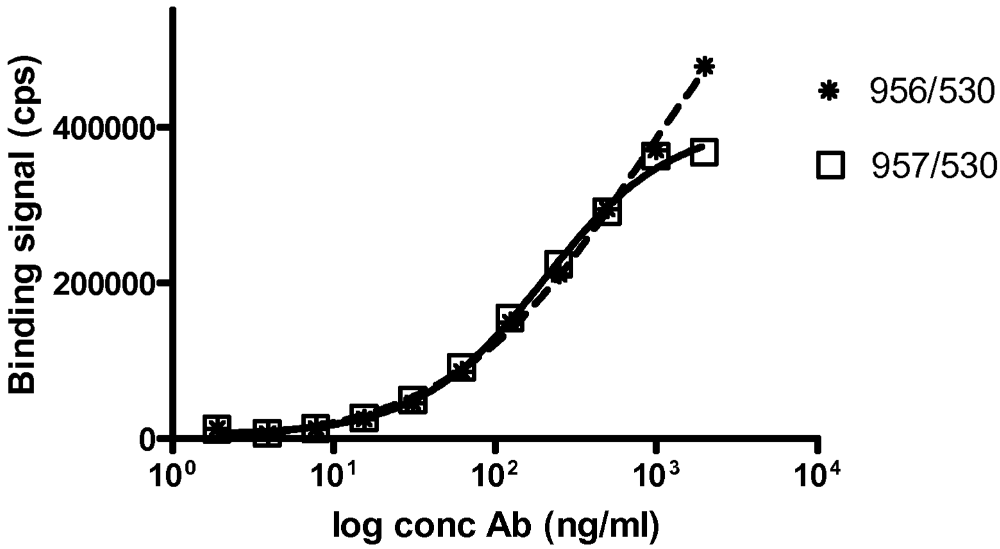

Binding signal (cps)
400000
200000
0
10^0
10^1
10^2
10^3
10^4
log conc Ab (ng/ml)
956/530
957/530


B

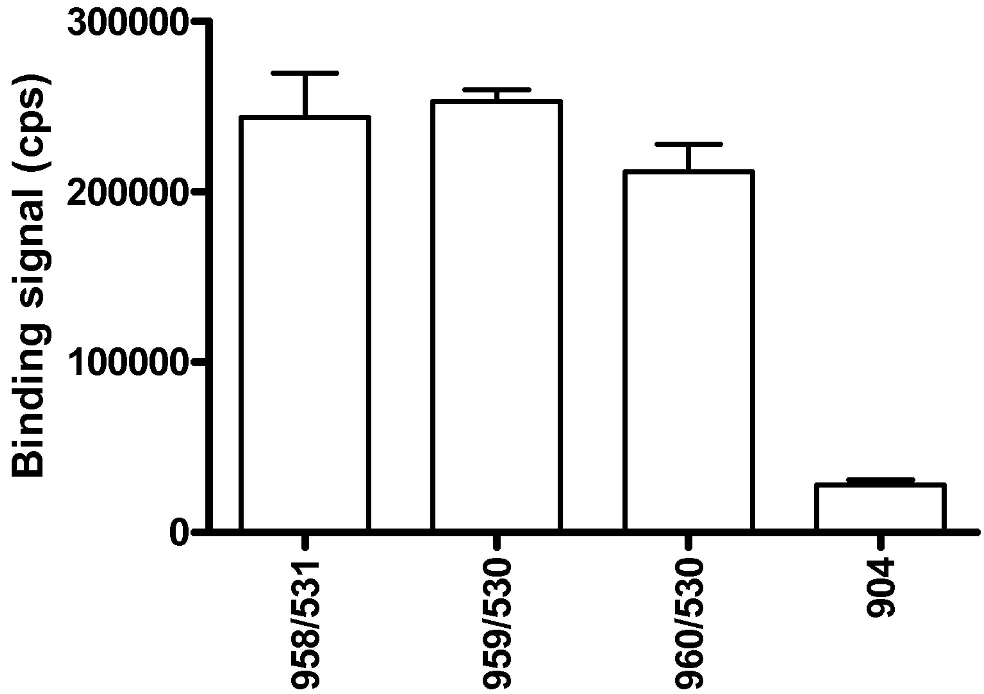

Binding signal (cps)
300000
200000
100000
0
958/531
959/530
960/530
904

FIGURE 7

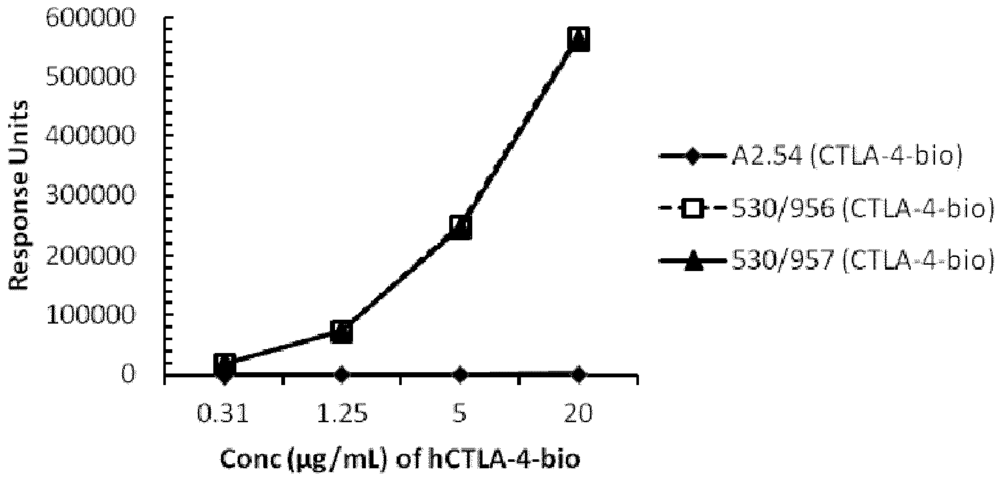
600000
500000
400000
300000
200000
100000
0
Response Units
0.31
1.25
5
20
Conc (µg/mL) of hCTLA-4-bio
A2.54 (CTLA-4-bio)
530/956 (CTLA-4-bio)
530/957 (CTLA-4-bio)

FIGURE 8

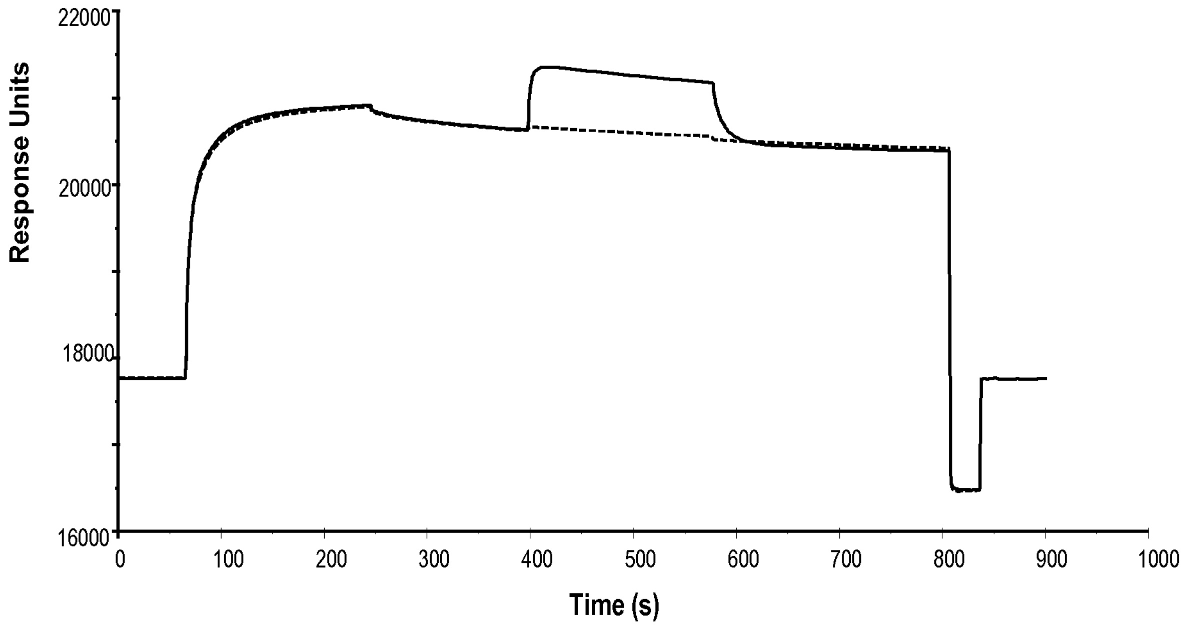
Response Units
22000
20000
18000
16000
0
100
200
300
400
500
600
700
800
900
1000
Time (s)

FIGURE 9

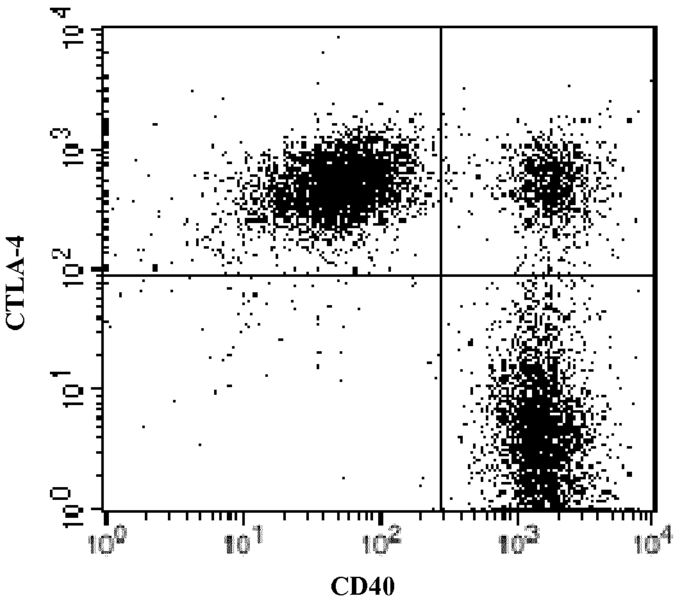

FIGURE 10

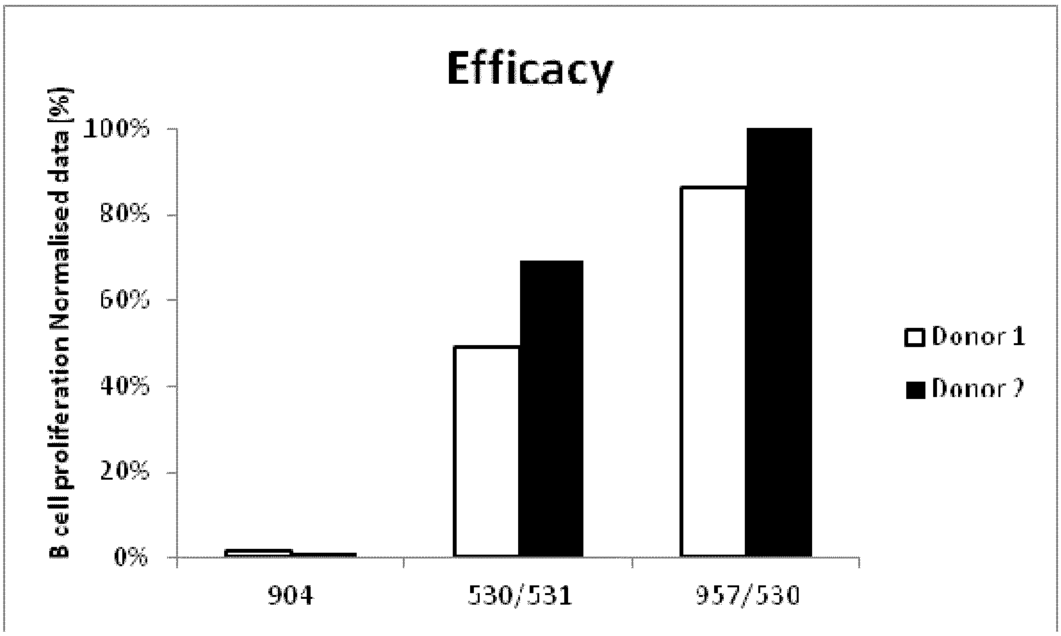
Efficacy
B cell proliferation Normalised data [%]
100%
80%
60%
40%
20%
0%
904
530/531
957/530
Donor 1
Donor 2

FIGURE 11

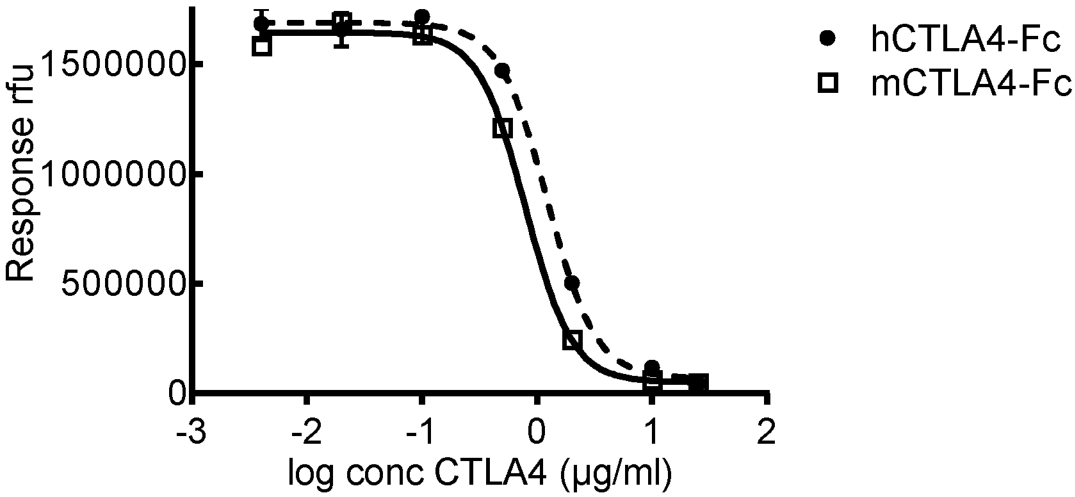
Response rfu
1500000
1000000
500000
0
-3
-2
-1
0
1
2
log conc CTLA4 (µg/ml)
hCTLA4-Fc
mCTLA4-Fc

FIGURE 12

A

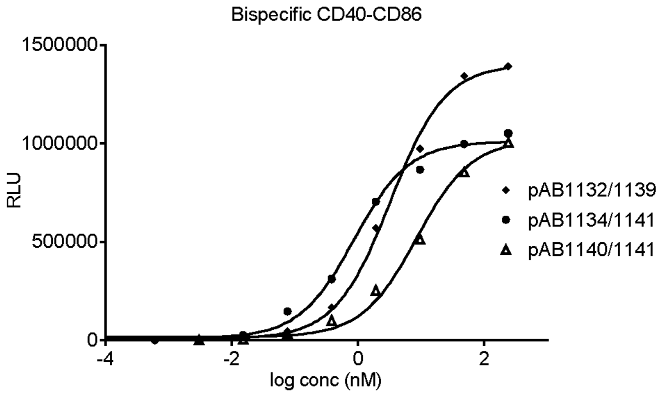


B

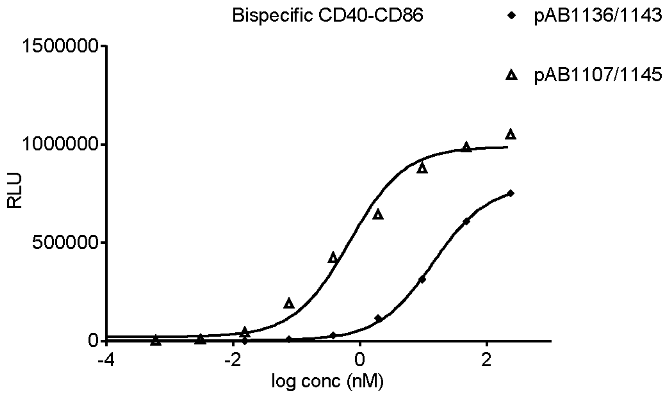


C

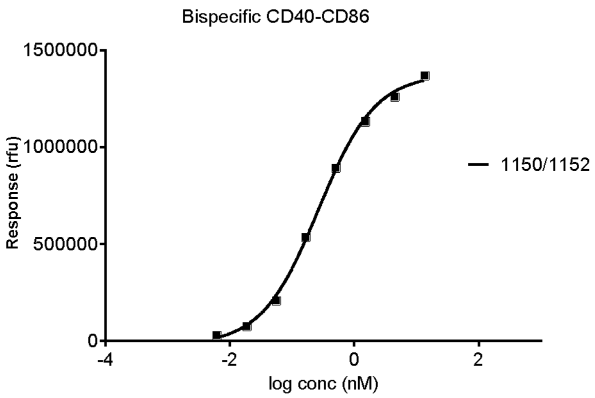

FIGURE 13

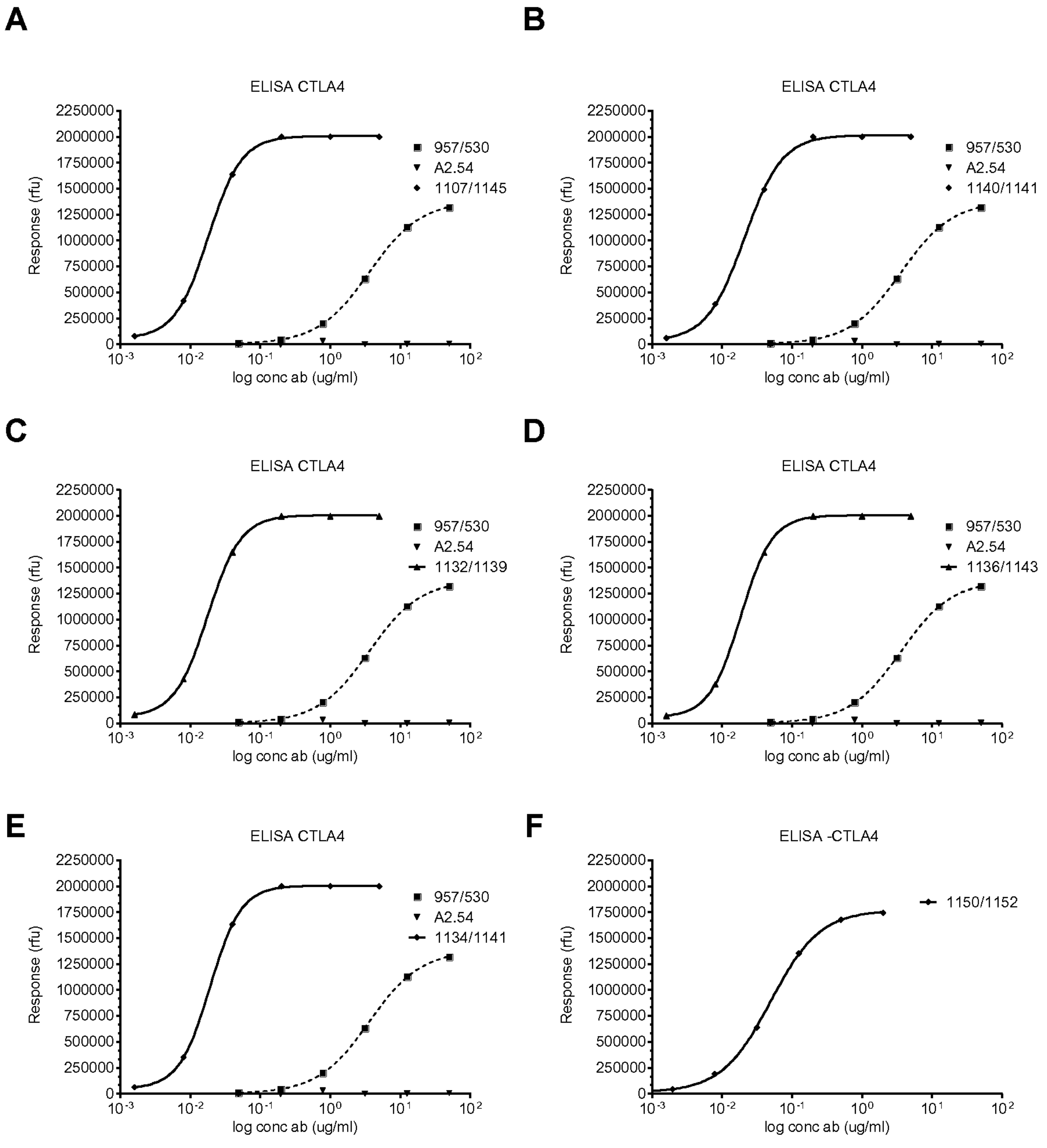
A
B
C
D
E
F
ELISA CTLA4
ELISA -CTLA4
Response (rfu)
log conc ab (ug/ml)
957/530
A2.54
1107/1145
1140/1141
1132/1139
1136/1143
1134/1141
1150/1152

FIGURE 14

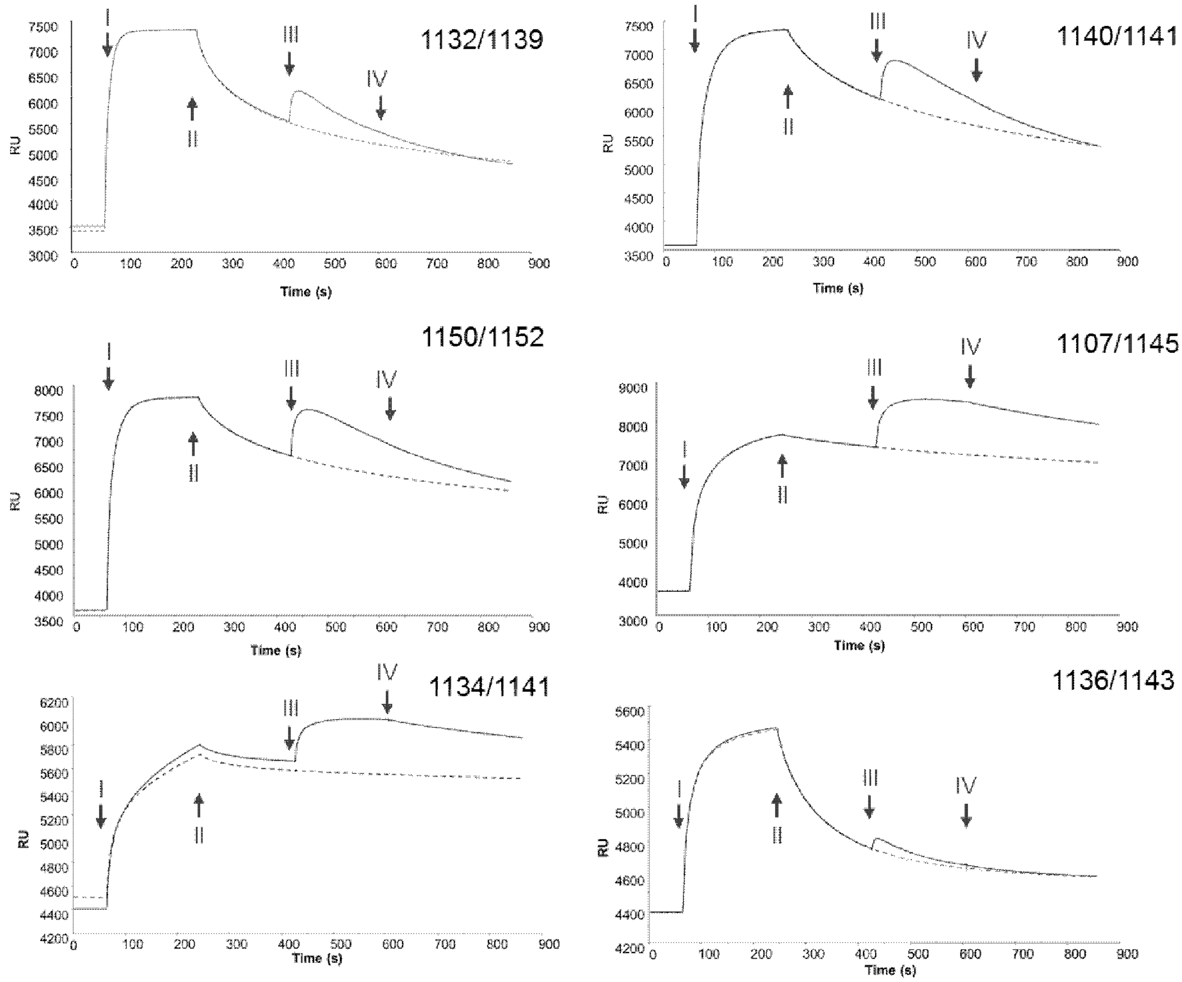
1132/1139
1140/1141
1150/1152
1107/1145
1134/1141
1136/1143
I
II
III
IV
RU
Time (s)

FIGURE 15

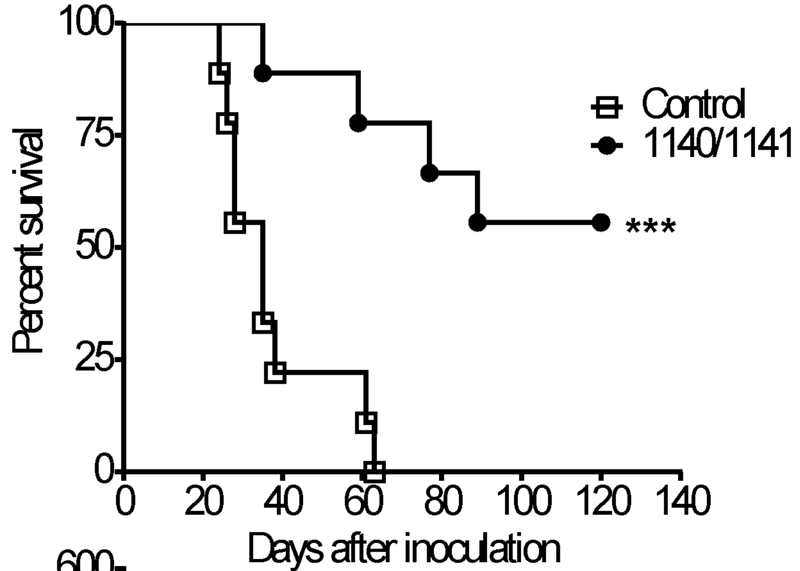

100
75
50
25
0
Percent survival
0 20 40 60 80 100 120 140
Days after inoculation
Control
1140/1141
***


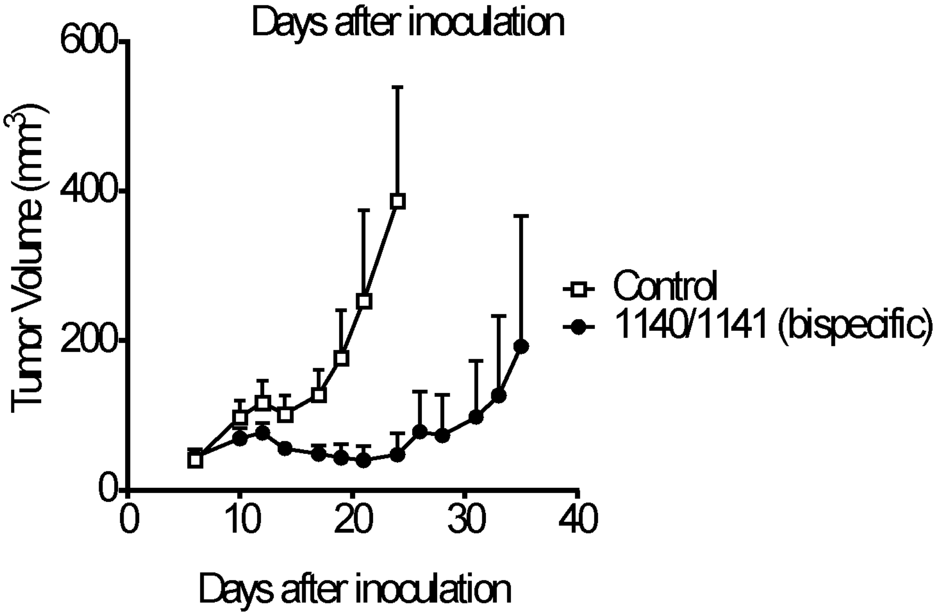

600
400
200
0
Tumor Volume (mm3)
0 10 20 30 40
Days after inoculation
Control
1140/1141 (bispecific)

FIGURE 16

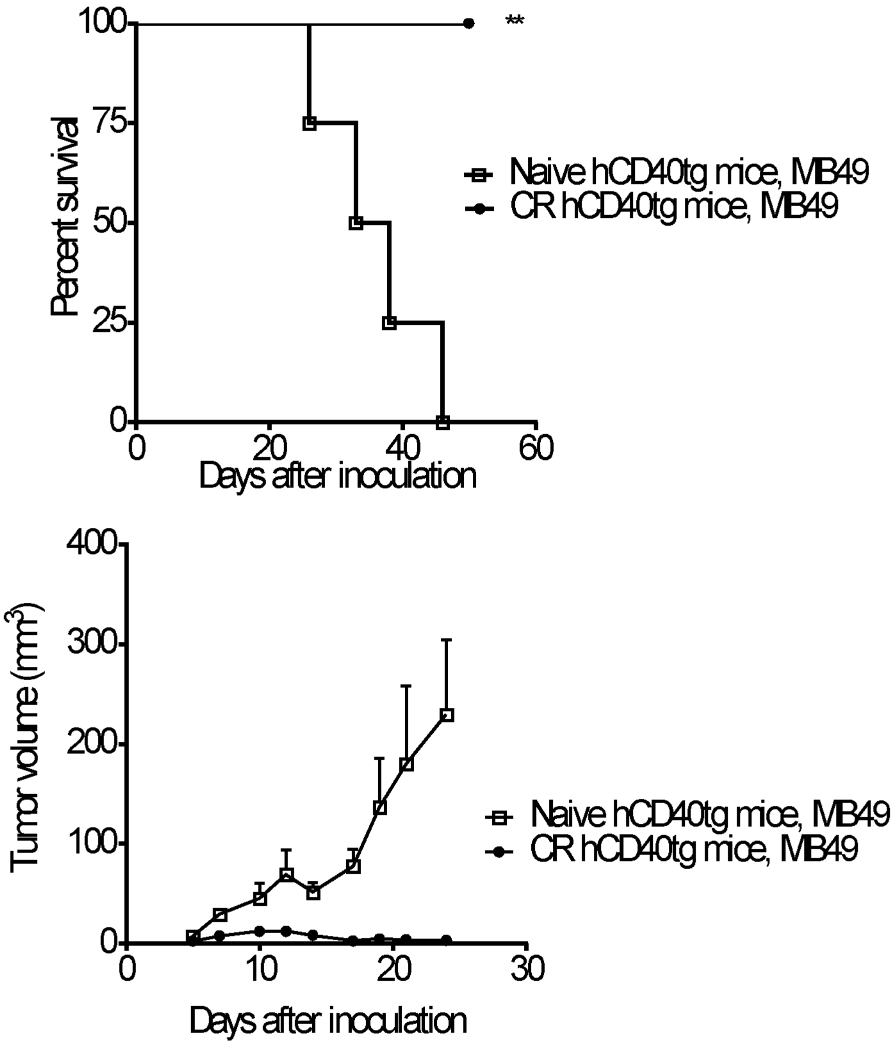
100
75
50
25
0
Percent survival
**
Naive hCD40tg mice, MB49
CR hCD40tg mice, MB49
0
20
40
60
Days after inoculation
400
300
200
100
0
Tumor volume (mm3)
Naive hCD40tg mice, MB49
CR hCD40tg mice, MB49
0
10
20
30
Days after inoculation

FIGURE 17

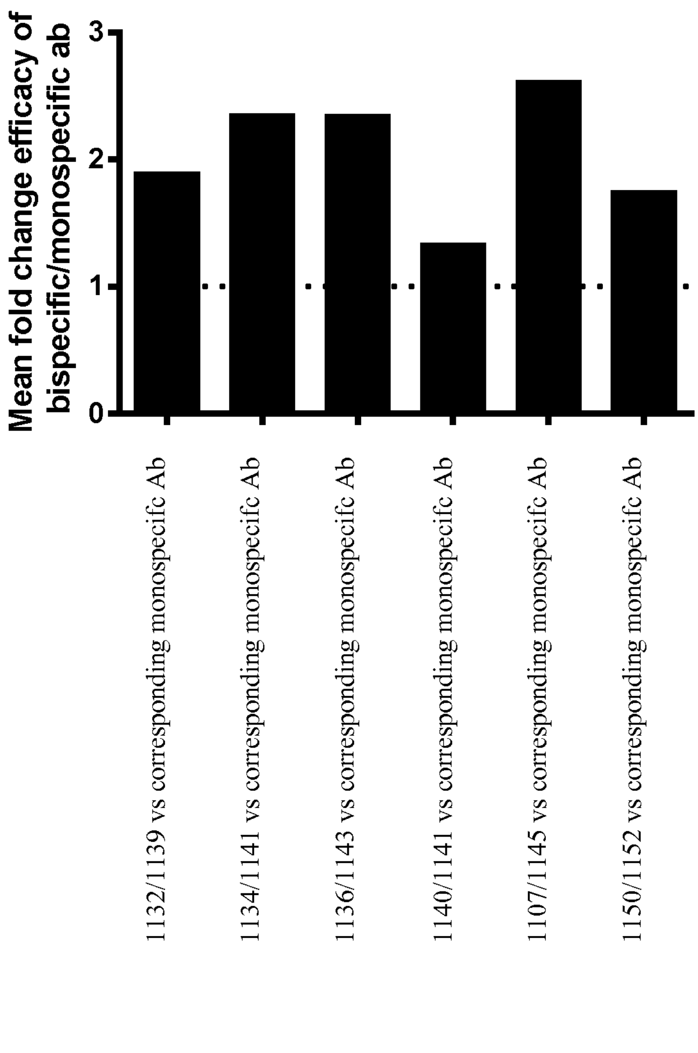
Mean fold change efficacy of bispecific/monospecific ab
3
2
1
0
1132/1139 vs corresponding monospecifc Ab
1134/1141 vs corresponding monospecifc Ab
1136/1143 vs corresponding monospecifc Ab
1140/1141 vs corresponding monospecifc Ab
1107/1145 vs corresponding monospecifc Ab
1150/1152 vs corresponding monospecifc Ab

FIGURE 18

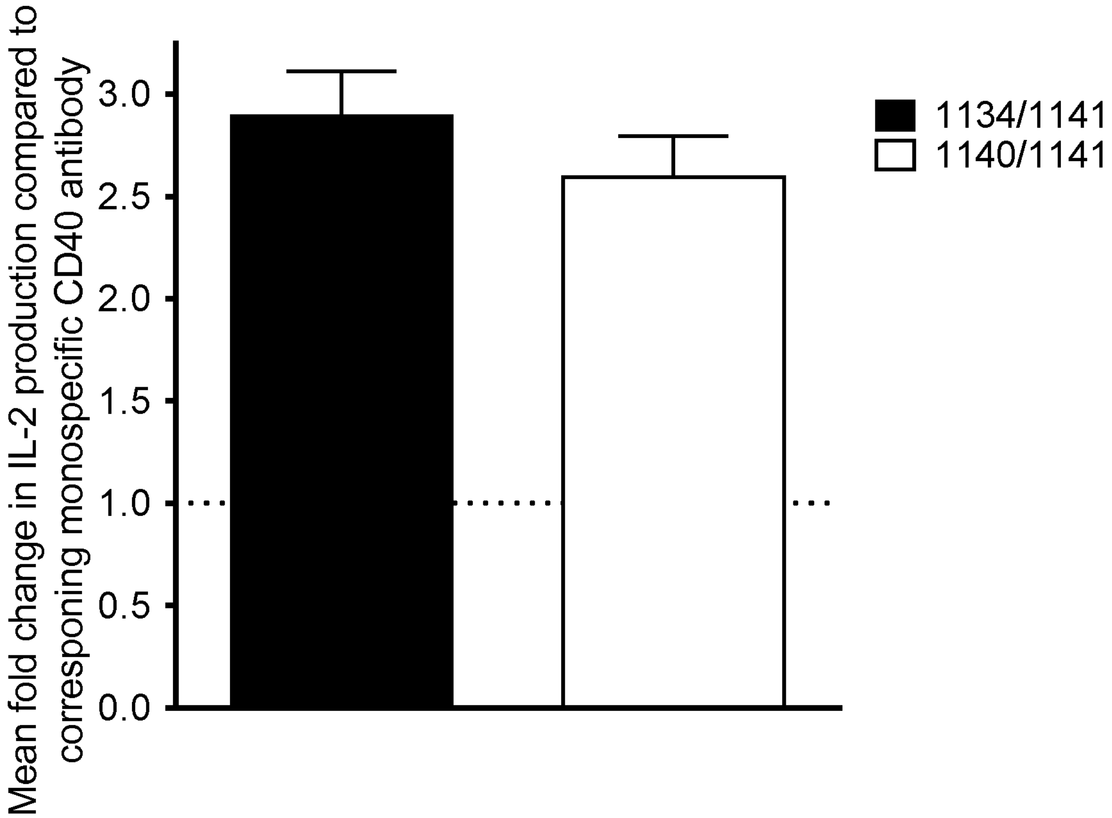
Mean fold change in IL-2 production compared to
corresponing monospecific CD40 antibody
3.0
2.5
2.0
1.5
1.0
0.5
0.0
1134/1141
1140/1141

FIGURE 19

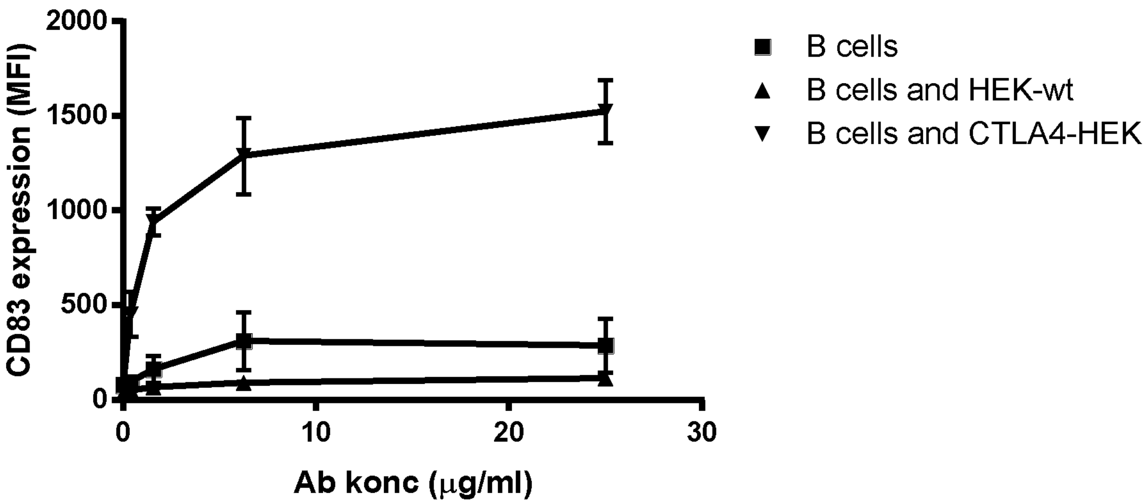
2000
1500
1000
500
0
CD83 expression (MFI)
0
10
20
30
Ab konc (μg/ml)
B cells
B cells and HEK-wt
B cells and CTLA4-HEK

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011061487 A
- US 20080233122 A
- WO 2002048351 A
- WO 200309734 A
- WO 2007057682 A
- BR 100369
- GB 1311487 A


### Non-patent literature cited in the description


- **KIRKWOOD et al.** *CA Cancer J Clin 2012,* 2012, vol. 62, 309-335
- **PEACH et al.** *Journal of Biological Chemistry,* 1995, vol. 270 (36), 21181-21187
- **SCHONBECK et al.** *Cell Mol Life Sci.,* 2001, vol. 58, 40-43
- **VAN KOOTEN et al.** *J. Leuk., Biol.,* 2000, vol. 67, 2-17
- **CACECI et al.** *Byte,* 1984, vol. 9, 340-362
- **WONG ; LOHMAN.** *Proc. Natl. Acad. Sci. USA,* 1993, vol. 90, 5428-5432
- **BAUMINGER ; WILCHEK.** *Methods Enzymol.,* 1980, vol. 70, 151-159
- **DEVEREUX et al.** *Nucleic Acids Research,* 1984, vol. 12, 387-395
- **ALTSCHUL S.F.** *J Mol Evol,* 1993, vol. 36, 290-300
- **ALTSCHUL, S, F et al.** *J Mol Biol,* 1990, vol. 215, 403-10
- **HENIKOFF.** *Proc. Natl. Acad. Sci. USA,* 1992, vol. 89, 10915-10919
- **KARLIN ; ALTSCHUL.** *Proc. Natl. Acad. Sci. USA,* 1993, vol. 90, 5873-5787
- **SAMBROOK et al.** Molecular Cloning - a laboratory manual. Cold Spring Harbor Press, 1989
- **SCHWARTZ et al.** *Nature,* 2001, vol. 410 (6828), 604-608
- **PEACH et al.** *Journal of Biological Chemistry,* 1995, vol. 270, 21181-21187
- *Biochem. J.,* 1985, vol. 229, 281-286
- *Eur. J. Biochem.,* 1985, vol. 150, 1-5
- *J. Biol. Chem.,* 1986, vol. 261, 13-17
- *Mol. Biol. Evol.,* 1986, vol. 3, 99-108
- *Nucl. Acids Res.,* 1985, vol. 13, 3021-3030
- *Proc. Nat. Acad. Sci. (U. S.),* 1986, vol. 83, 4-8
- Biochemical Nomenclature and Related Documents. Portland Press, 1992, 122-126
- **ELLMARK et al.** *Immunology,* 2003, vol. 108, 452-457
- **LI et al.** *Science,* 2011
- **WHITE et al.** *J Immunol,* 2011

SEQUENCE LISTING

<110> ALLIGATOR BIOSCIENCE AB

<120> BISPECIFIC MOLECULES

<130> N400377WO

<150> GB 1311487.1
<151> 2013-06-27

<160> 121

<170> PatentIn version 3.5

<210> 1
<211> 137
<212> PRT
<213> Human

<400> 1

```
Met His Val Ala Gln Pro Ala Val Val Leu Ala Ser Ser Arg Gly Ile
1               5                   10                  15

Ala Ser Phe Val Cys Glu Tyr Ala Ser Pro Gly Lys Ala Thr Glu Val
            20                  25                  30

Arg Val Thr Val Leu Arg Gln Ala Asp Ser Gln Val Thr Glu Val Cys
        35                  40                  45

Ala Ala Thr Tyr Met Met Gly Asn Glu Leu Thr Phe Leu Asp Asp Ser
    50                  55                  60

Ile Cys Thr Gly Thr Ser Ser Gly Asn Gln Val Asn Leu Thr Ile Gln
65                  70                  75                  80

Gly Leu Arg Ala Met Asp Thr Gly Leu Tyr Ile Cys Lys Val Glu Leu
                85                  90                  95

Met Tyr Pro Pro Pro Tyr Tyr Leu Gly Ile Gly Asn Gly Thr Gln Ile
            100                 105                 110

Tyr Val Ile Ala Lys Glu Lys Lys Pro Ser Tyr Asn Arg Gly Leu Cys
        115                 120                 125

Glu Asn Ala Pro Asn Arg Ala Arg Met
    130                 135
```

<210> 2
<211> 220
<212> PRT
<213> Human

<400> 2

```
Met Leu Arg Leu Leu Leu Ala Leu Asn Leu Phe Pro Ser Ile Gln Val
1               5                   10                  15

Thr Gly Asn Lys Ile Leu Val Lys Gln Ser Pro Met Leu Val Ala Tyr
            20                  25                  30

Asp Asn Ala Val Asn Leu Ser Cys Lys Tyr Ser Tyr Asn Leu Phe Ser
        35                  40                  45

Arg Glu Phe Arg Ala Ser Leu His Lys Gly Leu Asp Ser Ala Val Glu
    50                  55                  60

Val Cys Val Val Tyr Gly Asn Tyr Ser Gln Gln Leu Gln Val Tyr Ser
65                  70                  75                  80

Lys Thr Gly Phe Asn Cys Asp Gly Lys Leu Gly Asn Glu Ser Val Thr
                85                  90                  95

Phe Tyr Leu Gln Asn Leu Tyr Val Asn Gln Thr Asp Ile Tyr Phe Cys
            100                 105                 110

Lys Ile Glu Val Met Tyr Pro Pro Pro Tyr Leu Asp Asn Glu Lys Ser
        115                 120                 125

Asn Gly Thr Ile Ile His Val Lys Gly Lys His Leu Cys Pro Ser Pro
    130                 135                 140

Leu Phe Pro Gly Pro Ser Lys Pro Phe Trp Val Leu Val Val Val Gly
145                 150                 155                 160

Gly Val Leu Ala Cys Tyr Ser Leu Leu Val Thr Val Ala Phe Ile Ile
                165                 170                 175

Phe Trp Val Arg Ser Lys Arg Ser Arg Leu Leu His Ser Asp Tyr Met
            180                 185                 190

Asn Met Thr Pro Arg Arg Pro Gly Pro Thr Arg Lys His Tyr Gln Pro
        195                 200                 205

Tyr Ala Pro Pro Arg Asp Phe Ala Ala Tyr Arg Ser
    210                 215                 220
```

<210> 3
<211> 111
<212> **PRT**
<213> Human

<400> 3

```
Ala Pro Leu Lys Ile Gln Ala Tyr Phe Asn Glu Thr Ala Asp Leu Pro
1               5                   10                  15

Cys Gln Phe Ala Asn Ser Gln Asn Gln Ser Leu Ser Glu Leu Val Val
            20                  25                  30

Phe Trp Gln Asp Gln Glu Asn Leu Val Leu Asn Glu Val Tyr Leu Gly
        35                  40                  45

Lys Glu Lys Phe Asp Ser Val His Ser Lys Tyr Met Gly Arg Thr Ser
    50                  55                  60

Phe Asp Ser Asp Ser Trp Thr Leu Arg Leu His Asn Leu Gln Ile Lys
65                  70                  75                  80

Asp Lys Gly Leu Tyr Gln Cys Ile Ile His His Lys Lys Pro Thr Gly
                85                  90                  95

Met Ile Arg Ile His Gln Met Asn Ser Glu Leu Ser Val Leu Ala
            100                 105                 110
```

<210> 4
<211> 247
<212> PRT
<213> Human

<400> 4

```
Met Asp Pro Gln Cys Thr Met Gly Leu Ser Asn Ile Leu Phe Val Met
1               5                   10                  15

Ala Phe Leu Leu Ser Gly Ala Ala Pro Leu Lys Ile Gln Ala Tyr Phe
            20                  25                  30

Asn Glu Thr Ala Asp Leu Pro Cys Gln Phe Ala Asn Ser Gln Asn Gln
        35                  40                  45

Ser Leu Ser Glu Leu Val Val Phe Trp Gln Asp Gln Glu Asn Leu Val
    50                  55                  60

Leu Asn Glu Val Tyr Leu Gly Lys Glu Lys Phe Asp Ser Val His Ser
65                  70                  75                  80

Lys Tyr Met Gly Arg Thr Ser Phe Asp Ser Asp Ser Trp Thr Leu Arg
                85                  90                  95

Leu His Asn Leu Gln Ile Lys Asp Lys Gly Leu Tyr Gln Cys Ile Ile
            100                 105                 110
```

His His Lys Lys Pro Thr Gly Met Ile Arg Ile His Gln Met Asn Ser
115 120 125

Glu Leu Ser Val Leu Ala Asn Phe Ser Gln Pro Glu Ile Val Pro Ile
130 135 140

Ser Asn Ile Thr Glu Asn Val Tyr Ile Asn Leu Thr Cys Ser Ser Ile
145 150 155 160

His Gly Tyr Pro Glu Pro Lys Lys Met Ser Val Leu Leu Arg Thr Lys
165 170 175

Asn Ser Thr Ile Glu Tyr Asp Gly Ile Met Gln Lys Ser Gln Asp Asn
180 185 190

Val Thr Glu Leu Tyr Asp Val Ser Ile Ser Leu Ser Val Ser Phe Pro
195 200 205

Asp Val Thr Ser Asn Met Thr Ile Phe Cys Ile Leu Glu Thr Asp Lys
210 215 220

Thr Arg Leu Leu Ser Ser Pro Phe Ser Ile Glu Leu Glu Asp Pro Gln
225 230 235 240

Pro Pro Pro Asp His Ile Pro
245

<210> 5
<211> 111
<212> PRT
<213> Human

<400> 5

Ala Pro Leu Lys Ile Gln Ala Tyr Phe Asn Glu Thr Ala Asp Leu Pro
1 5 10 15

Cys Gln Phe Ala Asn Ser Gln Asn Gln Ser Leu Ser Glu Leu Val Val
20 25 30

Phe Trp Gln Asp Gln Glu Asn Leu Val Leu Asn Glu Val Tyr Leu Gly
35 40 45

Lys Glu Lys Phe Asp Ser Val Ala Ser Lys Tyr Met Gly Arg Thr Ser
50 55 60

Phe Asp Ser Asp Ser Trp Thr Leu Arg Leu His Asn Leu Gln Ile Lys
65 70 75 80

Asp Lys Gly Leu Tyr Gln Cys Ile Ile His His Lys Lys Pro Thr Gly
85 90 95

Met Ile Arg Ile His Gln Met Asn Ser Glu Leu Ser Val Leu Ala
100 105 110

<210> 6
<211> 109
<212> PRT
<213> Artificial

<220>
<223> Sequence of clone number 900

<400> 6

Leu Lys Ile Gln Ala Tyr Phe Asn Glu Thr Ala Asp Leu Pro Cys Gln
1 5 10 15

Phe Ala Asn Ser Gln Asn Gln Ser Leu Ser Glu Leu Val Val Phe Trp
20 25 30

Gln Asp Gln Glu Asn Leu Val Leu Asn Glu Val Tyr Leu Gly Lys Glu
35 40 45

Lys Phe Asp Ser Val Asp Ser Lys Tyr Met Gly Arg Thr Ser Phe Asp
50 55 60

Ser Asp Ser Trp Thr Leu Arg Leu His Asn Leu Gln Ile Lys Asp Lys
65 70 75 80

Gly Ile Tyr Gln Cys Val Ile His His Lys Lys Pro Ser Gly Leu Val
85 90 95

Lys Ile His Glu Met Asn Ser Glu Leu Ser Val Leu Ala
100 105

<210> 7
<211> 109
<212> PRT
<213> Artificial

<220>
<223> Sequence of clone number 901

<400> 7

Leu Lys Ile Gln Ala Tyr Phe Asn Glu Thr Ala Asp Leu Pro Cys Gln
1 5 10 15

Phe Ala Asn Ser Gln Asn Leu Thr Leu Ser Glu Leu Val Val Phe Trp

```
                20                  25                  30

Gln Asp Gln Glu Asn Leu Val Leu Asn Glu Val Tyr Leu Gly Lys Glu
        35                  40                  45

Lys Phe Asp Ser Val His Ser Lys Tyr Met Gly Arg Thr Ser Phe Asp
    50                  55                  60

Ser Asp Ser Trp Thr Leu Arg Leu His Asn Leu Gln Ile Lys Asp Lys
65                  70                  75                  80

Gly Ile Tyr Gln Cys Val Ile His His Lys Lys Pro Thr Gly Met Ile
                85                  90                  95

Lys Ile His Glu Met Asn Ser Glu Leu Ser Val Leu Thr
            100                 105
```

<210> 8
<211> 109
<212> PRT
<213> Artificial

<220>
<223> Sequence of clone number 904

<400> 8

```
Leu Lys Ile Gln Ala Tyr Phe Asn Glu Thr Ala Asp Leu Pro Cys Gln
1               5                   10                  15

Phe Ala Asn Ser Gln Asn Gln Ser Leu Ser Glu Leu Ile Val Phe Trp
            20                  25                  30

Gln Asp Gln Glu Asn Leu Val Leu Asn Glu Val Tyr Leu Gly Lys Glu
        35                  40                  45

Arg Phe Asp Ala Val Asp Ser Lys Tyr Met Gly Arg Thr Ser Phe Asp
    50                  55                  60

Ser Asp Ser Trp Thr Leu Arg Leu His Asn Leu Gln Ile Lys Asp Lys
65                  70                  75                  80

Gly Ile Tyr Gln Cys Ile Ile His His Lys Lys Pro Ser Gly Met Val
                85                  90                  95

Lys Ile His Gln Met Asp Ser Glu Leu Ser Val Leu Ala
            100                 105
```

<210> 9

<211> 109
<212> PRT
<213> Artificial

<220>
<223> Sequence of clone number number 906

<400> 9

```
Leu Lys Ile Gln Ala Tyr Ile Asn Glu Thr Ala Asp Leu Pro Cys Gln
1               5                   10                  15

Phe Ala Asn Ser Gln Asn Leu Ser Leu Ser Glu Leu Val Val Phe Trp
            20                  25                  30

Gln Asp Gln Glu Asn Leu Val Leu Asn Glu Val Tyr Leu Gly Lys Glu
        35                  40                  45

Arg Phe Asp Ser Val Asp Ser Lys Tyr Met Gly Arg Thr Ser Phe Asp
    50                  55                  60

Ser Asp Ser Trp Thr Leu Arg Leu His Asn Leu Gln Ile Lys Asp Lys
65                  70                  75                  80

Gly Phe Tyr Gln Cys Ile Ile His His Lys Lys Pro Thr Gly Leu Val
                85                  90                  95

Lys Ile His Glu Met Asn Ser Glu Leu Ser Val Leu Ala
            100                 105
```

<210> 10
<211> 109
<212> PRT
<213> Artificial

<220>
<223> Sequence of clone number 907

<400> 10

```
Leu Lys Ile Gln Ala Tyr Phe Asn Glu Thr Ala Asp Leu Pro Cys Gln
1               5                   10                  15

Phe Ala Asn Ser Gln Asn Gln Ser Leu Ser Glu Leu Val Val Phe Trp
            20                  25                  30

Gln Asp Gln Glu Asn Leu Val Leu Asn Glu Val Tyr Leu Gly Lys Glu
        35                  40                  45

Lys Phe Asp Ser Val His Ser Lys Tyr Met Gly Arg Thr Ser Phe Asp
    50                  55                  60

Ser Asp Ser Trp Thr Leu Arg Leu His Asn Leu Gln Ile Lys Asp Lys

65                  70                  75                  80

Gly Leu Tyr Gln Cys Ile Ile His His Lys Lys Pro Thr Gly Met Ile
                85                  90                  95

Lys Ile His Glu Met Asn Ser Glu Leu Ser Val Leu Ala
            100                 105
```

<210> 11
<211> 109
<212> PRT
<213> Artificial

<220>
<223> Sequence of clone number 908

<400> 11

```
Leu Lys Ile Gln Ala Tyr Phe Asn Glu Thr Ala Asp Leu Pro Cys Gln
1               5                   10                  15

Phe Ala Asn Ser Gln Asn Gln Ser Leu Ser Glu Leu Val Val Phe Trp
            20                  25                  30

Gln Asp Gln Glu Asn Leu Val Leu Asn Glu Val Tyr Leu Gly Lys Glu
        35                  40                  45

Lys Phe Asp Ser Val His Ser Lys Tyr Met Gly Arg Thr Ser Phe Asp
    50                  55                  60

Ser Asp Ser Trp Thr Leu Arg Leu His Asn Leu Gln Ile Lys Asp Lys
65                  70                  75                  80

Gly Ile Tyr Gln Cys Ile Ile His His Lys Lys Pro Thr Gly Met Val
                85                  90                  95

Lys Ile His Glu Met Asn Ser Glu Leu Ser Val Leu Ala
            100                 105
```

<210> 12
<211> 109
<212> PRT
<213> Artificial

<220>
<223> Sequence of clone number 910

<400> 12

```
Leu Lys Ile Gln Ala Tyr Phe Asn Glu Thr Ala Asp Leu Pro Cys Gln
1               5                   10                  15
```

```
Phe Ala Asn Ser Gln Asn Gln Ser Leu Ser Glu Leu Val Val Phe Trp
            20                  25                  30

Gln Asp Gln Glu Asn Leu Val Leu Asn Glu Val Tyr Leu Gly Lys Glu
        35                  40                  45

Lys Phe Asp Ser Val Asp Ser Lys Tyr Met Gly Arg Thr Ser Phe Asp
    50                  55                  60

Ser Asp Ser Trp Thr Leu Arg Leu His Asn Leu Gln Ile Lys Asp Lys
65                  70                  75                  80

Gly Ile Tyr Gln Cys Ile Ile His His Lys Lys Pro Thr Gly Met Val
                85                  90                  95

Lys Ile His Glu Met Asn Ser Glu Leu Ser Val Leu Ala
            100                 105
```

<210> 13
<211> 109
<212> PRT
<213> Artificial

<220>
<223> Sequence of clone number 915

<400> 13

```
Leu Lys Ile Gln Ala Tyr Phe Asn Glu Thr Ala Asp Leu Pro Cys Gln
1               5                   10                  15

Phe Ala Asn Ser Gln Asn Gln Ser Leu Ser Glu Leu Val Val Phe Trp
            20                  25                  30

Gln Asp Gln Glu Asn Leu Ile Leu Asn Glu Val Tyr Leu Gly Lys Glu
        35                  40                  45

Lys Phe Asp Ser Val Asp Ser Lys Tyr Met Gly Arg Thr Ser Phe Asp
    50                  55                  60

Ser Asp Ser Trp Thr Leu Arg Leu His Asn Leu Gln Ile Lys Asp Lys
65                  70                  75                  80

Gly Phe Tyr Gln Cys Ile Ile His His Lys Lys Pro Ser Gly Leu Ile
                85                  90                  95

Lys Ile His Gln Met Asp Ser Glu Leu Ser Val Leu Ala
            100                 105
```

<210> 14
<211> 109
<212> PRT
<213> Artificial

<220>
<223> Sequence of clone number 938

<400> 14

```
Leu Lys Ile Gln Ala Tyr Phe Asn Glu Thr Ala Asp Leu Pro Cys Gln
1               5                   10                  15

Phe Ala Asn Ser Gln Asn Gln Ser Leu Ser Glu Leu Val Val Phe Trp
            20                  25                  30

Gln Asp Gln Glu Asn Leu Ile Leu Asn Glu Val Tyr Leu Gly Lys Glu
        35                  40                  45

Lys Phe Asp Ser Val His Ser Lys Tyr Met Gly Arg Thr Ser Phe Asp
    50                  55                  60

Ser Asp Ser Trp Thr Leu Arg Leu His Asn Leu Gln Ile Lys Asp Lys
65                  70                  75                  80

Gly Ile Tyr Gln Cys Ile Ile His His Lys Lys Pro Thr Gly Met Val
                85                  90                  95

Lys Ile His Gln Met Asn Ser Glu Leu Ser Val Leu Ala
            100                 105
```

<210> 15
<211> 111
<212> PRT
<213> Artificial

<220>
<223> Sequence of clone number 1038

<400> 15

```
Ala Pro Leu Lys Ile Gln Ala Tyr Phe Asn Glu Thr Ala Asp Leu Pro
1               5                   10                  15

Cys Gln Phe Ala Asn Ser Gln Asn Leu Ser Leu Ser Glu Leu Val Val
            20                  25                  30

Phe Trp Gln Asp Gln Glu Asn Leu Val Leu Asn Glu Val Tyr Leu Gly
        35                  40                  45

Lys Glu Lys Phe Asp Ser Val Asp Ser Lys Tyr Met Gly Arg Thr Ser
    50                  55                  60

Phe Asp Ser Asp Ser Trp Thr Leu Arg Leu His Asn Leu Gln Ile Lys
65                  70                  75                  80

Asp Lys Gly Ile Tyr Gln Cys Ile Ile His His Lys Lys Pro Thr Gly
                85                  90                  95

Met Val Lys Ile His Glu Met Asn Ser Glu Leu Ser Val Leu Ala
            100                 105                 110
```

<210> 16
<211> 111
<212> PRT
<213> Artificial

<220>
<223> Sequence of clone number 1039

<400> 16

Ala Pro Leu Lys Ile Gln Ala Tyr Phe Asn Glu Thr Ala Asp Leu Pro
1 5 10 15

Cys Gln Phe Ala Asn Ser Gln Asn Leu Ser Leu Ser Glu Leu Val Val
20 25 30

Phe Trp Gln Asp Gln Glu Asn Leu Val Leu Asn Glu Val Tyr Leu Gly
35 40 45

Lys Glu Lys Phe Asp Ser Val Ser Ser Lys Tyr Met Gly Arg Thr Ser
50 55 60

Phe Asp Ser Asp Ser Trp Thr Leu Arg Leu His Asn Leu Gln Ile Lys
65 70 75 80

Asp Lys Gly Ile Tyr Gln Cys Ile Ile His His Lys Lys Pro Ser Gly
85 90 95

Met Val Lys Ile His Gln Met Asp Ser Glu Leu Ser Val Leu Ala
100 105 110

<210> 17
<211> 111
<212> PRT
<213> Artificial

<220>
<223> Sequence of clone number 1040

<400> 17

Ala Pro Leu Lys Ile Gln Ala Tyr Phe Asn Glu Thr Ala Asp Leu Pro
1 5 10 15

```
Cys Gln Phe Ala Asn Ser Gln Asn Leu Ser Leu Ser Glu Leu Val Val
            20                  25                  30

Phe Trp Gln Asp Gln Glu Asn Leu Val Leu Asn Glu Val Tyr Leu Gly
        35                  40                  45

Lys Glu Arg Phe Asp Ser Val Asp Ser Lys Tyr Met Gly Arg Thr Ser
    50                  55                  60

Phe Asp Ser Asp Ser Trp Thr Leu Arg Leu His Asn Leu Gln Ile Lys
65                  70                  75                  80

Asp Lys Gly Arg Tyr Gln Cys Ile Ile His His Lys Lys Pro Thr Gly
                85                  90                  95

Met Ile Asn Ile His Gln Met Asn Ser Glu Leu Ser Val Leu Ala
            100                 105                 110
```

<210> 18
<211> 111
<212> PRT
<213> Artificial

<220>
<223> Sequence of clone number 1041

<400> 18

```
Ala Pro Leu Lys Ile Gln Ala Tyr Leu Asn Glu Thr Ala Asp Leu Pro
1               5                   10                  15

Cys Gln Phe Ala Asn Ser Gln Asn Leu Ser Leu Ser Glu Leu Val Val
            20                  25                  30

Phe Trp Gln Asp Gln Glu Asn Leu Val Leu Asn Glu Val Tyr Leu Gly
        35                  40                  45

Lys Glu Lys Phe Asp Ser Val Asp Ser Lys Tyr Met Gly Arg Thr Ser
    50                  55                  60

Phe Asp Ser Asp Ser Trp Thr Leu Arg Leu His Asn Leu Gln Ile Lys
65                  70                  75                  80

Asp Lys Gly Ile Tyr Gln Cys Ile Ile His His Lys Lys Pro Thr Gly
                85                  90                  95

Leu Val Lys Ile His Glu Met Asn Ser Glu Leu Ser Val Leu Ala
            100                 105                 110
```

<210> 19
<211> 111
<212> PRT
<213> Artificial

<220>
<223> Sequence of clone number 1042

<400> 19

```
Ala Pro Leu Lys Ile Gln Ala Tyr Phe Asn Glu Thr Ala Asp Leu Pro
1               5                   10                  15

Cys Gln Phe Ala Asn Ser Gln Asn Leu Ser Leu Ser Glu Leu Val Val
            20                  25                  30

Phe Trp Gln Asp Gln Glu Asn Leu Val Leu Asn Glu Val Tyr Leu Gly
        35                  40                  45

Lys Glu Ile Phe Asp Ser Val Ser Ser Lys Tyr Met Gly Arg Thr Ser
    50                  55                  60

Phe Asp Ser Asp Ser Trp Thr Leu Arg Leu His Asn Leu Gln Ile Lys
65                  70                  75                  80

Asp Lys Gly Ile Tyr Gln Cys Ile Ile His His Lys Lys Pro Ser Gly
                85                  90                  95

Met Val Lys Ile His Gln Met Asp Ser Glu Leu Ser Val Leu Ala
            100                 105                 110
```

<210> 20
<211> 111
<212> PRT
<213> Artificial

<220>
<223> Sequence of clone number 1043

<400> 20

```
Ala Pro Leu Lys Ile Gln Ala Tyr Phe Asn Glu Thr Ala Asp Leu Pro
1               5                   10                  15

Cys Gln Phe Ala Asn Ser Gln Asn Leu Ser Leu Ser Glu Leu Val Val
            20                  25                  30

Phe Trp Gln Asp Gln Glu Asn Leu Val Leu Asn Glu Val Tyr Leu Gly
        35                  40                  45

Lys Glu Lys Phe Asp Ser Val Asp Ser Lys Tyr Met Gly Arg Thr Ser
    50                  55                  60

Phe Asp Ser Asp Ser Trp Thr Leu Arg Leu His Asn Leu Gln Ile Lys
65                  70                  75                  80

Asp Lys Gly Ile Tyr Gln Cys Ile Ile His His Lys Lys Pro Thr Gly
                85                  90                  95

Met Ile Lys Ile His Glu Met Asn Ser Glu Leu Ser Val Leu Ala
            100                 105                 110
```

<210> 21
<211> 111
<212> PRT
<213> Artificial

<220>
<223> Sequence of clone number 1044

<400> 21

```
Ala Pro Leu Lys Ile Gln Ala Tyr Phe Asn Glu Thr Ala Asp Leu Pro
1               5                   10                  15

Cys Gln Phe Ala Asn Ser Gln Asn Leu Thr Leu Ser Glu Leu Val Val
            20                  25                  30

Phe Trp Gln Asp Gln Glu Asn Leu Val Leu Asn Glu Val Tyr Leu Gly
        35                  40                  45

Lys Glu Lys Phe Asp Ser Val Ser Ser Lys Tyr Met Gly Arg Thr Ser
    50                  55                  60

Phe Asp Ser Asp Ser Trp Thr Leu Arg Leu His Asn Leu Gln Ile Lys
65                  70                  75                  80

Asp Lys Gly Ile Tyr Gln Cys Ile Ile His His Lys Lys Pro Thr Gly
                85                  90                  95

Met Ile Lys Ile His Glu Met Ser Ser Glu Leu Ser Val Leu Ala
            100                 105                 110
```

<210> 22
<211> 111
<212> PRT
<213> Artificial

<220>
<223> Sequence of clone number 1045

<400> 22

```
Ala Pro Leu Lys Ile Gln Ala Tyr Phe Asn Glu Thr Ala Asp Leu Pro
1               5                   10                  15
```

Cys Gln Phe Ala Asn Ser Gln Asn Leu Thr Leu Ser Glu Leu Val Val
20 25 30

Phe Trp Gln Asp Gln Glu Asn Leu Val Leu Asn Glu Val Tyr Leu Gly
35 40 45

Lys Glu Lys Phe Asp Ser Val Asp Ser Lys Tyr Met Gly Arg Thr Ser
50 55 60

Phe Asp Ser Asp Ser Trp Thr Leu Arg Leu His Asn Leu Gln Ile Lys
65 70 75 80

Asp Lys Gly Leu Tyr Gln Cys Ile Ile His His Lys Lys Pro Thr Gly
85 90 95

Leu Val Lys Ile His Glu Met Asn Ser Glu Leu Ser Val Leu Ala
100 105 110

<210> 23
<211> 111
<212> PRT
<213> Artificial

<220>
<223> Sequence of clone number 1046

<400> 23

Ala Pro Leu Lys Ile Gln Ala Tyr Phe Asn Glu Thr Ala Asp Leu Pro
1 5 10 15

Cys Gln Phe Ala Asn Ser Gln Asn Gln Ser Leu Ser Glu Leu Val Val
20 25 30

Phe Trp Gln Asp Gln Glu Asn Leu Val Leu Asn Glu Val Tyr Leu Gly
35 40 45

Lys Glu Lys Phe Asp Ser Val Asp Ser Lys Tyr Met Gly Arg Thr Ser
50 55 60

Phe Asp Ser Asp Ser Trp Thr Leu Arg Leu His Asn Leu Gln Ile Glu
65 70 75 80

Asp Lys Gly Ile Tyr Gln Cys Ile Ile His His Lys Lys Pro Ser Gly
85 90 95

Met Val Lys Ile His Gln Met Asp Ser Glu Leu Ser Val Leu Ala
100 105 110

<210> 24
<211> 111
<212> PRT
<213> Artificial

<220>
<223> Sequence of clone number 1047

<400> 24

```
Ala Pro Leu Lys Ile Gln Ala Tyr Phe Asn Glu Thr Ala Asp Leu Pro
1               5                   10                  15

Cys Gln Phe Ala Asn Ser Gln Asn Leu Ser Leu Ser Glu Leu Val Val
            20                  25                  30

Phe Trp Gln Asp Gln Glu Asn Leu Val Leu Asn Glu Val Tyr Leu Gly
        35                  40                  45

Lys Glu Lys Phe Asp Ser Val Asp Ser Lys Tyr Met Gly Arg Thr Ser
    50                  55                  60

Phe Asp Ser Asp Ser Trp Thr Leu Arg Leu His Asn Leu Gln Ile Lys
65                  70                  75                  80

Asp Lys Gly Ile Tyr Gln Cys Ile Ile His His Lys Lys Pro Thr Gly
                85                  90                  95

Leu Val Lys Ile His Glu Met Asn Ser Glu Leu Ser Val Leu Ala
            100                 105                 110
```

<210> 25
<211> 327
<212> DNA
<213> Artificial

<220>
<223> Sequence of clone number 900

<400> 25

```
ctcaaaatcc aagcgtactt caacgaaact gcagacttac cgtgtcagtt tgccaattcg      60

cagaatcaaa gcctgagcga actggtggtt ttctggcagg atcaggagaa cctggttctg     120

aacgaagtct atctgggcaa agagaaattc gacagcgtgg acagcaagta tatgggccgc     180

accagctttg atagcgacag ctggaccctg cgtctgcaca atctgcaaat caaagataag     240

ggtatctacc agtgcgtgat ccaccataag aagccgagcg gtctggtgaa gattcacgag     300

atgaactccg agttgtctgt cctggcg                                         327
```

<210> 26
<211> 327
<212> DNA
<213> Artificial

<220>
<223> Sequence of clone number 901

<400> 26

```
ctcaaaatcc aagcgtactt caacgaaact gcagacttac cgtgtcagtt tgccaattcg      60
cagaatctga ccctgagcga actggtggtt ttctggcagg atcaggagaa cctggttctg     120
aacgaagtct atctgggcaa agagaaattc gacagcgtgc atagcaagta tatgggccgc     180
accagctttg atagcgacag ctggaccctg cgtctgcaca atctgcaaat caaagataag     240
ggtatctacc agtgcgtgat ccaccataag aagccgacgg gtatgattaa gattcacgag     300
atgaactccg agttgtctgt cctgacc                                         327
```

<210> 27
<211> 327
<212> DNA
<213> Artificial

<220>
<223> Sequence of clone number 904

<400> 27

```
ctcaaaatcc aagcgtactt caacgaaact gcagacttac cgtgtcagtt tgccaattcg      60
cagaatcaaa gcctgagcga actgatcgtt ttctggcagg atcaggagaa cctggttctg     120
aacgaagtct atctgggcaa agagcggttc gacgccgtgg acagcaagta tatgggccgc     180
accagctttg atagcgacag ctggaccctg cgtctgcaca atctgcaaat caaagataag     240
ggtatctacc agtgcattat ccaccataag aagccgagcg gtatggtgaa gattcaccaa     300
atggactccg agttgtctgt cctggcg                                         327
```

<210> 28
<211> 327
<212> DNA
<213> Artificial

<220>
<223> Sequence of clone number 906

<400> 28

ctcaaaatcc aagcgtacat caacgaaact gcagacttac cgtgtcagtt tgccaattcg 60
cagaatctga gcctgagcga actggtggtt ttctggcagg atcaggagaa cctggttctg 120
aacgaagtct atctgggcaa agagcggttc gacagcgtgg acagcaagta tatgggccgc 180
accagctttg atagcgacag ctggaccctg cgtctgcaca atctgcaaat caaagataag 240
ggtttctacc agtgcattat ccaccataag aagccgacgg gtctggtgaa gattcacgag 300
atgaactccg agttgtctgt cctggcg 327

<210> 29
<211> 327
<212> DNA
<213> Artificial

<220>
<223> Sequence of clone number 907

<400> 29

ctcaaaatcc aagcgtactt caacgaaact gcagacttac cgtgtcagtt tgccaattcg 60
cagaatcaaa gcctgagcga actggtggtt ttctggcagg atcaggagaa cctggttctg 120
aacgaagtct atctgggcaa agagaaattc gacagcgtgc atagcaagta tatgggccgc 180
accagctttg atagcgacag ctggaccctg cgtctgcaca atctgcaaat caaagataag 240
ggtctgtacc agtgcattat ccaccataag aagccgacgg gtatgattaa gattcacgag 300
atgaactccg agttgtctgt cctggcg 327

<210> 30
<211> 327
<212> DNA
<213> Artificial

<220>
<223> Sequence of clone number 908

<400> 30

ctcaaaatcc aagcgtactt caacgaaact gcagacttac cgtgtcagtt tgccaattcg 60
cagaatcaaa gcctgagcga actggtggtt ttctggcagg atcaggagaa cctggttctg 120
aacgaagtct atctgggcaa agagaaattc gacagcgtgc atagcaagta tatgggccgc 180
accagctttg atagcgacag ctggaccctg cgtctgcaca atctgcaaat caaagataag 240
ggtatctacc agtgcattat ccaccataag aagccgacgg gtatggtgaa gattcacgag 300
atgaactccg agttgtctgt cctggcg 327

<210> 31
<211> 327

<212> DNA
<213> Artificial

<220>
<223> Sequence of clone number 910

<400> 31

```
ctcaaaatcc aagcgtactt caacgaaact gcagacttac cgtgtcagtt tgccaattcg      60
cagaatcaaa gcctgagcga actggtggtt ttctggcagg atcaggagaa cctggttctg     120
aacgaagtct atctgggcaa agagaaattc gacagcgtgg acagcaagta tatgggccgc     180
accagctttg atagcgacag ctggaccctg cgtctgcaca atctgcaaat caaagataag     240
ggtatctacc agtgcattat ccaccataag aagccgacgg gtatggtgaa gattcacgag     300
atgaactccg agttgtctgt cctggcg                                         327
```

<210> 32
<211> 327
<212> DNA
<213> Artificial

<220>
<223> Sequence for clone number 915

<400> 32

```
ctcaaaatcc aagcgtactt caacgaaact gcagacttac cgtgtcagtt tgccaattcg      60
cagaatcaaa gcctgagcga actggtggtt ttctggcagg atcaggagaa cctgatcctg     120
aacgaagtct atctgggcaa agagaaattc gacagcgtgg acagcaagta tatgggccgc     180
accagctttg atagcgacag ctggaccctg cgtctgcaca atctgcaaat caaagataag     240
ggtttctacc agtgcattat ccaccataag aagccgagcg gtctgattaa gattcaccaa     300
atggactccg agttgtctgt cctggcg                                         327
```

<210> 33
<211> 327
<212> DNA
<213> Artificial

<220>
<223> Sequence of clone number 938

<400> 33

ctcaaaatcc aagcgtactt caacgaaact gcagacttac cgtgtcagtt tgccaattcg 60
cagaatctga gcctgagcga actggtggtt ttctggcagg atcaggagaa cctgatcctg 120
aacgaagtct atctgggcaa agagcggttc gacagcgtgc atagcaagta tatgggccgc 180
accagctttg atagcgacag ctggaccctg cgtctgcaca atctgcaaat caaagataag 240
ggtctgtacc agtgcattat ccaccataag aagccgagcg gtatggtgaa gattcacgag 300
atgaactccg agttgtctgt cctggcg 327

<210> 34
<211> 333
<212> DNA
<213> Artificial

<220>
<223> Sequence of clone number 1038

<400> 34

gcccccctca aaatccaagc gtacttcaac gaaactgcag acttaccgtg tcagtttgcc 60
aattcgcaga atctgagcct gagcgaactg gtggttttct ggcaggatca ggagaacctg 120
gttctgaacg aagtctatct gggcaaagag aaattcgaca gcgtggacag caagtatatg 180
ggccgcacca gctttgatag cgacagctgg accctgcgtc tgcacaatct gcaaatcaaa 240
gataagggta tctaccagtg cattatccac cataagaagc cgacgggtat ggtgaagatt 300
cacgagatga actccgagtt gtctgtcctg gcg 333

<210> 35
<211> 333
<212> DNA
<213> Artificial

<220>
<223> Sequence of clone number 1039

<400> 35

gcccccctca aaatccaagc gtacttcaac gaaactgcag acttaccgtg tcagtttgcc 60
aattcgcaga atctgagcct gagcgaactg gtggttttct ggcaggatca ggagaacctg 120
gttctgaacg aagtctatct gggcaaagag aaattcgaca gcgtgagtag caagtatatg 180
ggccgcacca gctttgatag cgacagctgg accctgcgtc tgcacaatct gcaaatcaaa 240
gataagggta tctaccagtg cattatccac cataagaagc cgagcggtat ggtgaagatt 300
caccaaatgg actccgagtt gtctgtcctg gcg 333

<210> 36
<211> 333

<212> DNA
<213> Artificial

<220>
<223> Sequence of clone number 1040

<400> 36

```
gcccccctca aaatccaagc gtacttcaac gaaactgcag acttaccgtg tcagtttgcc      60
aattcgcaga atctgagcct gagcgaactg gtggttttct ggcaggatca ggagaacctg     120
gttctgaacg aagtctatct gggcaaagag cggttcgaca gcgtggacag caagtatatg     180
ggccgcacca gctttgatag cgacagctgg accctgcgtc tgcacaatct gcaaatcaaa     240
gataagggta ggtaccagtg cattatccac cataagaagc cgacgggtat gattaatatt     300
caccaaatga actccgagtt gtctgtcctg gcg                                  333
```

<210> 37
<211> 333
<212> DNA
<213> Artificial

<220>
<223> Sequence of clone number 1041

<400> 37

```
gcccccctca aaatccaagc gtacctcaac gaaactgcag acttaccgtg tcagtttgcc      60
aattcgcaga atctgagcct gagcgaactg gtggttttct ggcaggatca ggagaacctg     120
gttctgaacg aagtctatct gggcaaagag aaattcgaca gcgtggacag caagtatatg     180
ggccgcacca gctttgatag cgacagctgg accctgcgtc tgcacaatct gcaaatcaaa     240
gataagggta tctaccagtg cattatccac cataagaagc cgacgggtct ggtgaagatt     300
cacgagatga actccgagtt gtctgtcctg gcg                                  333
```

<210> 38
<211> 333
<212> DNA
<213> Artificial

<220>
<223> Sequence of clone number 1042

<400> 38

gcccccctca aaatccaagc gtacttcaac gaaactgcag acttaccgtg tcagtttgcc 60

aattcgcaga atctgagcct gagcgaactg gtggttttct ggcaggatca ggagaacctg 120

gttctgaacg aagtctatct gggcaaagag attttcgaca gcgtgagtag caagtatatg 180

ggccgcacca gctttgatag tgacagctgg accctgcgtc tgcacaatct gcaaatcaaa 240

gataagggta tctaccagtg cattatccac cataagaagc cgagcggtat ggtgaagatt 300

caccaaatgg actccgagtt gtctgtcctg gcg 333

<210> 39
<211> 333
<212> DNA
<213> Artificial

<220>
<223> Sequence of clone number 1043

<400> 39

gcccccctca aaatccaagc gtacttcaac gaaactgcag acttaccgtg tcagtttgcc 60

aattcgcaga atctgagcct gagcgaactg gtggttttct ggcaggatca ggagaacctg 120

gttctgaacg aagtctatct gggcaaagag aaattcgaca gcgtggatag caagtatatg 180

ggccgcacca gctttgatag cgacagctgg accctgcgtc tgcacaatct gcaaatcaaa 240

gataagggta tctaccagtg cattatccac cataagaagc cgacgggtat gattaagatt 300

cacgagatga actccgagtt gtctgtcctg gcg 333

<210> 40
<211> 333
<212> DNA
<213> Artificial

<220>
<223> Sequence of clone number 1044

<400> 40

gcccccctca aaatccaagc gtacttcaac gaaactgcag acttaccgtg tcagtttgcc 60

aattcgcaga atctgaccct gagcgaactg gtggttttct ggcaggatca ggagaacctg 120

gttctgaacg aagtctatct gggcaaagag aaattcgaca gcgtgtctag caagtatatg 180

ggccgcacca gctttgatag cgacagctgg accctgcgtc tgcacaatct gcaaatcaaa 240

gataagggta tctaccagtg cattatccac cataagaagc cgacgggtat gattaagatt 300

cacgagatga gctccgagtt gtctgtcctg gcg 333

<210> 41
<211> 333

<212> DNA
<213> Artificial

<220>
<223> Sequence of clone number 1045

<400> 41

```
gcccccctca aaatccaagc gtacttcaac gaaactgcag acttaccgtg tcagtttgcc        60
aattcgcaga atctgaccct gagcgaactg gtggttttct ggcaggatca ggagaacctg       120
gttctgaacg aagtctatct gggcaaagag aaattcgaca gcgtggacag caagtatatg       180
ggccgcacca gctttgatag cgacagctgg accctgcgtc tgcacaatct gcaaatcaaa       240
gataagggtc tgtaccagtg cattatccac cataagaagc cgacgggtct ggtgaagatt       300
cacgagatga actccgagtt gtctgtcctg gcg                                    333
```

<210> 42
<211> 333
<212> DNA
<213> Artificial

<220>
<223> Sequence of clone number 1046

<400> 42

```
gcccccctca aaatccaagc gtacttcaac gaaactgcag acttaccgtg tcagtttgcc        60
aattcgcaga atcaaagcct gagcgaactg gtggttttct ggcaggatca ggagaacctg       120
gttctgaacg aagtctatct gggcaaagag aaattcgaca gcgtggacag caagtatatg       180
ggccgcacca gctttgatag cgacagctgg accctgcgtc tgcacaatct gcaaatcgaa       240
gataagggta tctaccagtg cattatccac cataagaagc cgagcggtat ggtgaagatt       300
caccaaatgg actccgagtt gtctgtcctg gcg                                    333
```

<210> 43
<211> 333
<212> DNA
<213> Artificial

<220>
<223> Sequence of clone number 1047

<400> 43

```
gcccccctca aaatccaagc gtacttcaac gaaactgcag acttaccgtg tcagtttgcc        60
aattcgcaga atctgagcct gagcgaactg gtggttttct ggcaggatca ggagaacctg       120
gttctgaacg aagtctatct gggcaaagag aaattcgaca gcgtggacag caagtatatg       180
ggccgcacca gctttgatag cgacagctgg accctgcgtc tgcacaatct gcaaatcaaa       240
gataagggta tctaccagtg cattatccac cataagaagc cgacgggtct ggtgaagatt       300
cacgagatga actccgagtt gtctgtcctg gcg                                   333
```

<210> 44
<211> 329
<212> PRT
<213> Human

<400> 44

```
Met Asp Pro Gln Cys Thr Met Gly Leu Ser Asn Ile Leu Phe Val Met
1               5                   10                  15

Ala Phe Leu Leu Ser Gly Ala Ala Pro Leu Lys Ile Gln Ala Tyr Phe
            20                  25                  30

Asn Glu Thr Ala Asp Leu Pro Cys Gln Phe Ala Asn Ser Gln Asn Gln
        35                  40                  45

Ser Leu Ser Glu Leu Val Val Phe Trp Gln Asp Gln Glu Asn Leu Val
    50                  55                  60

Leu Asn Glu Val Tyr Leu Gly Lys Glu Lys Phe Asp Ser Val His Ser
65                  70                  75                  80

Lys Tyr Met Gly Arg Thr Ser Phe Asp Ser Asp Ser Trp Thr Leu Arg
                85                  90                  95

Leu His Asn Leu Gln Ile Lys Asp Lys Gly Leu Tyr Gln Cys Ile Ile
            100                 105                 110

His His Lys Lys Pro Thr Gly Met Ile Arg Ile His Gln Met Asn Ser
        115                 120                 125

Glu Leu Ser Val Leu Ala Asn Phe Ser Gln Pro Glu Ile Val Pro Ile
    130                 135                 140

Ser Asn Ile Thr Glu Asn Val Tyr Ile Asn Leu Thr Cys Ser Ser Ile
145                 150                 155                 160
```

```
His Gly Tyr Pro Glu Pro Lys Lys Met Ser Val Leu Leu Arg Thr Lys
                165                 170                 175

Asn Ser Thr Ile Glu Tyr Asp Gly Ile Met Gln Lys Ser Gln Asp Asn
            180                 185                 190

Val Thr Glu Leu Tyr Asp Val Ser Ile Ser Leu Ser Val Ser Phe Pro
        195                 200                 205

Asp Val Thr Ser Asn Met Thr Ile Phe Cys Ile Leu Glu Thr Asp Lys
    210                 215                 220

Thr Arg Leu Leu Ser Ser Pro Phe Ser Ile Glu Leu Glu Asp Pro Gln
225                 230                 235                 240

Pro Pro Pro Asp His Ile Pro Trp Ile Thr Ala Val Leu Pro Thr Val
                245                 250                 255

Ile Ile Cys Val Met Val Phe Cys Leu Ile Leu Trp Lys Trp Lys Lys
            260                 265                 270

Lys Lys Arg Pro Arg Asn Ser Tyr Lys Cys Gly Thr Asn Thr Met Glu
        275                 280                 285

Arg Glu Glu Ser Glu Gln Thr Lys Lys Arg Glu Lys Ile His Ile Pro
    290                 295                 300

Glu Arg Ser Asp Glu Ala Gln Arg Val Phe Lys Ser Ser Lys Thr Ser
305                 310                 315                 320

Ser Cys Asp Lys Ser Asp Thr Cys Phe
                325
```

<210> 45
<211> 277
<212> PRT
<213> Human

<400> 45

```
Met Val Arg Leu Pro Leu Gln Cys Val Leu Trp Gly Cys Leu Leu Thr
1               5                   10                  15

Ala Val His Pro Glu Pro Pro Thr Ala Cys Arg Glu Lys Gln Tyr Leu
            20                  25                  30

Ile Asn Ser Gln Cys Cys Ser Leu Cys Gln Pro Gly Gln Lys Leu Val
        35                  40                  45
```

```
Ser Asp Cys Thr Glu Phe Thr Glu Thr Glu Cys Leu Pro Cys Gly Glu
    50                  55                  60

Ser Glu Phe Leu Asp Thr Trp Asn Arg Glu Thr His Cys His Gln His
65                  70                  75                  80

Lys Tyr Cys Asp Pro Asn Leu Gly Leu Arg Val Gln Gln Lys Gly Thr
                85                  90                  95

Ser Glu Thr Asp Thr Ile Cys Thr Cys Glu Glu Gly Trp His Cys Thr
            100                 105                 110

Ser Glu Ala Cys Glu Ser Cys Val Leu His Arg Ser Cys Ser Pro Gly
        115                 120                 125

Phe Gly Val Lys Gln Ile Ala Thr Gly Val Ser Asp Thr Ile Cys Glu
    130                 135                 140

Pro Cys Pro Val Gly Phe Phe Ser Asn Val Ser Ser Ala Phe Glu Lys
145                 150                 155                 160

Cys His Pro Trp Thr Ser Cys Glu Thr Lys Asp Leu Val Val Gln Gln
                165                 170                 175

Ala Gly Thr Asn Lys Thr Asp Val Val Cys Gly Pro Gln Asp Arg Leu
            180                 185                 190

Arg Ala Leu Val Val Ile Pro Ile Ile Phe Gly Ile Leu Phe Ala Ile
        195                 200                 205

Leu Leu Val Leu Val Phe Ile Lys Lys Val Ala Lys Lys Pro Thr Asn
    210                 215                 220

Lys Ala Pro His Pro Lys Gln Glu Pro Gln Glu Ile Asn Phe Pro Asp
225                 230                 235                 240

Asp Leu Pro Gly Ser Asn Thr Ala Ala Pro Val Gln Glu Thr Leu His
                245                 250                 255

Gly Cys Gln Pro Val Thr Gln Glu Asp Gly Lys Glu Ser Arg Ile Ser
            260                 265                 270

Val Gln Glu Arg Gln
        275
```

<210> 46
<211> 11

<212> PRT
<213> Artificial

<220>
<223> Linker sequence

<400> 46

Ser Gly Gly Gly Gly Ser Gly Gly Gly Gly Ser
1 5 10

<210> 47
<211> 13
<212> PRT
<213> Artificial

<220>
<223> Linker sequence

<400> 47

Ser Gly Gly Gly Gly Ser Gly Gly Gly Gly Ser Ala Pro
1 5 10

<210> 48
<211> 5
<212> PRT
<213> Artificial

<220>
<223> Linker sequence

<400> 48

Asn Phe Ser Gln Pro
1 5

<210> 49
<211> 5
<212> PRT
<213> Artificial

<220>
<223> Linker sequence

<400> 49

Lys Arg Thr Val Ala
1 5

<210> 50
<211> 8
<212> PRT
<213> Artificial

<220>
<223> Linker sequence

<400> 50

Gly Phe Thr Phe Ser Asp Tyr Tyr
1 5

<210> 51
<211> 8
<212> PRT
<213> Artificial

<220>
<223> Linker sequence

<400> 51

Ile Ser Ser Asn Gly Ile Tyr Ile
1 5

<210> 52
<211> 15
<212> PRT
<213> Artificial

<220>
<223> Linker sequence

<400> 52

Ala Arg Ala Pro Val Asp Tyr Ser Asn Pro Ser Gly Met Asp Val
1 5 10 15

<210> 53
<211> 8
<212> PRT
<213> Artificial

<220>
<223> Linker sequence

<400> 53

Ser Ser Asn Ile Gly Ser Asn Thr
1 5

<210> 54
<211> 3
<212> PRT
<213> Artificial

<220>
<223> Linker sequence

<400> 54

Arg Asn Asn
1

<210> 55

<211> 9
<212> PRT
<213> Artificial

<220>
<223> Linker sequence

<400> 55

```
Ala Ala Trp Asp Asp Ser Leu Ser Gly
1               5
```

<210> 56
<211> 338
<212> PRT
<213> Human

<400> 56

```
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Thr Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Ser Leu
                85                  90                  95

Ser Gly Trp Arg Phe Gly Gly Gly Thr Lys Leu Thr Val Leu Gly Gly
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
```

```
                180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys Ser Gly Gly Gly Gly Ser
    210                 215                 220

Gly Gly Gly Gly Ser Leu Lys Ile Gln Ala Tyr Phe Asn Glu Thr Ala
225                 230                 235                 240

Asp Leu Pro Cys Gln Phe Ala Asn Ser Gln Asn Gln Ser Leu Ser Glu
                245                 250                 255

Leu Ile Val Phe Trp Gln Asp Gln Glu Asn Leu Val Leu Asn Glu Val
            260                 265                 270

Tyr Leu Gly Lys Glu Arg Phe Asp Ala Val Asp Ser Lys Tyr Met Gly
        275                 280                 285

Arg Thr Ser Phe Asp Ser Asp Ser Trp Thr Leu Arg Leu His Asn Leu
    290                 295                 300

Gln Ile Lys Asp Lys Gly Ile Tyr Gln Cys Ile Ile His His Lys Lys
305                 310                 315                 320

Pro Ser Gly Met Val Lys Ile His Gln Met Asp Ser Glu Leu Ser Val
                325                 330                 335

Leu Ala
```

<210> 57
<211> 340
<212> PRT
<213> Human

<400> 57

```
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Thr Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
```

```
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Ser Leu
                85                  90                  95

Ser Gly Trp Arg Phe Gly Gly Gly Thr Lys Leu Thr Val Leu Gly Gly
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys Ser Gly Gly Gly Gly Ser
    210                 215                 220

Gly Gly Gly Gly Ser Ala Pro Leu Lys Ile Gln Ala Tyr Phe Asn Glu
225                 230                 235                 240

Thr Ala Asp Leu Pro Cys Gln Phe Ala Asn Ser Gln Asn Gln Ser Leu
                245                 250                 255

Ser Glu Leu Ile Val Phe Trp Gln Asp Gln Glu Asn Leu Val Leu Asn
            260                 265                 270

Glu Val Tyr Leu Gly Lys Glu Arg Phe Asp Ala Val Asp Ser Lys Tyr
        275                 280                 285

Met Gly Arg Thr Ser Phe Asp Ser Asp Ser Trp Thr Leu Arg Leu His
    290                 295                 300
```

Asn Leu Gln Ile Lys Asp Lys Gly Ile Tyr Gln Cys Ile Ile His His
305 310 315 320

Lys Lys Pro Ser Gly Met Val Lys Ile His Gln Met Asp Ser Glu Leu
325 330 335

Ser Val Leu Ala
340

<210> 58
<211> 566
<212> PRT
<213> Human

<400> 58

Leu Lys Ile Gln Ala Tyr Phe Asn Glu Thr Ala Asp Leu Pro Cys Gln
1 5 10 15

Phe Ala Asn Ser Gln Asn Gln Ser Leu Ser Glu Leu Ile Val Phe Trp
20 25 30

Gln Asp Gln Glu Asn Leu Val Leu Asn Glu Val Tyr Leu Gly Lys Glu
35 40 45

Arg Phe Asp Ala Val Asp Ser Lys Tyr Met Gly Arg Thr Ser Phe Asp
50 55 60

Ser Asp Ser Trp Thr Leu Arg Leu His Asn Leu Gln Ile Lys Asp Lys
65 70 75 80

Gly Ile Tyr Gln Cys Ile Ile His His Lys Lys Pro Ser Gly Met Val
85 90 95

Lys Ile His Gln Met Asp Ser Glu Leu Ser Val Leu Ala Asn Phe Ser
100 105 110

Gln Pro Glu Val Gln Leu Leu Glu Ser Gly Gly Gly Leu Val Gln Pro
115 120 125

Gly Gly Ser Leu Arg Leu Ser Cys Ala Ala Ser Gly Phe Thr Phe Ser
130 135 140

Asp Tyr Tyr Met Ser Trp Val Arg Gln Ala Pro Gly Lys Gly Leu Glu
145 150 155 160

Trp Ile Ser Ser Ile Ser Ser Asn Gly Ile Tyr Ile Tyr Tyr Ala Asp
165 170 175

```
Ser Leu Lys Gly Arg Phe Thr Ile Ser Arg Asp Asn Ser Lys Asn Thr
            180                 185                 190

Leu Tyr Leu Gln Met Asn Ser Leu Arg Ala Glu Asp Thr Ala Val Tyr
        195                 200                 205

Tyr Cys Ala Arg Ala Pro Val Asp Tyr Ser Asn Pro Ser Gly Met Asp
    210                 215                 220

Val Trp Gly Gln Gly Thr Leu Val Thr Val Ser Ser Ala Ser Thr Lys
225                 230                 235                 240

Gly Pro Ser Val Phe Pro Leu Ala Pro Ser Ser Lys Ser Thr Ser Gly
                245                 250                 255

Gly Thr Ala Ala Leu Gly Cys Leu Val Lys Asp Tyr Phe Pro Glu Pro
            260                 265                 270

Val Thr Val Ser Trp Asn Ser Gly Ala Leu Thr Ser Gly Val His Thr
        275                 280                 285

Phe Pro Ala Val Leu Gln Ser Ser Gly Leu Tyr Ser Leu Ser Ser Val
    290                 295                 300

Val Thr Val Pro Ser Ser Ser Leu Gly Thr Gln Thr Tyr Ile Cys Asn
305                 310                 315                 320

Val Asn His Lys Pro Ser Asn Thr Lys Val Asp Lys Lys Val Glu Pro
                325                 330                 335

Lys Ser Cys Asp Lys Thr His Thr Cys Pro Pro Cys Pro Ala Pro Glu
            340                 345                 350

Leu Leu Gly Gly Pro Ser Val Phe Leu Phe Pro Pro Lys Pro Lys Asp
        355                 360                 365

Thr Leu Met Ile Ser Arg Thr Pro Glu Val Thr Cys Val Val Val Asp
    370                 375                 380

Val Ser His Glu Asp Pro Glu Val Lys Phe Asn Trp Tyr Val Asp Gly
385                 390                 395                 400

Val Glu Val His Asn Ala Lys Thr Lys Pro Arg Glu Glu Gln Tyr Asn
                405                 410                 415

Ser Thr Tyr Arg Val Val Ser Val Leu Thr Val Leu His Gln Asp Trp
            420                 425                 430
```

Leu Asn Gly Lys Glu Tyr Lys Cys Lys Val Ser Asn Lys Ala Leu Pro
435 440 445

Ala Pro Ile Glu Lys Thr Ile Ser Lys Ala Lys Gly Gln Pro Arg Glu
450 455 460

Pro Gln Val Tyr Thr Leu Pro Pro Ser Arg Asp Glu Leu Thr Lys Asn
465 470 475 480

Gln Val Ser Leu Thr Cys Leu Val Lys Gly Phe Tyr Pro Ser Asp Ile
485 490 495

Ala Val Glu Trp Glu Ser Asn Gly Gln Pro Glu Asn Asn Tyr Lys Thr
500 505 510

Thr Pro Pro Val Leu Asp Ser Asp Gly Ser Phe Phe Leu Tyr Ser Lys
515 520 525

Leu Thr Val Asp Lys Ser Arg Trp Gln Gln Gly Asn Val Phe Ser Cys
530 535 540

Ser Val Met His Glu Ala Leu His Asn His Tyr Thr Gln Lys Ser Leu
545 550 555 560

Ser Leu Ser Pro Gly Lys
565

<210> 59
<211> 332
<212> PRT
<213> Human

<400> 59

Leu Lys Ile Gln Ala Tyr Phe Asn Glu Thr Ala Asp Leu Pro Cys Gln
1 5 10 15

Phe Ala Asn Ser Gln Asn Gln Ser Leu Ser Glu Leu Ile Val Phe Trp
20 25 30

Gln Asp Gln Glu Asn Leu Val Leu Asn Glu Val Tyr Leu Gly Lys Glu
35 40 45

Arg Phe Asp Ala Val Asp Ser Lys Tyr Met Gly Arg Thr Ser Phe Asp
50 55 60

Ser Asp Ser Trp Thr Leu Arg Leu His Asn Leu Gln Ile Lys Asp Lys
65 70 75 80

```
Gly Ile Tyr Gln Cys Ile Ile His His Lys Lys Pro Ser Gly Met Val
                85                  90                  95

Lys Ile His Gln Met Asp Ser Glu Leu Ser Val Leu Ala Lys Arg Thr
            100                 105                 110

Val Ala Gln Ser Val Leu Thr Gln Pro Pro Ser Ala Ser Gly Thr Pro
        115                 120                 125

Gly Gln Arg Val Thr Ile Ser Cys Ser Gly Ser Ser Ser Asn Ile Gly
    130                 135                 140

Ser Asn Thr Val Asn Trp Tyr Gln Gln Leu Pro Gly Thr Ala Pro Lys
145                 150                 155                 160

Leu Leu Ile Tyr Arg Asn Asn Gln Arg Pro Ser Gly Val Pro Asp Arg
                165                 170                 175

Phe Ser Gly Ser Lys Ser Gly Thr Ser Ala Ser Leu Ala Ile Ser Gly
            180                 185                 190

Leu Arg Ser Glu Asp Glu Ala Asp Tyr Tyr Cys Ala Ala Trp Asp Asp
        195                 200                 205

Ser Leu Ser Gly Trp Arg Phe Gly Gly Gly Thr Lys Leu Thr Val Leu
    210                 215                 220

Gly Gly Thr Val Ala Ala Pro Ser Val Phe Ile Phe Pro Pro Ser Asp
225                 230                 235                 240

Glu Gln Leu Lys Ser Gly Thr Ala Ser Val Val Cys Leu Leu Asn Asn
                245                 250                 255

Phe Tyr Pro Arg Glu Ala Lys Val Gln Trp Lys Val Asp Asn Ala Leu
            260                 265                 270

Gln Ser Gly Asn Ser Gln Glu Ser Val Thr Glu Gln Asp Ser Lys Asp
        275                 280                 285

Ser Thr Tyr Ser Leu Ser Ser Thr Leu Thr Leu Ser Lys Ala Asp Tyr
    290                 295                 300

Glu Lys His Lys Val Tyr Ala Cys Glu Val Thr His Gln Gly Leu Ser
305                 310                 315                 320

Ser Pro Val Thr Lys Ser Phe Asn Arg Gly Glu Cys
                325                 330
```

<210> 60
<211> 332
<212> PRT
<213> Human

<400> 60

```
Leu Lys Ile Gln Ala Tyr Phe Asn Glu Thr Ala Asp Leu Pro Cys Gln
1               5                   10                  15

Phe Ala Asn Ser Gln Asn Gln Ser Leu Ser Glu Leu Ile Val Phe Trp
            20                  25                  30

Gln Asp Gln Glu Asn Leu Val Leu Asn Glu Val Tyr Leu Gly Lys Glu
        35                  40                  45

Arg Phe Asp Ala Val Asp Ser Lys Tyr Met Gly Arg Thr Ser Phe Asp
    50                  55                  60

Ser Asp Ser Trp Thr Leu Arg Leu His Asn Leu Gln Ile Lys Asp Lys
65                  70                  75                  80

Gly Ile Tyr Gln Cys Ile Ile His His Lys Lys Pro Ser Gly Met Val
                85                  90                  95

Lys Ile His Gln Met Asp Ser Glu Leu Ser Val Leu Ala Asn Phe Ser
            100                 105                 110

Gln Pro Gln Ser Val Leu Thr Gln Pro Pro Ser Ala Ser Gly Thr Pro
        115                 120                 125

Gly Gln Arg Val Thr Ile Ser Cys Ser Gly Ser Ser Ser Asn Ile Gly
    130                 135                 140

Ser Asn Thr Val Asn Trp Tyr Gln Gln Leu Pro Gly Thr Ala Pro Lys
145                 150                 155                 160

Leu Leu Ile Tyr Arg Asn Asn Gln Arg Pro Ser Gly Val Pro Asp Arg
                165                 170                 175

Phe Ser Gly Ser Lys Ser Gly Thr Ser Ala Ser Leu Ala Ile Ser Gly
            180                 185                 190

Leu Arg Ser Glu Asp Glu Ala Asp Tyr Tyr Cys Ala Ala Trp Asp Asp
        195                 200                 205

Ser Leu Ser Gly Trp Arg Phe Gly Gly Gly Thr Lys Leu Thr Val Leu
    210                 215                 220
```

```
Gly Gly Thr Val Ala Ala Pro Ser Val Phe Ile Phe Pro Pro Ser Asp
225                 230                 235                 240

Glu Gln Leu Lys Ser Gly Thr Ala Ser Val Val Cys Leu Leu Asn Asn
                245                 250                 255

Phe Tyr Pro Arg Glu Ala Lys Val Gln Trp Lys Val Asp Asn Ala Leu
            260                 265                 270

Gln Ser Gly Asn Ser Gln Glu Ser Val Thr Glu Gln Asp Ser Lys Asp
        275                 280                 285

Ser Thr Tyr Ser Leu Ser Ser Thr Leu Thr Leu Ser Lys Ala Asp Tyr
    290                 295                 300

Glu Lys His Lys Val Tyr Ala Cys Glu Val Thr His Gln Gly Leu Ser
305                 310                 315                 320

Ser Pro Val Thr Lys Ser Phe Asn Arg Gly Glu Cys
                325                 330
```

<210> 61
<211> 452
<212> PRT
<213> Human

<400> 61

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Tyr Met Ser Trp Val Arg Gln Ala Pro Gly Lys Gly Leu Glu Trp Ile
        35                  40                  45

Ser Ser Ile Ser Ser Asn Gly Ile Tyr Ile Tyr Tyr Ala Asp Ser Leu
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ala Pro Val Asp Tyr Ser Asn Pro Ser Gly Met Asp Val Trp
            100                 105                 110
```

```
Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
```

355 360 365

Ser Leu Thr Cys Leu Val Lys Gly Phe Tyr Pro Ser Asp Ile Ala Val
370 375 380

Glu Trp Glu Ser Asn Gly Gln Pro Glu Asn Asn Tyr Lys Thr Thr Pro
385 390 395 400

Pro Val Leu Asp Ser Asp Gly Ser Phe Phe Leu Tyr Ser Lys Leu Thr
405 410 415

Val Asp Lys Ser Arg Trp Gln Gln Gly Asn Val Phe Ser Cys Ser Val
420 425 430

Met His Glu Ala Leu His Asn His Tyr Thr Gln Lys Ser Leu Ser Leu
435 440 445

Ser Pro Gly Lys
450

<210> 62
<211> 218
<212> PRT
<213> Human

<400> 62

```
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Thr Val Asn Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Ser Leu
                85                  90                  95

Ser Gly Trp Arg Phe Gly Gly Gly Thr Lys Leu Thr Val Leu Gly Gly
            100                 105                 110

Thr Val Ala Ala Pro Ser Val Phe Ile Phe Pro Pro Ser Asp Glu Gln


        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215
```

<210> 63
<211> 4388
<212> DNA
<213> Human

<400> 63

```
gaggtgcagc tgctggagtc cggaggaggc ctggtgcagc ccggcggcag cctgagactg      60
agctgtgccg ccagcggatt caccttctcc gactactata tgagctgggt gaggcaggcc     120
ccaggcaagg gcctggagtg gatttcctcc atcagcagca atgggatcta catttactac     180
gccgacagcc tgaagggcag gttcacaatc agcagggaca actctaagaa cacactgtac     240
ctgcagatga actccctgcg cgccgaggac accgccgtgt attactgtgc cagggccccc     300
gtggactact ctaatcccag cggcatggac gtgtggggcc agggcaccct ggtgacagtg     360
agctcaggtg agtcgtacgc tagcaagctt tctggggcag gccaggcctg accttggctt     420
tggggcaggg agggggctaa ggtgaggcag gtggcgccag ccaggtgcac acccaatgcc     480
catgagccca gacactggac gctgaacctc gcggacagtt aagaacccag gggcctctgc     540
gccctgggcc cagctctgtc ccacaccgcg gtcacatggc accacctctc ttgcagcctc     600
caccaagggc ccatcggtct tccccctggc accctcctcc aagagcacct ctgggggcac     660
agcggccctg ggctgcctgg tcaaggacta cttccccgaa ccggtgacgg tgtcgtggaa     720
ctcaggcgcc ctgaccagcg gcgtgcacac cttcccggct gtcctacagt cctcaggact     780
ctactccctc agcagcgtgg tgaccgtgcc ctccagcagc ttgggcaccc agacctacat     840
ctgcaacgtg aatcacaagc ccagcaacac caaggtggac aagaaagttg gtgagaggcc     900
```

agcacaggga gggagggtgt ctgctggaag ccaggctcag cgctcctgcc tggacgcatc 960
ccggctatgc agccccagtc cagggcagca aggcaggccc cgtctgcctc ttcacccgga 1020
ggcctctgcc cgccccactc atgctcaggg agagggtctt ctggcttttt ccccaggctc 1080
tgggcaggca caggctaggt gcccctaacc caggccctgc acacaaaggg gcaggtgctg 1140
ggctcagacc tgccaagagc catatccggg aggaccctgc ccctgaccta agcccacccc 1200
aaaggccaaa ctctccactc cctcagctcg gacaccttct ctcctcccag attccagtaa 1260
ctcccaatct tctctctgca gagcccaaat cttgtgacaa aactcacaca tgcccaccgt 1320
gcccaggtaa gccagcccag gcctcgccct ccagctcaag gcgggacagg tgccctagag 1380
tagcctgcat ccagggacag gccccagccg ggtgctgaca cgtccacctc catctcttcc 1440
tcagcacctg aactcctggg gggaccgtca gtcttcctct tccccccaaa acccaaggac 1500
accctcatga tctcccggac ccctgaggtc acatgcgtgg tggtggacgt gagccacgaa 1560
gaccctgagg tcaagttcaa ctggtacgtg gacggcgtgg aggtgcataa tgccaagaca 1620
aagccgcggg aggagcagta caacagcacg taccgtgtgg tcagcgtcct caccgtcctg 1680
caccaggact ggctgaatgg caaggagtac aagtgcaagg tctccaacaa agccctccca 1740
gcccccatcg agaaaaccat ctccaaagcc aaaggtggga cccgtggggt gcgagggcca 1800
catggacaga ggccggctcg gcccaccctc tgccctgaga gtgaccgctg taccaacctc 1860
tgtccctaca gggcagcccc gagaaccaca ggtgtacacc ctgcccccat cccgggatga 1920
gctgaccaag aaccaggtca gcctgacctg cctggtcaaa ggcttctatc ccagcgacat 1980
cgccgtggag tgggagagca atgggcagcc ggagaacaac tacaagacca cgcctcccgt 2040
gctggactcc gacggctcct tcttcctcta cagcaagctc accgtggaca agagcaggtg 2100
gcagcagggg aacgtcttct catgctccgt gatgcatgag gctctgcaca accactacac 2160
gcagaagagc ctctccctgt ctccgggtaa atgagaggtg cagctgctgg agtccggagg 2220
aggcctggtg cagcccggcg gcagcctgag actgagctgt gccgccagcg gattcacctt 2280
ctccgactac tatatgagct gggtgaggca ggccccaggc aagggcctgg agtggatttc 2340
ctccatcagc agcaatggga tctacattta ctacgccgac agcctgaagg gcaggttcac 2400
aatcagcagg gacaactcta agaacacact gtacctgcag atgaactccc tgcgcgccga 2460
ggacaccgcc gtgtattact gtgccagggc cccgtggac tactctaatc ccagcggcat 2520
ggacgtgtgg ggccagggca ccctggtgac agtgagctca ggtgagtcgt acgctagcaa 2580
gctttctggg gcaggccagg cctgaccttg gctttggggc agggaggggg ctaaggtgag 2640
gcaggtggcg ccagccaggt gcacacccaa tgcccatgag cccagacact ggacgctgaa 2700
cctcgcggac agttaagaac ccaggggcct ctgcgccctg ggcccagctc tgtcccacac 2760
cgcggtcaca tggcaccacc tctcttgcag cctccaccaa gggcccatcg gtcttccccc 2820

tggcaccctc ctccaagagc acctctgggg gcacagcggc cctgggctgc ctggtcaagg 2880

actacttccc cgaaccggtg acggtgtcgt ggaactcagg cgccctgacc agcggcgtgc 2940

acaccttccc ggctgtccta cagtcctcag gactctactc cctcagcagc gtggtgaccg 3000

tgccctccag cagcttgggc acccagacct acatctgcaa cgtgaatcac aagcccagca 3060

acaccaaggt ggacaagaaa gttggtgaga ggccagcaca gggagggagg gtgtctgctg 3120

gaagccaggc tcagcgctcc tgcctggacg catcccggct atgcagcccc agtccagggc 3180

agcaaggcag gccccgtctg cctcttcacc cggaggcctc tgcccgcccc actcatgctc 3240

agggagaggg tcttctggct ttttccccag gctctgggca ggcacaggct aggtgcccct 3300

aacccaggcc ctgcacacaa aggggcaggt gctgggctca gacctgccaa gagccatatc 3360

cgggaggacc ctgcccctga cctaagccca ccccaaaggc caaactctcc actccctcag 3420

ctcggacacc ttctctcctc ccagattcca gtaactccca atcttctctc tgcagagccc 3480

aaatcttgtg acaaaactca cacatgccca ccgtgcccag gtaagccagc ccaggcctcg 3540

ccctccagct caaggcggga caggtgccct agagtagcct gcatccaggg acaggcccca 3600

gccgggtgct gacacgtcca cctccatctc ttcctcagca cctgaactcc tggggggacc 3660

gtcagtcttc ctcttccccc caaaacccaa ggacaccctc atgatctccc ggacccctga 3720

ggtcacatgc gtggtggtgg acgtgagcca cgaagaccct gaggtcaagt tcaactggta 3780

cgtggacggc gtggaggtgc ataatgccaa gacaaagccg cgggaggagc agtacaacag 3840

cacgtaccgt gtggtcagcg tcctcaccgt cctgcaccag gactggctga atggcaagga 3900

gtacaagtgc aaggtctcca acaaagccct cccagccccc atcgagaaaa ccatctccaa 3960

agccaaaggt gggacccgtg gggtgcgagg gccacatgga cagaggccgg ctcggcccac 4020

cctctgccct gagagtgacc gctgtaccaa cctctgtccc tacagggcag ccccgagaac 4080

cacaggtgta caccctgccc ccatcccggg atgagctgac caagaaccag gtcagcctga 4140

cctgcctggt caaaggcttc tatcccagcg acatcgccgt ggagtgggag agcaatgggc 4200

agccggagaa caactacaag accacgcctc ccgtgctgga ctccgacggc tccttcttcc 4260

tctacagcaa gctcaccgtg gacaagagca ggtggcagca ggggaacgtc ttctcatgct 4320

ccgtgatgca tgaggctctg cacaaccact acacgcagaa gagcctctcc ctgtctccgg 4380

gtaaatga 4388

<210> 64
<211> 1023
<212> DNA
<213> Human

<400> 64

cagtccgtgc tgacccagcc accctccgcc agcggcaccc ctggccagcg ggtgaccatc 60

tcttgtagcg gcagcagctc caacatcgga agcaataccg tgaattggta ccagcagctg 120

cccggcaccg cccccaagct gctgatctac cgcaataacc agaggccctc cggcgtgccc 180

gacaggttca gcggcagcaa gtctggcacc tccgcctctc tggccattag cggactgcgg 240

agcgaggacg aggccgatta ctactgcgcc gcctgggacg actccctgtc cgggtggcgc 300

tttggaggcg gcacaaagct gaccgtgctg ggaggtgagt agaacgtacg ctagcaagct 360

tgatatcgaa ttctaaactc tgagggggtc ggatgacgtg gccattcttt gcctaaagca 420

ttgagtttac tgcaaggtca gaaaagcatg caaagccctc agaatggctg caaagagctc 480

caacaaaaca atttagaact ttattaagga ataggggaa gctaggaaga aactcaaaac 540

atcaagattt taaatacgct tcttggtctc cttgctataa ttatctggga taagcatgct 600

gttttctgtc tgtccctaac atgccctgtg attatccgca aacaacacac ccaagggcag 660

aactttgtta cttaaacacc atcctgtttg cttctttcct caggaactgt ggctgcacca 720

tctgtcttca tcttcccgcc atctgatgag cagttgaaat ctggaactgc ctctgttgtg 780

tgcctgctga ataacttcta tcccagagag gccaaagtac agtggaaggt ggataacgcc 840

ctccaatcgg gtaactccca ggagagtgtc acagagcagg acagcaagga cagcacctac 900

agcctcagca gcaccctgac gctgagcaaa gcagactacg agaaacacaa agtctacgcc 960

tgcgaagtca cccatcaggg cctgagctcg cccgtcacaa agagcttcaa caggggagag 1020

tgt 1023

<210> 65
<211> 1383
<212> DNA
<213> Human

<400> 65

```
cagtccgtgc tgacccagcc accctccgcc agcggcaccc ctggccagcg ggtgaccatc      60
tcttgtagcg gcagcagctc caacatcgga agcaataccg tgaattggta ccagcagctg     120
cccggcaccg cccccaagct gctgatctac cgcaataacc agaggccctc cggcgtgccc     180
gacaggttca gcggcagcaa gtctggcacc tccgcctctc tggccattag cggactgcgg     240
agcgaggacg aggccgatta ctactgcgcc gcctgggacg actccctgtc cgggtggcgc     300
tttggaggcg gcacaaagct gaccgtgctg ggaggtgagt agaacgtacg ctagcaagct     360
tgatatcgaa ttctaaactc tgagggggtc ggatgacgtg gccattcttt gcctaaagca     420
ttgagtttac tgcaaggtca gaaaagcatg caaagccctc agaatggctg caaagagctc     480
caacaaaaca atttagaact ttattaagga ataggggaa gctaggaaga aactcaaaac      540
atcaagattt taaatacgct tcttggtctc cttgctataa ttatctggga taagcatgct     600
gttttctgtc tgtccctaac atgccctgtg attatccgca aacaacacac ccaagggcag     660
aactttgtta cttaaacacc atcctgtttg cttctttcct caggaactgt ggctgcacca     720
tctgtcttca tcttcccgcc atctgatgag cagttgaaat ctggaactgc ctctgttgtg     780
tgcctgctga ataacttcta tcccagagag gccaaagtac agtggaaggt ggataacgcc     840
ctccaatcgg gtaactccca ggagagtgtc acagagcagg acagcaagga cagcacctac     900
agcctcagca gcaccctgac gctgagcaaa gcagactacg agaaacacaa agtctacgcc     960
tgcgaagtca cccatcaggg cctgagctcg cccgtcacaa agagcttcaa caggggagag    1020
tgtagcggag gaggaggaag cggaggagga ggaagcctca agattcaggc gtactttaac    1080
gaaacagcgg acctcccgtg ccaattcgcc aatagccaga atcaatcgct cagtgagctc    1140
atcgtgtttt ggcaagatca ggaaaacctt gtcttgaatg aggtctatct gggtaaagag    1200
agattcgacg ccgtagattc caagtacatg gggaggactt cgttcgattc agattcgtgg    1260
acgctgagat tgcataatct ccaaatcaaa gacaaaggga tctaccagtg catcattcac    1320
cacaaaaagc ctagcggaat ggtgaagatc catcagatgg acagcgaatt gtcagttttg    1380
gcc                                                                  1383
```

<210> 66
<211> 1389
<212> DNA
<213> Human

<400> 66

```
cagtccgtgc tgacccagcc accctccgcc agcggcaccc ctggccagcg ggtgaccatc      60
tcttgtagcg gcagcagctc caacatcgga agcaataccg tgaattggta ccagcagctg     120
cccggcaccg cccccaagct gctgatctac cgcaataacc agaggccctc cggcgtgccc     180
gacaggttca gcggcagcaa gtctggcacc tccgcctctc tggccattag cggactgcgg     240
agcgaggacg aggccgatta ctactgcgcc gcctgggacg actccctgtc cgggtggcgc     300
tttggaggcg gcacaaagct gaccgtgctg ggaggtgagt agaacgtacg ctagcaagct     360
tgatatcgaa ttctaaactc tgaggggtc ggatgacgtg gccattcttt gcctaaagca      420
ttgagtttac tgcaaggtca gaaaagcatg caaagccctc agaatggctg caaagagctc     480
caacaaaaca atttagaact ttattaagga atagggggaa gctaggaaga aactcaaaac     540
atcaagattt taaatacgct tcttggtctc cttgctataa ttatctggga taagcatgct     600
gttttctgtc tgtccctaac atgccctgtg attatccgca aacaacacac ccaagggcag     660
aactttgtta cttaaacacc atcctgtttg cttctttcct caggaactgt ggctgcacca     720
tctgtcttca tcttcccgcc atctgatgag cagttgaaat ctggaactgc ctctgttgtg     780
tgcctgctga ataacttcta tcccagagag gccaaagtac agtggaaggt ggataacgcc     840
ctccaatcgg gtaactccca ggagagtgtc acagagcagg acagcaagga cagcacctac     900

agcctcagca gcaccctgac gctgagcaaa gcagactacg agaaacacaa agtctacgcc     960
tgcgaagtca cccatcaggg cctgagctcg cccgtcacaa agagcttcaa caggggagag    1020
tgtagcggag gaggaggaag cggaggagga ggaagcgccc ccctcaagat tcaggcgtac    1080
tttaacgaaa cagcggacct cccgtgccaa ttcgccaata gccagaatca atcgctcagt    1140
gagctcatcg tgttttggca agatcaggaa aaccttgtct tgaatgaggt ctatctgggt    1200
aaagagagat tcgacgccgt agattccaag tacatgggga ggacttcgtt cgattcagat    1260
tcgtggacgc tgagattgca taatctccaa atcaaagaca aagggatcta ccagtgcatc    1320
attcaccaca aaaagcctag cggaatggtg aagatccatc agatggacag cgaattgtca    1380
gttttggcc                                                            1389
```

<210> 67
<211> 2537
<212> DNA
<213> Human

<400> 67

```
ctcaagattc aggcgtactt taacgaaaca gcggacctcc cgtgccaatt cgccaatagc      60
cagaatcaat cgctcagtga gctcatcgtg ttttggcaag atcaggaaaa ccttgtcttg     120
aatgaggtct atctgggtaa agagagattc gacgccgtag attccaagta catggggagg     180
acttcgttcg attcagattc gtggacgctg agattgcata atctccaaat caaagacaaa     240
gggatctacc agtgcatcat tcaccacaaa aagcctagcg gaatggtgaa gatccatcag     300
atggacagcg aattgtcagt tttggccaac ttcagccagc ccgaggtgca gctgctggag     360
tccggaggag gcctggtgca gcccggcggc agcctgagac tgagctgtgc cgccagcgga     420
ttcaccttct ccgactacta tatgagctgg gtgaggcagg ccccaggcaa gggcctggag     480
tggatttcct ccatcagcag caatgggatc tacatttact acgccgacag cctgaagggc     540
aggttcacaa tcagcaggga caactctaag aacacactgt acctgcagat gaactccctg     600
cgcgccgagg acaccgccgt gtattactgt gccagggccc ccgtggacta ctctaatccc     660
agcggcatgg acgtgtgggg ccagggcacc ctggtgacag tgagctcagg tgagtcgtac     720
gctagcaagc tttctggggc aggccaggcc tgaccttggc tttggggcag ggagggggct     780
aaggtgaggc aggtggcgcc agccaggtgc acacccaatg cccatgagcc cagacactgg     840
acgctgaacc tcgcggacag ttaagaaccc aggggcctct gcgccctggg cccagctctg     900
tcccacaccg cggtcacatg gcaccacctc tcttgcagcc tccaccaagg gcccatcggt     960
cttccccctg gcaccctcct ccaagagcac ctctgggggc acagcggccc tgggctgcct    1020
ggtcaaggac tacttccccg aaccggtgac ggtgtcgtgg aactcaggcg ccctgaccag    1080
cggcgtgcac accttcccgg ctgtcctaca gtcctcagga ctctactccc tcagcagcgt    1140
```

ggtgaccgtg ccctccagca gcttgggcac ccagacctac atctgcaacg tgaatcacaa 1200
gcccagcaac accaaggtgg acaagaaagt tggtgagagg ccagcacagg gagggagggt 1260
gtctgctgga agccaggctc agcgctcctg cctggacgca tcccggctat gcagccccag 1320
tccagggcag caaggcaggc cccgtctgcc tcttcacccg gaggcctctg cccgccccac 1380
tcatgctcag ggagagggtc ttctggcttt ttccccaggc tctgggcagg cacaggctag 1440
gtgcccctaa cccaggccct gcacacaaag gggcaggtgc tgggctcaga cctgccaaga 1500
gccatatccg ggaggaccct gcccctgacc taagcccacc ccaaaggcca aactctccac 1560
tccctcagct cggacacctt ctctcctccc agattccagt aactcccaat cttctctctg 1620
cagagcccaa atcttgtgac aaaactcaca catgcccacc gtgcccaggt aagccagccc 1680
aggcctcgcc ctccagctca aggcgggaca ggtgccctag agtagcctgc atccagggac 1740
aggccccagc cgggtgctga cacgtccacc tccatctctt cctcagcacc tgaactcctg 1800
gggggaccgt cagtcttcct cttcccccca aaacccaagg acaccctcat gatctcccgg 1860
acccctgagg tcacatgcgt ggtggtggac gtgagccacg aagaccctga ggtcaagttc 1920
aactggtacg tggacggcgt ggaggtgcat aatgccaaga caaagccgcg ggaggagcag 1980
tacaacagca cgtaccgtgt ggtcagcgtc ctcaccgtcc tgcaccagga ctggctgaat 2040
ggcaaggagt acaagtgcaa ggtctccaac aaagccctcc cagcccccat cgagaaaacc 2100
atctccaaag ccaaaggtgg gacccgtggg gtgcgagggc cacatggaca gaggccggct 2160
cggcccaccc tctgccctga gagtgaccgc tgtaccaacc tctgtcccta cagggcagcc 2220
ccgagaacca caggtgtaca ccctgccccc atcccgggat gagctgacca agaaccaggt 2280
cagcctgacc tgcctggtca aaggcttcta tcccagcgac atcgccgtgg agtgggagag 2340
caatgggcag ccggagaaca actacaagac cacgcctccc gtgctggact ccgacggctc 2400
cttcttcctc tacagcaagc tcaccgtgga caagagcagg tggcagcagg ggaacgtctt 2460
ctcatgctcc gtgatgcatg aggctctgca caaccactac acgcagaaga gcctctccct 2520
gtctccgggt aaatgag 2537

<210> 68
<211> 1365
<212> DNA
<213> Human

<400> 68

ctcaagattc aggcgtactt taacgaaaca gcggacctcc cgtgccaatt cgccaatagc 60
cagaatcaat cgctcagtga gctcatcgtg ttttggcaag atcaggaaaa ccttgtcttg 120
aatgaggtct atctgggtaa agagagattc gacgccgtag attccaagta catggggagg 180
acttcgttcg attcagattc gtggacgctg agattgcata atctccaaat caaagacaaa 240
gggatctacc agtgcatcat tcaccacaaa aagcctagcg gaatggtgaa gatccatcag 300
atggacagcg aattgtcagt tttggccaag agaaccgtgg cccagtccgt gctgacccag 360
ccaccctccg ccagcggcac ccctggccag cgggtgacca tctcttgtag cggcagcagc 420
tccaacatcg gaagcaatac cgtgaattgg taccagcagc tgcccggcac cgcccccaag 480
ctgctgatct accgcaataa ccagaggccc tccggcgtgc ccgacaggtt cagcggcagc 540
aagtctggca cctccgcctc tctggccatt agcggactgc ggagcgagga cgaggccgat 600
tactactgcg ccgcctggga cgactccctg tccgggtggc gctttggagg cggcacaaag 660
ctgaccgtgc tgggaggtga gtagaacgta cgctagcaag cttgatatcg aattctaaac 720
tctgaggggg tcggatgacg tggccattct ttgcctaaag cattgagttt actgcaaggt 780
cagaaaagca tgcaaagccc tcagaatggc tgcaaagagc tccaacaaaa caatttagaa 840
ctttattaag gaataggggg aagctaggaa gaaactcaaa acatcaagat tttaaatacg 900
cttcttggtc tccttgctat aattatctgg gataagcatg ctgttttctg tctgtcccta 960
acatgccctg tgattatccg caaacaacac acccaagggc agaactttgt tacttaaaca 1020
ccatcctgtt tgcttctttc ctcaggaact gtggctgcac catctgtctt catcttcccg 1080
ccatctgatg agcagttgaa atctggaact gcctctgttg tgtgcctgct gaataacttc 1140
tatcccagag aggccaaagt acagtggaag gtggataacg ccctccaatc gggtaactcc 1200
caggagagtg tcacagagca ggacagcaag gacagcacct acagcctcag cagcaccctg 1260
acgctgagca aagcagacta cgagaaacac aaagtctacg cctgcgaagt cacccatcag 1320
ggcctgagct cgcccgtcac aaagagcttc aacaggggag agtgt 1365

<210> 69
<211> 1365
<212> DNA
<213> Human

<400> 69

```
ctcaagattc aggcgtactt taacgaaaca gcggacctcc cgtgccaatt cgccaatagc      60
cagaatcaat cgctcagtga gctcatcgtg ttttggcaag atcaggaaaa ccttgtcttg     120
aatgaggtct atctgggtaa agagagattc gacgccgtag attccaagta catggggagg     180
acttcgttcg attcagattc gtggacgctg agattgcata atctccaaat caaagacaaa     240
gggatctacc agtgcatcat tcaccacaaa aagcctagcg gaatggtgaa gatccatcag     300
atggacagcg aattgtcagt tttggccaac ttcagccagc cccagtccgt gctgacccag     360
ccaccctccg ccagcggcac ccctggccag cgggtgacca tctcttgtag cggcagcagc     420
tccaacatcg gaagcaatac cgtgaattgg taccagcagc tgcccggcac cgcccccaag     480
ctgctgatct accgcaataa ccagaggccc tccggcgtgc ccgacaggtt cagcggcagc     540

aagtctggca cctccgcctc tctggccatt agcggactgc ggagcgagga cgaggccgat     600
tactactgcg ccgcctggga cgactccctg tccgggtggc gctttggagg cggcacaaag     660
ctgaccgtgc tgggaggtga gtagaacgta cgctagcaag cttgatatcg aattctaaac     720
tctgaggggg tcggatgacg tggccattct ttgcctaaag cattgagttt actgcaaggt     780
cagaaaagca tgcaaagccc tcagaatggc tgcaaagagc tccaacaaaa caatttagaa     840
ctttattaag gaataggggg aagctaggaa gaaactcaaa acatcaagat tttaaatacg     900
cttcttggtc tccttgctat aattatctgg gataagcatg ctgttttctg tctgtcccta     960
acatgccctg tgattatccg caaacaacac acccaagggc agaactttgt tacttaaaca    1020
ccatcctgtt tgcttctttc ctcaggaact gtggctgcac catctgtctt catcttcccg    1080
ccatctgatg agcagttgaa atctggaact gcctctgttg tgtgcctgct gaataacttc    1140
tatcccagag aggccaaagt acagtggaag gtggataacg ccctccaatc gggtaactcc    1200
caggagagtg tcacagagca ggacagcaag gacagcacct acagcctcag cagcaccctg    1260
acgctgagca aagcagacta cgagaaacac aaagtctacg cctgcgaagt cacccatcag    1320
ggcctgagct cgcccgtcac aaagagcttc aacaggggag agtgt                    1365
```

<210> 70
<211> 446
<212> PRT
<213> Artificial

<220>
<223> antibody H chain 1107

<400> 70

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95
```

Ala Arg Arg Val Trp Gly Phe Asp Tyr Trp Gly Gln Gly Thr Leu Val
100 105 110

Thr Val Ser Ser Ala Ser Thr Lys Gly Pro Ser Val Phe Pro Leu Ala
115 120 125

Pro Ser Ser Lys Ser Thr Ser Gly Gly Thr Ala Ala Leu Gly Cys Leu
130 135 140

Val Lys Asp Tyr Phe Pro Glu Pro Val Thr Val Ser Trp Asn Ser Gly
145 150 155 160

Ala Leu Thr Ser Gly Val His Thr Phe Pro Ala Val Leu Gln Ser Ser
165 170 175

Gly Leu Tyr Ser Leu Ser Ser Val Val Thr Val Pro Ser Ser Ser Leu
180 185 190

Gly Thr Gln Thr Tyr Ile Cys Asn Val Asn His Lys Pro Ser Asn Thr
195 200 205

Lys Val Asp Lys Lys Val Glu Pro Lys Ser Cys Asp Lys Thr His Thr
210 215 220

Cys Pro Pro Cys Pro Ala Pro Glu Leu Leu Gly Gly Pro Ser Val Phe
225 230 235 240

Leu Phe Pro Pro Lys Pro Lys Asp Thr Leu Met Ile Ser Arg Thr Pro
245 250 255

Glu Val Thr Cys Val Val Val Asp Val Ser His Glu Asp Pro Glu Val
260 265 270

Lys Phe Asn Trp Tyr Val Asp Gly Val Glu Val His Asn Ala Lys Thr
275 280 285

Lys Pro Arg Glu Glu Gln Tyr Asn Ser Thr Tyr Arg Val Val Ser Val
290 295 300

Leu Thr Val Leu His Gln Asp Trp Leu Asn Gly Lys Glu Tyr Lys Cys
305 310 315 320

Lys Val Ser Asn Lys Ala Leu Pro Ala Pro Ile Glu Lys Thr Ile Ser
325 330 335

Lys Ala Lys Gly Gln Pro Arg Glu Pro Gln Val Tyr Thr Leu Pro Pro
340 345 350

Ser Arg Asp Glu Leu Thr Lys Asn Gln Val Ser Leu Thr Cys Leu Val
355 360 365

Lys Gly Phe Tyr Pro Ser Asp Ile Ala Val Glu Trp Glu Ser Asn Gly
370 375 380

Gln Pro Glu Asn Asn Tyr Lys Thr Thr Pro Pro Val Leu Asp Ser Asp
385 390 395 400

Gly Ser Phe Phe Leu Tyr Ser Lys Leu Thr Val Asp Lys Ser Arg Trp
405 410 415

Gln Gln Gly Asn Val Phe Ser Cys Ser Val Met His Glu Ala Leu His
420 425 430

Asn His Tyr Thr Gln Lys Ser Leu Ser Leu Ser Pro Gly Lys
435 440 445

<210> 71
<211> 214
<212> PRT
<213> artificial

<220>
<223> antibody L chain 1145

<400> 71

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Ser Ser Tyr
20 25 30

Leu Asn Trp Tyr Gln Gln Lys Pro Gly Lys Ala Pro Lys Leu Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Gly Val Tyr Pro Phe
85 90 95

Thr Phe Gly Gln Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 72
<211> 446
<212> PRT
<213> artificial

<220>
<223> antibody H chain 1136

<400> 72

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Ala Met Ser Trp Val Arg Gln Ala Pro Gly Lys Gly Leu Glu Trp Val
35 40 45

Ser Ala Ile Ser Gly Ser Gly Gly Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

```
Ala Arg Tyr Val Phe Gly Ile Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350
```

Ser Arg Asp Glu Leu Thr Lys Asn Gln Val Ser Leu Thr Cys Leu Val
355 360 365

Lys Gly Phe Tyr Pro Ser Asp Ile Ala Val Glu Trp Glu Ser Asn Gly
370 375 380

Gln Pro Glu Asn Asn Tyr Lys Thr Thr Pro Pro Val Leu Asp Ser Asp
385 390 395 400

Gly Ser Phe Phe Leu Tyr Ser Lys Leu Thr Val Asp Lys Ser Arg Trp
405 410 415

Gln Gln Gly Asn Val Phe Ser Cys Ser Val Met His Glu Ala Leu His
420 425 430

Asn His Tyr Thr Gln Lys Ser Leu Ser Leu Ser Pro Gly Lys
435 440 445

<210> 73
<211> 215
<212> PRT
<213> artificial

<220>
<223> antibody L chain 1143

<400> 73

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Ser Ser Tyr
20 25 30

Leu Asn Trp Tyr Gln Gln Lys Pro Gly Lys Ala Pro Lys Leu Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Ala Tyr Tyr Ala Gly Leu
85 90 95

Phe Thr Phe Gly Gln Gly Thr Lys Leu Glu Ile Lys Arg Thr Val Ala
100 105 110

```
Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215
```

<210> 74
<211> 447
<212> PRT
<213> artificial

<220>
<223> antibody H chain 1132

<400> 74

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Gly Ile Gly Ser Tyr Gly Gly Gly Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Tyr Val Asn Phe Gly Met Asp Tyr Trp Gly Gln Gly Thr Leu
```

100 105 110

Val Thr Val Ser Ser Ala Ser Thr Lys Gly Pro Ser Val Phe Pro Leu
115 120 125

Ala Pro Ser Ser Lys Ser Thr Ser Gly Gly Thr Ala Ala Leu Gly Cys
130 135 140

Leu Val Lys Asp Tyr Phe Pro Glu Pro Val Thr Val Ser Trp Asn Ser
145 150 155 160

Gly Ala Leu Thr Ser Gly Val His Thr Phe Pro Ala Val Leu Gln Ser
165 170 175

Ser Gly Leu Tyr Ser Leu Ser Ser Val Val Thr Val Pro Ser Ser Ser
180 185 190

Leu Gly Thr Gln Thr Tyr Ile Cys Asn Val Asn His Lys Pro Ser Asn
195 200 205

Thr Lys Val Asp Lys Lys Val Glu Pro Lys Ser Cys Asp Lys Thr His
210 215 220

Thr Cys Pro Pro Cys Pro Ala Pro Glu Leu Leu Gly Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Lys Phe Asn Trp Tyr Val Asp Gly Val Glu Val His Asn Ala Lys
275 280 285

Thr Lys Pro Arg Glu Glu Gln Tyr Asn Ser Thr Tyr Arg Val Val Ser
290 295 300

Val Leu Thr Val Leu His Gln Asp Trp Leu Asn Gly Lys Glu Tyr Lys
305 310 315 320

Cys Lys Val Ser Asn Lys Ala Leu Pro Ala Pro Ile Glu Lys Thr Ile
325 330 335

Ser Lys Ala Lys Gly Gln Pro Arg Glu Pro Gln Val Tyr Thr Leu Pro
340 345 350

Pro Ser Arg Asp Glu Leu Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Val Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Lys
435 440 445

<210> 75
<211> 214
<212> PRT
<213> artificial

<220>
<223> antibody L chain 1139

<400> 75

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Ser Ser Tyr
20 25 30

Leu Asn Trp Tyr Gln Gln Lys Pro Gly Lys Ala Pro Lys Leu Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Gly Arg Asn Pro Pro
85 90 95

Thr Phe Gly Gln Gly Thr Lys Leu Glu Ile Lys Arg Thr Val Ala Ala
100 105 110

Pro Ser Val Phe Ile Phe Pro Pro Ser Asp Glu Gln Leu Lys Ser Gly

115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 76
<211> 449
<212> PRT
<213> artificial

<220>
<223> antibody H chain 1140

<400> 76

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Pro Val Tyr Ser Ser Val Phe Asp Tyr Trp Gly Gln Gly
            100                 105                 110
```

```
Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
```

355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro Gly
435 440 445

Lys

<210> 77
<211> 214
<212> PRT
<213> artificial

<220>
<223> antibody L chain 1141

<400> 77

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Ser Ser Tyr
20 25 30

Leu Asn Trp Tyr Gln Gln Lys Pro Gly Lys Ala Pro Lys Leu Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Ser Tyr Ser Thr Pro Tyr
85 90 95

Thr Phe Gly Gln Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 78
<211> 447
<212> PRT
<213> artificial

<220>

<223> antibody H chain 1150

<400> 78

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Gly Ile Gly Gly Ser Ser Ser Tyr Thr Ser Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95
```

```
Ala Arg Tyr Tyr Ser Tyr His Met Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350
```

```
Pro Ser Arg Asp Glu Leu Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Lys
        435                 440                 445
```

<210> 79
<211> 214
<212> PRT
<213> artificial

<220>
<223> antibody L chain 1152

<400> 79

```
Asp Ile Gln Met Thr Gln Ser Pro Ser Ser Leu Ser Ala Ser Val Gly
1               5                   10                  15

Asp His Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Gly Ser Ala Pro Pro
                85                  90                  95

Thr Phe Gly Gln Gly Thr Lys Leu Glu Ile Lys Arg Thr Val Ala Ala
            100                 105                 110
```

```
Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210
```

<210> 80
<211> 449
<212> PRT
<213> artificial

<220>
<223> antibody H chain 1134

<400> 80

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ser Ile Tyr Ser Gly Gly Gly Gly Thr Ser Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95
```

```
Ala Arg Gly Pro Ala Tyr Ser Ser Phe Phe Asp Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350
```

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro Gly
435 440 445

Lys

<210> 81
<211> 214
<212> PRT
<213> artificial

<220>
<223> antibody L chain 1141

<400> 81

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Ser Ser Tyr
20 25 30

Leu Asn Trp Tyr Gln Gln Lys Pro Gly Lys Ala Pro Lys Leu Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Ser Tyr Ser Thr Pro Tyr
85 90 95

```
Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210
```

<210> 82
<211> 446
<212> PRT
<213> artificial

<220>
<223> antibody H chain 1146

<400> 82

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                      80

Leu Gln Met Asn Ser Leu Arg Ala Glu Asp Thr Ala Val Tyr Tyr Cys
```

```
                85                  90                  95

Ala Arg Arg Val Phe Gly Phe Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335
```

Lys Ala Lys Gly Gln Pro Arg Glu Pro Gln Val Tyr Thr Leu Pro Pro
340 345 350

Ser Arg Asp Glu Leu Thr Lys Asn Gln Val Ser Leu Thr Cys Leu Val
355 360 365

Lys Gly Phe Tyr Pro Ser Asp Ile Ala Val Glu Trp Glu Ser Asn Gly
370 375 380

Gln Pro Glu Asn Asn Tyr Lys Thr Thr Pro Pro Val Leu Asp Ser Asp
385 390 395 400

Gly Ser Phe Phe Leu Tyr Ser Lys Leu Thr Val Asp Lys Ser Arg Trp
405 410 415

Gln Gln Gly Asn Val Phe Ser Cys Ser Val Met His Glu Ala Leu His
420 425 430

Asn His Tyr Thr Gln Lys Ser Leu Ser Leu Ser Pro Gly Lys
435 440 445

<210> 83
<211> 214
<212> PRT
<213> artificial

<220>
<223> antibody L chain 1147

<400> 83

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Tyr Tyr Tyr Pro Phe
                85                  90                  95

Thr Phe Gly Gln Gly Thr Lys Leu Glu Ile Lys Arg Thr Val Ala Ala

            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 84
<211> 449
<212> PRT
<213> artificial

<220>
<223> antibody H chain 1142

<400> 84

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95
```

Ala Arg Gly Pro Ala Tyr Ser Thr Val Leu Asp Tyr Trp Gly Gln Gly
100 105 110

Thr Leu Val Thr Val Ser Ser Ala Ser Thr Lys Gly Pro Ser Val Phe
115 120 125

Pro Leu Ala Pro Ser Ser Lys Ser Thr Ser Gly Gly Thr Ala Ala Leu
130 135 140

Gly Cys Leu Val Lys Asp Tyr Phe Pro Glu Pro Val Thr Val Ser Trp
145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Ser Leu Gly Thr Gln Thr Tyr Ile Cys Asn Val Asn His Lys Pro
195 200 205

Ser Asn Thr Lys Val Asp Lys Lys Val Glu Pro Lys Ser Cys Asp Lys
210 215 220

Thr His Thr Cys Pro Pro Cys Pro Ala Pro Glu Leu Leu Gly Gly Pro
225 230 235 240

Ser Val Phe Leu Phe Pro Pro Lys Pro Lys Asp Thr Leu Met Ile Ser
245 250 255

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Ala Leu Pro Ala Pro Ile Glu Lys
325 330 335

Thr Ile Ser Lys Ala Lys Gly Gln Pro Arg Glu Pro Gln Val Tyr Thr

```
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys
```

<210> 85
<211> 214
<212> PRT
<213> artificial

<220>
<223> antibody L chain 1135

<400> 85

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Pro Tyr
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210
```

<210> 86
<211> 446
<212> PRT
<213> artificial

<220>

<223> antibody H chain 1148

<400> 86

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80
```

```
Leu Gln Met Asn Ser Leu Arg Ala Glu Asp Thr Ala Val Tyr Tyr Cys
                85                  90                  95

Ala Arg Ala Val Phe Gly Phe Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335
```

Lys Ala Lys Gly Gln Pro Arg Glu Pro Gln Val Tyr Thr Leu Pro Pro
340 345 350

Ser Arg Asp Glu Leu Thr Lys Asn Gln Val Ser Leu Thr Cys Leu Val
355 360 365

Lys Gly Phe Tyr Pro Ser Asp Ile Ala Val Glu Trp Glu Ser Asn Gly
370 375 380

Gln Pro Glu Asn Asn Tyr Lys Thr Thr Pro Pro Val Leu Asp Ser Asp
385 390 395 400

Gly Ser Phe Phe Leu Tyr Ser Lys Leu Thr Val Asp Lys Ser Arg Trp
405 410 415

Gln Gln Gly Asn Val Phe Ser Cys Ser Val Met His Glu Ala Leu His
420 425 430

Asn His Tyr Thr Gln Lys Ser Leu Ser Leu Ser Pro Gly Lys
435 440 445

<210> 87
<211> 214
<212> PRT
<213> artificial

<220>
<223> antibody L chain 1149

<400> 87

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Ser Ser Tyr
20 25 30

Leu Asn Trp Tyr Gln Gln Lys Pro Gly Lys Ala Pro Lys Leu Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Ala Tyr Tyr Phe Pro His
85 90 95

Thr Phe Gly Gln Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 88
<211> 449
<212> PRT
<213> artificial

<220>
<223> antibody H chain 1138

<400> 88

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Ala Met Ser Trp Val Arg Gln Ala Pro Gly Lys Gly Leu Glu Trp Val
35 40 45

Ser Ala Ile Ser Gly Ser Gly Gly Ser Thr Tyr Tyr Ala Asp Ser Val
50 55 60

Lys Gly Arg Phe Thr Ile Ser Arg Asp Asn Ser Lys Asn Thr Leu Tyr
65 70 75 80

```
Leu Gln Met Asn Ser Leu Arg Ala Glu Asp Thr Ala Val Tyr Tyr Cys
                85                  90                  95

Ala Arg Gly Phe Val Tyr Ser Ser Tyr Ile Asp Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335
```

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro Gly
435 440 445

Lys

<210> 89
<211> 214
<212> PRT
<213> artificial

<220>
<223> antibody L chain 1135

<400> 89

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Ser Ser Tyr
20 25 30

Leu Asn Trp Tyr Gln Gln Lys Pro Gly Lys Ala Pro Lys Leu Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

```
Glu Asp Phe Ala Thr Tyr Tyr Cys Gln Gln Ser Tyr Ser Thr Pro Tyr
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210
```

<210> 90
<211> 348
<212> DNA
<213> artificial

<220>
<223> antibody H chain 1107 V region

<400> 90

```
gaggtgcagc tgttggagag cgggggaggc ttggtacagc ctgggggggtc cctgcgcctc      60

tcctgtgcag ccagcggatt cacctttagc agctatgcca tgagctgggt ccgccaggct      120

ccagggaagg ggctggagtg ggtctcagct attagtggta gtggtggtag cacatactat      180

gcagactccg tgaagggccg gttcaccatc tcccgtgaca attccaagaa cacgctgtat      240

ctgcaaatga acagcctgcg tgccgaggac acggctgtat attattgtgc gcgccgtgtt      300

tggggttttg actattgggg ccagggaacc ctggtcaccg tctcctca                  348
```

<210> 91
<211> 949
<212> DNA

<213> artificial

<220>
<223> antibody L chain 1145

<400> 91

```
ccatcacttg ccgggcaagt cagagcatta gcagctattt aaattggtat cagcagaaac      60
cagggaaagc ccctaagctc ctgatctatg ctgcatccag tttgcaaagt ggggtcccat     120
cacgtttcag tggcagtgga agcgggacag atttcactct caccatcagc agtctgcaac     180
ctgaagattt tgcaacttat tactgtcaac agtacggtgt ttacccgttc acttttggcc     240
aggggaccaa gctggagatc aaacgtgagt cgtacgctag caagcttgat atcgaattct     300
aaactctgag gggtcggat gacgtggcca ttctttgcct aaagcattga gtttactgca     360
aggtcagaaa agcatgcaaa gccctcagaa tggctgcaaa gagctccaac aaaacaattt     420
agaactttat taaggaatag gggaagcta ggaagaaact caaaacatca agattttaaa     480
tacgcttctt ggtctccttg ctataattat ctgggataag catgctgttt tctgtctgtc     540
cctaacatgc cctgtgatta tccgcaaaca acacacccaa gggcagaact ttgttactta     600
aacaccatcc tgtttgcttc tttcctcagg aactgtggct gcaccatctg tcttcatctt     660
cccgccatct gatgagcagt tgaaatctgg aactgcctct gttgtgtgcc tgctgaataa     720
cttctatccc agagaggcca aagtacagtg gaaggtggat aacgccctcc aatcgggtaa     780
ctcccaggag agtgtcacag agcaggacag caaggacagc acctacagcc tcagcagcac     840
cctgacgctg agcaaagcag actacgagaa acacaaagtc tacgcctgcg aagtcaccca     900
tcagggcctg agctcgcccg tcacaaagag cttcaacagg ggagagtgt                 949
```

<210> 92
<211> 348
<212> DNA
<213> artificial

<220>
<223> antibody H chain 1136 V region

<400> 92

gaggtgcagc tgttggagag cgggggaggc ttggtacagc ctggggggtc cctgcgcctc 60
tcctgtgcag ccagcggatt cacctttagc agctatgcca tgagctgggt ccgccaggct 120
ccagggaagg ggctggagtg ggtctcagct attagtggta gtggtggtag cacatactat 180
gcagactccg tgaagggccg gttcaccatc tcccgtgaca attccaagaa cacgctgtat 240
ctgcaaatga acagcctgcg tgccgaggac acggctgtat attattgtgc gcgctacgtt 300
ttcggtattg actattgggg ccagggaacc ctggtcaccg tctcctca 348

<210> 93
<211> 1010
<212> DNA
<213> artificial

<220>
<223> antibody L chain 1143

<400> 93

gacatccaga tgacccagtc tccatcctcc ctgagcgcat ctgtaggaga ccgcgtcacc 60
atcacttgcc gggcaagtca gagcattagc agctatttaa attggtatca gcagaaacca 120
gggaaagccc ctaagctcct gatctatgct gcatccagtt tgcaaagtgg ggtcccatca 180
cgtttcagtg gcagtggaag cgggacagat ttcactctca ccatcagcag tctgcaacct 240
gaagattttg caacttatta ctgtcaacag gcttactacg ctggtctgtt cacttttggc 300
caggggacca agctggagat caaacgtgag tcgtacgcta gcaagcttga tatcgaattc 360
taaactctga gggggtcgga tgacgtggcc attctttgcc taaagcattg agtttactgc 420
aaggtcagaa aagcatgcaa agccctcaga atggctgcaa agagctccaa caaaacaatt 480
tagaacttta ttaaggaata gggggaagct aggaagaaac tcaaaacatc aagattttaa 540
atacgcttct tggtctcctt gctataatta tctgggataa gcatgctgtt ttctgtctgt 600
ccctaacatg ccctgtgatt atccgcaaac aacacaccca agggcagaac tttgttactt 660
aaacaccatc ctgtttgctt ctttcctcag gaactgtggc tgcaccatct gtcttcatct 720
tcccgccatc tgatgagcag ttgaaatctg gaactgcctc tgttgtgtgc ctgctgaata 780
acttctatcc cagagaggcc aaagtacagt ggaaggtgga taacgccctc caatcgggta 840
actcccagga gagtgtcaca gagcaggaca gcaaggacag cacctacagc ctcagcagca 900
ccctgacgct gagcaaagca gactacgaga aacacaaagt ctacgcctgc gaagtcaccc 960
atcagggcct gagctcgccc gtcacaaaga gcttcaacag gggagagtgt 1010

<210> 94
<211> 351
<212> DNA

<213> artificial

<220>
<223> antibody H chain 1132 V region

<400> 94

```
gaggtgcagc tgttggagag cgggggaggc ttggtacagc ctggggggtc cctgcgcctc      60
tcctgtgcag ccagcggatt cacctttagc agctatgcca tgagctgggt ccgccaggct     120
ccagggaagg ggctggagtg ggtctcaggt attggttctt acggtggtgg tacatactat     180
gcagactccg tgaagggccg gttcaccatc tcccgtgaca attccaagaa cacgctgtat     240
ctgcaaatga acagcctgcg tgccgaggac acggctgtat attattgtgc gcgctacgtt     300
aacttcggta tggactattg gggccaggga accctggtca ccgtctcctc a              351
```

<210> 95
<211> 1007
<212> DNA
<213> artificial

<220>
<223> antibdoy L chain 1139

<400> 95

```
gacatccaga tgacccagtc tccatcctcc ctgagcgcat ctgtaggaga ccgcgtcacc      60
atcacttgcc gggcaagtca gagcattagc agctatttaa attggtatca gcagaaacca     120
gggaaagccc ctaagctcct gatctatgct gcatccagtt tgcaaagtgg ggtcccatca     180
cgtttcagtg gcagtggaag cgggacagat ttcactctca ccatcagcag tctgcaacct     240
gaagattttg caacttatta ctgtcaacag tacggtcgta acccgcccac ttttggccag     300
gggaccaagc tggagatcaa acgtgagtcg tacgctagca agcttgatat cgaattctaa     360
actctgaggg ggtcggatga cgtggccatt ctttgcctaa agcattgagt ttactgcaag     420
gtcagaaaag catgcaaagc cctcagaatg gctgcaaaga gctccaacaa aacaatttag     480
aactttatta aggaataggg ggaagctagg aagaaactca aaacatcaag attttaaata     540
cgcttcttgg tctccttgct ataattatct gggataagca tgctgttttc tgtctgtccc     600
taacatgccc tgtgattatc cgcaaacaac acacccaagg gcagaacttt gttacttaaa     660
caccatcctg tttgcttctt tcctcaggaa ctgtggctgc accatctgtc ttcatcttcc     720
cgccatctga tgagcagttg aaatctggaa ctgcctctgt tgtgtgcctg ctgaataact     780
tctatcccag agaggccaaa gtacagtgga aggtggataa cgccctccaa tcgggtaact     840
cccaggagag tgtcacagag caggacagca aggacagcac ctacagcctc agcagcaccc     900
tgacgctgag caaagcagac tacgagaaac acaaagtcta cgcctgcgaa gtcacccatc     960
agggcctgag ctcgcccgtc acaaagagct tcaacagggg agagtgt                  1007
```

<210> 96
<211> 357
<212> DNA
<213> artificial

<220>
<223> antibody H chain 1140 V region

<400> 96

```
gaggtgcagc tgttggagag cgggggaggc ttggtacagc ctggggggtc cctgcgcctc      60
tcctgtgcag ccagcggatt cacctttagc agctatgcca tgagctgggt ccgccaggct     120
ccagggaagg ggctggagtg ggtctcagct attagtggta gtggtggtag cacatactat     180
gcagactccg tgaagggccg gttcaccatc tcccgtgaca attccaagaa cacgctgtat     240
ctgcaaatga acagcctgcg tgccgaggac acggctgtat attattgtgc gcgcggtccg     300
gtttactctt ctgtttttga ctattggggc cagggaaccc tggtcaccgt ctcctca        357
```

<210> 97
<211> 1007
<212> DNA

<213> artificial

<220>
<223> antibody L chain 1141

<400> 97

gacatccaga tgacccagtc tccatcctcc ctgagcgcat ctgtaggaga ccgcgtcacc 60

atcacttgcc gggcaagtca gagcattagc agctatttaa attggtatca gcagaaacca 120

gggaaagccc ctaagctcct gatctatgct gcatccagtt tgcaaagtgg ggtcccatca 180

cgtttcagtg gcagtggaag cgggacagat ttcactctca ccatcagcag tctgcaacct 240

gaagattttg caacttatta ctgtcaacag agttacagta ccccttatac ttttggccag 300

gggaccaagc tggagatcaa acgtgagtcg tacgctagca agcttgatat cgaattctaa 360

actctgaggg ggtcggatga cgtggccatt ctttgcctaa agcattgagt ttactgcaag 420

gtcagaaaag catgcaaagc cctcagaatg gctgcaaaga gctccaacaa aacaatttag 480

aactttatta aggaataggg ggaagctagg aagaaactca aaacatcaag attttaaata 540

cgcttcttgg tctccttgct ataattatct gggataagca tgctgttttc tgtctgtccc 600

taacatgccc tgtgattatc cgcaaacaac acacccaagg gcagaacttt gttacttaaa 660

caccatcctg tttgcttctt tcctcaggaa ctgtggctgc accatctgtc ttcatcttcc 720

cgccatctga tgagcagttg aaatctggaa ctgcctctgt tgtgtgcctg ctgaataact 780

tctatcccag agaggccaaa gtacagtgga aggtggataa cgccctccaa tcgggtaact 840

cccaggagag tgtcacagag caggacagca aggacagcac ctacagcctc agcagcaccc 900

tgacgctgag caaagcagac tacgagaaac acaaagtcta cgcctgcgaa gtcacccatc 960

agggcctgag ctcgcccgtc acaaagagct tcaacagggg agagtgt 1007

<210> 98
<211> 351
<212> DNA
<213> artificial

<220>
<223> antibody H chain 1150 V region

<400> 98

gaggtgcagc tgttggagag cgggggaggc ttggtacagc ctggggggtc cctgcgcctc 60

tcctgtgcag ccagcggatt cacctttagc agctatgcca tgagctgggt ccgccaggct 120

ccagggaagg ggctggagtg ggtctcaggt attggtggtt cttcttctta cacatcttat 180

gcagactccg tgaagggccg gttcaccatc tcccgtgaca attccaagaa cacgctgtat 240

ctgcaaatga acagcctgcg tgccgaggac acggctgtat attattgtgc gcgctactac 300

tcttaccata tggactattg gggccaggga accctggtca ccgtctcctc a 351

<210> 99
<211> 1007
<212> DNA
<213> artificial

<220>
<223> antibody L chain 1152

<400> 99

gacatccaga tgacccagtc tccatcctcc ctgagcgcat ctgtaggaga ccacgtcacc 60

atcacttgcc gggcaagtca gagcattagc agctatttaa attggtatca gcagaaacca 120

gggaaagccc ctaagctcct gatctatgct gcatccagtt tgcaaagtgg ggtcccatca 180

cgtttcagtg gcagtggaag cgggacagat ttcactctca ccatcagcag tctgcaacct 240

gaagattttg caacttatta ctgtcaacag tacggttctg ctccgcccac ttttggccag 300

gggaccaagc tggagatcaa acgtgagtcg tacgctagca agcttgatat cgaattctaa 360

actctgaggg ggtcggatga cgtggccatt ctttgcctaa agcattgagt ttactgcaag 420

gtcagaaaag catgcaaagc cctcagaatg gctgcaaaga gctccaacaa aacaatttag 480

aactttatta aggaataggg ggaagctagg aagaaactca aaacatcaag attttaaata 540

cgcttcttgg tctccttgct ataattatct gggataagca tgctgttttc tgtctgtccc 600

taacatgccc tgtgattatc cgcaaacaac acacccaagg gcagaacttt gttacttaaa 660

caccatcctg tttgcttctt tcctcaggaa ctgtggctgc accatctgtc ttcatcttcc 720

cgccatctga tgagcagttg aaatctggaa ctgcctctgt tgtgtgcctg ctgaataact 780

tctatcccag agaggccaaa gtacagtgga aggtggataa cgccctccaa tcgggtaact 840

cccaggagag tgtcacagag caggacagca aggacagcac ctacagcctc agcagcaccc 900

tgacgctgag caaagcagac tacgagaaac acaaagtcta cgcctgcgaa gtcacccatc 960

agggcctgag ctcgcccgtc acaaagagct tcaacagggg agagtgt 1007

<210> 100
<211> 357
<212> DNA
<213> artificial

<220>
<223> antibody H chain 1134 V region

<400> 100

gaggtgcagc tgttggagag cgggggaggc ttggtacagc ctggggggtc cctgcgcctc 60
tcctgtgcag ccagcggatt cacctttagc agctatgcca tgagctgggt ccgccaggct 120
ccagggaagg ggctggagtg ggtctcatct atttactctg gtggtggtgg tacatcttat 180
gcagactccg tgaagggccg gttcaccatc tcccgtgaca attccaagaa cacgctgtat 240
ctgcaaatga acagcctgcg tgccgaggac acggctgtat attattgtgc gcgcggtccg 300
gcttactctt ctttctttga ctattggggc cagggaaccc tggtcaccgt ctcctca 357

<210> 101
<211> 1007
<212> DNA
<213> artificial

<220>
<223> antibody L chain 1141

<400> 101

gacatccaga tgacccagtc tccatcctcc ctgagcgcat ctgtaggaga ccgcgtcacc 60
atcacttgcc gggcaagtca gagcattagc agctatttaa attggtatca gcagaaacca 120
gggaaagccc ctaagctcct gatctatgct gcatccagtt tgcaaagtgg ggtcccatca 180
cgtttcagtg gcagtggaag cgggacagat ttcactctca ccatcagcag tctgcaacct 240
gaagattttg caacttatta ctgtcaacag agttacagta ccccttatac ttttggccag 300
gggaccaagc tggagatcaa acgtgagtcg tacgctagca agcttgatat cgaattctaa 360
actctgaggg ggtcggatga cgtggccatt ctttgcctaa agcattgagt ttactgcaag 420
gtcagaaaag catgcaaagc cctcagaatg gctgcaaaga gctccaacaa aacaatttag 480
aactttatta aggaataggg ggaagctagg aagaaactca aaacatcaag attttaaata 540
cgcttcttgg tctccttgct ataattatct gggataagca tgctgttttc tgtctgtccc 600
taacatgccc tgtgattatc cgcaaacaac acacccaagg gcagaacttt gttacttaaa 660
caccatcctg tttgcttctt tcctcaggaa ctgtggctgc accatctgtc ttcatcttcc 720
cgccatctga tgagcagttg aaatctggaa ctgcctctgt tgtgtgcctg ctgaataact 780
tctatcccag agaggccaaa gtacagtgga aggtggataa cgccctccaa tcgggtaact 840
cccaggagag tgtcacagag caggacagca aggacagcac ctacagcctc agcagcaccc 900
tgacgctgag caaagcagac tacgagaaac acaaagtcta cgcctgcgaa gtcacccatc 960
agggcctgag ctcgcccgtc acaaagagct tcaacagggg agagtgt 1007

<210> 102
<211> 348
<212> DNA
<213> artificial

<220>
<223> antibody H chain 1146 V region

<400> 102

```
gaggtgcagc tgttggagag cgggggaggc ttggtacagc ctggggggtc cctgcgcctc      60
tcctgtgcag ccagcggatt cacctttagc agctatgcca tgagctgggt ccgccaggct     120
ccagggaagg ggctggagtg ggtctcagct attagtggta gtggtggtag cacatactat     180
gcagactccg tgaagggccg gttcaccatc tcccgtgaca attccaagaa cacgctgtat     240
ctgcaaatga acagcctgcg tgccgaggac acggctgtat attattgtgc gcgccgtgtt     300
ttcggttttg actattgggg ccagggaacc ctggtcaccg tctcctca                  348
```

<210> 103
<211> 321
<212> DNA
<213> Artificial

<220>
<223> antibody L chain 1147 V region

<400> 103

```
gacatccaga tgacccagtc tccatcctcc ctgagcgcat ctgtaggaga ccgcgtcacc      60
atcacttgcc gggcaagtca gagcattagc agctatttaa attggtatca gcagaaacca     120
gggaaagccc ctaagctcct gatctatgct gcatccagtt tgcaaagtgg ggtcccatca     180
cgtttcagtg gcagtggaag cgggacagat ttcactctca ccatcagcag tctgcaacct     240
gaagattttg caacttatta ctgtcaacag tactactact acccgttcac ttttggccag     300
gggaccaagc tggagatcaa a                                               321
```

<210> 104
<211> 357
<212> DNA
<213> artificial

<220>
<223> antibody H chain 1142 V region

<400> 104

gaggtgcagc tgttggagag cgggggaggc ttggtacagc ctggggggtc cctgcgcctc 60

tcctgtgcag ccagcggatt cacctttagc agctatgcca tgagctgggt ccgccaggct 120

ccagggaagg ggctggagtg ggtctcagct attagtggta gtggtggtag cacatactat 180

gcagactccg tgaagggccg gttcaccatc tcccgtgaca attccaagaa cacgctgtat 240

ctgcaaatga acagcctgcg tgccgaggac acggctgtat attattgtgc gcgcggtccg 300

gcttactcta ctgttttgga ctattggggc cagggaaccc tggtcaccgt ctcctca 357

<210> 105
<211> 321
<212> DNA
<213> artificial

<220>
<223> antibody L chain 1135 V region

<400> 105

gacatccaga tgacccagtc tccatcctcc ctgagcgcat ctgtaggaga ccgcgtcacc 60

atcacttgcc gggcaagtca gagcattagc agctatttaa attggtatca gcagaaacca 120

gggaaagccc ctaagctcct gatctatgct gcatccagtt tgcaaagtgg ggtcccatca 180

cgtttcagtg gcagtggaag cgggacagat ttcactctca ccatcagcag tctgcaacct 240

gaagattttg caacttatta ctgtcaacag agttacagta ccccttatac ttttggccag 300

gggaccaagc tggagatcaa a 321

<210> 106
<211> 348
<212> DNA
<213> artificial

<220>
<223> antibody H chain 1148 V region

<400> 106

gaggtgcagc tgttggagag cgggggaggc ttggtacagc ctggggggtc cctgcgcctc 60

tcctgtgcag ccagcggatt cacctttagc agctatgcca tgagctgggt ccgccaggct 120

ccagggaagg ggctggagtg ggtctcagct attagtggta gtggtggtag cacatactat 180

gcagactccg tgaagggccg gttcaccatc tcccgtgaca attccaagaa cacgctgtat 240

ctgcaaatga acagcctgcg tgccgaggac acggctgtat attattgtgc gcgcgctgtt 300

ttcggttttg actattgggg ccagggaacc ctggtcaccg tctcctca 348

<210> 107
<211> 321

<212> DNA
<213> artificial

<220>
<223> antibody L chain 1149 V region

<400> 107

```
gacatccaga tgacccagtc tccatcctcc ctgagcgcat ctgtaggaga ccgcgtcacc      60
atcacttgcc gggcaagtca gagcattagc agctatttaa attggtatca gcagaaacca     120
gggaaagccc ctaagctcct gatctatgct gcatccagtt tgcaaagtgg ggtcccatca     180
cgtttcagtg gcagtggaag cgggacagat ttcactctca ccatcagcag tctgcaacct     240
gaagattttg caacttatta ctgtcaacag gcttactact tcccgcacac ttttggccag     300
gggaccaagc tggagatcaa a                                                321
```

<210> 108
<211> 357
<212> DNA
<213> artificial

<220>
<223> antibody H chain 1138 V region

<400> 108

```
gaggtgcagc tgttggagag cgggggaggc ttggtacagc ctggggggtc cctgcgcctc      60
tcctgtgcag ccagcggatt cacctttagc agctatgcca tgagctgggt ccgccaggct     120
ccagggaagg ggctggagtg ggtctcagct attagtggta gtggtggtag cacatactat     180
gcagactccg tgaagggccg gttcaccatc tcccgtgaca attccaagaa cacgctgtat     240
ctgcaaatga acagcctgcg tgccgaggac acggctgtat attattgtgc gcgcggtttc     300
gtttactctt cttacattga ctattggggc cagggaaccc tggtcaccgt ctcctca        357
```

<210> 109
<211> 321
<212> DNA
<213> artificial

<220>
<223> antibody L chain 1135 V region

<400> 109

```
gacatccaga tgacccagtc tccatcctcc ctgagcgcat ctgtaggaga ccgcgtcacc      60
atcacttgcc gggcaagtca gagcattagc agctatttaa attggtatca gcagaaacca     120
gggaaagccc ctaagctcct gatctatgct gcatccagtt tgcaaagtgg ggtcccatca     180
cgtttcagtg gcagtggaag cgggacagat ttcactctca ccatcagcag tctgcaacct     240
gaagattttg caacttatta ctgtcaacag agttacagta ccccttatac ttttggccag     300
gggaccaagc tggagatcaa a                                               321
```

<210> 110
<211> 336
<212> PRT
<213> artificial

<220>
<223> bispecific molecule 1107/1145

<400> 110

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60
```

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Gly Val Tyr Pro Phe
85 90 95

Thr Phe Gly Gln Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys Ser Gly Gly Gly Gly Ser Gly Gly Gly Gly
210 215 220

Ser Ala Pro Leu Lys Ile Gln Ala Tyr Phe Asn Glu Thr Ala Asp Leu
225 230 235 240

Pro Cys Gln Phe Ala Asn Ser Gln Asn Leu Ser Leu Ser Glu Leu Val
245 250 255

Val Phe Trp Gln Asp Gln Glu Asn Leu Val Leu Asn Glu Val Tyr Leu
260 265 270

Gly Lys Glu Arg Phe Asp Ser Val Asp Ser Lys Tyr Met Gly Arg Thr
275 280 285

Ser Phe Asp Ser Asp Ser Trp Thr Leu Arg Leu His Asn Leu Gln Ile
290 295 300

Lys Asp Lys Gly Arg Tyr Gln Cys Ile Ile His His Lys Lys Pro Thr

305 310 315 320

Gly Met Ile Asn Ile His Gln Met Asn Ser Glu Leu Ser Val Leu Ala
325 330 335

<210> 111
<211> 337
<212> PRT
<213> artificial

<220>
<223> bispecific molecule 1136/1143

<400> 111

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ala Tyr Tyr Ala Gly Leu
                85                  90                  95

Phe Thr Phe Gly Gln Gly Thr Lys Leu Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190
```

Tyr Ala Cys Glu Val Thr His Gln Gly Leu Ser Ser Pro Val Thr Lys
195 200 205

Ser Phe Asn Arg Gly Glu Cys Ser Gly Gly Gly Gly Ser Gly Gly Gly
210 215 220

Gly Ser Ala Pro Leu Lys Ile Gln Ala Tyr Phe Asn Glu Thr Ala Asp
225 230 235 240

Leu Pro Cys Gln Phe Ala Asn Ser Gln Asn Leu Ser Leu Ser Glu Leu
245 250 255

Val Val Phe Trp Gln Asp Gln Glu Asn Leu Val Leu Asn Glu Val Tyr
260 265 270

Leu Gly Lys Glu Arg Phe Asp Ser Val Asp Ser Lys Tyr Met Gly Arg
275 280 285

Thr Ser Phe Asp Ser Asp Ser Trp Thr Leu Arg Leu His Asn Leu Gln
290 295 300

Ile Lys Asp Lys Gly Arg Tyr Gln Cys Ile Ile His His Lys Lys Pro
305 310 315 320

Thr Gly Met Ile Asn Ile His Gln Met Asn Ser Glu Leu Ser Val Leu
325 330 335

Ala

<210> 112
<211> 336
<212> PRT
<213> artificial

<220>
<223> bispecific molecule 1132/1139

<400> 112

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Ser Ser Tyr
20 25 30

Leu Asn Trp Tyr Gln Gln Lys Pro Gly Lys Ala Pro Lys Leu Leu Ile
35 40 45

```
Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Gly Arg Asn Pro Pro
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys Ser Gly Gly Gly Gly Ser Gly Gly Gly Gly
    210                 215                 220

Ser Ala Pro Leu Lys Ile Gln Ala Tyr Phe Asn Glu Thr Ala Asp Leu
225                 230                 235                 240

Pro Cys Gln Phe Ala Asn Ser Gln Asn Leu Ser Leu Ser Glu Leu Val
                245                 250                 255

Val Phe Trp Gln Asp Gln Glu Asn Leu Val Leu Asn Glu Val Tyr Leu
            260                 265                 270

Gly Lys Glu Arg Phe Asp Ser Val Asp Ser Lys Tyr Met Gly Arg Thr
        275                 280                 285

Ser Phe Asp Ser Asp Ser Trp Thr Leu Arg Leu His Asn Leu Gln Ile
    290                 295                 300
```

Lys Asp Lys Gly Arg Tyr Gln Cys Ile Ile His His Lys Lys Pro Thr
305 310 315 320

Gly Met Ile Asn Ile His Gln Met Asn Ser Glu Leu Ser Val Leu Ala
325 330 335

<210> 113
<211> 336
<212> PRT
<213> bispecific molecule 1140/1141

<400> 113

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Ser Ser Tyr
20 25 30

Leu Asn Trp Tyr Gln Gln Lys Pro Gly Lys Ala Pro Lys Leu Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Ser Tyr Ser Thr Pro Tyr
85 90 95

Thr Phe Gly Gln Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys Ser Gly Gly Gly Gly Ser Gly Gly Gly Gly
210 215 220

Ser Ala Pro Leu Lys Ile Gln Ala Tyr Phe Asn Glu Thr Ala Asp Leu
225 230 235 240

Pro Cys Gln Phe Ala Asn Ser Gln Asn Leu Ser Leu Ser Glu Leu Val
245 250 255

Val Phe Trp Gln Asp Gln Glu Asn Leu Val Leu Asn Glu Val Tyr Leu
260 265 270

Gly Lys Glu Arg Phe Asp Ser Val Asp Ser Lys Tyr Met Gly Arg Thr
275 280 285

Ser Phe Asp Ser Asp Ser Trp Thr Leu Arg Leu His Asn Leu Gln Ile
290 295 300

Lys Asp Lys Gly Arg Tyr Gln Cys Ile Ile His His Lys Lys Pro Thr
305 310 315 320

Gly Met Ile Asn Ile His Gln Met Asn Ser Glu Leu Ser Val Leu Ala
325 330 335

<210> 114
<211> 336
<212> PRT
<213> artificial

<220>
<223> bispecific molecule 1150/1152

<400> 114

Asp Ile Gln Met Thr Gln Ser Pro Ser Ser Leu Ser Ala Ser Val Gly
1 5 10 15

Asp His Val Thr Ile Thr Cys Arg Ala Ser Gln Ser Ile Ser Ser Tyr
20 25 30

Leu Asn Trp Tyr Gln Gln Lys Pro Gly Lys Ala Pro Lys Leu Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Gly Ser Ala Pro Pro
85 90 95

Thr Phe Gly Gln Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys Ser Gly Gly Gly Gly Ser Gly Gly Gly Gly
210 215 220

Ser Ala Pro Leu Lys Ile Gln Ala Tyr Phe Asn Glu Thr Ala Asp Leu
225 230 235 240

Pro Cys Gln Phe Ala Asn Ser Gln Asn Leu Ser Leu Ser Glu Leu Val
245 250 255

Val Phe Trp Gln Asp Gln Glu Asn Leu Val Leu Asn Glu Val Tyr Leu
260 265 270

Gly Lys Glu Arg Phe Asp Ser Val Asp Ser Lys Tyr Met Gly Arg Thr
275 280 285

Ser Phe Asp Ser Asp Ser Trp Thr Leu Arg Leu His Asn Leu Gln Ile
290 295 300

Lys Asp Lys Gly Arg Tyr Gln Cys Ile Ile His His Lys Lys Pro Thr
305 310 315 320

Gly Met Ile Asn Ile His Gln Met Asn Ser Glu Leu Ser Val Leu Ala
325 330 335

<210> 115
<211> 1373
<212> DNA
<213> artificial

<220>
<223> bispecific molecule 1107/1145

<400> 115

```
gacatccaga tgacccagtc tccatcctcc ctgagcgcat ctgtaggaga ccgcgtcacc      60
atcacttgcc gggcaagtca gagcattagc agctatttaa attggtatca gcagaaacca     120
gggaaagccc ctaagctcct gatctatgct gcatccagtt tgcaaagtgg ggtcccatca     180
cgtttcagtg gcagtggaag cgggacagat ttcactctca ccatcagcag tctgcaacct     240
gaagattttg caacttatta ctgtcaacag tacggtgttt acccgttcac ttttggccag     300
gggaccaagc tggagatcaa acgtgagtcg tacgctagca agcttgatat cgaattctaa     360
actctgaggg ggtcggatga cgtggccatt ctttgcctaa agcattgagt ttactgcaag     420
gtcagaaaag catgcaaagc cctcagaatg gctgcaaaga gctccaacaa aacaatttag     480
aactttatta aggaataggg ggaagctagg aagaaactca aaacatcaag attttaaata     540
cgcttcttgg tctccttgct ataattatct gggataagca tgctgttttc tgtctgtccc     600
taacatgccc tgtgattatc cgcaaacaac acacccaagg gcagaacttt gttacttaaa     660
caccatcctg tttgcttctt tcctcaggaa ctgtggctgc accatctgtc ttcatcttcc     720
cgccatctga tgagcagttg aaatctggaa ctgcctctgt tgtgtgcctg ctgaataact     780
tctatcccag agaggccaaa gtacagtgga aggtggataa cgccctccaa tcgggtaact     840
cccaggagag tgtcacagag caggacagca aggacagcac ctacagcctc agcagcaccc     900
tgacgctgag caaagcagac tacgagaaac acaaagtcta cgcctgcgaa gtcacccatc     960
agggcctgag ctcgcccgtc acaaagagct tcaacagggg agagtgtagc ggaggaggag    1020
gaagcggagg aggaggaagc gcccccctca aaatccaagc gtacttcaac gaaactgcag    1080
acttaccgtg tcagtttgcc aattcgcaga atctgagcct gagcgaactg gtggttttct    1140
ggcaggatca ggagaacctg gttctgaacg aagtctatct gggcaaagag cggttcgaca    1200
gcgtggacag caagtatatg ggccgcacca gctttgatag cgacagctgg accctgcgtc    1260
tgcacaatct gcaaatcaaa gataagggta ggtaccagtg cattatccac cataagaagc    1320
cgacgggtat gattaatatt caccaaatga actccgagtt gtctgtcctg gcg           1373
```

<210> 116

<211> 1376
<212> DNA
<213> artificial

<220>
<223> bispecific molecule 1136/1143

<400> 116

```
gacatccaga tgacccagtc tccatcctcc ctgagcgcat ctgtaggaga ccgcgtcacc      60
atcacttgcc gggcaagtca gagcattagc agctatttaa attggtatca gcagaaacca     120
gggaaagccc ctaagctcct gatctatgct gcatccagtt tgcaaagtgg ggtcccatca     180
cgtttcagtg gcagtggaag cgggacagat ttcactctca ccatcagcag tctgcaacct     240
gaagattttg caacttatta ctgtcaacag gcttactacg ctggtctgtt cacttttggc     300
caggggacca agctggagat caaacgtgag tcgtacgcta gcaagcttga tatcgaattc     360
taaactctga gggggtcgga tgacgtggcc attctttgcc taaagcattg agtttactgc     420
aaggtcagaa aagcatgcaa agccctcaga atggctgcaa agagctccaa caaaacaatt     480
tagaacttta ttaaggaata gggggaagct aggaagaaac tcaaaacatc aagattttaa     540
atacgcttct tggtctcctt gctataatta tctgggataa gcatgctgtt ttctgtctgt     600
ccctaacatg ccctgtgatt atccgcaaac aacacaccca agggcagaac tttgttactt     660
aaacaccatc ctgtttgctt ctttcctcag gaactgtggc tgcaccatct gtcttcatct     720
tcccgccatc tgatgagcag ttgaaatctg gaactgcctc tgttgtgtgc ctgctgaata     780
acttctatcc cagagaggcc aaagtacagt ggaaggtgga taacgccctc caatcgggta     840
actcccagga gagtgtcaca gagcaggaca gcaaggacag cacctacagc ctcagcagca     900
ccctgacgct gagcaaagca gactacgaga aacacaaagt ctacgcctgc gaagtcaccc     960
atcagggcct gagctcgccc gtcacaaaga gcttcaacag gggagagtgt agcggaggag    1020
gaggaagcgg aggaggagga agcgcccccc tcaaaatcca agcgtacttc aacgaaactg    1080
cagacttacc gtgtcagttt gccaattcgc agaatctgag cctgagcgaa ctggtggttt    1140
tctggcagga tcaggagaac ctggttctga acgaagtcta tctgggcaaa gagcggttcg    1200
acagcgtgga cagcaagtat atgggccgca ccagctttga tagcgacagc tggaccctgc    1260
gtctgcacaa tctgcaaatc aaagataagg gtaggtacca gtgcattatc caccataaga    1320
agccgacggg tatgattaat attcaccaaa tgaactccga gttgtctgtc ctggcg        1376
```

<210> 117
<211> 1373
<212> DNA
<213> artificial

<220>
<223> bispecifici molecule 1132/1139

<400> 117

```
gacatccaga tgacccagtc tccatcctcc ctgagcgcat ctgtaggaga ccgcgtcacc    60
atcacttgcc gggcaagtca gagcattagc agctatttaa attggtatca gcagaaacca   120
gggaaagccc ctaagctcct gatctatgct gcatccagtt tgcaaagtgg ggtcccatca   180
cgtttcagtg gcagtggaag cgggacagat ttcactctca ccatcagcag tctgcaacct   240
gaagattttg caacttatta ctgtcaacag tacggtcgta acccgcccac ttttggccag   300
gggaccaagc tggagatcaa acgtgagtcg tacgctagca agcttgatat cgaattctaa   360
actctgaggg ggtcggatga cgtggccatt ctttgcctaa agcattgagt ttactgcaag   420
gtcagaaaag catgcaaagc cctcagaatg gctgcaaaga gctccaacaa aacaatttag   480
aactttatta aggaataggg ggaagctagg aagaaactca aaacatcaag attttaaata   540
cgcttcttgg tctccttgct ataattatct gggataagca tgctgttttc tgtctgtccc   600
taacatgccc tgtgattatc cgcaaacaac acacccaagg gcagaacttt gttacttaaa   660
caccatcctg tttgcttctt tcctcaggaa ctgtggctgc accatctgtc ttcatcttcc   720
cgccatctga tgagcagttg aaatctggaa ctgcctctgt tgtgtgcctg ctgaataact   780
tctatcccag agaggccaaa gtacagtgga aggtggataa cgccctccaa tcgggtaact   840
cccaggagag tgtcacagag caggacagca aggacagcac ctacagcctc agcagcaccc   900
tgacgctgag caaagcagac tacgagaaac acaaagtcta cgcctgcgaa gtcacccatc   960
agggcctgag ctcgcccgtc acaaagagct tcaacagggg agagtgtagc ggaggaggag  1020
gaagcggagg aggaggaagc gccccccctca aaatccaagc gtacttcaac gaaactgcag  1080
acttaccgtg tcagtttgcc aattcgcaga atctgagcct gagcgaactg gtggttttct  1140
ggcaggatca ggagaacctg gttctgaacg aagtctatct gggcaaagag cggttcgaca  1200
gcgtggacag caagtatatg ggccgcacca gctttgatag cgacagctgg accctgcgtc  1260
tgcacaatct gcaaatcaaa gataagggta ggtaccagtg cattatccac cataagaagc  1320
cgacgggtat gattaatatt caccaaatga actccgagtt gtctgtcctg gcg          1373
```

<210> 118
<211> 1373
<212> DNA
<213> artificial

<220>
<223> bispecific molecule 1140/1141

<400> 118

```
gacatccaga tgacccagtc tccatcctcc ctgagcgcat ctgtaggaga ccgcgtcacc      60
atcacttgcc gggcaagtca gagcattagc agctatttaa attggtatca gcagaaacca     120
gggaaagccc ctaagctcct gatctatgct gcatccagtt tgcaaagtgg ggtcccatca     180
cgtttcagtg gcagtggaag cgggacagat ttcactctca ccatcagcag tctgcaacct     240
gaagattttg caacttatta ctgtcaacag agttacagta ccccttatac ttttggccag     300
gggaccaagc tggagatcaa acgtgagtcg tacgctagca agcttgatat cgaattctaa     360
actctgaggg ggtcggatga cgtggccatt ctttgcctaa agcattgagt ttactgcaag     420
gtcagaaaag catgcaaagc cctcagaatg gctgcaaaga gctccaacaa aacaatttag     480
aactttatta aggaataggg ggaagctagg aagaaactca aaacatcaag attttaaata     540
cgcttcttgg tctccttgct ataattatct gggataagca tgctgttttc tgtctgtccc     600
taacatgccc tgtgattatc cgcaaacaac acacccaagg gcagaacttt gttacttaaa     660
caccatcctg tttgcttctt tcctcaggaa ctgtggctgc accatctgtc ttcatcttcc     720
cgccatctga tgagcagttg aaatctggaa ctgcctctgt tgtgtgcctg ctgaataact     780
tctatcccag agaggccaaa gtacagtgga aggtggataa cgccctccaa tcgggtaact     840
cccaggagag tgtcacagag caggacagca aggacagcac ctacagcctc agcagcaccc     900
tgacgctgag caaagcagac tacgagaaac acaaagtcta cgcctgcgaa gtcacccatc     960
agggcctgag ctcgcccgtc acaaagagct tcaacagggg agagtgtagc ggaggaggag    1020
gaagcggagg aggaggaagc gcccccctca aaatccaagc gtacttcaac gaaactgcag    1080
acttaccgtg tcagtttgcc aattcgcaga atctgagcct gagcgaactg gtggttttct    1140
ggcaggatca ggagaacctg gttctgaacg aagtctatct gggcaaagag cggttcgaca    1200
gcgtggacag caagtatatg ggccgcacca gctttgatag cgacagctgg accctgcgtc    1260
tgcacaatct gcaaatcaaa gataagggta ggtaccagtg cattatccac cataagaagc    1320
cgacgggtat gattaatatt caccaaatga actccgagtt gtctgtcctg gcg             1373
```

<210> 119
<211> 1373
<212> DNA
<213> artificial

<220>
<223> bispecific molecule 1151/1150

<400> 119

```
gacatccaga tgacccagtc tccatcctcc ctgagcgcat ctgtaggaga ccacgtcacc      60
atcacttgcc gggcaagtca gagcattagc agctatttaa attggtatca gcagaaacca     120
gggaaagccc ctaagctcct gatctatgct gcatccagtt tgcaaagtgg ggtcccatca     180
cgtttcagtg gcagtggaag cgggacagat ttcactctca ccatcagcag tctgcaacct     240
gaagattttg caacttatta ctgtcaacag tacggttctg ctccgcccac ttttggccag     300
gggaccaagc tggagatcaa acgtgagtcg tacgctagca agcttgatat cgaattctaa     360
actctgaggg ggtcggatga cgtggccatt ctttgcctaa agcattgagt ttactgcaag     420

gtcagaaaag catgcaaagc cctcagaatg gctgcaaaga gctccaacaa aacaatttag     480
aactttatta aggaataggg ggaagctagg aagaaactca aaacatcaag attttaaata     540
cgcttcttgg tctccttgct ataattatct gggataagca tgctgttttc tgtctgtccc     600
taacatgccc tgtgattatc cgcaaacaac acacccaagg gcagaacttt gttacttaaa     660
caccatcctg tttgcttctt tcctcaggaa ctgtggctgc accatctgtc ttcatcttcc     720
cgccatctga tgagcagttg aaatctggaa ctgcctctgt tgtgtgcctg ctgaataact     780
tctatcccag agaggccaaa gtacagtgga aggtggataa cgccctccaa tcgggtaact     840
cccaggagag tgtcacagag caggacagca aggacagcac ctacagcctc agcagcaccc     900
tgacgctgag caaagcagac tacgagaaac acaaagtcta cgcctgcgaa gtcacccatc     960
agggcctgag ctcgcccgtc acaaagagct tcaacagggg agagtgtagc ggaggaggag    1020
gaagcggagg aggaggaagc gcccccctca aaatccaagc gtacttcaac gaaactgcag    1080
acttaccgtg tcagtttgcc aattcgcaga atctgagcct gagcgaactg gtggttttct    1140
ggcaggatca ggagaacctg gttctgaacg aagtctatct gggcaaagag cggttcgaca    1200
gcgtggacag caagtatatg ggccgcacca gctttgatag cgacagctgg accctgcgtc    1260
tgcacaatct gcaaatcaaa gataagggta ggtaccagtg cattatccac cataagaagc    1320
cgacgggtat gattaatatt caccaaatga actccgagtt gtctgtcctg gcg           1373
```

<210> 120
<211> 223
<212> PRT
<213> mus musculus

<400> 120

Met Ala Cys Leu Gly Leu Arg Arg Tyr Lys Ala Gln Leu Gln Leu Pro
1 5 10 15

Ser Arg Thr Trp Pro Phe Val Ala Leu Leu Thr Leu Leu Phe Ile Pro
20 25 30

Val Phe Ser Glu Ala Ile Gln Val Thr Gln Pro Ser Val Val Leu Ala
35 40 45

Ser Ser His Gly Val Ala Ser Phe Pro Cys Glu Tyr Ser Pro Ser His
50 55 60

Asn Thr Asp Glu Val Arg Val Thr Val Leu Arg Gln Thr Asn Asp Gln
65 70 75 80

Met Thr Glu Val Cys Ala Thr Thr Phe Thr Glu Lys Asn Thr Val Gly
85 90 95

Phe Leu Asp Tyr Pro Phe Cys Ser Gly Thr Phe Asn Glu Ser Arg Val
100 105 110

Asn Leu Thr Ile Gln Gly Leu Arg Ala Val Asp Thr Gly Leu Tyr Leu
115 120 125

Cys Lys Val Glu Leu Met Tyr Pro Pro Pro Tyr Phe Val Gly Met Gly
130 135 140

Asn Gly Thr Gln Ile Tyr Val Ile Asp Pro Glu Pro Cys Pro Asp Ser
145 150 155 160

Asp Phe Leu Leu Trp Ile Leu Val Ala Val Ser Leu Gly Leu Phe Phe
165 170 175

Tyr Ser Phe Leu Val Ser Ala Val Ser Leu Ser Lys Met Leu Lys Lys
180 185 190

Arg Ser Pro Leu Thr Thr Gly Val Tyr Val Lys Met Pro Pro Thr Glu
195 200 205

Pro Glu Cys Glu Lys Gln Phe Gln Pro Tyr Phe Ile Pro Ile Asn
210 215 220

<210> 121
<211> 218
<212> PRT
<213> mus musculus

<400> 121

```
Met Thr Leu Arg Leu Leu Phe Leu Ala Leu Asn Phe Phe Ser Val Gln
1               5                   10                  15

Val Thr Glu Asn Lys Ile Leu Val Lys Gln Ser Pro Leu Leu Val Val
            20                  25                  30

Asp Ser Asn Glu Val Ser Leu Ser Cys Arg Tyr Ser Tyr Asn Leu Leu
        35                  40                  45

Ala Lys Glu Phe Arg Ala Ser Leu Tyr Lys Gly Val Asn Ser Asp Val
    50                  55                  60

Glu Val Cys Val Gly Asn Gly Asn Phe Thr Tyr Gln Pro Gln Phe Arg
65                  70                  75                  80

Ser Asn Ala Glu Phe Asn Cys Asp Gly Asp Phe Asp Asn Glu Thr Val
                85                  90                  95

Thr Phe Arg Leu Trp Asn Leu His Val Asn His Thr Asp Ile Tyr Phe
            100                 105                 110

Cys Lys Ile Glu Phe Met Tyr Pro Pro Pro Tyr Leu Asp Asn Glu Arg
        115                 120                 125

Ser Asn Gly Thr Ile Ile His Ile Lys Glu Lys His Leu Cys His Thr
    130                 135                 140

Gln Ser Ser Pro Lys Leu Phe Trp Ala Leu Val Val Val Ala Gly Val
145                 150                 155                 160

Leu Phe Cys Tyr Gly Leu Leu Val Thr Val Ala Leu Cys Val Ile Trp
                165                 170                 175

Thr Asn Ser Arg Arg Asn Arg Leu Leu Gln Val Thr Thr Met Asn Met
            180                 185                 190

Thr Pro Arg Arg Pro Gly Leu Thr Arg Lys Pro Tyr Gln Pro Tyr Ala
        195                 200                 205

Pro Ala Arg Asp Phe Ala Ala Tyr Arg Pro
    210                 215
```